# EUROPEAN PATENT APPLICATION

(11) **EP 2 119 728 A1**
(43) Date of publication of application: **18.11.2009**
(21) Application number: 09010722.8
(22) Date of filing: 28.11.2003
(51) Int. Cl.: C07K 14/705, C07K 16/28, C07H 21/04, C12N 15/63, C12Q 1/06, A61K 38/17

(54) **Therapeutic and diagnostic agents**

(30) Priority: 29.11.2002 AU 2002952993
(62) Divisional of application: 03812096.0
(71) Applicant: The Corporation of The Trustees of The Order of The Sisters of Mercy In Queensland, South Brisbane, QLD 4101 (AU)
(72) Inventor: The designation of the inventor has not yet been filed
(74) Representative: Jones, Elizabeth Louise

(57) **Abstract**

The present invention relates generally to therapeutic and diagnostic agents. More particularly, the present invention provides molecules having structural features characteristic of immunoregulatory signalling (IRS) molecules and which are expressed by cells of haematopoietic lineages such as, in particular, leukocytes. The molecules of the present invention find broad application *inter alia* as diagnostic markers for cells, targets for cell therapy and as validated drug targets in order to modulate the immune response and to treat, prevent and diagnose a range of diseases conditions including cancer, genetic disease, inflammatory conditions and conditions associated with aberrant haematopoietic cell function or activity. The present invention extends to binding partners of the instant molecules such as, for example, antibodies, ligands, adaptor and other signalling associated molecules, agonists and antagonists and to methods of screening for same.

SEQUENCE LISTING

<110> The Corporation of the Trustees of the Order of the Sisters of
      Mercy in Queensland

<120>  Therapeutic and Diagnostic Agents

<130>  12373860/EJH

<150>  AU 2002952993

<151>  2002-11-29

<160>  28

<170>  PatentIn version 3.1

<210>  1

<211>  1151

<212>  DNA

<213>  Homo sapiens

<400>  1
ctctaaaggc cactagcacc catcccagag ctgtcagcac cggcctcagc ccaggcggct      60

ctctccctga gcttcctgta gccctgaccc tctccagcct cagacctgag acagggctgg     120

acaaggaagc agagagcaga agaaaagcag aagcgaagct cagatctgct gggaggaaga     180

ttacattttg tcccctcctg gggtcttgca cagtggcagg tgacattcgt gttacaggaa     240

tgactgccag ggcctgggcc tcgtggcggt cttcagctct gctcctcctg cttgtcccag     300

gctattttcc tctgagccac cccatgaccg tggcgggccc cgtgggggga tccctgagtg     360

tgcagtgtcg ctatgagaag gaacacagga ccctcaacaa attctggtgc agaccaccac     420

agattctccg atgtgacaag attgtggaga ccaaagggtc agcagggaaa aggaatggcc     480

gagtgtccat cagggacagt cctgcaaacc tcagcttcac agtgaccctg gagaatctca     540

cagaggagga cgcaggcacc tactggtgtg gggtggatac accgtggctc cgagactttc     600

atgatcccat tgtcgaggtt gaggtgtccg tgttcccggc cgggacgacc acagcctcca 660

gcccccagag ctccatgggc acctcaggtc ctcccacgaa gctgcccgtg cacacctggc 720

ccagcgtgac cagaaaggac agccccgaac ccagcccaca ccctggctcc ctgttcagca 780

atgtccgctt cctgctcctg gtcctcttgg agctgcccct gctcctgagc atgctgggtg 840

ccgtcctctg ggtgaacaga cctcagagaa gctctagaag caggcagaat tggcccaagg 900

gtgagaacca gtagcatctg ctgtccatca aggccctgtg ctgcaacaga gcccctctgg 960

ggactggaat gacctcctga ccatcaaggc ctgcaacaga gcccctctgg gggactggaa 1020

tgacctcctg accactccct cccgggctgc tctctccaac atctcctgga atcctttgtg 1080

agcctccttc agccttttcc ctgtgcccga tccaacatgt gacacatgag gactttagag 1140

cacaatggat c 1151

<210> 2

<211> 224

<212> PRT

<213> Homo sapiens

<400> 2

Met Thr Ala Arg Ala Trp Ala Ser Trp Arg Ser Ser Ala Leu Leu Leu
1               5                   10                  15

Leu Leu Val Pro Gly Tyr Phe Pro Leu Ser His Pro Met Thr Val Ala
            20                  25                  30

Gly Pro Val Gly Gly Ser Leu Ser Val Gln Cys Arg Tyr Glu Lys Glu
        35                  40                  45

His Arg Thr Leu Asn Lys Phe Trp Cys Arg Pro Pro Gln Ile Leu Arg
    50                  55                  60

Cys Asp Lys Ile Val Glu Thr Lys Gly Ser Ala Gly Lys Arg Asn Gly
65                  70                  75                  80

Arg Val Ser Ile Arg Asp Ser Pro Ala Asn Leu Ser Phe Thr Val Thr
                85                  90                  95

Leu Glu Asn Leu Thr Glu Glu Asp Ala Gly Thr Tyr Trp Cys Gly Val
        100                 105                 110

Asp Thr Pro Trp Leu Arg Asp Phe His Asp Pro Ile Val Glu Val Glu
    115                 120                 125

Val Ser Val Phe Pro Ala Gly Thr Thr Thr Ala Ser Ser Pro Gln Ser
    130                 135                 140

Ser Met Gly Thr Ser Gly Pro Pro Thr Lys Leu Pro Val His Thr Trp
145                 150                 155                 160

Pro Ser Val Thr Arg Lys Asp Ser Pro Glu Pro Ser Pro His Pro Gly
                165                 170                 175

Ser Leu Phe Ser Asn Val Arg Phe Leu Leu Leu Val Leu Leu Glu Leu
        180                 185                 190

Pro Leu Leu Leu Ser Met Leu Gly Ala Val Leu Trp Val Asn Arg Pro
    195                 200                 205

Gln Arg Ser Ser Arg Ser Arg Gln Asn Trp Pro Lys Gly Glu Asn Gln
    210                 215                 220

<210> 3

<211> 1560

<212> DNA

<213> Homo sapiens

<400> 3
ggaggagctg ggactctggc ttgtgttttc caggatgttt tgctctgagc aaatgcagga      60

ccgtggcggg ccccgtgggg ggatccctga gtgtgcagtg tccctatgag aaggaacaca     120

ggaccctcaa caaatactgg tgcagaccac cacagatttt cctatgtgac aagattgtgg     180

agaccaaagg gtcagcagga aaaaggaacg gccgagtgtc catcagggac agtcctgcaa     240

acctcagctt cacagtgacc ctggagaatc tcacagagga ggatgcaggc acctactggt     300

gtggggtgga tacaccgtgg ctccgagact ttcatgatcc cgttgtcgag gttgaggtgt     360

ccgtgttccc ggcatcaacg tcaatgacac ctgcaagtat cactgcggcc aagacctcaa 420

caatcacaac tgcatttcca cctgtatcat ccactaccct gtttgcagtg ggtgccaccc 480

acagtgccag catccaggag gaaactgagg aggtggtgaa ctcacagctc ccgctgctcc 540

tctccctgct ggcattgttg ctgcttctgt tggtgggggc ctccctgcta gcctggagga 600

tgtttcagaa atggatcaaa gctggtgacc attcagagct gtcccagaac cccaagcagg 660

ctgccacgca gagtgagctg cactacgcaa atctggagct gctgatgtgg cctctgcagg 720

aaaagccagc accaccaagg gaggtggagg tggaatacag cactgtggcc tcccccaggg 780

aagaacttca ctatgcctcg gtggtgtttg attctaacac caacaggata gctgctcaga 840

ggcctcggga ggaggaacca gattcagatt acagtgtgat aaggaagaca taggcttttg 900

tcctgcctcg ccatcggagc tctcatgggc cccaggaagt ccagggacag ctcccttata 960

cctggcccac gtccttctca gcctgccctc gacaacagtg accaacagac aggcagctgg 1020

gtttcccagg ccatccctct gttgccatca gcttgattgg cttccccgag ggccagcagg 1080

gctgggggct ccggagagca gcaggaagca ctcccagcca ccagtgcctg tcgcctcttt 1140

cccctttgcc cctgcttcat cccagctctg tgtgtggagg acaaagcttc ttcctgcgtg 1200

gctccaggaa aagatgtggc tcacgtaggt ggcacctgcc aatagctttg tcaatcacag 1260

ccccatagga acgtctggaa ttgcttggga gttggggaga actgtcaaga agagtgaaga 1320

gagtgccaaa gcggagatct gttcacctgg gggccatgga ggggggaccc actaaagatc 1380

aagatcaaag attctcccca tctcacagac aaggaaactg aggccagagg gaggagagaa 1440

ttgctcatgg ctccagaact ggtggcaagt ttctctggac tcttaggttt atttttaata 1500

tgaaatataa aaacagtttc aaatatctta ttgagggaga agtaaaaact tatttaaaca 1560

<210> 4

<211> 301

<212> PRT

<213> Homo sapiens

<400> 4

Met Trp Leu Pro Trp Ala Leu Leu Leu Leu Trp Val Pro Gly Cys Phe
1               5                   10                  15

Ala Leu Ser Lys Cys Arg Thr Val Ala Gly Pro Trp Gly Ser Leu Ser
            20                  25                  30

Val Gln Cys Pro Tyr Glu Lys Glu His Arg Thr Leu Asn Lys Tyr Trp
        35                  40                  45

Cys Arg Pro Pro Gln Ile Phe Leu Cys Asp Lys Ile Val Glu Thr Lys
    50                  55                  60

Gly Ser Ala Gly Lys Arg Asn Gly Arg Val Ser Ile Arg Asp Ser Pro
65                  70                  75                  80

Ala Asn Leu Ser Phe Thr Val Thr Leu Glu Asn Leu Thr Glu Glu Asp
                85                  90                  95

Ala Gly Thr Tyr Trp Cys Gly Val Asp Thr Pro Trp Leu Arg Asp Phe
            100                 105                 110

His Asp Pro Val Val Glu Val Glu Val Ser Val Phe Pro Ala Ser Thr
        115                 120                 125

Ser Met Thr Pro Ala Ser Ile Thr Ala Ala Lys Thr Ser Thr Ile Thr
    130                 135                 140

Thr Ala Phe Pro Pro Val Ser Ser Thr Thr Leu Phe Ala Val Gly Ala
145                 150                 155                 160

Thr His Ser Ala Ser Ile Gln Glu Glu Thr Glu Glu Val Val Asn Ser
                165                 170                 175

Gln Leu Pro Leu Leu Leu Ser Leu Leu Ala Leu Leu Leu Leu Leu Leu
            180                 185                 190

Val Gly Ala Ser Leu Leu Ala Trp Arg Met Phe Gln Lys Trp Ile Lys
        195                 200                 205

Trp Ile Lys Ala Gly Asp His Ser Glu Leu Ser Gln Asn Pro Lys Gln
    210                 215                 220

Ala Ala Thr Gln Ser Glu Leu His Tyr Ala Asn Leu Glu Leu Leu Met
225                 230                 235                 240

Trp Pro Leu Gln Glu Lys Pro Ala Pro Pro Arg Glu Val Glu Val Glu
                245                 250                 255

Tyr Ser Thr Val Ala Ser Pro Arg Glu Glu Leu His Tyr Ala Ser Val
            260                 265                 270

Val Phe Asp Ser Asn Thr Asn Arg Ile Ala Ala Gln Arg Pro Arg Glu
        275                 280                 285

Glu Glu Pro Asp Ser Asp Tyr Ser Val Ile Arg Lys Thr
    290                 295                 300

<210> 5

<211> 674

<212> DNA

<213> Homo sapiens

<400> 5
ttggattcca gctgggacct agatttgctg aggacggaag ccaaggagac aggaacatgt 60

ggctgctccc agctctactc cttctctgcc tctcaggctg tttgtctctg aagggccccg 120

gctctgtgac tggcactgcg ggggactctc tgacagtgtg gtgtcagtat gagagcatgt 180

acaagggata taacaagtac tggtgccgag gacagtacga cacgtcatgt gagagcattg 240

tggagaccaa gggagaagag aaggtggaga ggaatggccg cgtgtccatc agagaccacc 300

cggaggctct cgccttcact gtgaccatgc agaacctcaa tgaagatgat gctggatctt 360

actggtgcaa aattcagaca gtgtgggtcc tggattcatg gtcacgcgat ccctcggacc 420

tggttagggt gtatgtttcc ccagcaatta caaccccaag gaggaccaca catccagcca 480

cacctcccat cttcctggtg gtgaaccctg ggcgaaacct cagcaccagg gaggtgttga 540

cccaaaattc agggttccgg ctcagcagcc ctcacttcct gctcgtggtc cctctgaagc 600

tgcccctgct cctgagcatg ctgggtgctg ttttctgggt gaacaggcct cagtgggctc 660

ctcctggaag atag 674

<210> 6

<211> 205

<212> PRT

<213> Homo sapiens

<400> 6

<210>  7

<211>  510

<212>  DNA

<213>  Homo sapiens

<400>  7
atgtggctgt ccccagctct gctgcttctc atcctcccag gttactccat tgccgctaaa      60

atcactggtc caacaacagt gaatggctcg gagcagggct cattgactgt gcagtgtgct     120

tatggctcag gctgggagac ctacttgaag tggcggtgtc aaggagctga ttggaattac     180

tgtaacatcc ttgttaaaac aaatggatca gagcaggagg taaagaagaa tcgagtttcc     240

atcagggaca atcagaaaaa ccacgtgttc accgtgacca tggagaatct caaaagagat     300

gatgctgaca gttattggtg tgggactgag agacctggaa ttgatcttgg ggtcaaagtt     360

caagtgacca ttaacccagc tcagtgcctg agtctgttgc ccacagatga cagggtgatg     420

gttccagttt cagcccacag gccaaaggga cccccttccc tggtaaccag agaccccaat     480

ccctgccagt gccttcttgg aacttcttta                                      510

<210>  8

<211>  174

<212>  PRT

<213>  Homo sapiens

<400>  8

Met Trp Leu Ser Pro Ala Leu Leu Leu Leu Ile Leu Pro Gly Tyr Ser
1               5                   10                  15

Ile Ala Ala Lys Ile Thr Gly Pro Thr Thr Val Asn Gly Ser Glu Gln
            20                  25                  30

Gly Ser Glu Gln Gly Ser Leu Thr Val Gln Cys Ala Tyr Gly Ser Gly
        35                  40                  45

Trp Glu Thr Tyr Leu Lys Trp Arg Cys Gln Gly Ala Asp Trp Asn Tyr
    50                  55                  60

Cys Asn Ile Leu Val Lys Thr Asn Gly Ser Glu Gln Glu Val Lys Lys
65                  70                  75                  80

Asn Arg Val Ser Ile Arg Asp Asn Gln Lys Asn His Val Phe Thr Val
                85                  90                  95

Thr Met Glu Asn Leu Lys Arg Asp Asp Ala Asp Ser Tyr Trp Cys Gly
            100                 105                 110

Thr Glu Arg Pro Gly Ile Asp Leu Gly Val Lys Val Gln Val Thr Ile
        115                 120                 125

Asn Pro Ala Gln Cys Leu Ser Leu Leu Pro Thr Asp Asp Arg Val Met
    130                 135                 140

Val Pro Val Ser Ala His Arg Pro Lys Gly Pro Pro Ser Leu Val Thr
145                 150                 155                 160

Arg Asp Pro Asn Pro Cys Gln Cys Leu Leu Gly Thr Ser Leu
                165                 170

<210> 9

<211> 1026

<212> DNA

<213> Homo sapiens

<400> 9
ccacgcgtcc gctccggtac tctccaccag ctttgagaac ccaaacccca gaagaggcca 60

gagaaggaac cgagaagatg tagaaggaaa aagagcctca gacccttgct gcccacaagg 120

acttcccatg ttgtgagatg acccagaggg ctggggctgc catgctgcct tcagctctgc 180

tccttctctg tgtcccaggc tgtctgactg tgagtggccc cagcaccgtg atgggcgccg 240

tgggggaatc cctgagtgtt cagtgtcggt atgaagacaa atacaagacg tttaacaaat 300

actggtgcag acaaccatgc ttgccaattt ggcatgaaat ggtggagacc ggagggtctg 360

agggagtggt gaggagtgac caagtgatca tcacggacca tcctggagac ctcaccttca 420

ccgtgacctt ggagaacctc acggcagacg atgcaggaaa ataccgatgt gggattgcaa 480

caatactgca ggaagatggc ctgtctggtt tcctgcccga tcccttcttc caggttcaag 540

tgctggtctc atcggcctcc agtactgaga actctgtgaa gacacctgca tctcccacca 600

ggcccagcca atgccaaggg tccctgccca gcagcacctg cttcctgctt ctcccactcc 660

tgaaggtgcc tctgctcctg agcatactcg gtgctatcct ctgggtgaac aggccttgga 720

ggactccttg gacagagtca tgaacaggag aacttgcaac accccatgcc cattggaacc 780

ctgtccagag acacagcccc tctgactgca aaaaggactt ctgaccctga ccctcatatt 840

tctttccatc ttatcacccg gatacttttt aaaagttaaa aaaaaaatgt aggccgggtg 900

cggtggctta cacctgcaat cccagcactt tgggaggcca aggcaaggtg gatcacttga 960

gtccagggaa gtttgagagc ctgggcagca tggtcagacc tcatctctac aaaaaaaaaa 1020

aaaaag 1026

<210> 10

<211> 193

<212> PRT

<213> Homo sapiens

<400> 10

Met Leu Pro Ser Ala Leu Leu Leu Leu Cys Val Pro Gly Cys Leu Thr
1               5                   10                  15

Val Ser Gly Pro Ser Thr Val Met Gly Ala Val Gly Glu Ser Leu Ser
            20                  25                  30

Val Gln Cys Arg Tyr Glu Asp Lys Tyr Lys Thr Phe Asn Lys Tyr Trp
        35                  40                  45

Cys Arg Gln Pro Cys Leu Pro Ile Trp His Glu Met Val Glu Thr Gly
    50                  55                  60

Gly Ser Glu Gly Val Val Arg Ser Asp Gln Val Ile Ile Thr Asp His
65                  70                  75                  80

Pro Gly Asp Leu Thr Phe Thr Val Thr Leu Glu Asn Leu Thr Ala Asp
                85                  90                  95

Asp Ala Gly Lys Tyr Arg Cys Gly Ile Ala Thr Ile Leu Gln Glu Asp
            100                 105                 110

Gly Leu Ser Gly Phe Leu Pro Asp Pro Phe Phe Gln Val Gln Val Leu
        115                 120                 125

Val Ser Ser Ala Ser Ser Thr Glu Asn Ser Val Lys Thr Pro Ala Ser
    130                 135                 140

Pro Thr Arg Pro Ser Gln Cys Gln Gly Ser Leu Pro Ser Ser Thr Cys
145                 150                 155                 160

Phe Leu Leu Leu Pro Leu Leu Lys Val Pro Leu Leu Leu Ser Ile Leu
                165                 170                 175

Gly Ala Ile Leu Trp Val Asn Arg Pro Trp Arg Thr Pro Trp Thr Glu
            180                 185                 190

Ser

<210>  11

<211>  1352

<212>  DNA

<213>  Homo sapiens

<400>  11
gtctagatgt gcagaaggtg caagccagag ctcaggcaga acttccagag tgcatctggg      60

atctgcattt gccactggtt gcagatcagg cggacgagga gccgggaagg cagagccatg     120

tggctgcccc ctgctctgct ccttctcagc ctctcaggct gtttctccat ccaaggccca     180

gagtctgtga gagccccaga gcaggggtcc ctgacggttc aatgccacta taagcaagga     240

tgggagacct acattaagtg gtggtgccga ggggtgcgct gggatacatg caagatcctc 300

attgaaacca gagggtcgga gcaaggagag aagagtgacc gtgtgtccat caaggacaat 360

cagaaagacc gcacgttcac tgtgaccatg gaggggctca ggcgagatga cgcagatgtt 420

tactggtgtg ggattgaaag aagaggacct gaccttggga ctcaagtgaa agtgatcgtt 480

gacccagagg gagcggcttc cacaacagca agctcaccta ccaacagcaa tatggcagtg 540

ttcatcggct cccacaagag gaaccactac atgctcctgg tatttgtgaa ggtgcccatc 600

ttgctcatct tggtcactgc catcctctgg ttgaaggggt ctcagagggt ccctgaggag 660

ccaggggaac agcctatcta catgaacttc tccgaacctc tgactaaaga catggccact 720

tagagagatg gatctgcaga gccttcctgc cctggccacg tttccagaag agactcgggc 780

tgtggaagga acatctacga gtcctcggga tgcagtgact gagatagggg ccctgggcct 840

ccgccctggc cttggagctg gtgggcacct ccctgttctg cacagctcag ggacttagcc 900

aggtcctctc ctgagccacc atcacctcct ggggtgccag cacctgttct cttggtcagg 960

agctgtagag atggagctca agcactggac gactctgtcc ccactgctgg aataactcgg 1020

gcacagagca tgggaccaaa gtacagaaag aggttggggg agaccccccc agccctagac 1080

ttccatcatt ccggagacca actcaacacc gtctttgcct gagaacctga tatatccgtg 1140

tttttaaatt tttttttttc tagcaaagtt gggttttaat gacttatgtt cataggaaac 1200

ctctctgatc ccacacacaa ggagggtgat tctgggatga gttcctggtt ctagggcatg 1260

aggggctgga tggaccctgt ccccagggag gacatggctc tgagtccaca gggctgagga 1320

ggcaatggga acctccctgg cccggcccgg tg 1352

<210> 12

<211> 158

<212> PRT

<213> Homo sapiens

<400> 12

Met Trp Leu Pro Pro Ala Leu Leu Leu Leu Ser Leu Ser Gly Cys Phe
1               5                   10                  15

Ser Ile Gln Gly Pro Glu Ser Val Arg Ala Pro Glu Gln Gly Ser Leu
            20                  25                  30

Thr Val Gln Cys His Tyr Lys Gln Gly Trp Glu Thr Tyr Ile Lys Trp
        35                  40                  45

Trp Cys Arg Gly Val Arg Trp Asp Thr Cys Lys Ile Leu Ile Glu Thr
    50                  55                  60

Arg Gly Ser Glu Gln Gly Glu Lys Ser Asp Arg Val Ser Ile Lys Asp
65                  70                  75                  80

Asn Gln Lys Asp Arg Thr Phe Thr Val Thr Met Glu Gly Leu Arg Arg
                85                  90                  95

Asp Asp Ala Asp Val Tyr Trp Cys Gly Ile Glu Arg Arg Gly Pro Asp
            100                 105                 110

Leu Gly Thr Gln Val Lys Val Ile Val Asp Pro Glu Gly Ala Ala Ser
        115                 120                 125

Thr Thr Ala Ser Ser Pro Thr Asn Ser Asn Met Ala Val Phe Ile Gly
    130                 135                 140

Ser His Lys Arg Asn His Tyr Met Leu Leu Gly Thr Ser Leu
145                 150                 155

<210>  13

<211>  812

<212>  DNA

<213>  Homo sapiens

<400>  13
gaagttcaag ggcgagagtg agtaccagca gaaggctggg agtctgtagt ttgttcctgc      60

tgccaggctc cactgagggg aacggggacc tgtctgaaga gaagatgccc ctgctgacac     120

tctacctgct cctcttctgg ctctcaggct actccattgt cactcaaatc accggtccaa     180

caacagtgaa tggcttggag cggggctcct tgaccgtgca gtgtgtttac agatcaggct     240

gggagaccta cttgaagtgg tggtgtcgag gagctatttg gcgtgactgc aagatccttg     300

ttaaaaccag tgggtcagag caggaggtga agagggaccg ggtgtccatc aaggacaatc 360

agaaaaaccg cacgttcact gtgaccatgg aggatctcat gaaaactgat gctgacactt 420

actggtgtgg aattgagaaa actggaaatg accttggggt cacagttcaa gtgaccattg 480

acccagcacc agtcacccaa gaagaaacta gcagctcccc aactctgacc ggccaccact 540

tggacaacag gcacaagctc ctgaagctca gtgtcctcct gcccctcatc ttcaccatat 600

tgctgctgct tttggtggcc gcctcactct tggcttggag gatgatgaag taccagcaga 660

aaggtgagag gacctgggta ctgcagcccc tggagggcga cctctgctat gcagacctga 720

ccctgcagct ggccggaacc tccccgcaaa aggctaccac gaagctttcc tctgcccagg 780

ttgaccaggt ggaagtggaa tatgtcacca tg 812

<210> 14

<211> 287

<212> PRT

<213> Homo sapiens

<400> 14

Met Pro Leu Leu Thr Leu Tyr Leu Leu Leu Phe Trp Leu Ser Gly Tyr
1               5                   10                  15

Ser Ile Val Thr Gln Ile Thr Gly Pro Thr Thr Val Asn Gly Leu Glu
            20                  25                  30

Arg Gly Ser Leu Thr Val Gln Cys Val Tyr Arg Ser Gly Trp Glu Thr
        35                  40                  45

Tyr Leu Lys Trp Trp Cys Arg Gly Ala Ile Trp Arg Asp Cys Lys Ile
    50                  55                  60

Leu Val Lys Thr Ser Gly Ser Glu Gln Glu Val Lys Arg Asp Arg Val
65                  70                  75                  80

Ser Ile Lys Asp Asn Gln Lys Asn Arg Thr Phe Thr Val Thr Met Glu
                85                  90                  95

Asp Leu Met Lys Thr Asp Ala Asp Thr Tyr Trp Cys Gly Ile Glu Lys
            100                 105                 110

Thr Gly Asn Asp Leu Gly Val Thr Val Gln Val Thr Ile Asp Pro Ala
        115                 120                 125

Pro Val Thr Gln Glu Glu Thr Ser Ser Ser Pro Thr Leu Thr Gly His
    130                 135                 140

His Leu Asp Asn Arg His Lys Leu Leu Lys Leu Ser Val Leu Leu Pro
145                 150                 155                 160

Leu Ile Phe Thr Ile Leu Leu Leu Leu Leu Val Ala Ala Ser Leu Leu
                165                 170                 175

Ala Trp Arg Met Met Lys Tyr Gln Gln Lys Gly Glu Arg Thr Trp Val
            180                 185                 190

Leu Gln Pro Leu Glu Gly Asp Leu Cys Tyr Ala Asp Leu Thr Leu Gln
        195                 200                 205

Leu Ala Gly Thr Ser Pro Gln Lys Ala Thr Thr Lys Leu Ser Ser Ala
    210                 215                 220

Gln Val Asp Gln Val Glu Val Glu Tyr Val Thr Met Ala Ser Leu Pro
225                 230                 235                 240

Lys Glu Asp Ile Ser Tyr Ala Ser Leu Thr Leu Gly Ala Glu Asp Gln
                245                 250                 255

Glu Pro Thr Tyr Cys Asn Met Gly His Leu Ser Ser His Leu Pro Gly
            260                 265                 270

Arg Gly Pro Glu Glu Pro Thr Glu Tyr Ser Thr Ile Ser Arg Pro
        275                 280                 285

<210> 15

<211> 2389

<212> DNA

<213>  mouse

<400>  15
cagcccggca gaagctaaag ctcagaagag ctcccaattg caggcaactg cagtgtccag      60

cacccaccat gaggcctctg gtcctgctat ggggctgcct ggtgctccca ggttatgaag     120

ccctgaaggg tccaaaggag atcagtggat ttgaaggtga caccgtgtcc ctgcggtgta     180

cctacgtgga gaagatgaag gagcacagga agtattggtg ccggcagggt ggcatcctgg     240

tgtcacgctg cggtgacatt gtctacgcaa atcaggacca ggaggtgact cgaggcagga     300

tgtccatccg agacagtccc caagagctct cgatgaccgt gatcatgagg gaccttaccc     360

tgaaggattc agggaagtac tggtgtggga ttgacagact gggccgcgat gagtcttttg     420

aggttacact cattgtcttt ccagggagct cccgtccagt cgtctggctg ccccttacca     480

caccacagga ctccagggct gtagccagca gtgtctccaa gcccagtgtg tccatcccga     540

tggtccgcat gatggcccca gtcctgatac tcttgtccct gctgttggct gcaggactaa     600

ttgcctttgg cagccacatg ctccggtgga gaaagaaagc ttggctggcc acagagacac     660

agaagaacga gaaggtctac cttgaaacct cgctgccagg gaacggctgg accactgaag     720

actcgacgat agaccttgca gtgactcctg aatgtctcag aaacctcaac ccttctgctg     780

tgccctctcc tgagacacag aacctcagtc agtctacaga ggaggaagag gcagctcgtt     840

ccctggacga cgacaaggag gacgtgatgg caccccctcc cttgcagatg tctgcggagg     900

aactggcctt ctctgagttc atctctgtgt aattgcagaa tgccccgtgg tcggccaggg     960

attgtgaagc tgaacagctg agttctcatg aattcttggg ttctactcac agtccacggc    1020

tctgtccacc ttccttccgg ctctctttca tgccccagat ggagaagtgt cttggtccct    1080

gaagcccgga tggtacttaa caagtccagc cagaggctgg aacctcccgc atattctaat    1140

ccctgggaag agttaatggg tgtgtgggcc ttcatcgggg cctggccagg ctccatggat    1200

aaaggctgag tttgtgtgcg ttccaggaaa ttccctgggc atggatgtcc agcaacagtc    1260

ccacctccca tcctcggaag atcccacctt cacctcctcc agcaacagtc ccacctccca    1320

tcctcggaag atcccacctt cacctccctc taattcttct gcatcaattg ctatggagga    1380

gacaacatat gtgtgtctat gaaacacctg catcctggcc tcttagaaaa taattaaaac    1440

aaaattctgc agacccatca agactcacca aaccatctct agggcagggc ctgggactcc    1500

acagttctga caagtgaccc tgccattcct acccttgggt ctgatgaatc ctcagcccat    1560

tttagctaga atcttccttc cttccttcct tccttccttc cttccttcct tccttccttt 1620

ccttcctttc cttcctttcc ttcctttcct tcctttcctt cctttcttcg ttccttcctg 1680

ccttccctgt ggggtttcct atatgcttcc tagacctaga tcatgacagt acggtcccag 1740

taggcacttc ctgatgcctc tctggtcagg cacactatgg tgacagccag cccaaggcag 1800

ccagggatca gctgtctctc catcctcctt ccccaaggcc ctgtgtccct tgctttggta 1860

ggacactgga ggaagtctcg atatcattcc tgtccagagt ggttactcct ccatggggtc 1920

tggaggctga gggagaggag gaggaggagg ataccagagt gggaaggggg gcggggaaac 1980

agaagacact agactctagt tactagagga gaatactaaa tccagtactg ttgagtgagg 2040

gaaagatgga ctggctcaac tatttttttt cctttttcta ttttgttttg aaaagtaaga 2100

tgttgggaag ggaggtgttc agaatataaa acagaaatgt agggagaata caaaagaagt 2160

gctgtttcta ggatcatata taacctcacc aaaccttgtt gacggctctg cctgagcttg 2220

caggaccccc ctcccttccc ctccccttcc agtatttgca gatgctccgt ttacagaggg 2280

gtcctctcac catgcacagc ccactacgca tcacacgctg tctcgtcata agcatccctc 2340

cgtgttctac gaactttgta caataaactt tctcagctgt gtagtattt 2389

<210> 16

<211> 287

<212> PRT

<213> mouse

<400> 16

Met Arg Pro Leu Val Leu Leu Trp Gly Cys Leu Val Leu Pro Gly Tyr
1               5                   10                  15

Glu Ala Leu Lys Gly Pro Lys Glu Ile Ser Gly Phe Glu Gly Asp Thr
            20                  25                  30

Val Ser Leu Arg Cys Thr Tyr Val Glu Lys Met Lys Glu His Arg Lys
        35                  40                  45

Tyr Trp Cys Arg Gln Gly Gly Ile Leu Val Ser Arg Cys Gly Asp Ile
    50                  55                  60

Val Tyr Ala Asn Gln Asp Gln Glu Val Thr Arg Gly Arg Met Ser Ile
65                  70                  75                  80

Arg Asp Ser Pro Gln Glu Leu Ser Met Thr Val Ile Met Arg Asp Leu
                85                  90                  95

Thr Leu Lys Asp Ser Gly Lys Tyr Trp Cys Gly Ile Asp Arg Leu Gly
            100                 105                 110

Arg Asp Glu Ser Phe Glu Val Thr Leu Ile Val Phe Pro Gly Ser Ser
        115                 120                 125

Arg Pro Val Val Trp Leu Pro Leu Thr Thr Pro Gln Asp Ser Arg Ala
    130                 135                 140

Val Ala Ser Ser Val Ser Lys Pro Ser Val Ser Ile Pro Met Val Arg
145                 150                 155                 160

Met Met Ala Pro Val Leu Ile Leu Leu Ser Leu Leu Leu Ala Ala Gly
                165                 170                 175

Leu Ile Ala Phe Gly Ser His Met Leu Arg Trp Arg Lys Lys Ala Trp
            180                 185                 190

Leu Ala Thr Glu Thr Gln Lys Asn Glu Lys Val Tyr Leu Glu Thr Ser
        195                 200                 205

Leu Pro Gly Asn Gly Trp Thr Thr Glu Asp Ser Thr Ile Asp Leu Ala
    210                 215                 220

Val Thr Pro Glu Cys Leu Arg Asn Leu Asn Pro Ser Ala Val Pro Ser
225                 230                 235                 240

Pro Glu Thr Gln Asn Leu Ser Gln Ser Thr Glu Glu Glu Glu Ala Ala
                245                 250                 255

Arg Ser Leu Asp Asp Asp Lys Glu Asp Val Met Ala Pro Pro Pro Leu
            260                 265                 270

Gln Met Ser Ala Glu Glu Leu Ala Phe Ser Glu Phe Ile Ser Val
        275                 280                 285

<210> 17

<211> 1111

<212> DNA

<213> mouse

<400> 17
gaaatgaccc aactggcctc agctgtgtgg ctgcccacgc tgttgctgct gctgctgctt      60

ttttggcttc caggctgtgt ccctctgcat ggtcccagca ccatgacagg aagtgtgggt     120

caatccctga gtgtgtcgtg tcagtatgag gagaaattta agactaagga caaatactgg     180

tgcagagggt cacttaaggt actgtgcaaa gatattgtca agaccagcag ctcagaagaa     240

gctaggagtg gcagagtgac catcagggac catccagaca acctcacctt tacagtgacc     300

tatgagagcc tcaccctgga ggatgcagac acctacatgt gtgcggtgga tatatcactt     360

tttgatggct ccttggggtt cgataagtac ttcaagattg agttgtctgt ggttccaagt     420

gaggacccag gaccaacact agagacacct gtggtgtcca ccagtctgcc taccaagggt     480

cccgccctag gatccaacac agaggaccgc cgtgagcatg actattccca gggcttgagg     540

ctcccagcgc tgttgtctgt gttagctctc ctgctgtttc tgttggtggg gacctctctg     600

ctggcctgga ggatgttcca gaagcggctg gtcaaagctg ataggcatcc agagctgtcc     660

cagaacctca gacaggcttc tgagcagaat gagtgccagt atgtgaattt gcagctgcac     720

acgtggtctc tgagggaaga gccggtgcta ccaagtcagg tagaagtggt ggaatatagc     780

acattggcat taccccagga agagcttcac tattcatccg tggcattcaa ctcccagagg     840

caggattctc acgccaatgg agattctctt catcaacctc aggaccagaa agcagagtac     900

agtgagatcc agaagcccag aaaaggactc tctgaccttt acctgtgact ccttgtcacc     960

tgatcctctc agtggtgact accaggttcc aaggctccct gctggctgct gccctcaatg    1020

tcatgagcct cagtggcttc actaaagatg agcaggagcc agggctctgt gggcacagtc    1080

tcatcccact ggctctctcc tcttagcctg t                                   1111

<210> 18

<211>  314

<212>  PRT

<213>  mouse

<400>  18

Met Thr Gln Leu Ala Ser Ala Val Trp Leu Pro Thr Leu Leu Leu Leu
1               5                   10                  15

Leu Leu Leu Phe Trp Leu Pro Gly Cys Val Pro Leu His Gly Pro Ser
            20                  25                  30

Thr Met Thr Gly Ser Val Gly Gln Ser Leu Ser Val Ser Cys Gln Tyr
        35                  40                  45

Glu Glu Lys Phe Lys Thr Lys Asp Lys Tyr Trp Cys Arg Gly Ser Leu
    50                  55                  60

Lys Val Leu Cys Lys Asp Ile Val Lys Thr Ser Ser Ser Glu Glu Ala
65                  70                  75                  80

Arg Ser Gly Arg Val Thr Ile Arg Asp His Pro Asp Asn Leu Thr Phe
                85                  90                  95

Thr Val Thr Tyr Glu Ser Leu Thr Leu Glu Asp Ala Asp Thr Tyr Met
            100                 105                 110

Cys Ala Val Asp Ile Ser Leu Phe Asp Gly Ser Leu Gly Phe Asp Lys
        115                 120                 125

Tyr Phe Lys Ile Glu Leu Ser Val Val Pro Ser Glu Asp Pro Gly Pro
    130                 135                 140

Thr Leu Glu Thr Pro Val Val Ser Thr Ser Leu Pro Thr Lys Gly Pro
145                 150                 155                 160

Ala Leu Gly Ser Asn Thr Glu Asp Arg Arg Glu His Asp Tyr Ser Gln
                165                 170                 175

Gly Leu Arg Leu Pro Ala Leu Leu Ser Val Leu Ala Leu Leu Leu Phe
            180                 185                 190

Leu Leu Val Gly Thr Ser Leu Leu Ala Trp Arg Met Phe Gln Lys Arg
        195                 200                 205

Leu Val Lys Ala Asp Arg His Pro Glu Leu Ser Gln Asn Leu Arg Gln
    210                 215                 220

Ala Ser Glu Gln Asn Glu Cys Gln Tyr Val Asn Leu Gln Leu His Thr
225                 230                 235                 240

Trp Ser Leu Arg Glu Glu Pro Val Leu Pro Ser Gln Val Glu Val Val
                245                 250                 255

Glu Tyr Ser Thr Leu Ala Leu Pro Gln Glu Glu Leu His Tyr Ser Ser
            260                 265                 270

Val Ala Phe Asn Ser Gln Arg Gln Asp Ser His Ala Asn Gly Asp Ser
        275                 280                 285

Leu His Gln Pro Gln Asp Gln Lys Ala Glu Tyr Ser Glu Ile Gln Lys
    290                 295                 300

Pro Arg Lys Gly Leu Ser Asp Leu Tyr Leu
305                 310

<210> 19

<211> 711

<212> DNA

<213> mouse

<400> 19
atgtggctgt ccccagcttt gcttcttctc agttttccag gctgcctctc catccaaggc 60

ccagcattgg tgaggggtcc agagcagggg tcagtgactg tgcaatgtcg ctatagctca 120

agatggcaaa ccaacaagaa gtggtggtgc cggggagcaa gctggagcac ttgcagggtc 180

ctcatccgat ccactgggtc agagaaagaa acgaagagcg gccggctgtc catcagggac 240

aatcagaaaa atcactcatt ccaggttacc atggagatgc tcaggcaaaa tgacacggac 300

acttactggt gtggtattga aaagttcgga actgaccgtg ggaccagagt taaagtgaac 360

gtctacttcg gccatatgca gaccttcttc agttcagcag ccacactgac tcctgagagg 420

gcagcagaga tgtgggtaaa gataccatgt cgacttctaa tcaacttccc tggcccactg 480

tggacggcag tacagacatg gtgtcttctg acttgcagaa gaggacttga agccagtcta 540

gttggggcct ttgtgggtgg gctgatgcaa gttccttcct gttctctggc cgtcgccatc 600

tttaccttcg tgctaacact gactcctcct agttcccagg aagcacacag cacaccgtca 660

tcacactcag ccccagtggc ttccaaggaa gagatgaacc gtctcttcta a 711

<210> 20

<211> 236

<212> PRT

<213> mouse

<400> 20

Met Trp Leu Ser Pro Ala Leu Leu Leu Leu Ser Phe Pro Gly Cys Leu
1               5                   10                  15

Ser Ile Gln Gly Pro Ala Leu Val Arg Gly Pro Glu Gln Gly Ser Val
            20                  25                  30

Thr Val Gln Cys Arg Tyr Ser Ser Arg Trp Gln Thr Asn Lys Lys Trp
        35                  40                  45

Trp Cys Arg Gly Ala Ser Trp Ser Thr Cys Arg Val Leu Ile Arg Ser
    50                  55                  60

Thr Gly Ser Glu Lys Glu Thr Lys Ser Gly Arg Leu Ser Ile Arg Asp
65                  70                  75                  80

Asn Gln Lys Asn His Ser Phe Gln Val Thr Met Glu Met Leu Arg Gln
                85                  90                  95

Asn Asp Thr Asp Thr Tyr Trp Cys Gly Ile Glu Lys Phe Gly Thr Asp
            100                 105                 110

Arg Gly Thr Arg Val Lys Val Asn Val Tyr Phe Gly His Met Gln Thr
        115                 120                 125

Phe Phe Ser Ser Ala Ala Thr Leu Thr Pro Glu Arg Ala Ala Glu Met
    130                 135                 140

Trp Val Lys Ile Pro Cys Arg Leu Leu Ile Asn Phe Pro Gly Pro Leu
145                 150                 155                 160

Trp Thr Ala Val Gln Thr Trp Cys Leu Leu Thr Cys Arg Arg Gly Leu
                165                 170                 175

Glu Ala Ser Leu Val Gly Ala Phe Val Gly Gly Leu Met Gln Val Pro
            180                 185                 190

Ser Cys Ser Leu Ala Val Ala Ile Phe Thr Phe Val Leu Thr Leu Thr
        195                 200                 205

Pro Pro Ser Ser Gln Glu Ala His Ser Thr Pro Ser Ser His Ser Ala
    210                 215                 220

Pro Val Ala Ser Lys Glu Glu Met Asn Arg Leu Phe
225                 230                 235

<210>  21

<211>  819

<212>  DNA

<213>  mouse

<400>  21
aggaagtagc tcagagtgca aaggaagcag ataagaaaaa aacacatgga gagaacttga      60

acaagaaggt ggttgcctgg gctctgttac acacatctgg attccagcag cgacctggag     120

ttttctggag acagtaccca gtgaggcagg aggatgaggc tatgtgcagg tctgctcctt     180

ctctgcttcc aaggttgctt gtctctgacg ggccctggct ctgtgtctgg ctacgtagga     240

ggctctctcc gtgtgcagtg tcaatatagt ccatcatata agggctatat gaaatactgg     300

tgccgaggac cgcatgacac gacgtgtaaa actattgtag aaaccgacgg aagtgagaaa     360

gaaaagagga gtggcccagt gtccatcaga gaccatgctg cgaactccac catcacagtg     420

atcatggagg accttagcga agacgatgct gggtcttact ggtgcaagat tcagacttcc     480

tttatctggg attcgtggtc acgtgatcca tcggtcagcg taagggtgaa tgtttttcca 540

gtgaattctg ggcagaacct gaggattagt actaatgtga tgttcatctt ccaactgtgg 600

tccctgctca gcagcatcca gttccaggtc ctggtcttcc tgaagctgcc tctgtttctg 660

agcatgctct gtgctatctt ctgggtgaac agactttagg gggttcctgg gggcaatgta 720

gagtgaccca tccaagaact atgaagtgaa gcatcccagg aatgccctgg gaggaactca 780

gtcctgcatg cagactggac ttcattgttc tgtgtctca 819

<210> 22

<211> 181

<212> PRT

<213> mouse

<400> 22

Met Arg Leu Cys Ala Gly Leu Leu Leu Leu Cys Phe Gln Gly Cys Leu
1               5                   10                  15

Ser Leu Thr Gly Pro Gly Ser Val Ser Gly Tyr Val Gly Gly Ser Leu
            20                  25                  30

Arg Val Gln Cys Gln Tyr Ser Pro Ser Tyr Lys Gly Tyr Met Lys Tyr
        35                  40                  45

Trp Cys Arg Gly Pro His Asp Thr Thr Cys Lys Thr Ile Val Glu Thr
    50                  55                  60

Asp Gly Ser Glu Lys Glu Lys Arg Ser Gly Pro Val Ser Ile Arg Asp
65                  70                  75                  80

His Ala Ala Asn Ser Thr Ile Thr Val Ile Met Glu Asp Leu Ser Glu
                85                  90                  95

Asp Asp Ala Gly Ser Tyr Trp Cys Lys Ile Gln Thr Ser Phe Ile Trp
            100                 105                 110

Asp Ser Trp Ser Arg Asp Pro Ser Val Ser Val Arg Val Asn Val Phe
        115                 120                 125

Pro Val Asn Ser Gly Gln Asn Leu Arg Ile Ser Thr Asn Val Met Phe
    130                 135                 140

Ile Phe Gln Leu Trp Ser Leu Leu Ser Ser Ile Gln Phe Gln Val Leu
145                 150                 155                 160

Val Phe Leu Lys Leu Pro Leu Phe Leu Ser Met Leu Cys Ala Ile Phe
                165                 170                 175

Trp Val Asn Arg Leu
            180

<210>  23

<211>  2487

<212>  DNA

<213>  mouse

<400>  23
gaagttactg agagaagtga acaagagaga cctaaaggca actcaagctg agctgcgagt      60

cctcacaggg tcctgacatc tgtcgtcaac aaggacatga gaggagacga ccatgtggca     120

gttctctgct ctactcctat tcttcctccc aggctgctgc acggctcagg attcagtcac     180

aggtccagag gaggtgagcg gtcaggagca gggctccttg acagtgcagt gcagatattc     240

ctcatactgg aagggttaca agaagtactg gtgccgagga gttcctcaga gatcatgtga     300

tattcttgtt gaaaccgata aatcagagca gctggtgaag aagaaccgtg tgtccatcag     360

ggacaaccag agagacttca tcttcacagt gaccatggag gatctgagga tgagcgatgc     420

tggcatttac tggtgtggaa ttacgaaagg tggacctgat cccatgttta aagttaatgt     480

gaacattgac caagccccaa aaagttcaat gatgaccacc acagccacag ttctgaaatc     540

catacaacca agcgctgaga acactggcaa ggaacaagtg actcagagca aagaagtgac     600

tcagagcagg ccccacacca ggtccctgct gagcagcatc tacttcctgc tgatggtctt     660

tgtggagtta cccctgctcc tgagcatgct cagtgctgtc ctctgggtga ccaggcctca     720

gagatgcttt gggagaggtg aaaatgacct ggtgaagacc catagtcctg ttgcctagga     780

tagagagaaa cagttcccaa gaaatggaaa ataatctctg tctctctgtt gtctctgtct     840

ctgtctctgg ggtgtatgta tgtgtgtgca tgcaccttgc cggggcagat gtgtatgtgg 900
gagacatcta ctggaatcat tcccttagta tctgagacag ggtttctaat tgaccagcac 960
ctttgtgtgg taggtcagac agctggccag ggaactccag ggatctccct gcctctacca 1020
tccatcctga gattgcaagc atacacgagt gccctagctt aaaaacaaac aaacaaacaa 1080
acaccttagg ttgtagggat tgaactcatg tccttgtacc tgcaaggaag gtaggcgatt 1140
tacctgctga gccatctccc caatctggag aagactcaat ctagtaaaga acaactcatc 1200
agcagtacca tggctctgat gtgctgcaca accagactca gactaatccc actcctatag 1260
cagggacagc tgagttctgg aacccattca tgtgcccctc tctcaggaca tcctgcaata 1320
cctatctggg gctatcttcc actgatgact tccaaagaag aaaatacaag aaaacatcac 1380
atttcttctt agtgtactag ttccttagag gacacatgcc aatataagac tgcgggccac 1440
cagccagttg attgaccaaa tatctcggtg atgtggcctc accaagtagc ataaagtttg 1500
ccactgtcac actagctatc tgtcccttat tggcaggaca cacctgcttt tctttttctt 1560
caacacagcc cagtgactaa gcccattgca aacccagatg gagtagttga cctaagcttt 1620
gtaccacctg ctcaggtctt caagtagtag ttaagccttg gtccctgaaa tctagattgc 1680
tcagtgagac caaatgggga ggtcaactgc aggaatcagc tgatctcaca ggagtcacga 1740
acccacatca cccccaaacc cttccaggaa tggtctcttc accaggccct tccactctct 1800
cccttttact cagacaaatc tattgaatgt ctaagtagtt atcactctcc acatacatgc 1860
tccaaaataa gacagaccca attaaagtcc atagagaagg ccaatgggat caaaggtaaa 1920
tactcagggg aaatgagtag tctcagccca ccagtctcag acatcctgag ttctgcacca 1980
tgacacagtc ttcttcttga gtggggctct gacacccaca gccaaattca caactaacat 2040
gggtgttctc caactttgtg gaagaagagt ccccaggtta gcatcttctc agtgatgaca 2100
tgtgttggac tctagtgagc ttgcctcttg ttaagaggat ggttttcatt tgcttcaggg 2160
gtatacctgc cagtcagtca gccacattcc cactcatgct cagaccaaca atcatggtta 2220
aactctgtgg gacacacaca cacacacaca cacacacaca cacacacaca cacgacatat 2280
aatcaggaga gggactcatt agagcctgta ggtcaggcag tggtagcaca tgcctttaat 2340
ctcaacactc aggaggcaga ggcaggtgga tttctgagtt ctaggtcagt ctgctttaca 2400
gagtgagttc taggactaca cagagaaatc caaaaaaaca aggctacaca gagaaaccat 2460

gtcctggggt aaaaaagaaa aagaaaa                                          2487

<210>  24

<211>  221

<212>  PRT

<213>  mouse

<400>  24

Met Trp Gln Phe Ser Ala Leu Leu Leu Phe Phe Leu Pro Gly Cys Cys
1               5                   10                  15

Thr Ala Gln Asp Ser Val Thr Gly Pro Glu Glu Val Ser Gly Gln Glu
            20                  25                  30

Gln Gly Ser Leu Thr Val Gln Cys Arg Tyr Ser Ser Tyr Trp Lys Gly
        35                  40                  45

Tyr Lys Lys Tyr Trp Cys Arg Gly Val Pro Gln Arg Ser Cys Asp Ile
    50                  55                  60

Leu Val Glu Thr Asp Lys Ser Glu Gln Leu Val Lys Lys Asn Arg Val
65                  70                  75                  80

Ser Ile Arg Asp Asn Gln Arg Asp Phe Ile Phe Thr Val Thr Met Glu
                85                  90                  95

Asp Leu Arg Met Ser Asp Ala Gly Ile Tyr Trp Cys Gly Ile Thr Lys
            100                 105                 110

Gly Gly Pro Asp Pro Met Phe Lys Val Asn Val Asn Ile Asp Gln Ala
        115                 120                 125

Pro Lys Ser Ser Met Met Thr Thr Thr Ala Thr Val Leu Lys Ser Ile
    130                 135                 140

Gln Pro Ser Ala Glu Asn Thr Gly Lys Glu Gln Val Thr Gln Ser Lys
145                 150                 155                 160

Glu Val Thr Gln Ser Arg Pro His Thr Arg Ser Leu Leu Ser Ser Ile
                165                 170                 175

Tyr Phe Leu Leu Met Val Phe Val Glu Leu Pro Leu Leu Leu Ser Met
            180                 185                 190

Leu Ser Ala Val Leu Trp Val Thr Arg Pro Gln Arg Cys Phe Gly Arg
        195                 200                 205

Gly Glu Asn Asp Leu Val Lys Thr His Ser Pro Val Ala
    210                 215                 220

<210> 25

<211> 1307

<212> DNA

<213> mouse

<400> 25
cgggaagtgg ctaaaggagg aagtgccgag tgagagtgag ggaaaccaca ggaccaggag 60

acgcaggagt ggagcatgta gcctgttctc gctggcaggc tccaccaagg tgacccggtg 120

tgagaagatg catttgtcat tgctggtccc ctttctcttc tggatcacag gctgctgcac 180

ggctgaggat ccagtcacag gtccagagga ggtgagcggt caggagcagg gctccttgac 240

agtgcagtgc cgatatacct caggctggaa ggattacaag aagtactggt gccaaggagt 300

tcctcagaga tcatgtaaga ctcttgttga aaccgatgca tcagagcagc tggtgaagaa 360

gaaccgtgtg tccatcaggg acaaccagag agacttcatc ttcacagtga ccatggagga 420

tctgaggatg agcgatgctg gcatttactg gtgtggaatt acgaaagtgc caaccatgcc 480

ccccatcacc tccaccacca ccatcttcac agtgacaacc acagtaaaag agaccagcat 540

gtttccaacg ctgactagct actactctga taacgggcat ggcggtggtg acagtggcgg 600

tggtgaagat ggcgtcggtg atgggtttct ggatctcagt gtgctcctcc cagtcatctc 660

tgcagtcctg ttgcttctcc tgttggtggc ctcgctcttt gcttggagga tggtgaggag 720

acagaagaaa gacctgtccc tgaagcagcc cagaacctcc cctggctcct cttggaaaaa 780

gggctcctcc atgtcctcct ctggcaagga ccaccaagag gaagtggaat atgtcaccat 840

ggctcccttt cccagggagg aggtttcata tgccgctctg actttggccg gcttgggtca 900

ggagcctact tatggcaata ctggctgccc catcacccat gttcccagga caggccttga 960

agaggagacc acagagtaca gcagcatcag gaggcccttg cctgcagcca tgccttaatc 1020

ttggtctctg aaggcggctt ggagcatgga tctttacatc tgcctctgta cctgcttcct 1080

tacccggccc agctggtgac tggaactctg tccatccgtc tctcatggcc atcagctcta 1140

ccttgcttga gcttggagtt caacctcagg gggttccagg gaattaaggc tccttccaca 1200

tccccactta tagccaatgt accttggaag gtaccaggca ggctgcttca gggatgctgt 1260

gtaaatcgta tcaacgatga caataatagc aatcaacctt tatttat 1307

<210> 26

<211> 296

<212> PRT

<213> mouse

<400> 26

Met His Leu Ser Leu Leu Val Pro Phe Leu Phe Trp Ile Thr Gly Cys
1               5                   10                  15

Cys Thr Ala Glu Asp Pro Val Thr Gly Pro Glu Glu Val Ser Gly Gln
            20                  25                  30

Glu Gln Gly Ser Leu Thr Val Gln Cys Arg Tyr Thr Ser Gly Trp Lys
        35                  40                  45

Asp Tyr Lys Lys Tyr Trp Cys Gln Gly Val Pro Gln Arg Ser Cys Lys
    50                  55                  60

Thr Leu Val Glu Thr Asp Ala Ser Glu Gln Leu Val Lys Lys Asn Arg
65                  70                  75                  80

Val Ser Ile Arg Asp Asn Gln Arg Asp Phe Ile Phe Thr Val Thr Met
                85                  90                  95

Glu Asp Leu Arg Met Ser Asp Ala Gly Ile Tyr Trp Cys Gly Ile Thr
            100                 105                 110

Lys Val Pro Thr Met Pro Pro Ile Thr Ser Thr Thr Thr Ile Phe Thr
        115                 120                 125

Val Thr Thr Thr Val Lys Glu Thr Ser Met Phe Pro Thr Leu Thr Ser
    130                 135                 140

Tyr Tyr Ser Asp Asn Gly His Gly Gly Gly Asp Ser Gly Gly Gly Glu
145                 150                 155                 160

Asp Gly Val Gly Asp Gly Phe Leu Asp Leu Ser Val Leu Leu Pro Val
                165                 170                 175

Ile Ser Ala Val Leu Leu Leu Leu Leu Leu Val Ala Ser Leu Phe Ala
            180                 185                 190

Trp Arg Met Val Arg Arg Gln Lys Lys Asp Leu Ser Leu Lys Gln Pro
        195                 200                 205

Arg Thr Ser Pro Gly Ser Ser Trp Lys Lys Gly Ser Ser Met Ser Ser
    210                 215                 220

Ser Gly Lys Asp His Gln Glu Glu Val Glu Tyr Val Thr Met Ala Pro
225                 230                 235                 240

Phe Pro Arg Glu Glu Val Ser Tyr Ala Ala Leu Thr Leu Ala Gly Leu
                245                 250                 255

Gly Gln Glu Pro Thr Tyr Gly Asn Thr Gly Cys Pro Ile Thr His Val
            260                 265                 270

Pro Arg Thr Gly Leu Glu Glu Glu Thr Thr Glu Tyr Ser Ser Ile Arg
        275                 280                 285

Arg Pro Leu Pro Ala Ala Met Pro
    290                 295

<210>  27

<211>  114

<212>  PRT

<213>  mouse

<400>  27

Gly Cys Cys Thr Ala Gln Asp Pro Val Thr Gly Pro Glu Glu Val Ser
1               5                   10                  15

Gly Gln Glu Gln Gly Ser Leu Thr Val Gln Cys Arg Tyr Asp Ser Gly
            20                  25                  30

Trp Lys Asp Tyr Lys Lys Tyr Trp Cys Arg Gly Ala Tyr Trp Lys Ser
        35                  40                  45

Cys Glu Ile Leu Val Glu Thr Asp Ala Ser Glu Gln Leu Val Lys Glu
    50                  55                  60

Asn Arg Val Ser Ile Arg Asp Asp Gln Thr Asp Phe Ile Phe Thr Val
65                  70                  75                  80

Thr Met Glu Asp Leu Arg Met Ser Asp Ala Asp Ile Tyr Trp Cys Gly
                85                  90                  95

Ile Thr Lys Ala Gly Thr Asp Pro Met Phe Lys Val Asn Val Asn Ile
            100                 105                 110

Asp Pro

<210>  28

<211>  295

<212>  PRT

<213>  homosapiens

<400>  28

Met Pro Leu Leu Thr Leu Tyr Leu Leu Leu Phe Trp Leu Ser Gly Tyr
1               5                   10                  15

Ser Ile Val Thr Gln Ile Thr Gly Pro Thr Thr Val Asn Gly Leu Glu
            20                  25                  30

Arg Gly Ser Leu Thr Val Gln Cys Val Tyr Arg Ser Gly Trp Glu Thr
        35                  40                  45

Tyr Leu Lys Trp Trp Cys Arg Gly Ala Ile Trp Arg Asp Cys Lys Ile
    50                  55                  60

Leu Val Lys Thr Ser Gly Ser Glu Gln Glu Val Lys Arg Asp Arg Val
65                  70                  75                  80

Ser Ile Lys Asp Asn Gln Lys Asn Arg Thr Phe Thr Val Thr Met Glu
                85                  90                  95

Asp Leu Met Lys Thr Asp Ala Asp Thr Tyr Trp Cys Gly Ile Glu Lys
            100                 105                 110

Thr Gly Asn Asp Leu Gly Val Thr Val Gln Val Thr Ile Asp Pro Ala
        115                 120                 125

Pro Val Thr Gln Glu Glu Thr Ser Ser Ser Pro Thr Leu Thr Gly His
    130                 135                 140

His Leu Asp Asn Arg His Lys Leu Leu Lys Leu Ser Val Leu Leu Pro
145                 150                 155                 160

Leu Ile Phe Thr Ile Leu Leu Leu Leu Leu Val Ala Ala Ser Leu Leu
                165                 170                 175

Ala Trp Arg Met Met Lys Tyr Gln Gln Lys Gly Glu Arg Thr Trp Val
            180                 185                 190

Leu Gln Pro Leu Glu Gly Asp Leu Cys Tyr Ala Asp Leu Thr Leu Gln
        195                 200                 205

Leu Ala Gly Thr Ser Pro Gln Lys Ala Thr Thr Lys Leu Ser Ser Ala
    210                 215                 220

Gln Val Asp Gln Val Glu Val Glu Tyr Val Ala Ala Gly Met Ser Pro
225                 230                 235                 240

Glu Gln Thr Met Ala Ser Leu Pro Lys Glu Asp Ile Ser Tyr Ala Ser
                245                 250                 255

Leu Thr Leu Gly Ala Glu Asp Gln Glu Pro Thr Tyr Cys Asn Met Gly
            260                 265                 270

His Leu Ser Ser His Leu Pro Gly Arg Gly Pro Glu Glu Pro Thr Glu
        275                 280                 285

Tyr Ser Thr Ile Ser Arg Pro
    290                 295

## Description

### BACKGROUND OF THE INVENTION

### FIELD OF THE INVENTION

The present invention relates generally to therapeutic and diagnostic agents. More particularly, the present invention provides molecules having structural features characteristic of immunoregulatory signalling (IRS) molecules and which are expressed by cells of haematopoietic lineages such as, in particular, leukocytes. The molecules of the present invention find broad application *inter alia* as diagnostic markers for cells, targets for cell therapy and as validated drug targets in order to modulate the immune response and to treat, prevent and diagnose a range of diseases conditions including cancer, genetic disease, inflammatory conditions and conditions associated with aberrant haematopoietic cell function or activity. The present invention extends to binding partners of the instant molecules such as, for example, antibodies, ligands, adaptor and other signalling associated molecules, agonists and antagonists and to methods of screening for same.

### DESCRIPTION OF THE PRIOR ART

Bibliographic details of references provided in the subject specification are also listed at the end of the specification.

Reference to any prior art in this specification is not and should not be taken as an acknowledgment or any form of suggestion that this prior art forms part of the common general knowledge in any country.

The increasing sophistication of recombinant DNA technology is greatly facilitating research and development in a range of biotechnology-related industries. The availability of therapeutic or prophylactic reagents which regulate or manipulate immune responses in the body is developing, based largely on the ability to clone and study molecules which are expressed by cells of the immune system. Cell-surface and secreted molecules are particularly important expression products.

The Immunoregulatory Signalling (IRS) family is a group of cell surface molecules which regulate leukocyte function by delivering signals to the cells on which they are expressed. Members of the IRS family are typically either Immunoglobulin gene superfamily members or C-type lectins. Delivery of signals by these IRS molecules is through control of protein phosphorylation. Triggering IRS molecules typically associate with adaptor molecules that contain a cytoplasmic immuno tyrosine based activatory motif (ITAM) which interacts with SH2 domain-containing tyrosine kinases. To-date, a number of common adaptor molecules have been described; CD3ζ, FcεRγ, DAP12 and DAP10 (Wilson MJ, et al., J: Immunol Res 22:21, 2000). These triggering molecules contain either an arginine or lysine residue in the transmembrane region and their expression on the cell surface requires co-expression of the correct adaptor molecule. Inhibitory IRS molecules have one or more tyrosine based inhibitory motif (ITIM) in their cytoplasmic domains which interacts with SH2 domain-containing tyrosine phosphatases.

The leukocyte receptor complex is a large complex of IRS encoding genes on human chromosome 19q13.4 that has been characterized (Wende et al., Immunogenetics 51: 703, 2000; Wende et al., Mamm Genome 10(2): 154, 1999; Wilson et al., Methods Mol Biol 121: 251, 2000; Wagtmann et al., Current Biol 7:615, 1997). The complex contains more than twenty genes belonging to the IRS family and includes the genes for the immunoglobulin like transcript (ILT) molecules, the killer Ig-like receptor (KIR) molecules and the natural cytotoxic receptor (NCR) molecule NKp46.

The CMRF-35A and CMRF-35H molecules are also IRS molecules (Clark et al., Tissue Antigens 55: 101-109, 2000; Clark et al., Tissue Antigens 57: 415-423, 2001; Green et al., Int Immunol. 10: 891-899, 1998) having, in the case of CMRF-35H, ITIM in the cytoplasmic region.

35A and 35H are expressed throughout haematopoiesis from the early bone marrow precursors by most leukocyte lineages involved in innate and adaptive immunity. Both molecules are members of the Ig superfamily, each having a single V-like extracellular domain. They are most closely related to the Ig binding domains of the Fc receptor for polymeric IgA and IgM (Jackson et al., Eur. J. Immunol. 22: 1157-1163, 1992; Green et al., Int. Immunol. 10: 891-899, 1998P) but are also distantly related to the TREM molecules (Bouchon et al., J. Immunol. 164: 4991-4995, 2000), NKp44 (Vitale et al., J. Exp. Med. 187: 2065-2072, 1998) and NKp46 (Pessino et al., J. Exp. Med. 188: 953-960, 1998).

Like other IRS molecules, CMRF-35A and CMRF-35H are emerging as molecules which will shed light on how immune cells monitor and respond to their environment. In accordance with the present invention, molecules related to CMRF-35A and CMR-35H have been identified as a family of CMRF-35A and CMRF-35H-like molecules, which are expressed on defined cells and which are encoded by members of a gene family. The term "35-LM" is used in this specification to encompass CMRF-35-like molecules and includes CMRF-35A, CMRF-35H and all other closely related molecules.

### SUMMARY OF THE INVENTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Nucleotide and amino acid sequences are referred to by a sequence identifier number (SEQ ID NO:). The SEQ ID NOs: correspond numerically to the sequence identifiers <400> 1 (SEQ ID NO:1), <400>2 (SEQ ID NO:2), etc. A summary of the sequence identifiers is provided in Table 2. A sequence listing is provided at the end of the specification.

In accordance with the present invention, a family of closely linked genes on human chromosome 17 has been identified which comprises members encoding polypeptides which are structurally related to the leukocyte surface glycoproteins CMRF-35A and CMRF-35H.

For comparative purposes, the nucleotide and amino acid sequences of human CMRF-35A are set forth in SEQ ID NOs:1 and 2, respectively and the nucleotide and amino acid sequences of human CMRF-35H are set forth in SEQ ID NOs:3 and 4, respectively. In this context, reference to "h" is a reference to a molecule derived from human species; similarly, the prefix "m" is a reference to a molecule derived from mice. The term "35-LM"is used to encompass CMRF-35A, CMRF-35H and related molecules. Table 1 provides a summary of 35-LMs of the present invention.

In one embodiment the present invention provides a nucleic acid molecule or a derivative or homolog thereof corresponding to a gene family which is located on human chromosome 17q22-24 or the equivalent region in other species (e.g. chromosome 11 in mice). The nucleic acid molecules of the present invention, in a further embodiment, encode a polypeptide having one or more of the identifying characteristics of 35A or 35H selected from the following:
(i) sequence similarity to an Ig binding domain of CMRF-35A or CMRF-35H;
(ii) sequence similarity to a cytoplasmic ITIM motif; or
(iii) expression of polypeptide *in vivo* requires binding to an adaptor molecule comprising an ITAM motif.

The polypeptides may be expressed on the surface of defined populations of haematopoietic cells or may be excreted or be in soluble form.

A homolog includes a nucleic acid molecule comprising a nucleotide sequences having at least 40% similarity or higher to SEQ ID NO: (hCMRF-35A) or SEQ ID NO:3 (hCMRF-35H) SEQ ID NO:5 (h35-L1), SEQ ID NO:7 (h35-L2), SEQ ID NO:9 (h35-L3), SEQ ID NO:11 (h35-L4) or SEQ ID NO:13 (h35-L5) or SEQ ID NO:15 (m35a) or SEQ ID NO:17 (m35c) or SEQ ID NO:19 (m35d) or SEQ ID NO:21 (m35f) or SEQ ID NO:23 (m35a) or SEQ ID NO:25 (m35g), or to its complementary form or which is capable of hybridizing to SEQ ID NO:1 or SEQ ID NO:3 or SEQ ID NO:5, SEQ ID NO:7, SEQ ID NO:9, SEQ ID NO:11 or SEQ ID NO:13 or SEQ ID NO:15 or SEQ ID NO:17 or SEQ ID NO:19 or SEQ ID NO:21 or SEQ ID NO:23 or SEQ ID NO:25, or its complementary form under low stringency conditions.

In another embodiment, the present invention provides an isolated or recombinant polypeptide derived from the present nucleic acid molecules. In a preferred embodiment, the polypeptides are expressed on the surface of defined populations of haematopoietic cells and conveniently provide cell surface markers for these cell types. In one embodiment, the 35-LMs are expressed on the surface of leukocytes and are capable of influencing the ability of the leukocyte to respond to its environment. Specifically, expression of the 35-LMs influences the ability of the cells to proliferate, differentiate, activate, express cytokines, perform effector functions or undergo apoptosis.

In yet another embodiment, the polypeptide comprises a sequence of amino acids selected from those set forth in SEQ ID NO:2 (hCMRF-35A) or SEQ ID NO:4 (hCMRF-35H) or SEQ ID NO: 6 (h35-L1) or SEQ ID NO:8 (h35-L2)or SEQ ID NO:10 (h35-L3) or SEQ ID NO:12 (h35-L4)or SEQ ID NO:14 (h35-L5) or SEQ ID NO:16 (m35a) or SEQ ID NO:18 (m35c) or SEQ ID NO:20 (m35d) or SEQ ID NO:22 (m35f) or SEQ ID NO:24 (m35h) or SEQ ID NO:26 (m35g) or SEQ ID NO:27 (m350e, Ig domain) or SEQ ID NO:28 (35-L5b) or an amino acid sequence having at least 20% similarity to all or part of any one of the listed sequences. In another embodiment the instant polypeptide is encoded by a nucleotides sequence set forth in SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25 or by a nucleotide sequence having at least about 20% similarity thereto or a nucleotide sequence capable of hybridizing to SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23 or SEQ ID NO:25 or its complementary form under low stringency conditions. Binding partners may be used to activate or inhibit the immune system.

In another embodiment, binding partners including soluble forms of the instant polypeptides, antibodies, ligands, agonist and antagonists are usefully developed as diagnostic, therapeutic or prophylactic agents. As targets for cell therapy, the nucleic acid and polypeptide molecules of the present invention provide targets in screens for specific binding partners. Binding partners are contemplated for use in the treatment, prevention or diagnosis of conditions associated with aberrant cellular immunity or altered immune cell function or activity, as is found in cancer, autoimmune conditions, infections, immunosuppression and inflammation, among others.

**TABLE 1**

| ***Nomenclature for CMRF-35 family of molecule*** | | |
|---|---|---|
| **FAMILY NAME** | **NOMENCLATURE** | **MOUSE ORTHOLOG** |
| 35-LM | CMRF-35A | m35h |
| | CMRF-35H | m35c |
| | 35-L1 | m35f |
| | 35-L2 | m35d |
| | 35-L3 | DIgR1 |
| | 35-L4 | m35e |
| | 35-L5 and 35-L5b | m35g DIgR2 m35a |

A summary of sequence identifiers used throughout the subject specification is provided in Table 2.

**TABLE 2**

| ***Summary of Sequence Identifiers*** | |
|---|---|
| **SEQUENCE ID NO:** | **DESCRIPTION** |
| 1 | Nucleotide sequence of hCMRF-35A |
| 2 | Amino acid sequence of hCMRF-35A |
| 3 | Nucleotide sequence of hCMRF-35H |
| 4 | Amino acid sequence of hCMRF-35H |
| 5 | Nucleotide sequence of h35-L1 |
| 6 | Amino acid sequence of h35-L1 |
| 7 | Nucleotide sequence of h35-L2 |
| 8 | Amino acid sequence of h35-L2 |
| 9 | Nucleotide sequence of h35-L3 |
| 10 | Amino acid sequence of h35-L3 |
| 11 | Nucleotide sequence of h35-L4 |
| 12 | Amino acid sequence of h35-L4 |
| 13 | Nucleotide sequence of h35-L5 |
| 14 | Amino acid sequence of h35-L5 |
| 15 | Nucleotide sequence of m35-a |
| 16 | Amino acid sequence of m35-a |
| 17 | Nucleotide sequence of m35-c |
| 18 | Amino acid sequence of m35-c |
| 19 | Nucleotide sequence of m35-d |
| 20 | Amino acid sequence of m35-d |
| 21 | Nucleotide sequence of m35-f |
| 22 | Amino acid sequence of m35-f |
| 23 | Nucleotide sequence of m35-h |
| 24 | Amino acid sequence of m35-h |
| 25 | Nucleotide sequence of m35-g |
| 26 | Amino acid sequence of m35-g |
| 27 | Amino acid sequence of m35-e (Ig domain) |
| 28 | Amino acid sequence of h35-L5b |

### BRIEF DESCRIPTION OF THE FIGURES

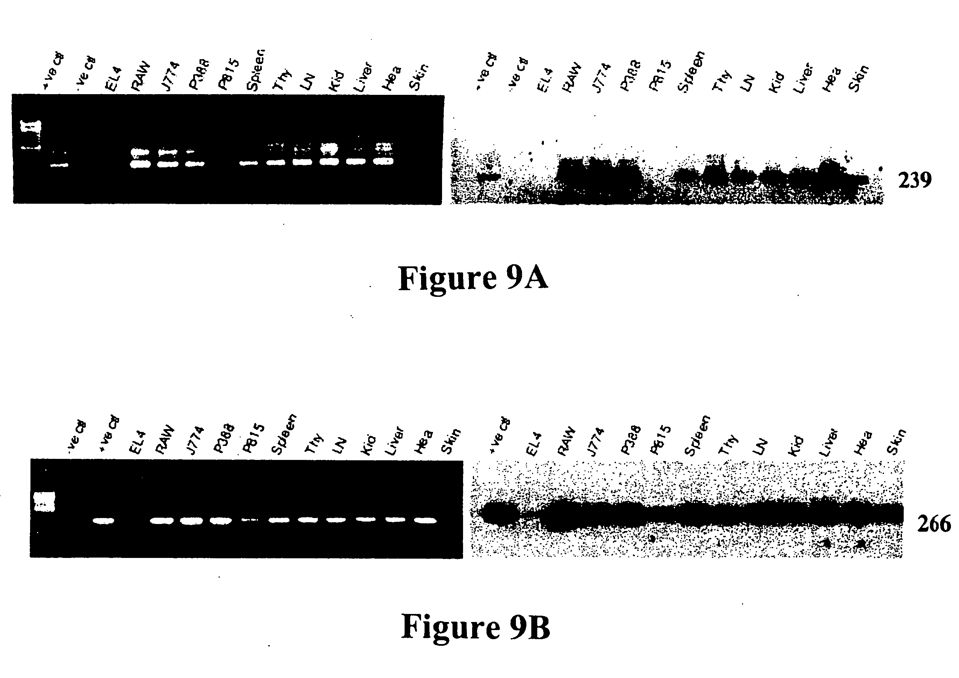

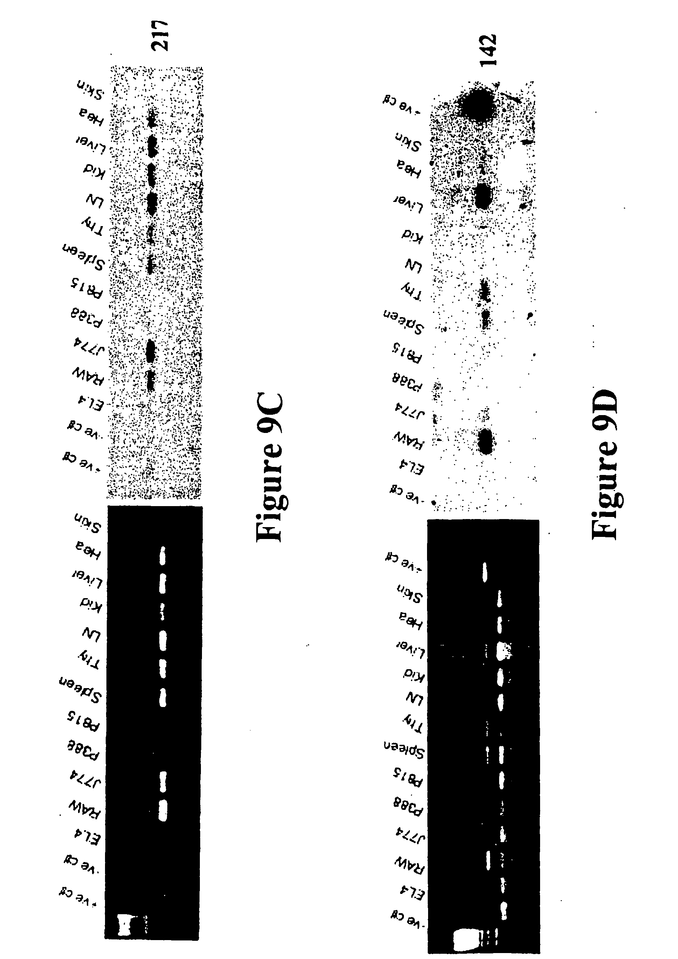

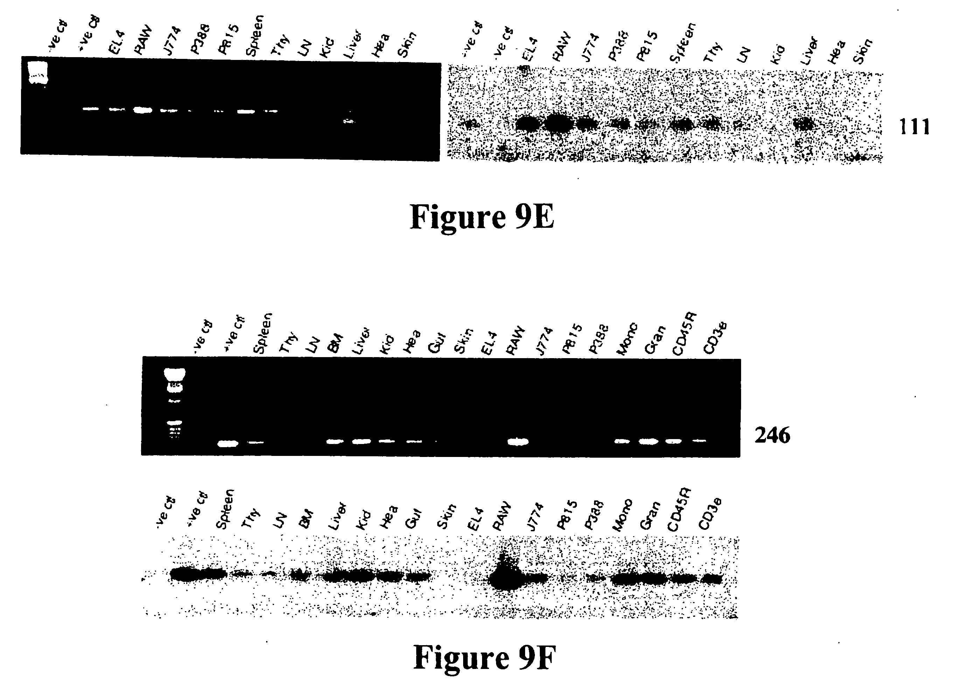

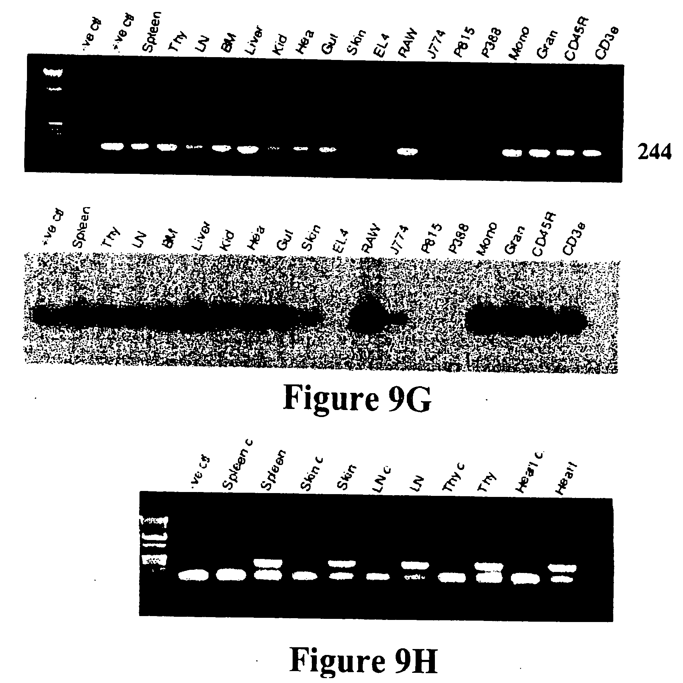

**Figure 1** is a representation of an alignment of the nucleic acid sequences of 35-LMs.
**Figure 2** is a representation of an alignment of predicted amino acid sequences of 35-LMs.
**Figure 3** is a diagrammatic representation showing the expression analysis of the h35-LMs on cell lines and freshly purified hemopoietic populations.
**Figure 4** is a photographic representation showing the expression of AW8 (also called 35-L3) RNA assayed by RT-PCR. Filters are probed with a specific AW8 oligonucleotide. M; marker, 1; B cells, 2; NK cells, 3; granulocytes, 4; monocytes, 5; lin-ve dendritic cells, 6; monocyte derived DC, 7; activated monocyte derived DC, 8; T cells, 9; negative control.
**Figure 5** is a representation of an alignment of the nucleic acid sequences of m35-LMs.

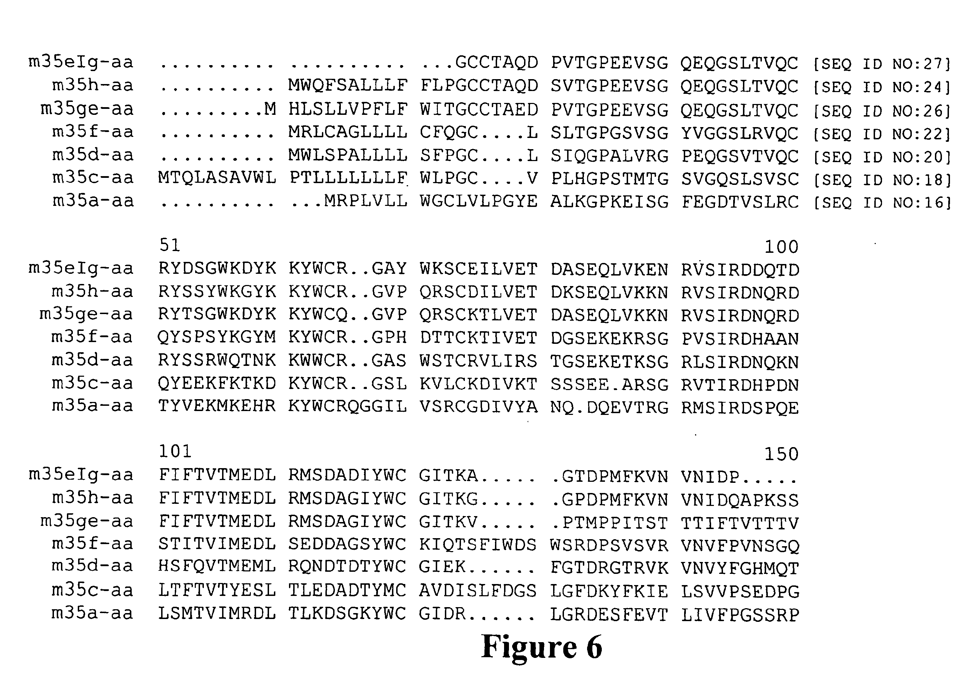

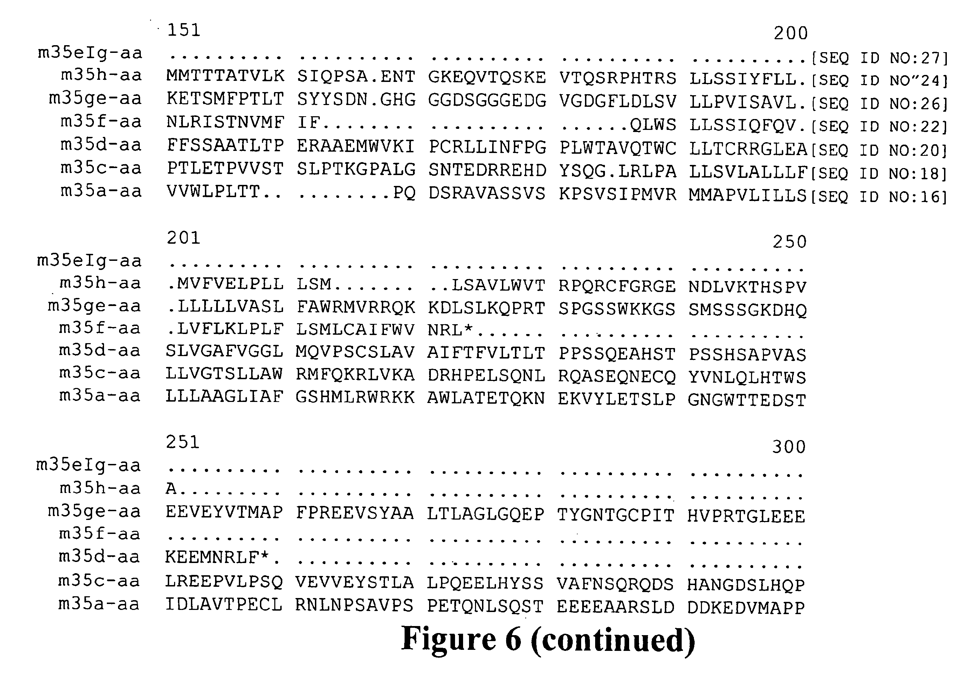

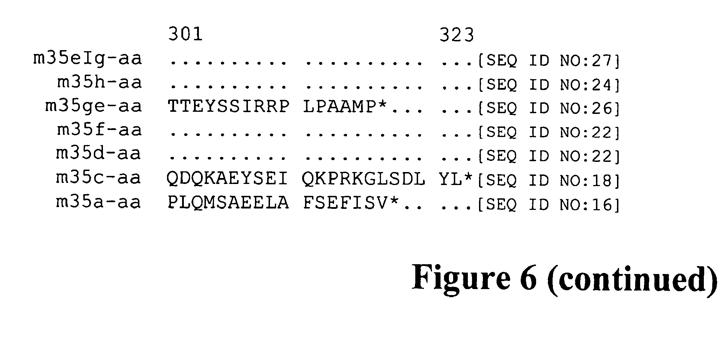

**Figure 6** is a representation of an alignment of the predicted amino acid sequences of the mouse.
**Figure 7** is a diagrammatic representation showing the expression analysis of the m35-LMs on cell lines and freshly purified haematopoietic populations.

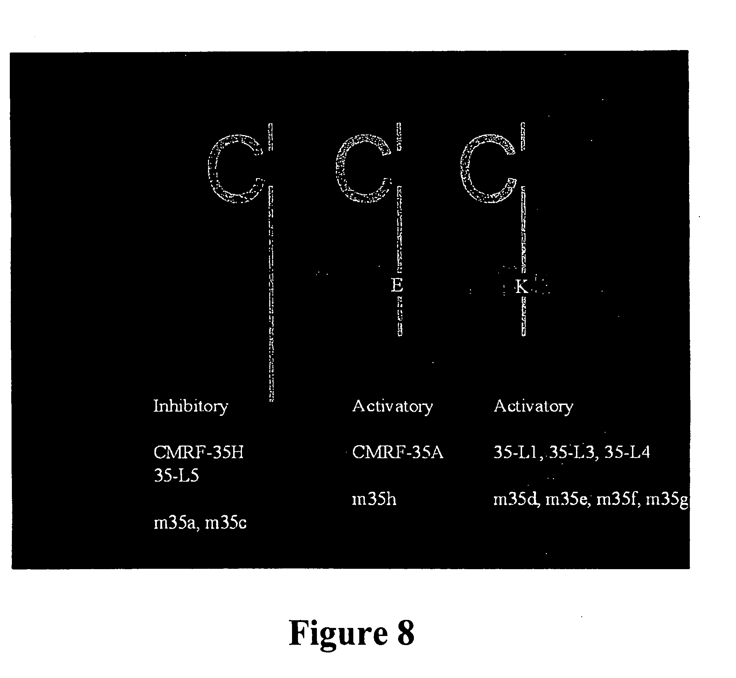

**Figure 8** is a diagrammatic representation showing the structure the three molecule types in the 35-LM family:-
   - Type I =: Inhibitory
   - Type II =: E residue in the transmembrane domain
   - Type III =: K residue in the transmembrane domain
**Figure 9** is a photographic representation showing family expression in various BALB/c tissue, cell lines and sorted spleen cell populations. Pictures show gel photos (dark background) and Southerns (light background). **(A) to (G)** show m35a, m35c, m35e, m35f, m35g, m35h and DIgR1 expression. Expected fragment size is indicated on the right hand side. (**H**) RT-PCR using mouse GAPDH primers on a selection of cDNA samples with and without (c, control) reverse transcriptase. Integrity of all cDNA samples was confirmed before use for expression analysis. (Thy, thymus; LN, lymph node; BM, bone marrow; Kid, kidney; Hea, heart; Mono, monocytes; Gran; granulocytes).
**Figure 10** is a graphical representation demonstrating 35-L1 surface expression on monocytes. Monocytes, B cells, Natural Killer cells and T cells were dual stained with 35-L1 and their respective surface marker and the cells analyzed using flow cytometry. Results from these experiments demonstrated that the majority of CD14+ monocytes co-stained for 35-L1 surface expression.

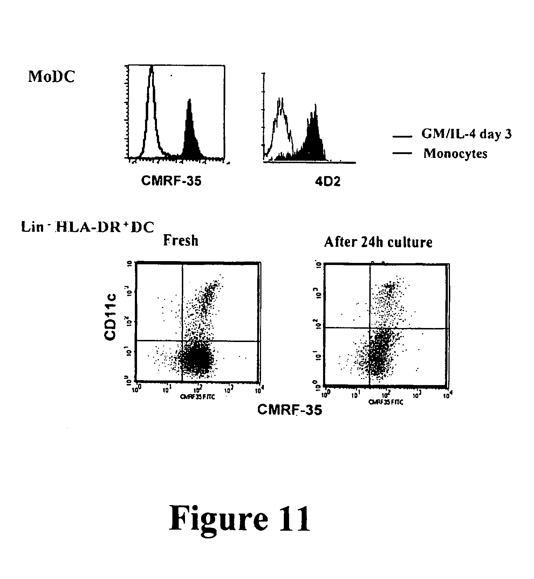

**Figure 11** is a graphical representation demonstrating that monocyte derived dendritic cells (MoDC) and blood DCs have differential expression for CMRF-35A/H and 35-L1. Analysis of cell surface expression of CMRF-35A/H and 35-L1 using flow cytometry revealed that MoDC expressed higher levels of surface CMRF-35A/H and 35-L1.

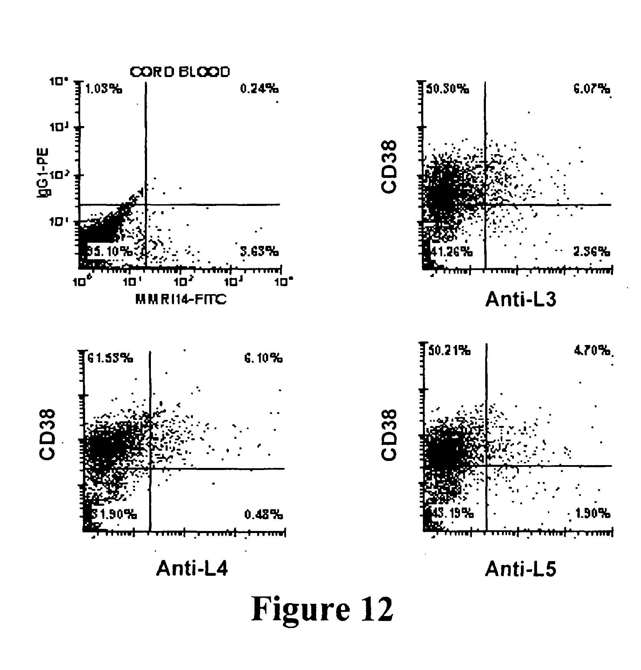

**Figure 12** is a graphical representation demonstrating cell surface expression of 35-L3, 35-L4 and 35-L5 on cord blood. CD38⁺ positive population of cells from cord blood was analyzed for cell surface expression of 35-L3, 35-L4 and 35-L5. Results demonstrated that 6.07% of cells stained positive for CD38/35-L3, 6.10% stained positive for CD38/35-L4 and 4.70% stained positive for CD38/35-L5.
**Figure 13** is a graphical representation demonstrating the cell surface expression of 35-L3 and 35-L1 on AML cells. Flow cytometric analysis demonstrated that a population of AML cells from sample #14 stained positive for 35-L3 and/or 35L-1.
**Figure 14** is a graphical representation demonstrating the cell surface expression of 35-L3 and 35-L5 on AML cells. Flow cytometric analysis demonstrated that a population of AML cells from sample #16 stained positive for 35-L3 and/or 35L-5.

### DETAILED DESCRIPTION OF THE PREFERRED EMBODIMENTS

The present invention provides members of a new family of immunoregulatory signalling-like molecules encoded by nucleic acid molecules which correspond to a gene family located on human chromosome 17q22-24 or the equivalent region in other species. These molecules are referred to as 35-LMs for "CMRF-35-like molecules".

Accordingly, one aspect of the present invention provides an isolated or recombinant nucleic acid molecule, or a derivative or homolog thereof, corresponding to a gene family which is located on human chromosome 17q22-24 or the equivalent region in other species. The equivalent region in mouse species, for example, is on chromosome 11.

The nucleic acid molecule may be isolated or derived from any suitable animal such as humans, primates, livestock animals (e.g. horses, cows, sheep, donkeys, pigs), laboratory test animals (e.g. mice, rats, rabbits, hamsters, guinea pigs), companion animals (e.g. dogs, cats), or captive wild animals (e.g. deer, foxes, kangaroo). Various databases are now available which compare chromosomal regions of synteny between two species, see for example the Seldin/Debry human/mouse homology map available through OPIM at http://www3.ncbi.lmn.hih.gov/omim, among others.

As used herein, the term "derived from" means that a particular element or group of elements has originated from the source described, but has not necessarily been obtained directly from the specified source.

The terms "nucleic acid molecule", "genetic sequence", "sequence of nucleotides" include RNA, cDNA, genomic DNA, synthetic forms and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (e.g. methyl phosphonates, phosphotriesters, phosphoamidates, carbamates, etc.), charged linkages (e.g. phosphorothioates, phosphorodithioates, etc.), pendent moieties (e.g. polypeptides), intercalators (e.g. acridine, psoralen, etc.), chelators, alkylators and modified linkages (e.g. α-anomeric nucleic acids, etc.). Also included are synthetic molecules that mimic polynucleotides in their ability to bind to a designated sequence *via* hydrogen binding and other chemical interactions. Such molecules are known in the art and include, for example, those in which peptide linkages substitute for phosphate linkages in the backbone of the molecule. The nucleic acid molecules of the present invention may be in single, double stranded form and other multiple forms thereof.

Reference herein to a nucleic acid molecule includes reference to a "gene".

The present nucleic acid molecules correspond to a gene family and may be independently or co-ordinately expressed therefrom. The nucleic acid molecules may be full length genes or they may be parts thereof.

The term "gene" is used in its broadest sense and includes cDNA corresponding to the exons of a gene. Reference herein to a "gene" is also taken to include:-
(i) a classical genomic gene consisting of transcriptional and/or translational regulatory sequences and/or a coding region and/or non-translated sequences (i.e. introns, 5'- and 3'- untranslated sequences); or
(ii) mRNA or cDNA corresponding to the coding regions (i.e. exons) and 5'- and 3'-untranslated sequences of the gene.

Reference to a "part " of a nucleic acid molecule according the present invention includes fragments of longer molecules defined as having a minimal size of at least about 10 nucleotides or preferably about 13 nucleotides or more preferably 17, 18, 19 or 20 nucleotides. There is no maximal size but a size of about 200 contiguous nucleotides is a useful maximum. Such parts may be useful as probes or primers. Alternatively such molecules may encode a polypeptide such as a soluble protein lacking a cytoplasmic or transmembrane domain. Accordingly, this definition includes all sizes in the range of 10-200 nucleotides as well as greater than 200 nucleotides. Thus, this definition includes nucleic acids of 12, 15, 17, 18, 19, 20, 25, 40, 60, 80, 100, 200, 300, 400, 500, 1000 or 1500 nucleotides or nucleic acids having any number of nucleotides within these values (e.g. 13, 16, 23, 30, 28, 50, 72, 121, etc. nucleotides) or nucleic acids having more than 1500 nucleotides or any number of nucleotides between 1500 and the number shown in SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23 or SEQ ID NO:25.

In a preferred aspect, the present invention provides for an isolated nucleic acid molecule comprising a sequence selected from the group consisting of:
(a) a sequence provided in SEQ ID NOs:1, 3, 5, 7, 9, 11, 13, 15, 17, 19, 21, 23 and 25;
(b) a complement of a sequence provided in SEQ ID NOs:1, 3, 5, 7, 9, 11, 13, 15, 17, 19, 21, 23 or 25;
(c) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
(d) a sequence which hybridizes to the complement of a sequence provided in SEQ ID NOs:1, 3, 5, 7, 9, 11, 13, 15, 17, 19, 21, 23 or 25, under conditions of low stringency;
(e) a sequence having at least 70% identity after optimal alignment to a sequence provided in SEQ ID NOs:1, 3, 5, 7, 9, 11, 13, 15, 17, 19, 21, 23 or 25;
(f) a derivative of a sequence provided in SEQ ID NOs:1, 3, 5, 7, 9, 11, 13, 15, 17, 19, 21, 23 or 25; and
(g) a homolog of a sequence provided in SEQ ID NOs:1, 3, 5, 7, 9, 11, 13, 15, 17, 19, 21, 23 or 25.

Members of the 35-LM family may be identified or cloned by any of a wide range of strategies including interaction of the polypeptides of the family with specific antibodies, homology cloning, *in silico* mining, through EST database or through further mapping and cloning procedures in relation to the 35-LM genomic complex. A number of strategies also exist for cloning full length cDNAs from the short sequences generated including screening cDNA libraries and 5' and 3' RACE strategies. General teaching on manipulating and cloning nucleic acid molecules may be found in Sambrook et al. (Molecular Cloning: A Laboratory Manual, Cold Spring Harbor Laboratory, 3rd Edition, 2001).

The isolated or recombinant nucleic acid molecule of the present invention may be deployed in appropriate vectors and cells for sequencing, cloning, expression or for administration to a cell, as described in standard laboratory manuals such as Ausubel et al., Current Protocols in Molecular Biology, John Wiley & Sons Inc, 1994-1998.

Homologs of the instant nucleic acid sequences include orthologous gene sequences from different species which are related by common phylogenic descent and gene sequences from other species which are similar to the instant nucleic acid molecules as a result of, for example, convergent evolution, wherein the homologs are functionally and structurally related to the instant nucleic acid sequences and are consequently readily identified and/or isolated by hybridization based methods or by sequence comparison with available genetic databases. A homolog includes a nucleic acid molecule comprising a nucleotide sequences having at least 40% similarity or higher to SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23 or SEQ ID NO:25, or to its complementary form or which is capable of hybridizing to SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23 or SEQ ID NO:25, or its complementary form under low stringency conditions.

The term "similarity" as used herein includes exact identity between compared sequences at the nucleotide or corresponding amino acid level. Where there is non-identity at the nucleotide level, "similarity" includes differences between sequences which result in different amino acids that are nevertheless related to each other at the structural, functional, biochemical and/or conformational levels. Where there is non-identity at the amino acid level, "similarity" includes amino acids that are nevertheless related to each other at the structural, functional, biochemical and/or conformational levels. In a particularly preferred embodiment, nucleotide and sequence comparisons are made at the level of identity rather than similarity.

Terms used to describe sequence relationships between two or more polynucleotides or polypeptides include "reference sequence", "comparison window", "sequence similarity", "sequence identity", "percentage of sequence similarity", "percentage of sequence identity", "substantially similar" and "substantial identity". A "reference sequence" is at least 12 but frequently 15 to 18 and often at least 25 or above, such as 30 monomer units, inclusive of nucleotides and amino acid residues, in length. Because two polynucleotides may each comprise (1) a sequence (i.e. only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of typically 12 contiguous residues that is compared to a reference sequence. The comparison window may comprise additions or deletions (i.e. gaps) of about 20% or less as compared to the reference sequence (which does not comprise additions or deletions) for optimal alignment of the two sequences. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e. resulting in the highest percentage homology over the comparison window) generated by any of the various methods selected. Reference also may be made to the BLAST family of programs as, for example, disclosed by Altschul el al., Nucl. Acids Res. 25: 3389. 1997. A detailed discussion of sequence analysis can be found in Unit 19.3 of Ausubel *et al*., 1994-1998, *supra*).

The terms "sequence similarity" and "sequence identity" as used herein refers to the extent that sequences are identical or functionally or structurally similar on a nucleotide-by-nucleotide basis or an amino acid-by-amino acid basis over a window of comparison. Thus, a "percentage of sequence identity", for example, is calculated by comparing two optimally aligned sequences over the window of comparison, determining the number of positions at which the identical nucleic acid base (e.g. A, T, C, G, I, U) or the identical amino acid residue (e.g. Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. For the purposes of the present invention, "sequence identity" will be understood to mean the "match percentage" calculated by the DNASIS computer program (Version 2.5 for windows; available from Hitachi Software engineering Co., Ltd., South San Francisco, California, USA) using standard defaults as used in the reference manual accompanying the software. Similar comments apply in relation to sequence similarity.

Preferably, the percentage similarity between a particular sequence and a reference sequence (nucleotide or amino acid) is at least about 30% or at least about 40% or at least about 50% or at least about 65% or at least about 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100%. A percentage identity of approximately 30-32% is particularly preferred.

Similarity at the nucleic acid level may be assessed in assays exploiting different stringency of hybridization conditions as is well known in the art and is, for example, described in Ausubel *et al., supra*, 1994-1998.

Reference herein to stringent hybridization conditions preferably means conditions which permit selective hybridization or annealing between molecules which are substantially similar. The hybridization temperature composition and ionic strength of the hybridization solution which meet this criteria will vary depending upon a number of well characterized factors such as length, degree of complementarity and GC content. For longer sequences it is generally possible to calculate the expected melting point of duplex nucleic acid sequences under various conditions. Hybridization may be to all or part of the instant polynucleotides with the minimum length being sufficient to provide specificity.

Low stringency hybridization conditions includes and encompasses from at least about 0 to at least about 15% v/v formamide and from at least about 1 M to at least about 2 M salt for hybridization, and at least about 1 M to at least about 2 M salt for washing conditions. Generally, low stringency is at from about 25-30°C to about 42°C. The temperature may be altered and higher temperatures used to replace formamide and/or to give alternative stringency conditions.

Medium stringency includes and encompasses from at least about 16% v/v to at least about 30% v/v formamide and from at least about 0.5 M to at least about 0.9 M salt for hybridization, and at least about 0.5 M to at least about 0.9 M salt for washing conditions. High stringency includes and encompasses from at least about 31% v/v to at least about 50% v/v formamide and from at least about 0.01 M to at least about 0.15 M salt for hybridization, and at least about 0.01 M to at least about 0.15 M salt for washing conditions. In general, washing is carried out Tₘ = 69.3 + 0.41 (G+C%). However, the Tₘ of a duplex DNA decreases by 1°C with every increase of 1% in the number of mismatch base pairs (Bonner and Laskey, Eur. J Biochem. 46: 83, 1974). Formamide is optional in these hybridization conditions. Accordingly, particularly preferred levels of stringency are defined as follows: low stringency is 6 x SSC buffer, 0.1% w/v SDS at 25-42°C; a moderate stringency is 2 x SSC buffer, 0.1% w/v SDS at a temperature in the range 20°C to 65°C; high stringency is 0.1 x SSC buffer, 0.1% w/v SDS at a temperature of at least 65°C.

As used herein, an "isolated" or "substantially pure" nucleic acid molecule (e.g. an RNA, DNA or a mixed polymer) is one which is substantially separated from other cellular components which naturally accompany a native sequence or protein, e.g. ribosomes, polymerases and many other genome sequences and proteins. The term embraces a nucleic acid sequence or protein which has been removed from its naturally occurring environment and includes recombinant or cloned DNA isolates and chemically synthesized analogs or analogs biologically synthesized by heterologous systems.

The present invention further provides recombinant nucleic acids including a recombinant construct comprising all or a part of the present gene family. The recombinant construct may be capable of replicating autonomously in a host cell. Alternatively, the recombinant construct may become integrated into the chromosomal DNA. of the host cell. Such a recombinant polynucleotide comprises a polynucleotide of genomic, cDNA, semisynthetic or synthetic origin which, by virtue of its origin or manipulation: (i) is not associated with all or a portion of a polynucleotide with which it is associated in nature; (ii) is linked to a polynucleotide other than that to which it is linked in nature; or (iii) does not occur in nature. Where nucleic acids according to the invention include RNA, reference to the sequence shown should be construed as reference to the RNA equivalent with U substituted for T. A "recombinant construct" includes an expression construct whereby the nucleotide sequence is expressed to form mRNA. The recombinant construct may be RNA or DNA.

Accordingly, recombinant nucleic acids comprising sequences otherwise not naturally occurring are provided by the present invention. Although the wild-type sequence may be employed, it will often be altered, e.g. by deletion, substitution or insertion of one or more nucleotides.

cDNA or genomic libraries of various types may be screened as natural sources of the nucleic acids of the present invention or such nucleic acids may be provided by amplification of sequences resident in genomic DNA or other natural sources, e.g. by PCR. The choice of cDNA libraries normally corresponds to a tissue source which is abundant in mRNA for the desired protein. Phage or plasmid libraries are normally preferred but other types of libraries may be used. Clones of a library are spread onto plates, transferred to a substrate for screening, denatured and probed for the presence of desired sequences.

The nucleic acid molecules of the present invention may be produced by replication in a suitable host cell. Natural or synthetic polynucleotide fragments coding for a desired fragment will be incorporated into recombinant polynucleotide constructs, usually DNA constructs, capable of introduction into and replication in a prokaryotic or eukaryotic cell. Usually the polynucleotide constructs will be suitable for replication in a unicellular host, such as yeast or bacteria, but may also be intended for introduction into (with or without integration within the genome) cultured mammalian or other eukaryotic cell lines. The purification of nucleic acids produced by the methods of the present invention are described, e.g. in Sambrook et al., Molecular Cloning: A Laboratory Manual, 2nd Ed., Cold Spring Harbor Laboratory, Cold Spring Harbor, New York, 1989 or Ausubel et al.,"Current Protocols in Molecular Biology" John Wiley & Sons Inc, 1992.

The polynucleotides of the present invention may also be produced by chemical synthesis, e.g. by the phosphoramidite method described by Beaucage and Carruthers (Tetra Letts 22: 1859-1862, 1981) or the triester method according to Matteucci and Caruthers (J. Am. Chem. Soc. 103: 3185, 1981) and may be performed on commercial, automated oligonucleotide synthesizers. A double-stranded fragment may be obtained from the single-stranded product of chemical synthesis either by synthesizing the complementary strand and annealing the strands together under appropriate conditions or by adding the complementary strand using DNA polymerase with an appropriate primer sequence.

An appropriate promoter and other necessary vector sequences, including selectable markers, will be selected so as to be functional in the host and may include, when appropriate, those naturally associated with the 35-LM gene family. Examples of workable combinations of cell lines and expression vectors are described in Sambrook *et al*., 1989, *supra* or Ausubel *et al*., 1992, *supra.* Many useful vectors are known in the art and may be obtained from such vectors as Stratagene, New England Biolabs, Promega Biotech and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization and others. Vectors and promoters suitable for use in yeast expression are further described in European Patent Publication No. 0 073 675. Appropriate non-native mammalian promoters might include the early and late promoters from SV40 (Fiers et al., Nature 273: 113-120, 1978) or promoters derived from murine molony leukemia virus, mouse tumor virus, avian sarcoma viruses, adenovirus II, bovine papilloma virus or polyoma. The CMV promoter is particularly useful in expressing 35-LM genes or cDNA. Insect promoters may be derived from baculovirus. In addition, the construct may be joined to an amplifiable gene (e.g. DHFR) so that multiple copies of the gene may be made. For appropriate enhancer and other expression control sequences, see also Enhancers and Eukaryotic Gene Expression, Cold Spring Harbor Press, Cold Spring Harbour, New York (1983). See also, e.g. U.S. Patent No. 5,691,198.

The vectors containing the nucleic acids of interest can be transcribed *in vitro* and the resulting RNA introduced into the host cell by well-known methods, e.g. by injection (see Kubo et al., FEBS Lett. 241: 119, 1988), or the vectors can be introduced directly into host cells by methods well known in the art, which vary depending on the type of cellular host, including electroporation; transfection employing calcium chloride, rubidium chloride, calcium phosphate, DEAE-dextran, or other substances; microprojectile bombardment; lipofection; infection (where the vector is an infectious agent, such as a retroviral genome); and other methods. See generally, Sambrook *et al.* (1989) *supra* and Ausubel *et al.* (1992) *supra.* The introduction of the polynucleotides into the host cell by any method known in the art, including, *inter alia*, those described above, will be referred to herein as "transformation". The cells into which have been introduced nucleic acids described above are meant to also include the progeny of such cells.

In one aspect, the vectors of the present invention comprise a nucleic acid molecule selected from the group consisting of:
(a) a sequence provided in SEQ ID NOs:1, 3, 5, 7, 9, 11, 13, 15, 17, 19, 21, 23 and 25;
(b) a complement of a sequence provided in SEQ ID NOs:1, 3, 5, 7, 9, 11, 13, 15, 17, 19, 21, 23 or 25;
(c) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
(d) a sequence which hybridizes to the complement of a sequence provided in SEQ ID NOs:1, 3, 5, 7, 9, 11, 13, 15, 17, 19, 21, 23 or 25, under conditions of low stringency;
(e) a sequence having at least 70% identity after optimal alignment to a sequence provided in SEQ ID NOs:1, 3, 5, 7, 9, 11, 13, 15, 17, 19, 21, 23 or 25;
(f) a derivative of a sequence provided in SEQ ID NOs:1, 3, 5, 7, 9, 11, 13, 15, 17, 19, 21, 23 or 25; and
(g) a homolog of a sequence provided in SEQ ID NOs:1, 3, 5, 7, 9, 11, 13, 15, 17, 19, 21, 23 or 25,
wherein the nucleic acid molecule is operably linked to an expression control sequence.

In a related aspect, the vectors of the present invention comprise a nucleic acid molecule which encodes a polypeptide selected from the group consisting of:
(a) a sequence provided in SEQ ID NOs:1, 3, 5, 7, 9, 11, 13, 15, 17, 19, 21, 23 and 25;
(b) a complement of a sequence provided in SEQ ID NOs:1, 3, 5, 7, 9, 11, 13, 15, 17, 19, 21, 23 or 25;
(c) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
(d) a sequence which hybridizes to the complement of a sequence provided in SEQ ID NOs:1, 3, 5, 7, 9, 11, 13, 15, 17, 19, 21, 23 or 25, under conditions of low stringency;
(e) a sequence having at least 70% identity after optimal alignment to a sequence provided in SEQ ID NOs:1, 3, 5, 7, 9, 11, 13, 15, 17, 19, 21, 23 or 25;
(f) a derivative of a sequence provided in SEQ ID NOs:1, 3, 5, 7, 9, 11, 13, 15, 17, 19, 21, 23 or 25; and
(g) a homolog of a sequence provided in SEQ ID NOs:1, 3, 5, 7, 9, 11, 13, 15, 17, 19, 21, 23 or 25,
wherein the nucleic acid molecule is operably linked to an expression control sequence.

In a preferred aspect, the vectors of the present invention are artificial chromosomes. Artificial chromosome nucleic acid molecules are DNA molecules. In one form, the artificial chromosome DNA molecule is in isolated form. In another form, the artificial chromosome DNA is resident within the cell of the mammalian, avian species or any other higher eukaryote. The term "resident" includes the DNA existing as a self-replicating unit relative to the cell's chromosome as well as being integrated into the cell's chromosome. Generally, the artificial chromosome is in the form of a vector. The vector comprises, therefore, a neocentromere or its centromeric equivalent and having a centromeric chromatin domain. The term "neocentromere" is not intended to exclude a centromere although the neocentromere or centromere of the present invention is substantially devoid of α-satellite or other repeat DNA that normally resides at a centromere. For brevity, reference to a "neocentromere" includes a centromere which substantially contains no α-satellite or other repetitive DNA-based centromeric sequences.

Large quantities of the nucleic acids and polypeptides (see below) of the present invention may be prepared by expressing the *35-LM* nucleic acids or parts thereof in vectors or other expression vehicles in compatible prokaryotic or eukaryotic host cells. The most commonly used prokaryotic hosts are strains of *E*. *coli*, although other prokaryotes, such as *Bacillus subtilis* or *Pseudomonas* may also be used.

Mammalian or other eukaryotic host cells, such as those of yeast, filamentous fungi, plant, insect or amphibian or avian species, may also be useful for production of the proteins of the present invention. Propagation of mammalian cells in culture is *per se* well known. See, Jakoby and Pastan (eds.), Cell Culture. Methods in Enzymology, Vol. 58, Academic Press, Inc., Harcour Brace Jovanovich, New York, 1979. Examples of commonly used mammalian host cell lines are VERO and HeLa cells, Chinese hamster ovary (CHO) cells, and WI38, BHK and COS cell lines. The Jurkat T-cell line is particularly useful in the practice of this aspect of the present invention. An example of a commonly used insect cell line is SF9. However, it will be appreciated by the skilled practitioner that other cell lines may be appropriate, e.g. to provide higher expression, desirable glycosylation patterns or other features.

In a related aspect, the present invention provides for a host cell transformed or transfected with a vector comprising a nucleic acid molecule selected from the group consisting of:
(a) a sequence provided in SEQ ID NOs:1, 3, 5, 7, 9, 11, 13, 15, 17, 19, 21, 23 and 25;
(b) a complement of a sequence provided in SEQ ID NOs:1, 3, 5, 7, 9, 11, 13, 15, 17, 19, 21, 23 or 25;
(c) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
(d) a sequence which hybridizes to the complement of a sequence provided in SEQ ID NOs:1, 3, 5, 7, 9, 11, 13, 15, 17, 19, 21, 23 or 25, under conditions of low stringency;
(e) a sequence having at least 70% identity after optimal alignment to a sequence provided in SEQ ID NOs:1, 3, 5, 7, 9, 11, 13, 15, 17, 19, 21, 23 or 25;
(f) a derivative of a sequence provided in SEQ ID NOs:1, 3, 5, 7, 9, 11, 13, 15, 17, 19, 21, 23 or 25; and
(g) a homolog of a sequence provided in SEQ ID NOs:1, 3, 5, 7, 9, 11, 13, 15, 17, 19, 21, 23 or 25,
wherein the sequence is operably Inked to an expression control sequence.

In a related aspect, the host cells of the present invention are transformed or transfected with a vector containing a polynucleotide of the present invention, wherein the vector is an artifical chromosome. In a preferred aspect, the host cell is transformed or transfected with a vector, wherein the vector is a human artificial chromosome.

Clones are selected by using markers depending on the mode of the vector construction. The marker may be on the same or a different DNA molecule, preferably the same DNA molecule. In prokaryotic hosts, the transformant may be selected, e.g. by resistance to ampicillin, tetracycline or other antibiotics. Production of a particular product based on temperature sensitivity may also serve as an appropriate marker.

Prokaryotic or eukaryotic cells transformed with the polynucleotides of the present invention will be useful not only for the production of the nucleic acids and polypeptides of the present invention but also, for example, in studying the characteristics of a *35-LM* expression product such as a polypeptide, mRNA, intron and exon.

Antisense polynucleotide sequences are useful in modulating the expression of members of the gene family. Polynucleotide vectors, for example, containing all or a part of the present nucleic acid molecule may be placed under the control of a promoter in an antisense orientation and introduced into a cell. Expression of such an antisense construct within a cell will interfere with the target *35-LM* transcription or translation. Furthermore, co-suppression and mechanisms to induce RNAi may also be employed. Such techniques may be useful to selectively inhibit inhibitory 35-LMs in subjects with for example immunosuppression and may also be useful to inhibit triggering 35-LMs in subjects with for example inflammatory or autoimmune conditions. Selective inhibition may involve the use of cell or tissue or cell cycle stage specific promoters to regulate expression of the antisense molecules in certain cell types or tissues, or over particular time periods.

Another embodiment of the present invention contemplates an isolated or recombinant nucleic acid molecule corresponding to a gene family which is located on human chromosome 17q22-24 or the equivalent region in another species and comprising a sequence of nucleotides encoding or complementary to a sequence encoding a polypeptide or a nucleotide sequence capable of hybridizing thereto under low stringency conditions wherein said polypeptide exhibits one or more of the identifying characteristics of hCMRF-35A or hCMRF-35H and wherein said polypeptide is expressed on the surface of defined populations of haematopoietic cells.

In a preferred embodiment, the polypeptide comprises a sequence of amino acids selected from those set forth in SEQ ID NO: 6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:27 or SEQ ID NO:28 or an amino acid sequence having at least 20% similarity to all or part of any one of the listed sequences.

Particularly preferred nucleic acid molecules comprise nucleotide sequences substantially as set forth in SEQ ID NO:5 (h35-L1), SEQ ID NO:7 (h35-L2), SEQ ID NO:9 (h35-L3), SEQ ID NO:11 (h35-L4), SEQ ID NO:13 (h35-L5), SEQ ID NO:15 (m35-a), SEQ ID NO:17 (m35-c), SEQ ID NO:19 (m35-d), SEQ ID NO:21 (m35-f), SEQ ID NO:23 (m35-h), SEQ ID NO:25 (m35-g), or a nucleotide sequence having at least about 15% similarity to all or a part of the sequences or a nucleotide sequence which hybridizes to any of these medium stringency conditions.

The term "polypeptide" refers to a polymer of amino acids and its equivalent and does not refer to a specific length of the product, thus, peptides, oligopeptides and proteins are included within the definition of a polypeptide. This term also does not exclude modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages as well as other modifications known in the art, both naturally and non-naturally occurring. Ordinarily, such polypeptides will be at least about 20% similar to the wild-type members of the 35-LM gene family, preferably in excess of 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100%. In a preferred aspect, the polypeptides of the present invention ar about 70% similar to the wild-type members of the 35'LM gene family. Also included are proteins encoding by DNAs which hybridize under high or low stringency conditions to *35-LM* nucleic acids and closely related polypeptides or proteins retrieved by, for example, antibodies to the 35-LM family member.

The polypeptide molecules may be in isolated and purified form, free or substantially free of material with which it is naturally associated. The polypeptide may, if produced by expression in a prokaryotic cell or produced synthetically, lack native post-translational processing, such as glycosylation. The present invention is also directed to polypeptides which are sequence variants, alleles or derivatives of the 35-LM polypeptides.

In a preferred aspect, the polypeptides of the present invention comprise an amino acid sequence selected from the group consisting of:
(a) a sequence provided in SEQ ID NOs:2, 4, 6, 8, 10, 12, 14, 16, 18, 20, 22, 24, 26, 27 and 28;
(b) a sequence having at least 70% similarity after optimal alignment to an amino acid sequence provided in SEQ ID NOs:2, 4, 6, 8, 10, 12, 14, 16, 18, 20, 22, 24, 26, 27 and 28;
(c) a derivative, homolog, analog, chemical equivalent or mimetic of a sequence provided in SEQ ID NOs:2, 4, 6, 8, 10, 12, 14, 16, 18, 20, 22, 24, 26, 27 and 28;
(d) a sequence encoded by a nucleic acid molecule selected from the group consisting of:
   (i) a sequence provided in SEQ ID NOs:1, 3, 5, 7, 9, 11, 13, 15, 17, 19, 21, 23 and 25;
   (ii) a complement of a sequence provided in SEQ ID NOs:1, 3, 5, 7, 9, 11, 13, 15, 17, 19, 21, 23 or 25;
   (iii) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
   (iv) a sequence which hybridizes to the complement of a sequence provided in SEQ ID NOs:1, 3, 5, 7, 9, 11, 13, 15, 17, 19, 21, 23 or 25, under conditions of low stringency;
   (v) a sequence having at least 70% identity after optimal alignment to a sequence provided in SEQ ID NOs:1, 3, 5, 7, 9, 11, 13, 15, 17, 19, 21, 23 or 25;
   (vi) a derivative of a sequence provided in SEQ ID NOs:1, 3, 5, 7, 9, 11, 13, 15, 17, 19, 21, 23 or 25; and
   (vii) a homolog of a sequence provided in SEQ ID NOs:1, 3, 5, 7, 9, 11, 13, 15, 17, 19, 21, 23 or 25; and
(e) a sequence having at least 70% similarity after optimal alignment to a sequence encoded by a nucleic acid molecule selected from the group consisting of:
   (i) a sequence provided in SEQ ID NOs:1, 3, 5, 7, 9, 11, 13, 15, 17, 19, 21, 23 and 25;
   (ii) a complement of a sequence provided in SEQ ID NOs:1, 3, 5, 7, 9, 11, 13, 15, 17, 19, 21, 23 or 25;
   (iii) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
   (iv) a sequence which hybridizes to the complement of a sequence provided in SEQ ID NOs:1, 3, 5, 7, 9, 11, 13, 15, 17, 19, 21, 23 or 25, under conditions of low stringency;
   (v) a sequence having at least 70% identity after optimal alignment to a sequence provided in SEQ ID NOs:1, 3, 5, 7, 9, 11, 13, 15, 17, 19, 21, 23 or 25;
   (vi) a derivative of a sequence provided in SEQ ID NOs:1, 3, 5, 7, 9, 11, 13, 15, 17, 19, 21, 23 or 25; and
   (vii) a homolog of a sequence provided in SEQ ID NOs:1, 3, 5, 7, 9, 11, 13, 15, 17, 19, 21, 23 or 25.

Substitutional variants typically contain the exchange of one amino acid for another at one or more sites within the protein and may be designed to modulate one or more properties of the polypeptide such as stability against proteolytic cleavage without the loss of other functions or properties. Amino acid substitutions may be made on the basis of similarity in polarity, charge, solubility, hydrophobicity, hydrophilicity and/or the amphipathic nature of the residues involved. Preferred substitutions are ones which are conservative, that is, one amino acid is replaced with one of similar shape and charge. Conservative substitutions are well known in the art and typically include substitutions within the following groups: glycine, alanine; valine, isoleucine, leucine; aspartic acid, glutamic acid; asparagine, glutamine; serine, threonine; lysine, arginine; and tyrosine, phenylalanine.

Certain amino acids may be substituted for other amino acids in a protein structure without appreciable loss of interactive binding capacity with structures such as, for example, epitope-binding regions of antibodies or binding sites on substrate molecules or binding sites on proteins interacting with the 35-LM polypeptide. The interactive capacity and nature of a protein may define that protein's biological functional activity, and certain amino acid substitutions can be made in a protein sequence or its underlying DNA coding sequence and nevertheless obtain a protein with like properties. In making such changes, the hydropathic index of amino acids may be considered. The importance of the hydrophobic amino acid index in conferring interactive biological function on a protein is generally understood in the art (Kyte and Doolittle, J. Mol. Biol. 157: 105-132, 1982). Alternatively, the substitution of like amino acids can be made effectively on the basis of hydrophilicity. The importance of hydrophilicity in conferring interactive biological function of a protein is generally understood in the art (U.S. Patent No. 4,554,101). The use of the hydrophobic index or hydrophilicity in designing polypeptides is further discussed in U.S. Patent No. 5,691,198.

The length of the polypeptide sequences compared for homology will generally be at least about 16 amino acids, usually at least about 20 residues, more usually at least about 24 residues, typically at least about 28 residues and preferably more than about 35 residues. In related aspects, sequences compared for homology will generally be, for example, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100 amino acids.

The present invention further contemplates chemical analogs of a 35-LM polypeptide.

Analogues contemplated herein include but are not limited to modification to side chains, incorporating of unnatural amino acids and/or their derivatives during peptide, polypeptide or protein synthesis and the use of crosslinkers and other methods which impose conformational constraints on the proteinaceous molecule or their analogs.

Examples of side chain modifications contemplated by the present invention include modifications of amino groups such as by reductive alkylation by reaction with an aldehyde followed by reduction with NaBH₄; amidination with methylacetimidate; acetylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation *via* O-acylisourea formation followed by subsequent derivitization, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 3.

**TABLE 3**

| ***Codes for non-conventional amino acids*** | | | |
|---|---|---|---|
| Non-conventional amino acid | Code | Non-conventional amino acid | Code |
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbomyl- | Norb | L-N-methylglutamine | Nmgln |
| carboxylate | | L-N-methylglutamic acid | Nmglu |
| cyclohexylalanine | Chexa | L-Nmethylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmom |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Nom |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmom | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-y-aminobutyrate | Nmgabu |
| N-methylcyclohexylalanine | Nmchexa | D-N-methylmethionine | Dnmmet |
| D-N-methylomithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Mom |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) | Nnbhm | N-(N-(3,3-diphenylpropyl) | Nnbhe |
| carbamylmethyl)glycine | | carbamylmethyl)glycine | |
| 1-carboxy-l-(2,2-diphenyl- | Nmbc | | |
| ethylamino)cyclopropane | | | |

Crosslinkers can be used, for example, to stabilize 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of C_{α} and N_{α}-methylamino acids, introduction of double bonds between C_{α} and C_{β} atoms of amino acids and the formation of cyclic peptides or analogues by introducing covalent bonds such as forming an amide bond between the N and C termini, between two side chains or between a side chain and the N or C terminus.

The term "peptide mimetic" or "mimetic" is intended to refer to a substance which has the essential biological activity of the 35-LM family member polypeptide. A peptide mimetic may be a peptide-containing molecule that mimics elements of protein secondary structure. The underlying rationale behind the use of peptide mimetics is that the peptide backbone of proteins exists chiefly to orient amino acid side chains in such a way as to facilitate molecular interactions such as those of antibody and antigen, enzyme and substrate or scaffolding proteins. A peptide mimetic is designed to permit molecular interactions similar to the natural molecule. A mimetic may not be a peptide at all, but it will retain the essential biological activity of a natural 35-LM polypeptide.

The present invention is particularly useful, therefore, for screening compounds by using one or more 35-LM family member polypeptide or binding fragment thereof in any of a variety of drug screening techniques, such as those described herein and in International Publication No. WO 97/02048.

The 35-LM family member polypeptide or fragment employed in such a test may either be free in solution, affixed to a solid support, or borne on a cell surface. One method of drug screening utilizes eukaryotic or prokaryotic host cells which are stably transformed with recombinant polynucleotides expressing the polypeptide or fragment, preferably in competitive binding assays. Such cells, either in viable or fixed form, can be used for standard binding assays. One may measure, for example, the formation of complexes between a polypeptide or fragment and the agent being tested, or examine the degree to which the formation of a complex between a 35-LM polypeptide or a part thereof and a specific antibody is aided or interfered with by the agent being tested.

Reference to an "immunointeractive molecule" should be understood as a reference to any molecule comprising an antigen binding portion or a derivative thereof. In a preferred aspect, the immunointeractive molecules of the present invention are antibodies. Antobodies contemplated by the present invention may be polyclonal, monoclonal, humanized or deimmunized antibodies.

Polyclonal antibodies may conveniently be used, however, the use of monoclonal antibodies in an immunoassay is particularly preferred because of the ability to produce them in large quantities and the homogeneity of the product. The preparation of hybridoma cell lines for monoclonal antibody production is derived by fusing an immortal cell line and lymphocytes sensitized against the immunogenic preparation (i.e. comprising 35-LM polypeptide) or can be done by techniques which are well known to those who are skilled in the art. (See, for example, Douillard and Hoffman, Basic Facts about Hybridomas, in Compendium of Immunology Vol. II, ed. by Schwartz, 1981; Kohler and Milstein, Nature 256: 495-499, 1975; Kohler and Milstein, European Journal of Immunology 6: 511-519, 1976). Single chain antibodies or transgenic mice expressing humanized antibodies or other recognition proteins may also be used. Useful proteins in this regard include diabodies, peptide mimetics and antibody fragments such as scFv fragments and Fab fragments.

Monoclonal antibodies which bind specifically to members of the 35-LM family provide a convenient method for detecting and targeting the cells which express one or more 35-LM. For detecting one or more cells expressing particular 35-LMs either alone or in conjunction with other cell surface molecules, an large number of assays are available. For example, populations of cells may be routinely assessed for their 35-LM polypeptide cell surface markers using identifiable polypeptide specific binding partners such as primary antibodies to cell surface markers and secondary antibodies labeled with detectable markers. Antibodies may further differentiate between allelic or altered forms of 35-LM polypeptides. The presence of members of the 35-LM members may be accomplished in a number of ways such as by Western blotting and ELISA procedures. A wide range of immunoassay techniques are available as can be seen by reference to U.S. Patent Nos. 4,016,043, 4,424,279 and 4,018,653. These include both single-site and two-site or "sandwich" assays of the non-competitive types, as well as in the traditional competitive binding assays. These assays also include direct binding of a labeled antibody to a target. Monoclonal antibodies may be used as agonists or antagonists of 35-LM polypeptide activity. They may also be formulated as a composition suitable for administration to an individual in a method of treatment or prophylaxis.

The antibodies of the present invention are useful in a range of other methodologies including flow cytometry, which typically detects optical parameters. For example, a flow cytometer may be used to determine forward scatter (which is a measure of size of a carrier), side scatter (which is sensitive to refractive index and size of a particle [see Shapiro, "Practical flow cytometry", 3rd ed. Brisbane, Wiley-Liss, 1995]) and fluorescent emission.

In one aspect, the present invention provides a method for detecting a target cell which produces a member of the 35-LM family of proteins, comprising the steps of:
(a) obtaining a sample comprising cells;
(b) contacting said sample with an molecule that binds to a member of the 35-LM family of proteins; and
(c) detecting the presence of a target cell conjugated to said molecule specific for a member of the 35-LM family of proteins.

In a preffered aspect, the 35-LM line molecule detected is selected from the group consisting of 35-L1, 35-L2, 35-L3, 35-L4 and/or 35-L5.

Further contemplated by the present invention are methods for assessing a disease or condition, including the ability for a subject to mount an immune response, wherein the method comprises determining the level or pattern of expression of the nucleic acid molecule selected from the group consisting of:
(a) a sequence provided in SEQ ID NOs:1, 3, 5, 7, 9, 11, 13, 15, 17, 19, 21, 23 and 25;
(b) a complement of a sequence provided in SEQ ID NOs:1, 3, 5, 7, 9, 11, 13, 15, 17, 19, 21, 23 or 25;
(c) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
(d) a sequence which hybridizes to the complement of a sequence provided in SEQ ID NOs:1, 3, 5, 7, 9, 11, 13, 15, 17, 19, 21, 23 or 25, under conditions of low stringency;
(e) a sequence having at least 70% identity after optimal alignment to a sequence provided in SEQ ID NOs:1, 3, 5, 7, 9, 11, 13, 15, 17, 19, 21, 23 or 25;
(f) a derivative of a sequence provided in SEQ ID NOs:1, 3, 5, 7, 9, 11, 13, 15, 17, 19, 21, 23 or 25; and
(g) a homolog of a sequence provided in SEQ ID NOs:1, 3, 5, 7, 9, 11, 13, 15, 17, 19, 21, 23 or 25,
wherein the pattern of presence or absence of expression correlates with a disease condition, a propensity for developing a disease condition and/or an ability for a subject to maintain an immune response.

In a related aspect, the present invention provides a method for assessing a disease or condition including the ability for a subject to mount an immune response, said method comprising determining the level or pattern of the protein, wherein the polypeptide is selected from the group consisting of:
(a) a sequence provided in SEQ ID NOs:2, 4, 6, 8, 10, 12, 14, 16, 18, 20, 22, 24, 26, 27 and 28;
(b) a sequence having at least 70% similarity after optimal alignment to an amino acid sequence provided in SEQ ID NOs:2, 4, 6, 8, 10, 12, 14, 16, 18, 20, 22, 24, 26, 27 and 28;
(c) a derivative, homolog, analog, chemical equivalent or mimetic of a sequence provided in SEQ ID NOs:2, 4, 6, 8, 10, 12, 14, 16, 18, 20, 22, 24, 26, 27 and 28;
(d) a sequence encoded by a nucleic acid molecule selected from the group consisting of:
   (i) a sequence provided in SEQ ID NOs:1, 3, 5, 7, 9, 11, 13, 15, 17, 19, 21, 23 and 25;
   (ii) a complement of a sequence provided in SEQ ID NOs:1, 3, 5, 7, 9, 11, 13, 15, 17, 19, 21, 23 or 25;
   (iii) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
   (iv) a sequence which hybridizes to the complement of a sequence provided in SEQ ID NOs:1, 3, 5, 7, 9, 11, 13, 15, 17, 19, 21, 23 or 25, under conditions of low stringency;
   (v) a sequence having at least 70% identity after optimal alignment to a sequence provided in SEQ ID NOs:1, 3, 5, 7, 9, 11, 13, 15, 17, 19, 21, 23 or 25;
   (vi) a derivative of a sequence provided in SEQ ID NOs:1, 3, 5, 7, 9, 11, 13, 15, 17, 19, 21, 23 or 25; and
   (vii) a homolog of a sequence provided in SEQ ID NOs:1, 3, 5, 7, 9, 11, 13, 15, 17, 19, 21, 23 or 25; and
(e) a sequence having at least 70% similarity after optimal alignment to a sequence encoded by a nucleic acid molecule selected from the group consisting of:
   (i) a sequence provided in SEQ ID NOs:1, 3, 5, 7, 9, 11, 13, 15, 17, 19, 21, 23 and 25;
   (ii) a complement of a sequence provided in SEQ ID NOs:1, 3, 5, 7, 9, 11, 13, 15, 17, 19, 21, 23 or 25;
   (iii) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
   (iv) a sequence which hybridizes to the complement of a sequence provided in SEQ ID NOs:1, 3, 5, 7, 9, 11, 13, 15, 17, 19, 21, 23 or 25, under conditions of low stringency;
   (v) a sequence having at least 70% identity after optimal alignment to a sequence provided in SEQ ID NOs:1, 3, 5, 7, 9, 11, 13, 15, 17, 19, 21, 23 or 25;
   (vi) a derivative of a sequence provided in SEQ ID NOs:1, 3, 5, 7, 9, 11, 13, 15, 17, 19, 21, 23 or 25; and
   (vii) a homolog of a sequence provided in SEQ ID NOs:1, 3, 5, 7, 9, 11, 13, 15, 17, 19, 21, 23 or 25,
wherein the pattern of presence or absence or level of said protein correlates with a disease condition, a propensity for developing a disease condition and/or an ability for a subject to maintain an immune response.

As is known in the art, flow cytometry is a high throughput technique which involves rapidly analyzing the physical and chemical characteristics of cells or other particles as they pass through the path of one or more laser beams while suspended in a fluid stream. As each cell or particle intercepts the laser beam, the scattered light and fluorescent light emitted by each cell or particle is detected and recorded using any suitable tracking algorithm.

A modern flow cytometer is able to perform these tasks up to 100,000 cells/particles s⁻¹. Through the use of an optical array of filters and dichroic mirrors, different wavelengths of fluorescent light can be separated and detected simultaneously. In addition, a number of lasers with different excitation wavelengths may be used. Hence, a variety of fluorophores can be used to target and examine, for example, intra- and extra-cellular properties of individual cells. The scattered light measurements can also classify an individual carrier's size, shape, granularity and/or complexity and, hence, belonging to a particular population of interest (Shapiro, 1995, *supra*).

Suitable flow cytometers which may be used in the methods of the present invention include those which measure five to nine optical parameters (see Table 4) using a single excitation laser, commonly an argon ion air-cooled laser operating at 15 mW on its 488 nm spectral line. More advanced flow cytometers are capable of using multiple excitation lasers such as a HeNe laser (633 nm) or a HeCd laser (325 nm) in addition to the argon ion laser (488 or 514 nm). Optical parameters, corresponding to different optically detectable/quantifiable attributes, for a carrier, may be measured by a flow cytometer to provide a matrix of qualitative and/or quantitative information, providing a code (or addressability in a multi-dimensional space) for the carrier.

**TABLE 4**

| ***Exemplary optical parameters which may be measured by a flow cytometer.*** | | | |
|---|---|---|---|
| **Parameter** | **Acronym** | **Detection angle form incident laser beam** | **Wavelength (nm)** |
| Forward scattered light | FS | 2-5° | 488^{*} |
| Side scattered light | SS | 90° | 488^{*} |
| "Green" fluorescence | FL1 | 90° | 510-540^{†} |
| "Yellow" fluorescence | FL2 | 90° | 560-580^{†} |
| "Red" fluorescence | FL3 | 90° | >650^{#} |

| | | | |
|---|---|---|---|
| ^{*} using a 488 nm excitation laser ^{†} width of bandpass filter ^{#} longpass filter | | | |

For example, Biggs el al. (Cytometry 36: 36-45, 1999) have constructed an 11-parameter flow cytometer using three excitation lasers and have demonstrated the use of nine distinguishable fluorophores in addition to forward and side scatter measurements for purposes of immunophenotyping (i.e. classifying) cells. The maximum number of parameters commercially available currently is 17: forward scatter, side scatter and three excitation lasers each with five fluorescence detectors. Whether all of the parameters can be adequately used depends heavily on the extinction coefficients, quantum yields and amount of spectral overlap between all fluorophores (Malemed et al., "Flow cytometry and sorting", 2nd Ed., New York, Wiley-Liss, 1990). However, it will be understood that the present invention is not restricted to any particular flow cytometer or any particular set of parameters. In this regard, the invention also contemplates use in place of a conventional flow cytometer, a microfabricated flow cytometer as, for example, disclosed by Fu et al. (Nature Biotechnology 17: 1109-1111, 1999).

A flow cytometer with this capacity to sort is known as a "fluorescence-activated cell sorter" (FACS). Accordingly, the step of sorting in the present method of obtaining a population of detectably unique carriers may be effected by flow cytometric techniques such as by fluorescence activated cell sorting (FACS) although with respect to the present invention, FACS is more accurately "fluorescence activated carrier or solid support sorting" (see, for example, "Methods in Cell Biology" Vol. 33, Darzynkiewica, Z. and Crissman, H.A., eds., Academic Press) and Dangl and Herzenberg, J. Immunol. Methods 52: 1-14, 1982.

The present invention further relates to modified antibodies. Modified antibodies of particular interest are single chain fragments carrying the variable (V) region of an antibody. This is called an scFv antibody fragment. scFv antibody fragments are derived from Fragment antigen binding (Fab) portions of an antibody and comprise only the V region of a heavy chain linked by a stretch of synthetic peptide to a V region light chain.

In a particularly preferred embodiment, antibodies may also be used to purge target cells, either alone or in conjunction with other immune or cytotoxic molecules.

The present invention further provides a method of treating a disease or disorder in a subject by administering to the subject an antibody which specifically recognizes and targets cells affected by the disease or disorder contemplated for treatment by the present invention. The antibody may be evaluated for its ability act directly on cells to bring out the desired effect and/or it may be evaluated for its suitability for use in a conjugated form such as to an immunotoxin. The antibody may be evaluated for its potential usefulness in a therapeutic product to treat a disorder or disease state in a subject, preferably a human, or it may be evaluated for its potential usefulness in a therapeutic product to enhance cell function or confer a beneficial effect on a subject, preferably a human.

The therapeutic product may be a therapeutic antibody containing an antibody or antibody fragment and if needed, carriers, buffers, excipients and the like. Alternately, a therapeutic product may contain an antibody or antibody fragment conjugated to at least one bioactive substance such as a cytotoxin or a stimulant, and if needed, carriers, buffers, excipients and the like. The term "immunotoxin" refers to a therapeutic product containing an antibody conjugated to at least one cytotoxin, where the antibody and cytotoxin(s) may be conjugated or combined by any suitable means, with or without the use of cross-linking agents. An immunotoxin may be used to deliver a toxin to a target cell, in order to destroy or inhibit the target cell. A therapeutic product containing an antibody conjugated to or otherwise combined with a stimulant may be used to stimulate or enhance the functioning of a target cell.

Antibodies are regarded as an important resource for developing effective therapeutic products because of their combination of variability and specificity, i.e., antibodies can be elicited against a wide variety of target antigens and antibodies recognize a single epitope on the target antigen. This specificity is best used against a target antigen that appears to be limited to a specific disease condition, such as a surface antigen found only on cancer cells, or a surface antigen specific to a disease-causing organism.

Antibodies can function in therapeutic products through various mechanisms. In the simplest model, antibody binding to a target antigen on the surface of a cell triggers destruction, malfunctioning, or neutralization of the cell. Antibody binding may trigger cell destruction through apoptosis, necrosis, or by eliciting other cells such as macrophages to destroy and remove the cell. Antibodies may cause malfunctioning of a diseased cell, in particular a cell which expresses or has on it surface a CMRF-35 like molecule, and preferably 35-L1, 35-L2, 35-L3, 35-L4 and/or 35-L5, by interfering with normal processes. For example, antibodies may bind to and inhibit receptors or kinases which are expressed only in cancer cells, or which are overexpressed in certain diseased cells, such as AML cells. Antibodies may also have a neutralizing effect in which they bind to toxic antigens or antigens involved in various essential cell processes such as transcription or signal transduction, and block the action of these antigens. Therapeutic antibodies may induce effector mechanisms such as antibody-dependent cellular cytotoxicity (ADCC) and complement-dependent cytolysis.

In a different model, antibodies are conjugated to a cytotoxin to produce a therapeutic product known as an immunotoxin. This approach utilizes the specificity and affinity of antibodies to deliver cytotoxic agents to a target cell in an approach sometimes known as the "magic bullet". Antibodies, typically a tumor-directed antibody or antibody fragment, are conjugated with a cytotoxic agent or toxic moiety active against the target cell. The antibody acts as a targeting agent to find and bind to a cell bearing the target antigen, thereby delivering the toxin which selectively kills the cell carrying the target antigen. If necessary, crosslinkers can be chosen which endow immunotoxins with high in vivo stability.

In a preferred aspect, the antibodies of the present invention either alone or conjugated to an immunotoxin are immunoreactive against CMRF-35-like molecules. In a particularly preferred aspect, the antibodies are immunoreactive against 35-L1, 35-L2, 35-L3, 35-L4 and/or 35-L5.

35-LM expression and variation may also be assessed at the nucleic acid level. For example RT-PCR based methods may be employed to monitor expression of nucleic acid molecules in different cell types and tissues. Nucleic acid sequence variation may be detected by direct DNA sequencing, either manual sequencing or automated fluorescent sequencing, can detect sequence variation. Another approach is the single-stranded conformation polymorphism assay (SSCP) [Orita et al., Proc. Nat. Acad Sci. USA 86: 2776-2770, 1989]. This method can be optimized to detect most DNA sequence variation. The increased throughput possible with SSCP makes it an attractive, viable alternative to direct sequencing for mutation detection on a research basis. The fragments which have shifted mobility on SSCP gels are then sequenced to determine the exact nature of the DNA sequence variation. Other approaches based on the detection of mismatches between the two complementary DNA strands include clamped denaturing gel electrophoresis (CDGE) [Sheffield el al., Am. J. Hum. Genet. 49: 699-706, 1991], heteroduplex analysis (HA) [White et al., Genomics 12: 301-306, 1992] and chemical mismatch cleavage (CMC) [Grompe et al., Proc. Natl. Acad. Sci. USA 86: 5855-5892, 1989]. Other methods which might detect mutations in regulatory regions or which might comprise large deletions, duplications or insertions include the protein truncation assay or the asymmetric assay. A review of methods of detecting DNA sequence variation can be found in Grompe [Nature Genetics 5: 111-117, 1993]. Once a mutation is known, an allele specific detection approach such as allele specific oligonucleotide (ASO) hybridization can be utilized to rapidly screen large numbers of other samples for that same mutation. Such a technique can utilize probes which are labeled with gold nanoparticles to yield a visual color result [Elghanian et al., Science 277: 1078-1081, 1997]. Techniques are available to screen RNA products or proteinaceous products.

Preferably, the polypeptides encoded by the present nucleic acid molecules are expressed on the surface of defined populations of hematopoietic cells. Cells of leukocyte lineages are contemplated, including, for example, monocytes, dendritic cells, NK cells, granulocytes, T-lymphocytes, B-lymphocytes, monocyte derived dendritic cells and precursors thereof.

The phrase, "differentially expressed" is a broad reference to expression of mRNA or a polypeptide in a particular cell type, organ or tissue, stage of development, differentiation cell cycle, or, wherein expression is varied as a result of age, infection, immune or other status or an individual.

The present invention provides methods of screening for agents which interact with the 35-LM nucleic acid molecules or polypeptides of the present invention. Competitive binding assays are preferred. Conveniently, high throughput screening of test peptides is used to identify peptides with suitable affinity and selectivity. Purified 35-LM polypeptide may be immobilized or cells or membranes expressing 35-LM polypeptide may be employed.

Following identification of antibodies or natural or artificial agonists and antagonists including scFv fragments, one or more substances may be manufactured or formulated as a composition suitable for administration to individuals in a method of treatment or prophylaxis.

In another aspect, the present invention provides methods for detecting the presence of a disease condition in a subject, comprising the steps of:
(a) obtaining a biological sample from said subject;
(b) contacting said biological sample with an molecule that binds to a nucleic acid molecule of Claim 1 or a polypeptide Claim 6;
(c) detecting in said biological sample the presence of binding of said molecule; and
(d) comparing the presence of bound molecule with a pre-determined cut-off value to make a determination as to the presence or absence of a disease or condition in said subject.

In a preferred aspect, the moecule used in the methods of the present invention is an immunointeractive molecule. In a particularly preferred aspect, the immuninteractive molecule is an antibody.

In another aspect, the present invention relates to a method of diagnosing or treating a subject suffering from a genetic disease or condition including, without being limited to, A-Beta-Lipoproteinemia, A-V, A Beta-2-Microglobulin Amyloidosis, A-T, A1AD, A1AT, Aagenaes, Aarskog syndrome, Aarskog-Scott Syndrome, Aase-smith syndrome, Aase Syndrome, AAT, Abderhalden-Kaufmann-Lignac Syndrome, Abdominal Muscle Deficiency Syndrome, Abdominal Wall Defect, Abdominal Epilepsy, Abdominal Migraine, Abductor Spasmodic Dysphonia, Abductor Spastic Dysphonia, Abercrombie Syndrome, blepharon-Macrostomia Syndrome, ABS, Absence of HPRT, Absence of Corpus Callosum Schinzel Typ, Absence Defect of Limbs Scalp and Skull, Absence of Menstruation Primar, Absence of HGPRT, Absorptive Hyperoxaluriaor Enteric, Abt-Letterer-Siwe Disease, ACADL, ACADM Deficiency, ACADM, ACADS, Acanthocytosis-Neurologic Disorder, Acanthocytosis, Acantholysis Bullosa, Acanthosis Nigricans, Acanthosis Bullosa, Acanthosis Nigricans With Insulin Resistance Type A, Acanthosis Nigricans With Insulin Resistance Type B, Acanthotic Nevus, Acatalasemia, Acatalasia, ACC, Accessory Atrioventricular Pathways, Acephaly, ACF with Cardiac Defects, Achalasia, Achard-Thiers Syndrome, ACHARD (Marfan variant), Achard's syndrome, Acholuric Jaundice, Achondrogenesis, Achondrogenesis Type IV, Achondrogenesis Type III, Achondroplasia, Achondroplasia Tarda, Achondroplastic Dwarfism, Achoo Syndrome, Achromat, Achromatope, Achromatopic, Achromatopsia, Achromic Nevi, Acid Ceramidase Deficiency, Acid Maltase Deficiency, Acid Beta-glucosidase Deficiency, Acidemia Methylmalonic, Acidemia Propionic, Acidemia with Episodic Ataxia and Weakness, Acidosis, Aclasis Tarsoepiphyseal, ACM, Acoustic Neurilemoma, Acoustic Neuroma, ACPS with Leg Hypoplasia, ACPS II, ACPS IV, ACPS III, Acquired Aphasia with Convulsive Disorder, Acquired Brown Syndrome, Acquired Epileptic Aphasia, Acquired Factor XIII Deficiency, Acquired Form of ACC (caused by infection while still in womb), Acquired Hyperoxaluria, Acquired Hypogammaglobulinemia, Acquired Immunodeficiency Syndrome (AIDS), Acquired Iron Overload, Acquired Lipodystrophy, Acquired Partial Lipodystrophy, Acquired Wandering Spleen, ACR, Acral Dysostosis with Facial and Genital Abnormalities, Acro Renal, Acrocallosal Syndrome Schinzel Type, Acrocephalosyndactyly, Acrocephalosyndactyly Type I, Acrocephalosyndactyly Type I Subtype I, Acrocephalopolysyndactyly Type II, Acrocephalopolysyndactyly Type III, Acrocephalopolysyndactyly Type IV, Acrocephalosyndactyly V (ACS5 or ACS V) Subtype I, Acrocephaly Skull Asymmetry and Mild Syndactyly, Acrocephaly, Acrochondrohyperplasia, Acrodermatitis Enteropathica, Acrodysostosis, Acrodystrophic Neuropathy, Acrodystrophic Neuropathy, Acrofacial Dysostosis Nager Type, Acrofacial Dysostosis Nager Type, Acrofacial Dysostosis Postaxial Type, Acrofacial Dysostosis Type Genee-Wiedep, Acrogeria Familial, Acromegaly, Acromelalgia Hereditary, Acromesomelic Dysplasia, Acromesomelic Dwarfism, Acromicric Skeletal Dysplasia, Acromicric Dysplasia, Acroosteolysis with Osteoporosis and Changes in Skull and Mandible, Acroosteolysis, Acroparesthesia, ACS 1, ACS Type II, ACS Type III, ACS, ACS3, ACTH Deficiency, Action Myoclonus, Acute Brachial Neuritis Syndrome, Acute Brachial Radiculitis Syndrome, Acute Cerebral Gaucher Disease, Acute Cholangitis, Acute Disseminated Encephalomyeloradiculopathy, Acute Disseminated Histiocytosis-X, Acute Hemorrhagic Polioencephalitis, Acute Idiopathic Polyneuritis, Acute Immune-Mediation Polyneuritis, Acute Infantile Pelizaeus-Merzbacher Brain Sclerosis, Acute Intermittant Porphyria, Acute Porphyrias, Acute Sarcoidosis, Acute Shoulder Neuritis, Acute Toxic Epidermolysis, Acyl-CoA Dehydrogenase Deficiency Long-Chain, Acyl-CoA Dehydrogenase Deficiency Short-Chain, Acyl-CoA Dihydroxyacetone Acyltransferase, Acyl-coenzyme A Oxidase Deficiency, ADA, ADA Deficiency, Adam Complex, Adamantiades-Behcet's Syndrome, Adamantinoma, Adams Oliver Syndrome, Adaptive Colitis, ADD combined type, ADD, Addison Disease with Cerebral Sclerosis, Addison's Anemia, Addison's Disease, Addison-Biermer Anemia, Addison-Schilder Disease, Addisonian Pernicious Anemia, Addisonian Pernicious Anemia, Adducted Thumbs-Mental Retardation, Adductor Spasmodic Dysphonia, Adductor Spastic Dysphonia, Adenoma Associated Virilism of Older Women, Adenomatosis of the Colon and Rectum, Adenomatous polyposis of the Colon, Adenomatous Polyposis Familial, Adenosine Deaminase Deficiency, Adenylosuccinase deficiency, ADHD predominantly hyperactive-impulsive type, ADHD predominantly inattentive type, ADHD, Adhesive Arachnoiditis, Adie Syndrome, Adie's Syndrome, Adie's Tonic Pupil, Adie's Pupil, Adipogenital Retinitis Pigmentosa Polydactyly, Adipogenital-Retinitis Pigmentosa Syndrome, Adiposa Dolorosa, Adiposis Dolorosa, Adiposogenital Dystrophy, Adolescent Cystinosis, ADPKD, Adrenal Cortex Adenoma, Adrenal Disease, Adrenal Hyperfunction resulting from Pituitary ACTH Excess, Adrenal Hypoplasia, Adrenal Insufficiency, Adrenal Neoplasm, Adrenal Virilism, Adreno-Retinitis Pigmentosa-Polydactyly Syndrome, Adrenocortical Insufficiency, Adrenocortical Hypofunction, Adrenocorticotropic Hormone Deficiency Isolated, Adrenogenital Syndrome, Adrenoleukodystrophy, Adrenomyeloneuropathy, Adreno-Retinitis Pigmentosa-Polydactyly Syndrome, Adult Cystinosis, Adult Dermatomyositis, Adult Hypophosphatasia, Adult Macula Lutea Retinae Degeneration, Adult Onset ALD, Adult-Onset Ceroidosis, Adult Onset Medullary Cystic Disease, Adult Onset Pernicious Anemia, Adult Onset Schindler Disease, Adult-Onset Subacute Necrotizing Encephalomyelopathy, Adult Onset Pernicious Anemia, Adult Polycystic Kidney Disease, Adult Onset Medullary Cystic Disease, Adynlosuccinate Lyase Deficiency, AE, AEC Syndrome, AFD, Afibrinogenemia, African Siderosis, AGA, Aganglionic Megacolon, Age Related Macular Degeneration, Agenesis of Commissura Magna Cerebri, Agenesis of Corpus Callosum, Agenesis of Corpus Callosum-Infantile Spasms-Ocular Anomalies, Agenesis of Corpus Callosum and Chorioretinal Abnormality, Agenesis of Corpus Callosum-Chorioretinitis Abnormality, Aggressive mastocytosis, Agnosis Primary, AGR Triad, AGU, Agyria, Agyria-pachygria-band spectrum, AHC, AHD, AHDS, AHF Deficiency, AHG Deficiency, AHO, Ahumada Del Castillo, Aicardi Syndrome, AIED, AIMP, AIP, AIS, Akinetic Seizure, ALA-D Porphyria, Alactasia, Alagille Syndrome, Aland Island Eye Disease (X-Linked), Alaninuria, Albers-Schonberg Disease, Albinism, Albinismus, Albinoidism, Albright Hereditary Osteodystrophy, Alcaptonuria, Alcohol-Related Birth Defects, Alcoholic Embryopathy, ALD, Aldosterone, Aldosteronism With Normal Blood Pressure, Aldrich Syndrome, Alexander's Disease, Algodystrophy, Algoneurodystrophy, Alkaptonuria, Alkaptonuric Ochronosis, Alkyl DHAP synthase deficiency, Allan-Herndon-Dudley Syndrome, Allan-Herndon Syndrome, Allan-Herndon-Dudley Mental Retardation, Allergic Granulomatous Antitis, Allergic Granulomatous Angiitis of Cronkhite-Canada, Alobar Holoprosencephaly, Alopecia Areata, Alopecia Celsi, Alopecia Cicatrisata, Alopecia Circumscripta, Alopecia-Poliosis-Uveitis-Vitiligo-Deafness-Cutaneous-Uveo-O, Alopecia Seminuniversalis, Alopecia Totalis, Alopecia Universalis, Alpers Disease, Alpers Diffuse Degeneration of Cerebral Gray Matter with Hepatic Cirrhosis, Alpers Progressive Infantile Poliodystrophy, Alpha-1-Antitrypsin Deficiency, Alpha-1 4 Glucosidase Deficiency, Alpha-Galactosidase A Deficiency, Alpha-Galactosidase B Deficiency, Alpha High-Density Lipoprotein Deficieny, Alpha-L-Fucosidase Deficiency Fucosidosis Type 3, Alpha-GalNAc Deficiency Schindler Type, Alphalipoproteinemia, Alpha Mannosidosis, Alpha-N-Acetylgalactosaminidase Deficiency Schindler Type, Alpha-NAGA Deficiency Schindler Type, Alpha-Neuraminidase Deficiency, Alpha-Thalassemia/mental retardation syndorme non-deletion type, Alphalipoproteinemia, Alport Syndrome, ALS, Alstroem's Syndrome, Alstroem, Alstrom Syndrome, Alternating Hemiplegia Syndrome, Alternating Hemiplegia of Childhood, Alzheimer's Disease, Amaurotic Familial Idiocy, Amaurotic Familial Idiocy Adult, Amaurotic Familial Infantile Idiocy, Ambiguous Genitalia, AMC, AMD, Ameloblastoma, Amelogenesis Imperfecta, Amenorrhea-Galactorrhea Nonpuerperal, Amenorrhea-Galactorrhea-FSH Decrease Syndrome, Amenorrhea, Amino Acid Disorders, Aminoaciduria-Osteomalacia-Hyperphosphaturia Syndrome, AMN, Amniocentesis, Amniotic Band Syndrome, Amniotic Band Disruption Complex, Amniotic Band Sequence, Amniotic Rupture Sequence, Amputation Congenital, AMS, Amsterdam Dwarf Syndrome de Lange, Amylo-1 6-Glucosidase Deficiency, Amyloid Arthropathy of Chronic Hemodialysis, Amyloid Corneal Dystrophy, Amyloid Polyneuropathy, Amyloidosis, Amyloidosis of Familial Mediterranean Fever, Amylopectinosis, Amyoplasia Congenita, Amyotrophic Lateral Sclerosis, Amyotrophic Lateral Sclerosis-Polyglucosan Bodies, AN, AN 1, AN 2, Anal Atresia, Anal Membrane, Anal Rectal Malformations, Anal Stenosis, Analine 60 Amyloidosis, Analphalipoproteinemia, Analrectal, Anaplastic Astrocytoma, Andersen Disease, Anderson-Fabry Disease, Andersen Glycogenosis, Anderson-Warburg Syndrome, Andre Syndrome, Andre Syndrome Type II, Androgen Insensitivity, Androgen Insensitivity Syndrome Partial, Androgen Insensitivity Syndrome, Anemia Autoimmune Hemolytic, Anemia Blackfan Diamond, Anemia, Congenital, Triphalangeal Thumb Syndrome, Anemia Hemolytic Cold Antibody, Anemia Hemolytic with PGK Deficiency, Anemia Pernicious, Anencephaly, Angelman Syndrome, Angio-Osteohypertrophy Syndrome, Angiofollicular Lymph Node Hyperplasia, Angiohemophilia, Angiokeratoma Corporis, Angiokeratoma Corporis Diffusum, Angiokeratoma Diffuse, Angiomatosis Retina, Angiomatous Lymphoid, Angioneurotic Edema Hereditary, Anhidrotic Ectodermal Dysplasia, Anhidrotic X-Linked Ectodermal Dysplasias, Aniridia, Aniridia-Ambiguous Genitalia-Mental Retardation, Aniridia Associated with Mental Retardation, Aniridia-Cerebellar Ataxia-Mental Deficiency, Aniridia Partial-Cerebellar Ataxia-Mental Retardation, Aniridia Partial-Cerebellar Ataxia-Oligophrenia, Aniridia Type I, Aniridia Type II, Aniridia-Wilms' Tumor Association, Aniridia-Wilms' Tumor-Gonadoblastoma, Ankyloblepharon-Ectodermal Defects-Cleft Lip/Palate, Ankylosing Spondylitis, Annular groves, Anodontia, Anodontia Vera, Anomalous Trichromasy, Anomalous Dysplasia of Dentin, Coronal Dentin Dysplasia, Anomic Aphasia, Anophthalmia, Anosmia, Anterior Bowing of the Legs with Dwarfism, Anterior Membrane Corneal Dystrophy, Anti-Convulsant Syndrome, Anti-Epstein-Barr Virus Nuclear Antigen (EBNA) Antibody Deficiency, Antibody Deficiency, Antibody Deficiency with near normal Immunoglobulins, Antihemophilic Factor Deficiency, Antihemophilic Globulin Deficiency, Antiphospholipid Syndrome, Antiphospholipid Antibody Syndrome, Antithrombin III Deficiency, Antithrombin III Deficiency Classical (Type I), Antitrypsin Deficiency, Antley-Bixler Syndrome, Antoni's Palsy, Anxietas Tibialis, Aorta Arch Syndrome, Aortic and Mitral Atresia with Hypoplasic Left Heart Syndrome, Aortic Stenosis, Aparoschisis, APC, APECED Syndrome, Apert Syndrome, Aperts, Aphasia, Aplasia Axialis Extracorticales Congenital, Aplasia Cutis Congenita, Aplasia Cutis Congenita with Terminal Transverse Limb Defects, Aplastic Anemia, Aplastic Anemia with Congenital Anomalies, APLS, Apnea, Appalachian Type Amyloidosis, Apple Peel Syndrome, Apraxia, Apraxia Buccofacial, Apraxia Constructional, Apraxia Ideational, Apraxia Ideokinetic, Apraxia Ideomotor, Apraxia Motor, Apraxia Oculomotor, APS, Arachnitis, Arachnodactyly Contractural Beals Type, Arachnodactyly, Arachnoid Cysts, Arachnoiditis Ossificans, Arachnoiditis, Aran-Duchenne, Aran-Duchenne Muscular Atrophy, Aregenerative Anemia, Arginase Deficiency, Argininemia, Arginino Succinase Deficiency, Argininosuccinase Deficiency, Argininosuccinate Lyase Deficiency, Argininosuccinic Acid Lyase-ASL, Argininosuccinic Acid Synthetase Deficiency, Argininosuccinic Aciduria, Argonz-Del Castillo Syndrome, Arhinencephaly, Armenian Syndrome, Arnold-Chiari Malformation, Arnold-Chiari Syndrome, ARPKD, Arrhythmic Myoclonus, Arrhythmogenic Right Ventricular Dysplasia, Arteriohepatic Dysplasia, Arteriovenous Malformation, Arteriovenous Malformation of the Brain, Arteritis Giant Cell, Arthritis, Arthritis Urethritica, Arthro-Dento-Osteodysplasia, Arthro-Ophthalmopathy, Arthrochalasis Multiplex Congenita, Arthrogryposis Multiplex Congenita, Distal, Type IIA, ARVD, Arylsulfatase-B Deficiency, AS, ASA Deficiency, Ascending Paralysis, ASD,Atrioseptal Defects, ASH, Ashermans Syndrome, Ashkenazi Type Amyloidosis, ASL Deficiency, Aspartylglucosaminuria, Asperger's Syndrome, Asperger's Type Autism, Asphyxiating Thoracic Dysplasia, Asplenia Syndrome, ASS Deficiency, Asthma, Astrocytoma Grade I (Benign), Astrocytoma Grade II (Benign), Asymmetric Crying Facies with Cardiac Defects, Asymmetrical septal hypertrophy, Asymptomatic Callosal Agenesis, AT, AT III Deficiency, AT III Variant IA, AT III Variant Ib, AT 3, Ataxia, Ataxia Telangiectasia, Ataxia with Lactic Acidosis Type II, Ataxia Cerebral Palsy, Ataxiadynamia, Ataxiophemia, ATD, Athetoid Cerebral Palsy, Atopic Eczema, Atresia of Esophagus with or without Tracheoesophageal Fistula, Atrial Septal Defects, Atrial Septal Defect Primum, Atrial and Septal and Small Ventricular Septal Defect, Atrial Flutter, Atrial Fibrillation, Atriodigital Dysplasia, Atrioseptal Defects, Atrioventricular Block, Atrioventricular Canal Defect, Atrioventricular Septal Defect, Atrophia Bulborum Hereditaria, Atrophic Beriberi, Atrophy Olivopontocerebellar, Attention Deficit Hyperactivity Disorder, Attentuated Adenomatous Polyposis Coli, Atypical Amyloidosis, Atypical Hyperphenylalaninemia, Auditory Canal Atresia, Auriculotemporal Syndrome, Autism, Autism Asperger's Type, Autism Dementia Ataxia and Loss of Purposeful Hand Use, Autism Infantile Autism, Autoimmune Addison's Disease, Autoimmune Hemolytic Anemia, Autoimmune Hepatitis, Autoimmune-Polyendocrinopathy-Candidias, Autoimmune Polyglandular Disease Type I, Autosomal Dominant Albinism, Autosomal Dominant Compelling Helioophthalmic Outburst Syndrome, Autosomal Dominant Desmin Distal myopathy with Late Onset, Autosomal Dominant EDS, Autosomal Dominant Emery-Dreifuss Muscular Dystrophy, Autosomal Dominant Keratoconus, Autosomal Dominant Pelizaeus-Merzbacher Brain Sclerosis, Autosomal Dominant Polycystic Kidney Disease, Autosomal Dominant Spinocerebellar Degeneration, Autosomal Recessive Agammaglobulinemia, Autosomal Recessive Centronuclear myopathy, Autosomal Recessive Conradi-Hunermann Syndrome, Autosomal Recessive EDS, Autosomal Recessive Emery-Dreifuss Muscular Dystrophy, Autosomal Recessive Forms of Ocular Albinism, Autosomal Recessive Inheritance Agenesis of Corpus Callosum, Autosomal Recessive Keratoconus, Autosomal Recessive Polycystic Kidney Disease, Autosomal Recessive Severe Combined Immunodeficiency, AV, AVM, AVSD, AWTA, Axilla Abscess, Axonal Neuropathy Giant, Azorean Neurologic Disease, B-K Mole Syndrome, Babinski-Froelich Syndrome, BADS, Baillarger's Syndrome, Balkan Disease, Baller-Gerold Syndrome, Ballooning Mitral Valve, Balo Disease Concentric Sclerosis, Baltic Myoclonus Epilepsy, Bannayan-Zonana syndrome (BZS), Bannayan-Riley-Ruvalcaba syndrome, Banti's Disease, Bardet-Biedl Syndrome, Bare Lymphocyte Syndrome, Barlow's syndrome, Barraquer-Simons Disease, Barrett Esophagus, Barrett Ulcer, Barth syndrome, Bartter's Syndrome, Basal Cell Nevus Syndrome, Basedow Disease, Bassen-Kornzweig Syndrome, Batten Disease, Batten-Mayou Syndrome, Batten-Spielmeyer-Vogt's Disease, Batten Turner Syndrome, Batten Turner Type Congenital myopathy, Batten-Vogt Syndrome, BBB Syndrome, BBBG Syndrome, BCKD Deficiency, BD, BDLS, BE, Beals Syndrome, Beals-Hecht Syndrome, Bean Syndrome, BEB, Bechterew Syndrome, Becker Disease, Becker Muscular Dystrophy, Becker Nevus, Beckwith Wiedemann Syndrome, Beckwith-Syndrome, Begnez-Cesar's Syndrome, Behcet's syndrome, Behcet's Disease, Behr 1, Behr 2, Bell's Palsy, Benign Acanthosis Nigricans, Benign Astrocytoma, Benign Cranial Nerve Tumors, Benign Cystinosis, Benign Essential Blepharospasm, Benign Essential Tremor, Benign Familial Hematuria, Benign Focal Amyotrophy, Benign Focal Amyotrophy of ALS, Benign Hydrocephalus, Benign Hypermobility Syndrome, Benign Keratosis Nigricans, Benign Paroxysmal Peritonitis, Benign Recurrent Hematuria, Benign Recurrent Intrahepatic Cholestasis, Benign Spinal Muscular Atrophy with Hypertrophy of the Calves, Benign Symmetrical Lipomatosis, Benign Tumors of the Central Nervous System, Berardinelli-Seip Syndrome, Berger's Disease, Beriberi, Berman Syndrome, Bernard-Horner Syndrome, Bernard-Soulier Syndrome, Besnier Prurigo, Best Disease, Beta-Alanine-Pyruvate Aminotransferase, Beta-Galactosidase Deficiency Morquio Syndrome, Beta-Glucuronidase Deficiency, Beta Oxidation Defects, Beta Thalassemia Major, Beta Thalassemia Minor, Betalipoprotein Deficiency, Bethlem myopathy, Beuren Syndrome, BH4 Deficiency, BH4 Deficiency, Biber-Haab-Dimmer Corneal Dystrophy, Bicuspid Aortic Valve, Biedl-Bardet, Bifid Cranium, Bifunctional Enzyme Deficiency, Bilateral Acoustic Neurofibromatosis, Bilateral Acoustic Neuroma, Bilateral Right-Sidedness Sequence, Bilateral Renal Agenesis, Bilateral Temporal Lobe Disorder, Bilious Attacks, Bilirubin Glucuronosyltransferase Deficiency Type I, Binder Syndrome, Binswanger's Disease, Binswanger's Encephalopathy, Biotinidase deficiency, Bird-Headed Dwarfism Seckel Type, Bitemporal Forceps Marks Syndrome, Biventricular Fibrosis, Bjornstad Syndrome, B-K Mole Syndrome, Black Locks-Albinism-Deafness of Sensoneural Type (BADS), Blackfan-Diamond Anemia, Blennorrheal Idiopathic Arthritis, Blepharophimosis-Ptosis-Epicanthus Inversus Syndrome, Blepharospasm, Blepharospasm Benign Essential, Blepharospasm Oromandibular Dystonia, Blessig Cysts, BLFS, Blindness, Bloch-Siemens Incontinentia Pigmenti Melanoblastosis Cutis Linearis, Bloch-Siemens-Sulzberger Syndrome, Bloch-Sulzberger Syndrome, Bloom Syndrome, Bloom-Torre-Mackacek Syndrome, Blue Rubber Bleb Nevus, Blue Baby, Blue Diaper Syndrome, BMD, BOD, BOFS, Bone Tumor-Epidermoid Cyst-Polyposis, Bonnet-Dechaume-Blanc Syndrome, Bonnevie-Ulrich Syndrome, Book Syndrome, BOR Syndrome, BORJ, Borjeson Syndrome, Borjeson-Forssman-Lehmann Syndrome, Bowen Syndrome, Bowen-Conradi Syndrome, Bowen-Conradi Hutterite, Bowen-Conradi Type Hutterite Syndrome, Bowman's Layer, BPEI, BPES, Brachial Neuritis, Brachial Neuritis Syndrome, Brachial Plexus Neuritis, Brachial-Plexus-Neuropathy, Brachiocephalic Ischemia, Brachmann-de Lange Syndrome, Brachycephaly, Brachymorphic Type Congenital, Bradycardia, Brain Tumors, Brain Tumors Benign, Brain Tumors Malignant, Branched Chain Alpha-Ketoacid Dehydrogenase Deficiency, Branched Chain Ketonuria I, Brancher Deficiency, Branchio-Oculo-Facial Syndrome, Branchio-Oto-Renal Dysplasia, Branchio-Oto-Renal Syndrome, Branchiooculofacial Syndrome, Branchiootic Syndrome, Brandt Syndrome, Brandywine Type Dentinogenesis Imperfecta, Breast Cancer, BRIC Syndrome, Brittle Bone Disease, Broad Beta Disease, Broad Thumb Syndrome, Broad Thumbs and Great Toes Characteristic Facies and Mental Retardation, Broad Thumb-Hallux, Broca's Aphasia, Brocq-Duhring Disease, Bronze Diabetes, Bronze Schilder's Disease, Brown Albinism, Brown Enamel Hereditary, Brown-Sequard Syndrome, Brown Syndrome, BRRS, Brueghel Syndrome, Bruton's Agammaglobulinemia Common, BS, BSS, Buchanan's Syndrome, Budd's Syndrome, Budd-Chiari Syndrome, Buerger-Gruetz Syndrome, Bulbospinal Muscular Atrophy-X-linked, Bulldog Syndrome, Bullosa Hereditaria, Bullous CIE, Bullous Congenital Ichthyosiform Erythroderma, Bullous Ichthyosis, Bullous Pemphigoid, Burkitt's Lymphoma, Burkitt's Lymphoma African type, Burkitt's Lymphoma Non-african type, BWS, Byler's Disease, C Syndrome, C1 Esterase Inhibitor Dysfunction Type II Angioedema, C1-INH, C1 Esterase Inhibitor Deficiency Type I Angioedema, CINH, Cacchi-Ricci Disease, CAD, CADASIL, CAH, Calcaneal Valgus, Calcaneovalgus, Calcium Pyrophosphate Dihydrate Deposits, Callosal Agenesis and Ocular Abnormalities, Calves-Hypertrophy of Spinal Muscular Atrophy, Campomelic Dysplasia, Campomelic Dwarfism, Campomelic Syndrome, Camptodactyly-Cleft Palate-Clubfoot, Camptodactyly-Limited Jaw Excursion, Camptomelic Dwarfism, Camptomelic Syndrome, Camptomelic Syndrome Long-Limb Type, Camurati-Engelmann Disease, Canada-Cronkhite Disease, Canavan disease, Canavan's Disease Included, Canavan's Leukodystrophy, Cancer, Cancer Family Syndrome Lynch Type, Cantrell Syndrome, Cantrell-Haller-Ravich Syndrome, Cantrell Pentalogy, Carbamyl Phosphate Synthetase Deficiency, Carbohydrate Deficient Glycoprotein Syndrome, Carbohydrate-Deficient Glycoprotein Syndrome Type Ia, Carbohydrate-Induced Hyperlipemia, Carbohydrate Intolerance of Glucose Galactose, Carbon Dioxide Acidosis, Carboxylase Deficiency Multiple, Cardiac-Limb Syndrome, Cardio-auditory Syndrome, Cardioauditory Syndrome of Jervell and and Lange-Nielsen, Cardiocutaneous Syndrome, Cardio-facial-cutaneous syndrome, Cardiofacial Syndrome Cayler Type, Cardiomegalia Glycogenica Diffusa, Cardiomyopathic Lentiginosis, Cardio myopathy, Cardio myopathy Associated with Desmin Storage myopathy, Cardio myopathy Due to Desmin Defect, Cardio myopathy-Neutropenia Syndrome, Cardio myopathy-Neutropenia Syndrome Lethal Infantile Cardio myopathy, Cardiopathic Amyloidosis, Cardiospasm, Cardocardiac Syndrome, Carnitine-Acylcarnitine Translocase Deficiency, Camitine Deficiency and Disorders, Carnitine Deficiency Primary, Camitine Deficiency Secondary, Carnitine Deficiency Secondary to MCAD Deficiency, Carnitine Deficiency Syndrome, Carnitine Palmitoyl Transferase I & II (CPT I & II), Camitine Palmitoyltransferase Deficiency, Camitine Palmitoyltransferase Deficiency Type 1, Camitine Palmitoyltransferase Deficiency Type 2 benign classical muscular form included severe infantile form included, Carnitine Transport Defect (Primary Camitine Deficiency), Camosinase Deficiency, Camosinemia, Caroli Disease, Carpenter syndrome, Carpenter's, Cartilage-Hair Hypoplasia, Cartilage-Hair Hypoplasia, Castleman's Disease, Castleman's Disease Hyaline Vascular Type, Castleman's Disease Plasma Cell Type, Castleman Tumor, Cat Eye Syndrome, Cat's Cry Syndrome, Catalayse deficiency, Cataract-Dental Syndrome, Cataract X-Linked with Hutchinsonian Teeth, Catecholamine hormones, Catel-Manzke Syndrome, Catel-Manzke Type Palatodigital Syndrome, Caudal Dysplasia, Caudal Dysplasia Sequence, Caudal Regression Syndrome, Causalgia Syndrome Major, Cavernomas, Cavernous Angioma, Cavernous Hemangioma, Cavernous Lymphangioma, Cavernous Malformations, Cayler Syndrome, Cazenave's Vitiligo, CBGD, CBPS, CCA, CCD, CCD, CCHS, CCM Syndrome, CCMS, CCO, CD, CDGIa, CDG1A, CDGS Type Ia, CDI, CdLS, Celiac Disease, Celiac sprue, Celiac Sprue-Dermatitis, Cellelar Immunodeficiency with Purine Nucleoside Phosphorylase Deficiency, Celsus' Vitiligo, Central Apnea, Central Core Disease, Central Core Disease, Central Diabetes Insipidus, Central Form Neurofibromatosis, Central Hypoventilation, Central Sleep Apnea, Centrifugal Lipodystrophy, Centronuclear myopathy, CEP, Cephalocele, Cephalothoracic Lipodystrophy, Ceramide Trihexosidase Deficiency, Cerebellar Agenesis, Cerebellar Aplasia, Cerebellar Hemiagenesis, Cerebellar Hypoplasia, Cerebellar Vermis Aplasia, Cerebellar Vermis Agenesis-Hypernea-Episodic Eye Moves-Ataxia-Retardation, Cerebellar Syndrome, Cerebellarparenchymal Disorder IV, Cerebellomedullary Malformation Syndrome, Cerebello-Oculocutaneous Telangiectasia, Cerebelloparenchymal Disorder IV Familial, Cerebellopontine Angle Tumor, Cerebral Arachnoiditis, Cerebral Autosomal Dominant Arteriopathy with Subcortical Infarcts and Leukodystrophy, Cerebral Beriberi, Cerebral Diplegia, Cerebral Gigantism, Cerebral Malformations Vascular, Cerebral Palsy, Cerebro-Oculorenal Dystrophy, Cerebro-Oculo-Facio-Skeletal Syndrome, Cerebrocostomandibular syndrome, Cerebrohepatorenal Syndrome, Cerebromacular Degeneration, Cerebromuscular Dystrophy Fukuyama Type, Cerebroocular Dysgenesis, Cerebroocular Dysplasia-Muscular Dystrophy Syndrome, Cerebrooculofacioskeletal Syndrome, Cerebroretinal Arteriovenous Aneurysm, Cerebroside Lipidosis, Cerebrosidosis, Cerebrotendinous Xanthomatosis, Cerebrovascular Ferrocalcinosis, Ceroid-Lipofuscinosis Adult form, Cervical Dystonia, Cervical Dystonia, Cervico-Oculo-Acoustic Syndrome, Cervical Spinal Stenosis, Cervical Vertebral Fusion, CES, CF, CFC syndrome, CFIDS, CFND, CGD, CGF, CGF, Chalasodermia Generalized, Chanarin Dorfman Disease, Chanarin Dorfman Syndrome, Chanarin Dorfman Ichthyosis Syndrome, Chandler's Syndrome, Charcot's Disease, Charcot-Marie-Tooth, Charcot-Marie-Tooth Disease, Charcot-Marie-Tooth Disease Variant, Charcot-Marie-Tooth-Roussy-Levy Disease, CHARGE Association, CHARGE Syndrome, Chaund's Ectodermal Dysplasias, Chediak-Higashi Syndrome, Chediak-Steinbrinck-Higashi Syndrome, Cheilitis Granulomatosa, Cheiloschisis, Chemke Syndrome, Cheney Syndrome, Cherry Red Spot and Myoclonus Syndrome, CHF, CHH, Chiari's Disease, Chiari Malformation I, Chiari Type II (Chiari Malformation II), Chiari I Syndrome, Chiari-Budd Syndrome, Chiari-Frommel Syndrome, Chiari Malformation II, CHILD Syndrome, CHILD Ichthyosis Syndrome, CHILD Syndrome Ichthyosis, Childhood Adrenoleukodystrophy, Childhood Dermatomyositis, Childhood-onset Dystonia, Childhood Cyclic Vomiting, Childhood Giant Axonal Neuropathy, Childhood Hypophasphatasia, Childhood Muscular Dystrophy, CHN, Cholestasis, Cholestasis Hereditary Norwegian Type, Cholestasis Intrahepatic, Cholestasis Neonatal, Cholestasis of Oral Contraceptive Users, Cholestasis with Peripheral Pulmonary Stenosis, Cholestasis of Pregnancy, Cholesterol Desmolase Deficiency, Chondrodysplasia Punctata, Chondrodystrophia Calcificans Congenita, Chondrodystrophia Fetalis, Chondrodystrophic Myotonia, Chondrodystrophy, Chondrodystrophy with Clubfeet, Chondrodystrophy Epiphyseal, Chondrodystrophy Hyperplastic Form, Chondroectodermal Dysplasias, Chondrogenesis Imperfecta, Chondrohystrophia, Chondroosteodystrophy, Choreoacanthocytosis, Chorionic Villi Sampling, Chorioretinal Anomalies, Chorioretinal Anomalies with ACC, Chorireninal Coloboma-Joubert Syndrome, Choroidal Sclerosis, Choroideremia, Chotzen Syndrome, Chotzen Syndrome, Christ-Siemens-Touraine Syndrome, Christ-Siemans-Touraine Syndrome, Christmas Disease, Christmas Tree Syndrome, Chromosome 3 Deletion of Distal 3p, Chromosome 3 Distal 3p Monosomy, Chromosome 3-Distal 3q2 Duplication, Chromosome 3-Distal 3q2 Trisomy, Chromosome 3 Monosomy 3p2, Chromosome 3q Partial Duplication Syndrome, Chromosome 3q, Partial Trisomy Syndrome, Chromosome 3-Trisomy 3q2, Chromosome 4 Deletion 4q31-qter Syndrome, Chromosome 4 Deletion 4q32-qter Syndrome, Chromosome 4 Deletion 4q33-qter Syndrome, Chromosome 4 Long Arm Deletion, Chromosome 4 Long Arm Deletion, Chromosome 4 Monosomy 4q, Chromosome 4-Monosomy 4q, Chromosome 4 Monosomy Distal 4q, Chromosome 4 Partial Deletion 4p, Chromosome 4, Partial Deletion of the Short Arm, Chromosome 4 Partial Monosomy of Distal 4q, Chromosome 4 Partial Monosomy 4p, Chromosome 4 Partial Trisomy 4 (q25-qter), Chromosome 4 Partial Trisomy 4 (q26 or q27-qter), Chromosome 4 Partial Trisomy 4 (q31 or 32-qter), Chromosome 4 Partial Trisomy 4p, Chromosome 4 Partial Trisomies 4q2 and 4q3, Chromosome 4 Partial Trisomy Distal 4, Chromosome 4 Ring, Chromosome 4 4q Terminal Deletion Syndrome, Chromosome 4q- Syndrome, Chromosome 4 Trisomy 4, Chromosome 4 Trisomy 4p, Chromosome 4 XY/47 XXY (Mosiac), Chromosome 5 Monosomy 5p, Chromosome 5, Partial Deletion of the Short Arm Syndrome, Chromosome 5 Trisomy 5p, Chromosome 5 Trisomy 5p Complete (5p11-pter), Chromosome 5 Trisomy 5p Partial (Sp13 or 14-pter), Chromosome 5p-Syndrome, Chromosome 6 Partial Trisomy 6q, Chromosome 6 Ring, Chromosome 6 Trisomy 6q2, Chromosome 7 Monosomy 7p2, Chromosome 7 Partial Deletion of Short Arm (7p2-), Chromosome 7 Terminal 7p Deletion [del (7) (p21-p22)], Chromosome 8 Monosomy 8p2, Chromosome 8 Monosomy 8p21-pter, Chromosome 8 Partial Deletion (short arm), Chromosome 8 Partial Monosomy 8p2, Chromosome 9 Complete Trisomy 9P, Chromosome 9 Partial Deletion of Short Arm, Chromosome 9 Partial Monosomy 9p, Chromosome 9 Partial Monosomy 9p22, Chromosome 9 Partial Monosomy 9p22-pter, Chromosome 9 Partial Trisomy 9P Included, Chromosome 9 Ring, Chromosome 9 Tetrasomy 9p, Chromosome 9 Tetrasomy 9p Mosaicism, Chromosome 9 Trisomy 9p (Multiple Variants), Chromosome 9 Trisomy 9 (pter-p21 to q32) Included, Chromosome 9 Trisomy Mosaic, Chromosome 9 Trisomy Mosaic, Chromosome 10 Distal Trisomy 10q, Chromosome 10 Monosomy, Chromosome 10 Monosomy 10p, Chromosome 10, Partial Deletion (short arm), Choromsome 10, 10p-Partial, Chromosome 10 Partial Trisomy 10q24-qter, Chromosome 10 Trisomy 10q2, Partial Monosomy of Long Arm of Chromosome 11, Chromosome 11 Partial Monosomy 11q, Chromosome 11 Partial Trisomy, Chromosome 11 Partial Trisomy 11q13-qter, Chromosome 11 Partial Trisomy 11q21-qter, Chromosome 11 Partial Trisomy 11q23-qter, Chromosome 11q, Partial Trisomy, Chromosome 12 Isochromosome 12p Mosaic, Chromosome 13 Partial Monosomy 13q, Chromosome 13, Partial Monosomy of the Long Arm, Chromosome 14 Ring, Chromosome 14 Trisomy, Chromosome 15 Distal Trisomy 15q, Chromosome r15, Chromosome 15 Ring, Chromosome 15 Trisomy 15q2, Chromosome 15q, Partial Duplication Syndrome, Chromosome 17 Interstitial Deletion 17p, Chromosome 18 Long Arm Deletion Syndrome, Chromosome 18 Monosomy 18p, Chromosome 18 Monosomy 18Q, Chromosome 18 Ring, Chromosome 18 Tetrasomy 18p, Chromosome 18q- Syndrome, Chromosome 21 Mosaic 21 Syndrome, Chromosome 21 Ring, Chromosome 21 Translocation 21 Syndrome, Chromosome 22 Inverted Duplication (22pter-22q11), Chromosome 22 Partial Trisomy (22pter-22q11), Chromosome 22 Ring, Chromosome 22 Trisomy Mosaic, Chromosome 48 XXYY, Chromosome 48 XXXY, Chromosome r15, Chromosomal Triplication, Chromosome Triplication, Chromosome Triploidy Syndrome, Chromosome X, Chromosome XXY, Chronic Acholuric Jaundice, Chronic Adhesive Arachnoiditis, Chronic Adrenocortical Insufficiency, Chronic Cavernositis, Chronic Congenital Aregenerative Anemia, Chronic Dysphagocytosis, Chronic Familial Granulomatosis, Chronic Familial Icterus, Chronic Fatigue Immune Dysfunction Syndrome (CFIDS), Chronic Granulomatous Disease, Chronic Guillain-Barre Syndrome, Chronic Idiopathic Jaundice, Chronic Idiopathic Polyneuritis (CIP), Chronic Inflammatory Demyelinating Polyneuropathy, Chronic Inflammatory Demyelinating Polyradiculoneuropathy, Chronic Motor Tic, Chronic Mucocutaneous Candidiasis, Chronic Multiple Tics, Chronic Non-Specific Ulcerative Colitis, Chronic Obliterative Cholangitis, Chronic Peptic Ulcer and Esophagitis Syndrome, Chronic Progressive Chorea, Chronic Progressive External Ophthalmoplegia Syndrome, Chronic Progressive External Ophthalmoplegia and myopathy, Chronic Progressive External Ophthalmoplegia with Ragged Red Fibers, Chronic Relapsing Polyneuropathy, Chronic Sarcoidosis, Chronic Spasmodic Dysphonia, Chronic Vomiting in Childhood, CHS, Churg-Strauss Syndrome, Cicatricial Pemphigoid, CIP, Cirrhosis Congenital Pigmentary, Cirrhosis, Cistinuria, Citrullinemia, CJD, Classic Schindler Disease, Classic Type Pfeiffer Syndrome, Classical Maple Syrup Urine Disease, Classical Hemophilia, Classical Form Cockayne Syndrome Type I (Type A), Classical Leigh's Disease, Classical Phenylketonuria, Classical X-Linked Pelizaeus-Merzbacher Brain Sclerosis, CLE, Cleft Lip/Palate Mucous Cysts Lower Lip PP Digital and Genital Anomalies, Cleft Lip-Palate Blepharophimosis Lagophthalmos and Hypertelorism, Cleft Lip/Palate with Abnormal Thumbs and Microcephaly, Cleft palate-joint contractures-dandy walker malformations, Cleft Palate and Cleft Lip, Cleidocranial Dysplasia w/ Micrognathia, Absent Thumbs, & Distal Aphalangia, Cleidocranial Dysostosis, Cleidocranial Dysplasia, Click murmur syndrome, CLN1, Clonic Spasmodic, Cloustons Syndrome, Clubfoot, CMDI, CMM, CMT, CMTC, CMTX, COA Syndrome, Coarctation of the aorta, Coats' Disease, Cobblestone dysplasia, Cochin Jewish Disorder, Cockayne Syndrome, COD-MD Syndrome, COD, Coffin Lowry Syndrome, Coffin Syndrome, Coffin Siris Syndrome, COFS Syndrome, Cogan Corneal Dystrophy, Cogan Reese Syndrome, Cohen Syndrome, Cold Agglutinin Disease, Cold Antibody Disease, Cold Antibody Hemolytic Anemia, Cold Agglutinin Disease, Colitis Ulcerative, Colitis Gravis, Colitis Ulcerative Chronic Non-Specific Ulcerative Colitis, Collodion Baby, Coloboma Heart Defects Atresia of the Choanae Retardation of Growth and Development Genital and Urinary Anomalies and Ear Anomalies, Coloboma, Colonic Neurosis; Color blindness, Colpocephaly, Columnar-Like Esophagus, Combined Cone-Rod Degeneration, Combined Immunodeficiency with Immunoglobulins, Combined Mesoectodermal Dysplasia, Common Variable Hypogammaglobulinemia, Common Variable Immunodeficiency, Common Ventricle, Communicating Hydrocephalus, Complete Absense of Hypoxanthine-Guanine Phosphoribosyltranferase, Complete Atrioventricular Septal Defect, Complement Component 1 Inhibitor Deficiciency, Complement Component C1 Regulatory Component Deficiency, Complete Heart Block, Complex Carbohydrate Intolerance, Complex Regional Pain Syndrome, Complex V ATP Synthase Deficiency, Complex I, Complex I NADH dehydrogenase deficiency, Complex II, Complex II Succinate dehydrogenase deficiency, Complex III, Complex III Ubiquinone-cytochrome c oxidoreductase deficiency, Complex IV, Complex IV Cytochrome c oxidase deficiency, Complex IV Deficiency, Complex V, Cone-Rod Degeneration, Cone-Rod Degeneration Progressive, Cone Dystrophy, Cone-Rod Dystrophy, Confluent Reticular Papillomatosis, Congenital with low PK Kinetics, Congenital Absence of Abdominal Muscles, Congenital Absence of the Thymus and Parathyroids, Congenital Achromia, Congenital Addison's Disease, Congenital Adrenal Hyperplasia, Congenital Afibrinogenemia, Congenital Alveolar Hypoventilation, Congenital Anemia of Newborn, Congenital Bilateral Persylvian Syndrome, Congenital Brown Syndrome, Congenital Cardiovascular Defects, Congenital Central Hypoventilation Syndrome, Congenital Cerebral Palsy, Congenital Cervical Synostosis, Congenital Clasped Thumb with Mental Retardation, Congenital Contractural Arachnodactyly, Congenital Contractures Multiple with Arachnodactyly, Congenital Cyanosis, Congenital Defect of the Skull and Scalp, Congenital Dilatation of Intrahepatic Bile Duct, Congenital Dysmyelinating Neuropathy, Congenital Dysphagocytosis, Congenital Dysplastic Angiectasia, Congenital Erythropoietic Porphyria, Congenital Erythropoietic Porphyria, Congenital Factor XIII Deficiency, Congenital Failure of Autonomic Control of Respiration, Congenital Familial Nonhemolytic Jaundice Type I, Congenital Familial Protracted Diarrhea, Congenital Form Cockayne Syndrome Type II (Type B), Congenital Generalized Fibromatosis, Congenital German Measles, Congenital Giant Axonal Neuropathy, Congenital Heart Block, Congenital Heart Defects, Congenital Hemidysplasia with Ichthyosis Erythroderma and Limb Defects, Congenital Hemolytic Jaundice, Congenital Hemolytic Anemia, Congenital Hepatic Fibrosis, Congenital Hereditary Corneal Dystrophy, Congenital Hereditary Lymphedema, Congenital Hyperchondroplasia, Congenital Hypomyelinating Polyneuropathy, Congenital Hypomyelination Neuropathy, Congenital Hypomyelination, Congenital Hypomyelination Neuropathy, Congenital Hypomyelination (Onion Bulb) Polyneuropathy, Congenital Ichthyosiform Erythroderma, Congenital Keratoconus, Congenital Lactic Acidosis, Congenital Lactose Intolerance, Congenital Lipodystrophy, Congenital Liver Cirrhosis, Congenital Lobar Emphysema, Congenital Localized Emphysema, Congenital Macroglossia, Congenital Medullary Stenosis, Congenital Megacolon, Congenital Melanocytic Nevus, Congenital Mesodermal Dysmorphodystrophy, Congenital Mesodermal Dystrophy, Congenital Microvillus Atrophy, Congenital Multiple Arthrogryposis, Congenital Myotonic Dystrophy, Congenital Neuropathy caused by Hypomyelination, Congenital Pancytopenia, Congenital Pernicious Anemia, Congenital Pernicious Anemia due to Defect of Intrinsic Factor, Congenital Pernicious Anemia due to Defect of Intrinsic Factor, Congenital Pigmentary Cirrhosis, Congenital Porphyria, Congenital Proximal myopathy Associated with Desmin Storage myopathy, Congenital Pulmonary Emphysema, Congenital Pure Red Cell Anemia, Congenital Pure Red Cell Aplasia, Congenital Retinal Blindness, Congenital Retinal Cyst, Congenital Retinitis Pigmentosa, Congenital Retinoschisis, Congenital Rod Disease, Congenital Rubella Syndrome, Congenital Scalp Defects with Distal Limb Reduction Anomalies, Congenital Sensory Neuropathy, Congenital SMA with arthrogryposis, Congenital Spherocytic Anemia, Congenital Spondyloepiphyseal Dysplasia, Congenital Tethered Cervical Spinal Cord Syndrome, Congenital Tyrosinosis, Congenital Varicella Syndrome, Congenital Vascular Cavernous Malformations, Congenital Vascular Veils in the Retina, Congenital Word Blindness, Congenital Wandering Spleen (Pediatric), Congestive Cardio myopathy, Conical Cornea, Conjugated Hyperbilirubinemia, Conjunctivitis, Conjunctivitis Ligneous, Conjunctivo-Urethro-Synovial Syndrome, Conn's Syndrome, Connective Tissue Disease, Conradi Disease, Conradi Hunermann Syndrome, Constitutional Aplastic Anemia, Constitutional Erythroid Hypoplasia, Constitutional Eczema, Constitutional Liver Dysfunction, Constitutional Thrombopathy, Constricting Bands Congenital, Constrictive Pericarditis with Dwarfism, Continuous Muscle Fiber Activity Syndrome, Contractural Arachnodactyly, Contractures of Feet Muscle Atrophy and Oculomotor Apraxia, Convulsions, Cooley's anemia, Copper Transport Disease, Coproporphyria Porphyria Hepatica, Cor Triatriatum, Cor Triatriatum Sinistrum, Cor Triloculare Biatriatum, Cor Biloculare, Cori Disease, Cornea Dystrophy, Corneal Amyloidosis, Corneal Clouding-Cutis Laxa-Mental Retardation, Corneal Dystrophy, Cornelia de Lange Syndrome, Coronal Dentine Dysplasia, Coronary Artery Disease, Coronary Heart Disease, Corpus Callosum Agenesis, Cortical-Basal Ganglionic Degeneration, Corticalis Deformaris, Cortico-Basal Ganglionic Degeneration (CBGD), Corticobasal Degeneration, Corticosterone Methloxidase Deficiency Type I, Corticosterone Methyloxidase Deficiency Type II, Cortisol, Costello Syndrome, Cot Death, COVESDEM Syndrome, COX, COX Deficiency, COX Deficiency French-Canadian Type, COX Deficiency Infantile Mitochondrial myopathy de Toni-Fanconi-Debre included, COX Deficiency Type Benign Infantile Mitochondrial Mypoathy, CP, CPEO, CPEO with myopathy, CPEO with Ragged-Red Fibers, CPPD Familial Form, CPT Deficiency, CPTD, Cranial Arteritis, Cranial Meningoencephalocele, Cranio-Oro-Digital Syndrome, Craniocarpotarsal dystrophy, Craniocele, Craniodigital Syndrome-Mental Retardation Scott Type, Craniofacial Dysostosis, Craniofacial Dysostosis-PD Arteriosus-Hypertrichosis-Hypoplasia of Labia, Craniofrontonasal Dysplasia, Craniometaphyseal Dysplasia, Cranioorodigital Syndrome, Cranioorodigital Syndrome Type II, Craniostenosis Crouzon Type, Craniostenosis, Craniosynostosis-Choanal Atresia-Radial Humeral Synostosis, Craniosynostosis-Hypertrichosis-Facial and Other Anomalies, Craniosynostosis Midfacial Hypoplasia and Foot Abnormalities, Craniosynostosis Primary, Craniosynostosis-Radial Aplasia Syndrome, Craniosynostosis with Radial Defects, Cranium Bifidum, CREST Syndrome, Creutzfeldt Jakob Disease, Cri du Chat Syndrome, Crib Death, Crigler Najjar Syndrome Type I, Crohn's Disease, Cronkhite-Canada Syndrome, Cross Syndrome, Cross' Syndrome, Cross-McKusick-Breen Syndrome, Crouzon, Crouzon Syndrome, Crouzon Craniofacial Dysostosis, Cryoglobulinemia Essential Mixed, Cryptophthalmos-Syndactyly Syndrome, Cryptorchidism-Dwarfism-Subnormal Mentality, Crystalline Corneal Dystrophy of Schnyder, CS, CSD, CSID, CSO, CST Syndrome, Curly Hair-Ankyloblephanon-Nail Dysplasia, Curschmann-Batten-Steinert Syndrome, Curth Macklin Type Ichthyosis Hystric, Curth-Macklin Type, Cushing's, Cushing Syndrome, Cushing's III, Cutaneous Malignant Melanoma Hereditary, Cutaneous Porphyrias, Cutis Laxa, Cutis Laxa-Growth Deficiency Syndrome, Cutis Marmorata Telangiectatica Congenita, CVI, CVID, CVS, Cyclic vomiting syndrome, Cystic Disease of the Renal Medulla, Cystic Disease of the Renal Medulla, Cystic Hygroma, Cystic Fibrosis, Cystic Lymphangioma, Cystine-Lysine-Arginine-Omithinuria, Cystine Storage Disease, Cystinosis, Cystinuria, Cystinuria with Dibasic Aminoaciduria, Cystinuria Type I, Cystinuria Type II, Cystinuria Type III, Cysts of the Renal Medulla Congenital, Cysts of the Renal Medulla Congenital, Cytochrome C Oxidase Deficiency, D.C., Dacryosialoadenopathy, Dacryosialoadenopathia, Dalpro, Dalton, Daltonism, Danbolt-Cross Syndrome, Dancing Eyes-Dancing Feet Syndrome, Dandy-Walker Syndrome, Dandy-Walker Cyst, Dandy-Walker Deformity, Dandy Walker Malformation, Danish Cardiac Type Amyloidosis (Type III), Darier Disease, Davidson's Disease, Davies' Disease, DBA, DBS, DC, DD, De Barsy Syndrome, De Barsy-Moens-Diercks Syndrome, de Lange Syndrome, De Morsier Syndrome, De Santis Cacchione Syndrome, de Toni-Fanconi Syndrome, Deafness Congenital and Functional Heart Disease, Deafness-Dwarfism-Retinal Atrophy, Deafness-Functional Heart Disease, Deafness Onychodystrophy Osteodystrophyand Mental Retardation, Deafness and Pili Torti Bjornstad Type, Deafness Sensorineural with Imperforate Anus and Hypoplastic Thumbs, Debrancher Deficiency, Deciduous Skin, Defect of Enterocyte Intrinsic Factor Receptor, Defect of Enterocyte Intrinsic Factor Receptor, Defect in Natural Killer Lymphocytes, Defect of Renal Reabsorption of Carnitine, Deficiency of Glycoprotein Neuraminidase, Deficiency of Mitochondrial Respiratory Chain Complex IV, Deficiency of Platelet Glycoprotein Ib, Deficiency of Von Willebrand Factor Receptor, Deficiency of Short-Chain Acyl-CoA Dehydrogenase (ACADS, Deformity with Mesomelic Dwarfism, Degenerative Chorea, Degenerative Lumbar Spinal Stenosis, Degos Disease, Degos-Kohlmeier Disease, Degos Syndrome, DEH, Dejerine-Roussy Syndrome, Dejerine Sottas Disease, Deletion 9p Syndrome Partial, Deletion 11q Syndrome Partial, Deletion 13q Syndrome Partial, Delleman-Oorthuys Syndrome, Delleman Syndrome, Dementia with Lobar Atrophy and Neuronal Cytoplasmic Inclusions, Demyelinating Disease, DeMyer Syndrome, Dentin Dysplasia Coronal, Dentin Dysplasia Radicular, Dentin Dysplasia Type I, Dentin Dysplasia Type II, Dentinogenesis Imperfecta Brandywine type, Dentinogenesis Imperfecta Shields Type, Dentinogenesis Imperfecta Shields Type, Dentinogenesis Imperfecta Type III, Dentinogenesis Imperfecta Type III, Dento-Oculo-Osseous Dysplasia, Dento-Oculo-Osseous Dysplasia, Dentooculocutaneous Syndrome, Denys-Drash Syndrome, Depakene, DepakeneTM exposure, Depakote, Depakote Sprinkle, Depigmentation-Gingival Fibromatosis-Microphthalmia, Dercum Disease, Dermatitis Atopic, Dermatitis Exfoliativa, Dermatitis Herpetiformis, Dermatitis Multiformis, Dermatochalasia Generalized, Dermatolysis Generalized, Dermatomegaly, Dermatomyositis sine myositis, Dermatomyositis, Dermatosparaxis, Dermatostomatitis Stevens Johnson Type, Desbuquois Syndrome, Desmin Storage myopathy, Desquamation of Newborn, Deuteranomaly, Deuteranomaly, Developmental Reading Disorder, Developmental Gerstmann Syndrome, Devergie Disease, Devic Disease, Devic Syndrome, Dextrocardia- Bronchiectasis and Sinusitis, Dextrocardia with Situs Inversus, DGS, DGSX Golabi-Rosen Syndrome Included, DH, DHAP alkyl transferase deficiency, DHBS Deficiency, DHOF, DHPR Deficiency, Diabetes Insipidus, Diabetes Insipidus Diabetes Mellitus Optic Atrophy and Deafness, Diabetes Insipidus Neurohypophyseal, Diabetes Insulin Dependent, Diabetes Mellitus, Diabetes Mellitus Addison's Disease Myxedema, Diabetic Acidosis, Diabetic Bearded Woman Syndrome, Diamond-Blackfan Anemia, Diaphragmatic Apnea, Diaphyseal Aclasis, Diastrophic Dwarfism, Diastrophic Dysplasia, Diastrophic Nanism Syndrome, Dicarboxylic Aminoaciduria, Dicarboxylicaciduria Caused by Defect in Beta-Oxidation of Fatty Acids, Dicarboxylicaciduria due to Defect in Beta-Oxidation of Fatty Acids, Dicarboxylicaciduria due to MCADH Deficiency, Dichromasy, Dicker-Opitz, DIDMOAD, Diencephalic Syndrome, Diencephalic Syndrome of Childhood, Diencephalic Syndrome of Emaciation, Dienoyl-CoA Reductase Deficiency, Diffuse Cerebral Degeneration in Infancy, Diffuse Degenerative Cerebral Disease, Diffuse Idiopathic Skeletal Hyperostosis, Diffusum-Glycopeptiduria, DiGeorge Syndrome, DiGeorge Syndrome, Digital-Oro-Cranio Syndrome, Digito-Oto-Palatal Syndrome, Digito-Oto-Palatal Syndrome Type I, Digito-Oto-Palatal Syndrome Type II, Dihydrobiopterin Synthetase Deficiency, Dihydrobiopterin Synthetase Deficiency, Dihydropteridine Reductase Deficiency, Dihydropteridine Reductase Deficiency, Dihydroxyacetonephosphate synthase, Dilated (Congestive) Cardio myopathy, Dimitri Disease, Diplegia of Cerebral Palsy, Diplo-Y Syndrome, Disaccharidase Deficiency, Disaccharide Intolerance I, Discoid Lupus, Discoid Lupus Erythematosus, DISH, Disorder of Cornification, Disorder of Cornification Type I, Disorder of Cornification 4, Disorder of Cornification 6, Disorder of Cornification 8, Disorder of Cornifcation 9 Netherton's Type, Disorder of Cornification 11 Phytanic Acid Type, Disorder of Cornification 12 (Neutral Lipid Storage Type), Disorder of Conification 13, Disorder of Cornification 14, Disorder of Cornification 14 Trichothiodystrophy Type, Disorder of Cornification 15 (Keratitis Deafness Type), Disorder of Cornification 16, Disorder of Cornification 18 Erythrokeratodermia Variabilis Type, Disorder of Cornification 19, Disorder of Cornification 20, Disorder of Cornification 24, Displaced Spleen, Disseminated Lupus Erythematosus, Disseminated Neurodermatitis, Disseminated Sclerosis, Distal 11q Monosomy, Distal 11q- Syndrome, Distal Arthrogryposis Multiplex Congenita Type IIA, Distal Arthrogryposis Multiplex Congenita Type IIA, Distal Arthrogryposis Type IIA, Distal Arthrogryposis Type 2A, Distal Duplication 6q, Distal Duplication 10q, Dup(10q) Syndrome, Distal Duplication 15q, Distal Monosomy 9p, Distal Trisomy 6q, Distal Trisomy 10q Syndrome, Distal Trisomy 11q, Divalproex, DJS, DKC, DLE, DLPIII, DM, DMC Syndrome, DMC Disease, DMD, DNS Hereditary, DOC I, DOC 2, DOC 4, DOC 6 (Harlequin Type), DOC 8 Curth-Macklin Type, DOC 11 Phytanic Acid Type, DOC 12 (Neutral Lipid Storage Type), DOC 13, DOC 14, DOC 14 Trichothiodystrophy Type, DOC 15 (Keratitis Deafness Type), DOC 16, DOC 16 Unilateral Hemidysplasia Type, DOC 18, DOC 19, DOC 20, DOC 24, Dohle's Bodies-Myelopathy, Dolichospondylic Dysplasia, Dolichostenomelia, Dolichostenomelia Syndrome, Dominant Type Kenny-Caffe Syndrome, Dominant Type Myotonia Congenita, Donahue Syndrome, Donath-Landsteiner Hemolytic Anemia, Donath-Landsteiner Syndrome, DOOR Syndrome, DOORS Syndrome, Dopa-responsive Dystonia (DRD), Dorfman Chanarin Syndrome, Dowling-Meara Syndrome, Down Syndrome, DR Syndrome, Drash Syndrome, DRD, Dreifuss-Emery Type Muscular Dystrophy with Contractures, Dressler Syndrome, Drifting Spleen, Drug-induced Acanthosis Nigricans, Drug-induced Lupus Erythematosus, Drug-related Adrenal Insufficiency, Drummond's Syndrome, Dry Beriberi, Dry Eye, DTD, Duane's Retraction Syndrome, Duane Syndrome, Duane Syndrome Type 1A 1B and 1C, Duane Syndrome Type 2A 2B and 2C, Duane Syndrome Type 3A 3B and 3C, Dubin Johnson Syndrome, Dubowitz Syndrome, Duchenne, Duchenne Muscular Dystrophy, Duchenne's Paralysis, Duhring's Disease, Duncan's Disease, Duodenal Atresia, Duodenal Stenosis, Duodenitis, Duplication 4p Syndrome, Duplication 6q Partial, Dupuy's Syndrome, Dupuytren's Contracture, Dutch-Kennedy Syndrome, Dwarfism, Dwarfism Campomelic, Dwarfism Cortical Thickening of the Tubular Bones & Transient Hypocalcemia, Dwarfism Levi's Type, Dwarfism Metatropic, Dwarfism-Onychodysplasia, Dwarfism-Pericarditis, Dwarfism with Renal Atrophy and Deafness, Dwarfism with Rickets, DWM, Dyggve Melchior Clausen Syndrome, Dysautonomia Familial, Dysbetalipoproteinemia Familial, Dyschondrodysplasia with Hemangiomas, Dyschondrosteosis, Dyschromatosis Universalis Hereditaria, Dysencephalia Splanchnocystica, Dyskeratosis Congenita, Dyskeratosis Congenita Autosomal Recessive, Dyskeratosis Congenita Scoggins Type, Dyskeratosis Congenita Syndrome, Dyskeratosis Follicularis Vegetans, Dyslexia, Dysmyelogenic Leukodystrophy, Dysmyelogenic Leukodystrophy-Megalobare, Dysphonia Spastica, Dysplasia Epiphysialis Punctata, Dysplasia Epiphyseal Hemimelica, Dysplasia of Nails With Hypodontia, Dysplasia Cleidocranial, Dysplasia Fibrous, Dysplasia Gigantism SyndromeX-Linked, Dysplasia Osteodental, Dysplastic Nevus Syndrome, Dysplastic Nevus Syndrome, Dysplastic Nevus Type, Dyssynergia Cerebellaris Myoclonica, Dyssynergia Esophagus, Dystonia, Dystonia, Dystopia Canthorum, Dystopia Canthorum, Dystrophia Adiposogenitalis, Dystrophia Endothelialis Cornea, Dystrophia Mesodermalis, Dystrophic Epidermolysis Bullosa, Dystrophy, Asphyxiating Thoracic, Dystrophy Myotonic, E-D Syndrome, Eagle-Barrett Syndrome, Eales Retinopathy, Eales Disease, Ear Anomalies-Contractures-Dysplasia of Bone with Kyphoscoliosis, Ear Patella Short Stature Syndrome, Early Constraint Defects, Early Hypercalcemia Syndrome with Elfin Facie, Early-onset Dystonia, Eaton Lambert Syndrome, EB, Ebstein's anomaly, EBV Susceptibility (EBVS), EBVS, ECD, ECPSG, Ectodermal Dysplasias, Ectodermal Dysplasia Anhidrotic with Cleft Lip and Cleft Palate, Ectodermal Dysplasia-Exocrine Pancreatic Insufficiency, Ectodermal Dysplasia Rapp-Hodgkin type, Ectodermal and Mesodermal Dysplasia Congenital, Ectodermal and Mesodermal Dysplasia with Osseous Involvement, Ectodermosis Erosiva Pluriorificialis, Ectopia Lentis, Ectopia Vesicae, Ectopic ACTH Syndrome, Ectopic Adrenocorticotropic Hormone Syndrome, Ectopic Anus, Ectrodactilia of the Hand, Ectrodactyly, Ectrodactyly-Ectodermal Dysplasia-Clefting Syndrome, Ectrodactyly Ectodermal Dysplasia Cleft Lip/Cleft Palate, Eczema, Eczema-Thrombocytopenia-Immunodeficiency Syndrome, EDA, EDMD, EDS, EDS Arterial-Ecchymotic Type, EDS Arthrochalasia, EDS Classic Severe Form, EDS Dysfibronectinemic, EDS Gravis Type, EDS Hypermobility, EDS Kyphoscoliotic, EDS Kyphoscoliosis, EDS Mitis Type, EDS Ocular-Scoliotic, EDS Progeroid, EDS Periodontosis, EDS Vascular, EEC Syndrome, EFE, EHBA, EHK, Ehlers Danlos Syndrome, Ehlers-Danlos syndrome, Ehlers Danlos IX, Eisenmenger Complex, Eisenmenger's complex, Eisenmenger Disease, Eisenmenger Reaction, Eisenmenger Syndrome, Ekbom Syndrome, Ekman-Lobstein Disease, Ektrodactyly of the Hand, Ektrodactyly of the Hand, EKV, Elastin fiber disorders, Elastorrhexis Generalized, Elastosis Dystrophica Syndrome, Elective Mutism (obsolete), Elective Mutism, Electrocardiogram (ECG or EKG), Electron Transfer Flavoprotein (ETF) Dehydrogenase Deficiency: (GAII & MADD), Electrophysiologic study (EPS), Elephant Nails From Birth, Elephantiasis Congenita Angiomatosa, Hemangiectatic Hypertrophy, Elfin Facies with Hypercalcemia, Ellis-van Creveld Syndrome, Embryoma Kidney, Embryonal Adenomyosarcoma Kidney, Embryonal Carcinosarcoma Kidney, Embryonal Mixed Tumor Kidney, EMC, Emery Dreyfus Muscular Dystrophy, Emery-Dreifuss Muscular Dystrophy, Emery-Dreifuss Syndrome, EMF, EMG Syndrome, Empty Sella Syndrome, Encephalitis Periaxialis Diffusa, Encephalitis Periaxialis Concentrica, Encephalocele, Encephalofacial Angiomatosis, Encephalopathy, Encephalotrigeminal Angiomatosis, Enchondromatosis with Multiple Cavernous Hemangiomas, Endemic Polyneuritis, Endocardial Cushion Defect, Endocardial Cushion Defects, Endocardial Dysplasia, Endocardial Fibroelastosis (EFE), Endogenous Hypertriglyceridemia, Endolymphatic Hydrops, Endometrial Growths, Endometriosis, Endomyocardial Fibrosis, Endothelial Corneal Dystrophy Congenital, Endothelial Epithelial Corneal Dystrophy, Endothelium, Engelmann Disease, Enlarged Tongue, Enterocolitis, Enterocyte Cobalamin Malabsorption, Eosinophia Syndrome, Eosinophilic Cellulitis, Eosinophilic Fasciitis, Eosinophilic Granuloma, Eosinophilic Syndrome, Epidermal Nevus Syndrome, Epidermolysis bullosa, Epidermolysis Bullosa, Epidermolysis Bullosa Acquisita, Epidermolysis Bullosa Hereditaria, Epidermolysis Bullosa Letalias, Epidermolysis Hereditaria Tarda, Epidermolytic Hyperkeratosis, Epidermolytic Hyperkeratosis (Bullous CIE), Epilepsia Procursiva, Epilepsy, Epinephrine, Epiphyseal Changes and High Myopia, Epiphyseal Osteochondroma Benign, Epiphysealis Hemimelica Dysplasia, Episodic-Abnormal Eye Movement, Epithelial Basement Membrane Corneal Dystrophy, Epithelial Corneal Dystrophy of Meesmann Juvenile, Epitheliomatosis Multiplex with Nevus, Epithelium, Epival, EPS, Epstein-Barr Virus-Induced Lymphoproliferative Disease in Males, Erb-Goldflam syndrome, Erdheim Chester Disease, Erythema Multiforme Exudativum, Erythema Polymorphe Stevens Johnson Type, Erythroblastophthisis, Erythroblastosis Fetalis, Erythroblastosis Neonatorum, Erythroblastotic Anemia of Childhood, Erythrocyte Phosphoglycerate Kinase Deficiency, Erythrogenesis Imperfecta, Erythrokeratodermia Progressiva Symmetrica, Erythrokeratodermia Progressiva Symmetrica Ichthyosis, Erythrokeratodermia Variabilis, Erythrokeratodermia Variabilis, Erythrokeratodermia Variabilis Type, Erythrokeratolysis Hiemalis, Erythrokeratolysis Hiemalis, Erythropoietic Porphyrias, Erythropoietic Porphyria, Escobar Syndrome, Esophageal Atresia, Esophageal Aperistalsis, Esophagitis-Peptic Ulcer, Esophagus Atresia and/or Tracheoesophageal Fistula, Essential Familial Hyperlipemia, Essential Fructosuria, Essential Hematuria, Essential Hemorrhagic Thrombocythemia, Essential Mixed Cryoglobulinemia, Essential Moschowitz Disease, Essential Thrombocythemia, Essential Thrombocythemia, Essential Thrombocytopenia, Essential Thrombocytosis, Essential Tremor, Esterase Inhibitor Deficiency, Estren-Dameshek variant of Fanconi Anemia, Estrogen-related Cholestasis, ET, ETF, Ethylmalonic Adipicaciduria, Eulenburg Disease, pc, EVCS, Exaggerated Startle Reaction, Exencephaly, Exogenous Hypertriglyceridemia, Exomphalos-Macroglossia-Gigantism Syndrom, Exophthalmic Goiter, Expanded Rubella Syndrome, Exstrophy of the Bladder, EXT, External Chondromatosis Syndrome, Extrahepatic Biliary Atresia, Extramedullary Plasmacytoma, Exudative Retinitis, Eye Retraction Syndrome, FA1, FAA, Fabry Disease, FAC, FACB, FACD, FACE, FACF, FACG, FACH, Facial Nerve Palsy, Facial Paralysis, Facial Ectodermal Dysplasias, Facial Ectodermal Dysplasia, Facio-Scapulo-Humeral Dystrophy, Facio-Auriculo-Vertebral Spectrum, Facio-cardio-cutaneous syndrome, Facio-Fronto-Nasal Dysplasia, Faciocutaneoskeletal Syndrome, Faciodigitogenital syndrome, Faciogenital dysplasia, Faciogenitopopliteal Syndrome, Faciopalatoosseous Syndrome, Faciopalatoosseous Syndrome Type II, Facioscapulohumeral muscular dystrophy, Factitious Hypoglycemia, Factor VIII Deficiency, Factor IX Deficiency, Factor IX Deficiency, Factor XI Deficiency, Factor XII deficiency, Factor XIII Deficiency, Fahr Disease, Fahr's Disease, Failure of Secretion Gastric Intrinsic Factor, Fairbank Disease, Fallot's Tetralogy, Familial Acrogeria, Familial Acrogeria, Familial Acromicria, Familial Acromicria, Familial Adenomatous Colon Polyposis, Familial Adenomatous Polyposis with Extraintestinal Manifestations, Familial Alobar Holoprosencephaly, Familial Alpha-Lipoprotein Deficiency, Familial Amyotrophic Chorea with Acanthocytosis, Familial Arrhythmic Myoclonus, Familial Articular Chondrocalcinosis, Familial Atypical Mole-Malignant Melanoma Syndrome, Familial Broad Beta Disease, Familial Calcium Gout, Familial Calcium Pyrophosphate Arthropathy, Familial Chronic Obstructive Lung Disease, Familial Continuous Skin Peeling, Familial Cutaneous Amyloidosis, Familial Dysproteinemia, Familial Emphysema, Familial Enteropathy Microvillus, Familial Foveal Retinoschisis, Familial Hibernation Syndrome, Familial High Cholesterol, Familial Hemochromatosis, Familial High Blood Cholesterol, Familial High-Density Lipoprotein Deficiency, Familial High Serum Cholesterol, Familial Hyperlipidema, Familial Hypoproteinemia with Lymphangietatic Enteropathy, Familial Jaundice, Familial Juvenile Nephronophtisis-Associated Ocular Anomaly, Familial Lichen Amyloidosis (Type IX), Familial Lumbar Stenosis, Familial Lymphedema Praecox, Familial Mediterranean Fever, Familial Multiple Polyposis, Familial Nuchal Bleb, Familial Paroxysmal Polyserositis, Familial Polyposis Coli, Familial Primary Pulmonary Hypertension, Familial Renal Glycosuria, Familial Splenic Anemia, Familial Startle Disease, Familial Visceral Amyloidosis (Type VIII), FAMMM, FANCA, FANCB, FANCC, FANCD, FANCE, Fanconi Panmyelopathy, Fanconi Pancytopenia, Fanconi II, Fanconi's Anemia, Fanconi's Anemia Type I, Fanconi's Anemia Complementation Group, Fanconi's Anemia Complementation Group A, Fanconi's Anemia Complementation Group B, Fanconi's Anemia Complementation Group C, Fanconi's Anemia Complementation Group D, Fanconi's Anemia Complementation Group E, Fanconi's Anemia Complementation Group G, Fanconi's Anemia Complementation Group H, Fanconi's Anemia Estren-Dameshek Variant, FANF, FANG, FANH, FAP, FAPG, Farber's Disease, Farber's Lipogranulomatosis, FAS, Fasting Hypoglycemia, Fat-Induced Hyperlipemia, Fatal Granulomatous Disease of Childhood, Fatty Oxidation Disorders, Fatty Liver with Encephalopathy, FAV, FCH, FCMD, FCS Syndrome, FD, FDH, Febrile Mucocutaneous Syndrome Stevens Johnson Type, Febrile Neutrophilic Dermatosis Acute, Febrile Seizures, Feinberg's syndrome, Feissinger-Leroy-Reiter Syndrome, Female Pseudo-Turner Syndrome, Femoral Dysgenesis Bilateral-Robin Anomaly, Femoral Dysgenesis Bilateral, Femoral Facial Syndrome, Femoral Hypoplasia-Unusual Facies Syndrome, Fetal Alcohol Syndrome, Fetal Anti-Convulsant Syndrome, Fetal Cystic Hygroma, Fetal Effects of Alcohol, Fetal Effects of Chickenpox, Fetal Effects of Thalidomide, Fetal Effects of Varicella Zoster Virus, Fetal Endomyocardial Fibrosis, Fetal Face Syndrome, Fetal Iritis Syndrome, Fetal Transfusion Syndrome, Fetal Valproate Syndrome, Fetal Valproic Acid Exposure Syndrome, Fetal Varicella Infection, Fetal Varicella Zoster Syndrome, FFDD Type II, FG Syndrome, FGDY, FHS, Fibrin Stabilizing Factor Deficiency, Fibrinase Deficiency, Fibrinoid Degeneration of Astrocytes, Fibrinoid Leukodystrophy, Fibrinoligase Deficiency, Fibroblastoma Perineural, Fibrocystic Disease of Pancreas, Fibrodysplasia Ossificans Progressiva, Fibroelastic Endocarditis, Fibromyalgia, Fibromyalgia-Fibromyositis, Fibromyositis, Fibrosing Cholangitis, Fibrositis, Fibrous Ankylosis of Multiple Joints, Fibrous Cavernositis, Fibrous Dysplasia, Fibrous Plaques of the Penis, Fibrous Sclerosis of the Penis, Fickler-Winkler Type, Fiedler Disease, Fifth Digit Syndrome, Filippi Syndrome, Finnish Type Amyloidosis (Type V), First Degree Congenital Heart Block, First and Second Branchial Arch Syndrome, Fischer's Syndrome, Fish Odor Syndrome, Fissured Tongue, Flat Adenoma Syndrome, Flatau-Schilder Disease, Flavin Containing Monooxygenase 2, Floating Beta Disease, Floating-Harbor Syndrome, Floating Spleen, Floppy Infant Syndrome, Floppy Valve Syndrome, Fluent aphasia, FMD, FMF, FMO Adult Liver Form, FMO2, FND, Focal Dermal Dysplasia Syndrome, Focal Dermal Hypoplasia, Focal Dermato-Phalangeal Dysplasia, Focal Dystonia, Focal Epilepsy, Focal Facial Dermal Dysplasia Type II, Focal Neuromyotonia, FODH, Folling Syndrome, Fong Disease, FOP, Forbes Disease, Forbes-Albright Syndrome, Forestier's Disease, Forsius-Eriksson Syndrome (X-Linked), Fothergill Disease, Fountain Syndrome, Foveal Dystrophy Progressive, FPO Syndrome Type II, FPO, Fraccaro Type Achondrogenesis (Type IB), Fragile X syndrome, Franceschetti-Zwalen-Klein Syndrome, Francois Dyscephaly Syndrome, Francois-Neetens Speckled Dystrophy, Flecked Corneal Dystrophy, Fraser Syndrome, FRAXA, FRDA, Fredrickson Type I Hyperlipoproteinemia, Freeman-Sheldon Syndrome, Freire-Maia Syndrome, Frey's Syndrome, Friedreich's Ataxia, Friedreich's Ataxia, Friedreich's Disease, Friedreich's Tabes, FRNS, Froelich's Syndrome, Frommel-Chiari Syndrome, Frommel-Chiari Syndrome Lactation-Uterus Atrophy, Frontodigital Syndrome, Frontofacionasal Dysostosis, Frontofacionasal Dysplasia, Frontonasal Dysplasia, Frontonasal Dysplasia with Coronal Craniosynostosis, Fructose-1-Phosphate Aldolase Deficiency, Fructosemia, Fructosuria, Fryns Syndrome, FSH, FSHD, FSS, Fuchs Dystrophy, Fucosidosis Type 1, Fucosidosis Type 2, Fucosidosis Type 3, Fukuhara Syndrome, Fukuyama Disease, Fukuyama Type Muscular Dystrophy, Fumarylacetoacetase deficiency, Furrowed Tongue, G Syndrome, G6PD Deficiency, G6PD, GA I, GA IIB, GA IIA, GA II, GAII & MADD, Galactorrhea-Amenorrhea Syndrome Nonpuerperal, Galactorrhea-Amenorrhea without Pregnancy, Galactosamine-6-Sulfatase Deficiency, Galactose-1-Phosphate Uridyl Transferase Deficiency, Galactosemia, GALB Deficiency, Galloway-Mowat Syndrome, Galloway Syndrome, GALT Deficiency, Gammaglobulin Deficiency, GAN, Ganglioside Neuraminidase Deficiency, Ganglioside Sialidase Deficiency, Gangliosidosis GM1 Type 1, Gangliosidosis GM2 Type 2, Gangliosidosis Beta Hexosaminidase B Defeciency, Gardner Syndrome, Gardner Syndrome, Gargoylism, Garies-Mason Syndrome, Gasser Syndrome, Gastric Intrinsic Factor Failure of Secretion, Enterocyte Cobalamin, Gastrinoma, Gastritis, Gastroesophageal Laceration-Hemorrhage, Gastrointestinal Polyposis and Ectodermal Changes, Gastroschisis, Gaucher Disease, Gaucher-Schlagenhaufer, Gayet-Wernicke Syndrome, GBS, GCA, GCM Syndrome, GCPS, Gee-Herter Disease, Gee-Thaysen Disease, Gehrig's Disease, Gelineau's Syndrome, Genee-Wiedemann Syndrome, Generalized Dystonia, Generalized Familial Neuromyotonia, Generalized Fibromatosis, Generalized Flexion Epilepsy, Generalized Glycogenosis, Generalized Hyperhidrosis, Generalized Lipofuscinosis, Generalized Myasthenia Gravis, Generalized Myotonia, Generalized Sporadic Neuromytonia, Genetic Disorders, Genital Defects, Genital and Urinary Tract Defects, Genital and Urinary Tract Defects, Gerstmann Syndrome, Gerstmann Tetrad, GHBP, GHD, GHR, Giant Axonal Disease, Giant Axonal Neuropathy, Giant Benign Lymphoma, Giant Cell Glioblastoma Astrocytoma, Giant Cell Arteritis, Giant Cell Disease of the Liver, Giant Cell Hepatitis, Giant Cell of Newborns Cirrhosis, Giant Cyst of the Retina, Giant Lymph Node Hyperplasia, Giant Platelet Syndrome Hereditary, Giant Tongue, gic Macular Dystrophy, Gilbert's Disease, Gilbert Syndrome, Gilbert-Dreyfus Syndrome, Gilbert-Lereboullet Syndrome, Gilford Syndrome, Gilles de la Tourette's syndrome, Gillespie Syndrome, Gingival Fibromatosis-Abnormal Fingers Nails Nose Ear Splenomegaly, GLA Deficiency, GLA, GLB1, Glioma Retina, Global aphasia, Globoid Leukodystrophy, Glossoptosis Micrognathia and Cleft Palate, Glucocerebrosidase deficiency, Glucocerebrosidosis, Glucose-6-Phosphate Dehydrogenase Deficiency, Glucose-6-Phosphate Tranport Defect, Glucose-6-Phospate Translocase Deficiency, Glucose-G-Phosphatase Deficiency, Glucose-Galactose Malabsorption, Glucose-Galactose Malabsorption, Glucosyl Ceramide Lipidosis, Glutaric Aciduria I, Glutaric Acidemia I, Glutaric Acidemia II, Glutaric Aciduria II, Glutaric Aciduria Type II, Glutaric Aciduria Type III, Glutaricacidemia I, Glutaricacidemia II, Glutaricaciduria I, Glutaricaciduria II, Glutaricaciduria Type IIA, Glutaricaciduria Type IIB, Glutaryl-CoA Dehydrogenase Deficiency, Glutaurate-Aspartate Transport Defect, Gluten-Sensitive Enteropathy, Glycogen Disease of Muscle Type VII, Glycogen Storage Disease I, Glycogen Storage Disease III, Glycogen Storage Disease IV, Glycogen Storage Disease Type V, Glycogen Storage Disease VI, Glycogen Storage Disease VII, Glycogen Storage Disease VIII, Glycogen Storage Disease Type II, Glycogenosis, Glycogenosis Type I, Glycogenosis Type IA, Glycogenosis Type IB, Glycogenosis Type II, Glycogenosis Type III, Glycogenosis Type IV, Glycogenosis Type V, Glycogenosis Type VI, Glycogenosis Type VII, Glycogenosis Type VIII, Glycolic Aciduria, Glycolic Aciduria, Glycolipid Lipidosis, GM2 Gangliosidosis Type 1, GM2 Gangliosidosis Type 1, GNPTA, Goitrous Autoimmune Thyroiditis, Goldenhar Syndrome, Goldenhar-Gorlin Syndrome, Goldscheider's Disease, Goltz Syndrome, Goltz-Gorlin Syndrome, Gonadal Dysgenesis 45 X, Gonadal Dysgenesis XO, Goniodysgenesis-Hypodontia, Goodman Syndrome, Goodman, Goodpasture Syndrome, Gordon Syndrome, Gorlin's Syndrome, Gorlin-Chaudhry-Moss Syndrome, Gottron Erythrokeratodermia Congenitalis Progressiva Symmetrica, Gottron's Syndrome, Gougerot-Carteaud Syndrome, Grand Mal Epilepsy, Granular Type Corneal Dystrophy, Granulomatous Arteritis, Granulomatous Colitis, Granulomatous Dermatitis with Eosinophilia, Granulomatous Ileitis, Graves Disease, Graves' Hyperthyroidism, Graves' Disease, Greig Cephalopolysyndactyly Syndrome, Groenouw Type I Corneal Dystrophy, Groenouw Type II Corneal Dystrophy, Gronblad-Strandberg Syndrome, Grotton Syndrome, Growth Hormone Receptor Deficiency, Growth Hormone Binding Protein Deficiency, Growth Hormone Deficiency, Growth-Mental Deficiency Syndrome of Myhre, Growth Retardation-Rieger Anomaly, GRS, Gruber Syndrome, GS, GSD6, GSD8, GTS, Guanosine Triphosphate-Cyclohydrolase Deficiency, Guanosine Triphosphate-Cyclohydrolase Deficiency, Guenther Porphyria, Guerin-Stem Syndrome, Guillain-Barré, Guillain-Barre Syndrome, Gunther Disease, H Disease, H. Gottron's Syndrome, H. Gottron's Syndrome, Habit Spasms, HAE, Hageman Factor Deficiency, Hageman factor, Haim-Munk Syndrome, Hajdu-Cheney Syndrome, Hajdu Cheney, HAL Deficiency, Hall-Pallister Syndrome, Hallermann-Streiff-Francois syndrome, Hallermann-Streiff Syndrome, Hallervorden-Spatz Disease, Hallervorden-Spatz Syndrome, Hallopeau-Siemens Disease, Hallux Duplication Postaxial Polydactyly and Absence of Corpus Callosum, Halushi-Behcet's Syndrome, Hamartoma of the Lymphatics, Hand-Schueller-Christian Syndrome, HANE, Hanhart Syndrome, Happy Puppet Syndrome, Harada Syndrome, HARD +/-E Syndrome, HARD Syndrome, Hare Lip, Harlequin Fetus, Harlequin Type DOC 6, Harlequin Type Ichthyosis, Harley Syndrome, Harrington Syndrome, Hart Syndrome, Hartnup Disease, Hartnup Disorder, Hartnup Syndrome, Hashimoto's Disease, Hashimoto-Pritzker Syndrome, Hashimoto's Syndrome, Hashimoto's Thyroiditis, Hashimoto's Thyroiditis, Hashimoto-Pritzker Syndrome, Hay Well's Syndrome, Hay-Wells Syndrome of Ectodermal Dysplasia, HCMM, HCP, HCTD, HD, Heart-Hand Syndrome (Holt-Oram Type), Heart Disease, Hecht Syndrome, HED, Heerferdt-Waldenstrom and Lofgren's Syndromes, Hegglin's Disease, Heinrichsbauer Syndrome, Hemangiomas, Hemangioma Familial, Hemangioma-Thrombocytopenia Syndrome, Hemangiomatosis Chondrodystrophica, Hemangiomatous Branchial Clefts-Lip Pseudocleft Syndrome, Hemifacial Microsomia, Hemimegalencephaly, Hemiparesis of Cerebral Palsy, Hemiplegia of Cerebral Palsy, Hemisection of the Spinal Cord, Hemochromatosis, Hemochromatosis Syndrome, Hemodialysis-Related Amyloidosis, Hemoglobin Lepore Syndromes, Hemolytic Anemia of Newborn, Hemolytic Cold Antibody Anemia, Hemolytic Disease of Newborn, Hemolytic-Uremic Syndrome, Hemolytic-Uremic Syndrome, Hemophilia, Hemophilia A, Hemophilia B, Hemophilia B Factor IX, Hemophilia C, Hemorrhagic Dystrophic Thrombocytopenia, Hemorrhagica Aleukia, Hemosiderosis, Hepatic Fructokinase Deficiency, Hepatic Phosphorylase Kinase Deficiency, Hepatic Porphyria, Hepatic Porphyrias, Hepatic Veno-Occlusive Disease, Hepato-Renal Syndrome, Hepatolenticular Degeneration, Hepatophosphorylase Deficiency, Hepatorenal Glycogenosis, Hepatorenal Syndrome, Hepatorenal Tyrosinemia, Hereditary Acromelalgia, Hereditary Alkaptonuria, Hereditary Amyloidosis, Hereditary Angioedema, Hereditary Areflexic Dystasia, Heredopathia Atactica Polyneuritiformis, Hereditary Ataxia, Hereditary Ataxia Friedrich's Type, Hereditary Benign Acanthosis Nigricans, Hereditary Cerebellar Ataxia, Hereditary Chorea, Hereditary Chronic Progressive Chorea, Hereditary Connective Tissue Disorders, Hereditary Coproporphyria, Hereditary Coproporphyria Porphyria, Hereditary Cutaneous Malignant Melanoma, Hereditary Deafness-Retinitis Pigmentosa, Heritable Disorder of Zinc Deficiency, Hereditary DNS, Hereditary Dystopic Lipidosis, Hereditary Emphysema, Hereditary Fructose Intolerance, Hereditary Hemorrhagic Telangiectasia, Hereditary Hemorrhagic Telangiectasia Type I, Hereditary Hemorrhagic Telangiectasia Type II, Hereditary Hemorrhagic Telangiectasia Type III, Hereditary Hyperuricemia and Choreoathetosis Syndrome, Hereditary Leptocytosis Major, Hereditary Leptocytosis Minor, Hereditary Lymphedema, Hereditary Lymphedema Tarda, Hereditary Lymphedema Type I, Hereditary Lymphedema Type II, Hereditary Motor Sensory Neuropathy, Hereditary Motor Sensory Neuropathy I, Hereditary Motor Sensory Neuropathy Type III, Hereditary Nephritis, Hereditary Nephritis and Nerve Deafness, Hereditary Nephropathic Amyloidosis, Hereditary Nephropathy and Deafness, Hereditary Nonpolyposis Colorectal Cancer, Hereditary Nonpolyposis Colorectal Carcinoma, Hereditary Nonspherocytic Hemolytic Anemia, Hereditary Onychoosteodysplasia, Hereditary Optic Neuroretinopathy, Hereditary Polyposis Coli, Hereditary Sensory and Autonomic Neuropathy Type I, Hereditary Sensory and Autonomic Neuropathy Type II, Hereditary Sensory and Autonomic Neuropathy Type III, Hereditary Sensory Motor Neuropathy, Hereditary Sensory Neuropathy Type I, Hereditary Sensory Neuropathy Type II, Hereditary Sensory Neuropathy Type III, Hereditary Sensory Radicular Neuropathy Type I, Hereditary Sensory Radicular Neuropathy Type II, Hereditary Site Specific Cancer, Hereditary Spherocytic Hemolytic Anemia, Hereditary Spherocytosis, Hereditary Tyrosinemia Type 1, Heritable Connective Tissue Disorders, Herlitz Syndrome, Hermans-Herzberg Phakomatosis, Hermansky-Pudlak Syndrome, Hermansky-Pudlak Syndrome, Hermaphroditism, Herpes Zoster, Herpes Iris Stevens-Johnson Type, Hers Disease, Heterozygous Beta Thalassemia, Hexoaminidase Alpha-Subunit Deficiency (Variant B), Hexoaminidase Alpha-Subunit Deficiency (Variant B), HFA, HFM, HGPS, HH, HHHO, HHRH, HHT, Hiatal Hernia-Microcephaly-Nephrosis Galloway Type, Hidradenitis Suppurativa, Hidrosadenitis Axillaris, Hidrosadenitis Suppurativa, Hidrotic Ectodermal Dysplasias, HIE Syndrome, High Imperforate Anus, High Potassium, High Scapula, HIM, Hirschsprung's Disease, Hirschsprung's Disease Acquired, Hirschsprung Disease Polydactyly of Ulnar & Big Toe and VSD, Hirschsprung Disease with Type D Brachydactyly, Hirsutism, HIS Deficiency, Histidine Ammonia-Lyase (HAL) Deficiency, Histidase Deficiency, Histidinemia, Histidinemia, Histiocytosis, Histiocytosis X, HLHS, HLP Type II, HMG, HMI, HMSN I, HNHA, HOCM, Hodgkin Disease, Hodgkin's Disease, Hodgkin's Lymphoma, Hollaender-Simons Disease, Holmes-Adie Syndrome, Holocarboxylase Synthetase Deficiency, Holoprosencephaly, Holoprosencephaly Malformation Complex, Holoprosencephaly Sequence, Holt-Oram Syndrome, Holt-Oram Type Heart-Hand Syndrome, Homocystinemia, Homocystinuria, Homocystinuria, Homogentisic Acid Oxidase Deficiency, Homogentisic Acidura, Homozygous Alpha-1-Antitrypsin Deficiency, HOOD, Horner Syndrome, Horton's disease, HOS, HOS1, Houston-Harris Type Achrondrogenesis (Type IA), HPS, HRS, HS, HSAN Type I, HSAN Type II, HSAN-III, HSMN, HSMN Type III, HSN I, HSN-III, Huebner-Herter Disease, Hunner's Patch, Hunner's Ulcer, Hunter Syndrome, Hunter Syndrome, Hunter-Thompson Type Acromesomelic Dysplasia, Huntington's Chorea, Huntington's Disease, Hurler Disease, Hurler Disease, Hurler Syndrome, Hurler-Scheie Syndrome, HUS, Hutchinson-Gilford Progeria Syndrome, Hutchinson-Gilford Syndrome, Hutchinson-Weber-Peutz Syndrome, Hutterite Syndrome Bowen-Conradi Type, Hyaline Panneuropathy, Hydranencephaly, Hydrocephalus, Hydrocephalus Agyria and Retinal Dysplasia, Hydrocephalus Internal Dandy-Walker Type, Hydrocephalus Noncommunicating Dandy-Walker Type, Hydrocephaly, Hydronephrosis With Peculiar Facial Expression, Hydroxylase Deficiency, Hygroma Colli, Hyper-IgE Syndrome, Hyper IgM Syndrome, Hyperaldosteronism, Hyperaldosteronism With Hypokalemic Alkatosis, Hyperaldosteronism Without Hypertension, Hyperammonemia, Hyperammonemia Due to Carbamylphosphate Synthetase Deficiency, Hyperammonemia Due to Ornithine Transcarbamylase Deficiency, Hyperammonemia Type II, Hyper-Beta Carnosinemia, Hyperbilirubinemia I, Hyperbilirubinemia II, Hypercalcemia Familial with Nephrocalcinosis and Indicanuria, Hypercalcemia-Supravalvar Aortic Stenosis, Hypercalciuric Rickets, Hypercapnic acidosis, Hypercatabolic Protein-Losing Enteropathy, Hyperchloremic acidosis, Hypercholesterolemia, Hypercholesterolemia Type IV, Hyperchylomicronemia, Hypercystinuria, Hyperekplexia, Hyperextensible joints, Hyperglobulinemic Purpura, Hyperglycinemia with Ketoacidosis and Lactic Acidosis Propionic Type, Hyperglycinemia Nonketotic, Hypergonadotropic Hypogonadism, Hyperimmunoglobulin E Syndrome, Hyperimmunoglobulin E-Recurrent Infection Syndrome, Hyperimmunoglobulinemia E-Staphylococcal, Hyperkalemia, Hyperkinetic Syndrome, Hyperlipemic Retinitis, Hyperlipidemia I, Hyperlipidemia IV, Hyperlipoproteinemia Type I, Hyperlipoproteinemia Type III, Hyperlipoproteinemia Type IV, Hyperoxaluria, Hyperphalangy-Clinodactyly of Index Finger with Pierre Robin Syndrome, Hyperphenylalanemia, Hyperplastic Epidermolysis Bullosa, Hyperpnea, Hyperpotassemia, Hyperprebeta-Lipoproteinemia, Hyperprolinemia Type I, Hyperprolinemia Type II, Hypersplenism, Hypertelorism with Esophageal Abnormalities and Hypospadias, Hypertelorism-Hypospadias Syndrome, Hypertrophic Cardio myopathy, Hypertrophic Interstitial Neuropathy, Hypertrophic Interstitial Neuritis, Hypertrophic Interstitial Radiculoneuropathy, Hypertrophic Neuropathy of Refsum, Hypertrophic Obstructive Cardio myopathy, Hyperuricemia Choreoathetosis Self-multilation Syndrome, Hyperuricemia-Oligophrenia, Hypervalinemia, Hypocalcified (Hypomineralized) Type, Hypochondrogenesis, Hypochrondroplasia, Hypogammaglobulinemia, Hypogammaglobulinemia Transient of Infancy, Hypogenital Dystrophy with Diabetic Tendency, Hypoglossia-Hypodactylia Syndrome, Hypoglycemia, Hypoglycemia, Exogenous Hypoglycemia, Hypoglycemia with Macroglossia, Hypoglycosylation Syndrome Type 1a, Hypoglycosylation Syndrome Type 1a, Hypogonadism with Anosmia, Hypogonadotropic Hypogonadism and Anosmia, Hypohidrotic Ectodermal Dysplasia, Hypohidrotic Ectodermal Dysplasia Autosomal Dominant type, Hypohidrotic Ectodermal Dysplasias Autorecessive, Hypokalemia, Hypokalemic Alkalosis with Hypercalciuria, Hypokalemic Syndrome, Hypolactasia, Hypomaturation Type (Snow-Capped Teeth), Hypomelanosis of Ito, Hypomelia-Hypotrichosis-Facial Hemangioma Syndrome, Hypomyelination Neuropathy, Hypoparathyroidism, Hypophosphatasia, Hypophosphatemic Rickets with Hypercalcemia, Hypopigmentation, Hypopigmentation, Hypopigmented macular lesion, Hypoplasia of the Depressor Anguli Oris Muscle with Cardiac Defects, Hypoplastic Anemia, Hypoplastic Congenital Anemia, Hypoplastic Chondrodystrophy, Hypoplastic Enamel-Onycholysis-Hypohidrosis, Hypoplastic (Hypoplastic-Explastic) Type, Hypoplastic Left Heart Syndrome, Hypoplastic Left Heart Syndrome, Hypoplastic-Triphalangeal Thumbs, Hypopotassemia Syndrome, Hypospadias-Dysphagia Syndrome, Hyposmia, Hypothalamic Hamartoblastoma Hypopituitarism Imperforate Anus Polydactyly, Hypothalamic Infantilism-Obesity, Hypothyroidism; Hypotonia-Hypomentia-Hypogonadism-Obesity Syndrome, Hypoxanthine-Guanine Phosphoribosyltranferase Defect (Complete Absense of), I-Cell Disease, latrogenic Hypoglycemia, IBGC, IBIDS Syndrome, IBM, IBS, IC, I-Cell Disease, ICD, ICE Syndrome Cogan-Reese Type, Icelandic Type Amyloidosis (Type VI), I-Cell Disease, Ichthyosiform Erythroderma Corneal Involvement and Deafness, Ichthyosiform Erythroderma Hair Abnormality Growth and Men, Ichthyosiform Erythroderma with Leukocyte Vacuolation, Ichthyosis, Ichthyosis Congenita, Ichthyosis Congenital with Trichothiodystrophy, Ichthyosis Hystrix, Ichthyosis Hystrix Gravior, Ichthyosis Linearis Circumflexa, Ichthyosis Simplex, Ichthyosis Tay Syndrome, Ichthyosis Vulgaris, Ichthyosis Vulgaris, Ichthyotic Neutral Lipid Storage Disease, Icteric Leptospirosis, Icterohemorrhagic Leptospirosis, Icterus (Chronic Familial), Icterus Gravis Neonatorum, Icterus Intermittens Juvenalis, Idiopathic Alveolar Hypoventilation, Idiopathic Amyloidosis, Idiopathic Arteritis of Takayasu, Idiopathic Basal Ganglia Calcification (IBGC), Idiopathic Brachial Plexus Neuropathy, Idiopathic Cervical Dystonia, Idiopathic Dilatation of the Pulmonary Artery, Idiopathic Dilatation of the Pulmonary Artery, Idiopathic Facial Palsy, Idiopathic Familial Hyperlipemia, Idiopathic Hypertrophic Subaortic Stenosis, Idiopathic Hypoproteinemia, Idiopathic Immunoglobulin Deficiency, Idiopathic Neonatal Hepatitis, Idiopathic Non-Specific Ulcerative Colitis, Idiopathic Non-Specific Ulcerative Colitis, Idiopathic Peripheral Periphlebitis, Idiopathic Pulmonary Fibrosis, Idiopathic Refractory Sideroblastic Anemia, Idiopathic Refractory Sideroblastic Anemia, Idiopathic Renal Hematuria, Idiopathic Steatorrhea, Idiopathic Thrombocythemia, Idiopathic Thrombocytopenic Purpura, Idiopathic Thrombocytopenia Purpura (ITP), IDPA, IgA Nephropathy, IgA Nephropathy, IHSS, Ileitis, Ileocolitis, Illinois Type Amyloidosis, ILS, IM, IMD2, IMD5, IMD5, Immune Defect due to Absence of Thymus, Immune Hemolytic Anemia Paroxysmal Cold, Immunodeficiency with Ataxia Telangiectasia, Immunodeficiency Cellular with Abnormal Immunoglobulin Synthesis, Immunodeficiency Common Variable Unclassifiable, Immunodeficiency with Hyper-IgM, Immunodeficiency with Leukopenia, Immunodeficiency-2, Immunodeficiency-5 (IMD5), Immunoglobulin Deficiency, Imperforate Anus, Imperforate Anus with Hand Foot and Ear Anomalies, Imperforate Nasolacrimal Duct and Premature Aging Syndrome, Impotent Neutrophil Syndrome, Inability To Open Mouth Completely And Short Finger-Flexor, INAD, Inborn Error of Urea Synthesis Arginase Type, Inborn Error of Urea Synthesis Arginino Succinic Type, Inborn Errors of Urea Synthesis Carbamyl Phosphate Type, Inborn Error of Urea Synthesis Citrullinemia Type, Inborn Errors of Urea Synthesis Glutamate Synthetase Type, INCL, Inclusion body myositis, Incomplete Atrioventricular Septal Defect, Incomplete Testicular Feminization, Incomplete Testicular Feminization, Incontinentia Pigmenti, Incontinentia Pigmenti, Incontinenti Pigmenti Achromians, Index Finger Anomaly with Pierre Robin Syndrome, Indiana Type Amyloidosis (Type II), Indolent systemic mastocytosis, Infantile Acquired Aphasia, Infantile Autosomal Recessive Polycystic Kidney Disease, Infantile Beriberi, Infantile Cerebral Ganglioside, Infantile Cerebral Ganglioside, Infantile Cerebral Paralysis, Infantile Cystinosis, Infantile Epileptic, Infantile Fanconi Syndrome with Cystinosis, Infantile Finnish Type Neuronal Ceroid Lipofuscinosis, Infantile Gaucher Disease, Infantile Hypoglycemia, Infantile Hypophasphatasia, Infantile Lobar Emphysema, Infantile Myoclonic Encephalopathy, Infantile Myoclonic Encephalopathy and Polymyoclonia, Infantile Myofibromatosis, Infantile Necrotizing Encephalopathy, Infantile Neuronal Ceroid Lipofuscinosis, Infantile Neuroaxonal Dystrophy, Infantile Onset Schindler Disease, Infantile Phytanic Acid Storage Disease, Infantile Refsum Disease (IRD), Infantile Sipoidosis GM-2 Gangliosideosis (Type S), Infantile Sipoidosis GM-2 Gangliosideosis (Type S, Infantile Sleep Apnea, Infantile Spasms, Infantile Spinal Muscular Atrophy (all types), Infantile Spinal Muscular Atrophy ALS, Infantile Spinal Muscular Atrophy Type I, Infantile Type Neuronal Ceroid Lipofuscinosis, Infectious Jaundice, Inflammatory Breast Cancer, Inflammatory Linear Nevus Sebaceous Syndrome, Iniencephaly, Insulin Resistant Acanthosis Nigricans, Insulin Lipodystrophy, Insulin dependent Diabetes, Intention Myoclonus, Intermediate Cystinosis, Intermediate Maple Syrup Urine Disease, Intermittent Ataxia with Pyruvate Dehydrogenase Deficiency, Intermittent Ataxia with Pyruvate Dehydrogenase Deficiency, Intermittent Maple Syrup Urine Disease, Internal Hydrocephalus, Interstitial Cystitis, Interstitial Deletion of 4q Included, Interstitial Deletion of 4q- Included, Intestinal Lipodystrophy, Intestinal Lipophagic Granulomatosis, Intestinal Lymphangiectasia, Intestinal Polyposis I, Intestinal Polyposis II, Intestinal Polyposis II, Intestinal Polyposis III, Intestinal Polyposis-Cutaneous Pigmentation Syndrome, Intestinal Polyposis-Cutaneous Pigmentation Syndrome, Intestinal Pseudoobstruction with External Ophthalmoplegia, Intracranial Neoplasm, Intracranial Tumors, Intracranial Vascular Malformations, Intrauterine Dwarfism, Intrauterine Synechiae, Inverted Smile And Occult Neuropathic Bladder, Iowa Type Amyloidosis (Type IV), IP, IPA, Iridocorneal Endothelial Syndrome, Iridocorneal Endothelial (ICE) Syndrome Cogan-Resse Type, Iridogoniodysgenesis With Somatic Anomalies, Iris Atrophy with Corneal Edema and Glaucoma, Iris Nevus Syndrome, Iron Overload Anemia, Iron Overload Disease, Irritable Bowel Syndrome, Irritable Colon Syndrome, Isaacs Syndrome, Isaacs-Merten Syndrome, Ischemic Cardio myopathy, Isolated Lissencephaly Sequence, Isoleucine 33 Amyloidosis, Isovaleric Acid CoA Dehydrogenase Deficiency, Isovaleric Acidaemia, Isovalericacidemia, Isovaleryl CoA Carboxylase Deficiency, ITO Hypomelanosis, ITO, ITP, IVA, Ivemark Syndrome, Iwanoff Cysts, Jackknife Convulsion, Jackson-Weiss Craniosynostosis, Jackson-Weiss Syndrome, Jacksonian Epilepsy, Jacobsen Syndrome, Jadassohn-Lewandowsky Syndrome, Jaffe-Lichenstein Disease, Jakob's Disease, Jakob-Creutzfeldt Disease, Janeway I, Janeway Dysgammaglobulinemia, Jansen Metaphyseal Dysostosis, Jansen Type Metaphyseal Chondrodysplasia, Jarcho-Levin Syndrome, Jaw-Winking, JBS, JDMS, Jegher's Syndrome, Jegher's Syndrome, Jejunal Atresia, Jejunitis, Jejunoileitis, Jervell and Lange-Nielsen Syndrome, Jeune Syndrome, JMS, Job Syndrome, Job-Buckley Syndrome, Johanson-Blizzard Syndrome, John Dalton, Johnson-Stevens Disease, Jonston's Alopecia, Joseph's Disease, Joseph's Disease Type I, Joseph's Disease Type II, Joseph's Disease Type III, Joubert Syndrome, Joubert-Bolthauser Syndrome, JRA, Juberg Hayward Syndrome, Juberg-Marsidi Syndrome, Juberg-Marsidi Mental Retardation Syndrome, Jumping Frenchmen, Jumping Frenchmen of Maine, Juvenile Arthritis, Juvenile Arthritis, Juvenile Autosomal Recessive Polycystic Kidney Disease, Juvenile Cystinosis, Juvenile (Childhood) Dermatomyositis (JDMS), Juvenile Diabetes, Juvenile Gaucher Disease, Juvenile Gout Choreoathetosis and Mental Retardation Syndrome, Juvenile Intestinal Malabsorption of Vit B12, Juvenile Intestinal Malabsorption of Vitamin B12, Juvenile Macular Degeneration, Juvenile Pernicious Anemia, Juvenile Retinoschisis, Juvenile Rheumatoid Arthritis, Juvenile Rheumatoid Arthritis, Juvenile Spinal Muscular Atrophy Included, Juvenile Spinal Muscular Atrophy ALS Included, Juvenile Spinal Muscular Atrophy Type III, Juxta-Articular Adiposis Dolorosa, Juxta-Articular Adiposis Dolorosa, Juxtaglomerular Hyperplasia, Kabuki Make-Up Syndrome, Kahler Disease, Kallmann Syndrome, Kanner Syndrome, Kanzaki Disease, Kaposi Disease (not Kaposi Sarcoma), Kappa Light Chain Deficiency, Karsch-Neugebauer Syndrome, Karsch-Neugebauer Syndrome, Kartagener Syndrome-Chronic Sinobronchial Disease and Dextrocardia, Kartagener Triad, Kasabach-Merritt Syndrome, Kast Syndrome, Kawasaki Disease, Kawasaki Syndrome, KBG Syndrome, KD, Kearns-Sayre Disease, Kearns-Sayre Syndrome, Kearns-Sayre Syndrome, Kennedy Disease, Kennedy Syndrome, Kennedy Type Spinal and Bulbar Muscular Atrophy, Kennedy-Stefanis Disease, Kenny Disease, Kenny Syndrome, Kenny Type Tubular Stenosis, Kenny-Caffe Syndrome, Kera. Palmoplant. Con. Pes Planus Ony. Periodon. Arach., Keratitis Ichthyosis Deafness Syndrome, Keratoconus, Keratoconus Posticus Circumscriptus, Keratolysis, Keratolysis Exfoliativa Congenita, Keratolytic Winter Erythema, Keratomalacia, Keratosis Follicularis, Keratosis Follicularis Spinulosa Decalvans, Keratosis Follicularis Spinulosa Decalvans Ichthyosis, Keratosis Nigricans, Keratosis Palmoplantaris with Periodontopathia and Onychogryposis, Keratosis Palmoplantaris Congenital Pes Planus Onychogryposis Periodontosis Arachnodactyly, Keratosis Palmoplantaris Congenital, Pes Planus, Onychogryphosis, Periodontosis, Arachnodactyly, Acroosteolysis, Keratosis Rubra Figurata, Keratosis Seborrheica, Ketoacid Decarboxylase Deficiency, Ketoaciduria, Ketotic Glycinemia, Ketotic Glycinemia, KFS, KID Syndrome, Kidney Agenesis, Kidneys Cystic-Retinal Aplasia Joubert Syndrome, Killian Syndrome, Killianffeschler-Nicola Syndrome, Kiloh-Nevin syndrome III, Kinky Hair Disease, Kinsbourne Syndrome, Kleeblattschadel Deformity, Kleine-Levin Syndrome, Kleine-Levin Hibernation Syndrome, Klinefelter, Klippel-Feil Syndrome, Klippel-Feil Syndrome Type I, Klippel-Feil Syndrome Type II, Klippel-Feil Syndrome Type III, Klippel Trenaunay Syndrome, Klippel-Trenaunay-Weber Syndrome, Kluver-Bucy Syndrome, KMS, Kniest Dysplasia, Kniest Syndrome, Kobner's Disease, Koebberling-Dunnigan Syndrome, Kohlmeier-Degos Disease, Kok Disease, Korsakoff Psychosis, Korsakoff's Syndrome, Krabbe's Disease Included, Krabbe's Leukodystrophy, Kramer Syndrome, KSS, KTS, KTW Syndrome, Kufs Disease, Kugelberg-Welander Disease, Kugelberg-Welander Disease, Kugelberg-Welander Syndrome, Kugelberg-Welander Syndrome, Kussmaul-Landry Paralysis, KWS, L-3-Hydroxy-Acyl-CoA Dehydrogenase (LCHAD) Deficiency, Laband Syndrome, Labhart-Willi Syndrome, Labyrinthine Syndrome, Labyrinthine Hydrops, Lacrimo-Auriculo-Dento-Digital Syndrome, Lactase Isolated Intolerance, Lactase Deficiency, Lactation-Uterus Atrophy, Lactic Acidosis Leber Hereditary Optic Neuropathy, Lactic and Pyruvate Acidemia with Carbohydrate Sensitivity, Lactic and Pyruvate Acidemia with Episodic Ataxia and Weakness, Lactic and Pyruvate Acidemia with Carbohydrate Sensitivity,Lactic and Pyruvate, Lactic acidosis, Lactose Intolerance of Adulthood, Lactose Intolerance, Lactose Intolerance of Childhood, Lactose Intolerance, LADD Syndrome, LADD, Lafora Disease Included, Lafora Body Disease, Laki-Lorand Factor Deficiency, LAM, Lambert Type Ichthyosis, Lambert-Eaton Syndrome, Lambert-Eaton Myasthenic Syndrome, Lamellar Recessive Ichthyosis, Lancereaux-Mathieu-Weil Spirochetosis, Landau-Kleffner Syndrome, Landouzy Dejerine Muscular Dystrophy, Landry Ascending Paralysis, Langer-Salidino Type Achondrogensis (Type II), Langer Giedion Syndrome, Langerhans-Cell Granulomatosis, Langerhans-Cell Histiocytosis (LCH), Large Atrial and Ventricular Defect, Laron Dwarfism, Laron Type Pituitary Dwarfism, Larsen Syndrome, Laryngeal Dystonia, Latah (Observed in Malaysia), Late Infantile Neuroaxonal Dystrophy, Late Infantile Neuroaxonal Dystrophy, Late Onset Cockayne Syndrome Type III (Type C), Late-Onset Dystonia, Late-Onset Immunoglobulin Deficiency, Late-Onset Immunoglobulin Deficiency, Late Onset Pelizaeus-Merzbacher Brain Sclerosis, Lattice Corneal Dystrophy, Lattice Dystrophy, Launois-Bensaude, Launois-Cleret Syndrome, Laurence Syndrome, Laurence-Moon Syndrome, Laurence-Moon/Bardet-Biedl, Lawrence-Seip Syndrome, LCA, LCAD Deficiency, LCAD, LCADH Deficiency, LOCH, LCHAD, LCPD, Le Jeune Syndrome, Leband Syndrome, Leber's Amaurosis, Leber's Congenital Amaurosis,Congenital Absence of the Rods and Cones, Leber's Congenital Tapetoretinal Degeneration, Leber's Congenital Tapetoretinal Dysplasia, Leber's Disease, Leber's Optic Atrophy, Leber's Optic Neuropathy, Left Ventricular Fibrosis, Leg Ulcer, Legg-Calve-Perthes Disease, Leigh's Disease, Leigh's Syndrome, Leigh's Syndrome (Subacute Necrotizing Encephalomyelopathy), Leigh Necrotizing Encephalopathy, Lennox-Gastaut Syndrome, Lentigio-Polypose-Digestive Syndrome, Lentigio-Polypose-Digestive Syndrome, Lenz Dysmorphogenetic Syndrome, Lenz Dysplasia, Lenz Microphthalmia Syndrome, Lenz Syndrome, LEOPARD Syndrome, Leprechaunism, Leprechaunism, Leptomeningeal Angiomatosis, Leptospiral Jaundice, Leri-Weill Disease, Leri-Weil Dyschondrosteosis, Leri-Weil Syndrome, Lermoyez Syndrome, Leroy Disease, Lesch Nyhan Syndrome, Lethal Infantile Cardio myopathy, Lethal Neonatal Dwarfism, Lethal Osteochondrodysplasia, Letterer-Siwe Disease, Leukocytic Anomaly Albinism, Leukocytic Inclusions with Platelet Abnormality, Leukodystrophy, Leukodystrophy with Rosenthal Fibers, Leukoencephalitis Periaxialis Concentric, Levine-Critchley Syndrome, Levulosuria, Levy-Hollister Syndrome, LGMD, LGS, LHON, LIC, Lichen Ruber Acuminatus, Lichen Acuminatus, Lichen Amyloidosis, Lichen Planus, Lichen Psoriasis, Lignac-Debre-Fanconi Syndrome, Lignac-Fanconi Syndrome, Ligneous Conjunctivitis, Limb-Girdle Muscular Dystrophy, Limb Girdle Muscular Dystrophy, Limb Malformations-Dento-Digital Syndrome, Limit Dextrinosis, Linear Nevoid Hypermelanosis, Linear Nevus Sebacous Syndrome, Linear Scleroderma, Linear Sebaceous Nevus Sequence, Linear Sebaceous Nevus Syndrome, Lingua Fissurata, Lingua Plicata, Lingua Scrotalis, Linguofacial Dyskinesia, Lip Pseudocieft-hemangiomatous Branchial Cyst Syndrome, Lipid Granulomatosis, Lipid Histiocytosis, Lipid Kerasin Type, Lipid Storage Disease, Lipid-Storage myopathy Associated with SCAD Deficiency, Lipidosis Ganglioside Infantile, Lipidosis Ganglioside Infantile, Lipoatrophic Diabetes Mellitus, Lipodystrophy, Lipoid Corneal Dystrophy, Lipoid Hyperplasia-Male Pseudohermaphroditism, Lipoid Hyperplasia-Male Pseudohermaphroditism, Lipomatosis of Pancreas Congenital, Lipomucopolysaccharidosis Type I, Lipomyelomeningocele, Lipoprotein Lipase Deficiency Familial, LIS, LIS1, Lissencephaly 1, Lissencephaly Type I, Lissencephaly variants with agenesis of the corpus callosum cerebellar hypoplasia or other anomalies, Little Disease, Liver Phosphorylase Deficiency, LKS, LM Syndrome, Lobar Atrophy, Lobar Atrophy of the Brain, Lobar Holoprosencephaly, Lobar Tension Emphysema in Infancy, Lobstein Disease (Type I), Lobster Claw Deformity, Lobster Claw Deformity, Localized Epidermolysis Bullosa, Localized Lipodystrophy, Localized Neuritis of the Shoulder Girdle, Loeffler's Disease, Loeffler Endomyocardial Fibrosis with Eosinophilia, Loeffler Fibroplastic Parietal Endocarditis, Loken Syndrome, Loken-Senior Syndrome, Long-Chain 3-hydroxyacyl-CoA Dehydrogenase (LCHAD), Long Chain Acyl CoA Dehydrogenase Deficiency, Long-Chain Acyl-CoA Dehydrogenase (ACADL), Long-Chain Acyl-CoA Dehydrogenase Deficiency, Long QT Syndrome without Deafness, Lou Gehrig's Disease, Lou Gehrig's Disease Included, Louis-Bar Syndrome, Low Blood Sugar, Low-Density Beta Lipoprotein Deficiency, Low Imperforate Anus, Low Potassium Syndrome, Lowe's Syndrome, Lowe-Bickel Syndrome, Lowe-Terry-MacLachlan Syndrome, LS, LTD, Lubs Syndrome, Luft Disease, Lumbar Canal Stenosis, Lumbar Spinal Stenosis, Lumbosacral Spinal Stenosis, Lundborg-Unverricht Disease, Lundborg-Unverricht Disease Included, Lupus, Lupus Erythematosus, Luschka-Magendie Foramina Atresia, Lyell Syndrome, Lyelles Syndrome, Lymphadenoid Goiter, Lymphangiectatic Protein-Losing Enteropathy, Lymphangioleiomatosis, Lymphangioleimyomatosis, Lymphangiomas, Lymphatic Malformations, Lynch Syndromes, Lynch Syndrome I, Lynch Syndrome II, Lysosomal Alpha-N-Acetylgalactosaminidase Deficiency Schindler Type, Lysosomal Glycoaminoacid Storage Disease-Angiokeratoma Corporis Diffusum, Lysosomal Glucosidase Deficiency, Lysosomal Glucosidase Deficiency, MAA, Machado Disease, Machado-Joseph Disease, Macrencephaly, Macrocephaly, Macrocephaly Hemihypertrophy, Macrocephaly with Multiple Lipomas and Hemangiomata, Macrocephaly with Pseudopapilledema and Multiple Hemangiomata, Macroglobulinemia, Macroglossia, Macroglossia-Omphalocele-Visceromegaly Syndrome, Macrostomia Ablepheron Syndrome, Macrothrombocytopenia Familial Bernard-Soulier Type, Macula Lutea degeneration, Macular Amyloidosis, Macular Degeneration, Macular Degeneration Disciform, Macular Degeneration Senile, Macular Dystrophy, Macular Type Corneal Dystrophy, MAD, Madelung's Disease, Maffucci Syndrome, Major Epilepsy, Malabsorption, Malabsorption-Ectodermal Dysplasia-Nasal Alar Hypoplasia, Maladie de Roger, Maladie de Tics, Male Malformation of Limbs and Kidneys, Male Turner Syndrome, Malignant Acanthosis, Malignant Acanthosis Nigricans, Malignant Astrocytoma, Malignant Atrophic Papulosis, Malignant Fever, Malignant Hyperphenylalaninemia, Malignant Hyperpyrexia, Malignant Hyperthermia, Malignant Melanoma, Malignant Tumors of the Central Nervous System, Mallory-Weiss Laceration, Mallory-Weiss Tear, Mallory-Weiss Syndrome, Mammary Paget's Disease, Mandibular Ameloblastoma, Mandibulofacial Dysostosis, Mannosidosis, Map-Dot-Fingerprint Type Corneal Dystrophy, Maple Syrup Urine Disease, Marble Bones, Marchiafava-Micheli Syndrome, Marcus Gunn Jaw-Winking Syndrome, Marcus Gunn Phenomenon, Marcus Gunn Ptosis with jaw-winking, Marcus Gunn Syndrome, Marcus Gunn (Jaw-Winking) Syndrome, Marcus Gunn Ptosis (with jaw-winking), Marden-Walker Syndrome, Marden-Walker Type Connective Tissue Disorder, Marfan's Abiotrophy, Marfan-Achard syndrome, Marfan Syndrome, Marfan's Syndrome I, Marfan's Variant, Marfan-Achard syndrome, Marfanoid Hypermobility Syndrome, Marginal Corneal Dystrophy, Marie's Ataxia, Marie Disease, Marie-Sainton Disease, Marie Strumpell Disease, Marie-Strumpell Spondylitis, Marinesco-Sjogren Syndrome, Marinesco-Sjogren-Gorland Syndrome, Marker X Syndrome, Maroteaux Lamy Syndrome, Maroteaux Type Acromesomelic Dysplasia, Marshall's Ectodermal Dysplasias With Ocular and Hearing Defects, Marshall-Smith Syndrome, Marshall Syndrome, Marshall Type Deafness-Myopia-Cataract-Saddle Nose, Martin-Albright Syndrome, Martin-Bell Syndrome, Martorell Syndrome, MASA Syndrome, Massive Myoclonia, Mast Cell Leukemia, Mastocytosis, Mastocytosis With an Associated Hematologic Disorder, Maumenee Corneal Dystrophy, Maxillary Ameloblastoma, Maxillofacial Dysostosis, Maxillonasal Dysplasia, Maxillonasal Dysplasia Binder Type, Maxillopalpebral Synkinesis, May-Hegglin Anomaly, MCAD Deficiency, MCAD, McArdle Disease, McCune-Albright, MCD, McKusick Type Metaphyseal Chondrodysplasia, McKusick Type Metaphyseal Chondrodysplasia, MCR, MCTD, Meckel Syndrome, Meckel-Gruber Syndrome, Median Cleft Face Syndrome, Mediterranean Anemia, Medium-Chain Acyl-CoA dehydrogenase (ACADM), Medium Chain Acyl-CoA Dehydrogenase (MCAD) Deficiency, Medium-Chain Acyl-CoA Dehydrogenase Deficiency, Medium Chain Acyl CoA Dehydrogenase Deficiency, Medullary Cystic Disease, Medullary Cystic Disease, Medullary Sponge Kidney, MEF, Megaesophagus, Megalencephaly, Megalencephaly with Hyaline Inclusion, Megalencephaly with Hyaline Panneuropathy, Megaloblastic Anemia, Megaloblastic Anemia of Pregnancy, Megalocornea-Mental Retardation Syndrome, Meier-Gorlin Syndrome, Meige's Lymphedema, Meige's Syndrome, Melanodermic Leukodystrophy, Melanoplakia-Intestinal Polyposis, Melanoplakia-Intestinal Polyposis, MELAS Syndrome, MELAS, Melkersson Syndrome, Melnick-Fraser Syndrome, Melnick-Needies Osteodysplasty, Melnick-Needles Syndrome, Membranous Lipodystrophy, Mendes Da Costa Syndrome, Ménière's Disease, Meningeal Capillary Angiomatosis, Menkes Disease, Menke's Syndrome I, Mental Retardation Aphasia Shuffling Gait Adducted Thumbs (MASA), Mental Retardation-Deafness-Skeletal Abnormalities-Coarse Face with Full Lips, Mental Retardation with Hypoplastic 5th Fingernails and Toenails, Mental Retardation with Osteocartilaginous Abnormalities, Mental Retradation-X-linked with Growth Delay-Deafness-Microgenitalism, Menzel Type OPCA, Mermaid Syndrome, MERRF, MERRF Syndrome, Merten-Singleton Syndrome, MES, Mesangial IGA Nephropathy, Mesenteric Lipodystrophy, Mesiodens-Cataract Syndrome, Mesodermal Dysmorphodystrophy, Mesomelic Dwarfism-Madelung Deformity, Metabolic Acidosis, Metachromatic Leukodystrophy, Metatarsus Varus, Metatropic Dwarfism Syndrome, Metatropic Dysplasia, Metatropic Dysplasia I, Metatropic Dysplasia II, Methylmalonic Acidemia, Methylmalonic Aciduria, Meulengracht's Disease, MFD1, MG, MH, MHA, Micrencephaly, Microcephalic Primordial Dwarfism I, Microcephaly, Microcephaly-Hiatal Hernia-Nephrosis Galloway Type, Microcephaly-Hiatal Hernia-Nephrotic Syndrome, Microcystic Corneal Dystrophy, Microcythemia, Microlissencephaly, Microphthalmia, Microphthalmia, Microphthalmia or Anophthalmos with Associated Anomalies, Micropolygyria With Muscular Dystrophy, Microtia Absent Patellae Micrognathia Syndrome, Microvillus Inclusion Disease, MID, Midsystolic-click-late systolic murmur syndrome, Miescher's Type I Syndrome, Mikulicz Syndrome, Mikulicz-Radecki Syndrome, Mikulicz-Sjogren Syndrome, Mild Autosomal Recessive, Mild Intermediate Maple Syrup Urine Disease, Mild Maple Syrup Urine Disease, Miller Syndrome, Miller-Dieker Syndrome, Miller-Fisher Syndrome, Milroy Disease, Minkowski-Chauffard Syndrome, Minor Epilepsy, Minot-Von Willebrand Disease, Mirror-Image Dextrocardia, Mitochondrial Beta-Oxidation Disorders, Mitrochondrial and Cytosolic, Mitochondrial Cytopathy, Mitochondrial Cytopathy, Kearn-Sayre Type, Mitochondrial Encephalopathy, Mitochondrial Encephalo myopathy Lactic Acidosis and Strokelike Episodes, Mitochondrial myopathy, Mitochondrial myopathy Encephalopathy Lactic Acidosis Stroke-Like Episode, Mitochondrial PEPCK Deficiency, Mitral-valve prolapse, Mixed Apnea, Mixed Connective Tissue Disease, Mixed Connective Tissue Disease, Mixed Hepatic Porphyria, Mixed Non-Fluent Aphasia, Mixed Sleep Apnea, Mixed Tonic and Clonic Torticollis, MJD, MKS, ML I, ML II, ML II, ML III, ML IV, ML Disorder Type I, ML Disorder Type II, ML Disorder Type III, ML Disorder Type IV, MLNS, MMR Syndrome, MND, MNGIE, MNS, Mobitz I, Mobitz II, Mobius Syndrome, Moebius Syndrome, Moersch-Woltmann Syndrome, Mohr Syndrome, Monilethrix, Monomodal Visual Amnesia, Mononeuritis Multiplex, Mononeuritis Peripheral, Mononeuropathym Peripheral, Monosomy 3p2, Monosomy 9p Partial, Monosomy 11q Partial, Monosomy 13q Partial, Monosomy 18q Syndrome, Monosomy X, Monostotic Fibrous Dysplasia, Morgagni-Tumer-Albright Syndrome, Morphea, Morquio Disease, Morquio Syndrome, Morquio Syndrome A, Morquio Syndrome B, Morquio-Brailsford Syndrome, Morvan Disease, Mosaic Tetrasomy 9p, Motor Neuron Disease, Motor Neuron Syndrome, Motor Neurone Disease, Motoneuron Disease, Motoneurone Disease, Motor System Disease (Focal and Slow), Moya-moya Disease, Moyamoya Disease, MPS, MPS I, MPS I H, MPS 1 H/S Hurler/Scheie Syndrome, MPS I S Scheie Syndrome, MPS II, MPS IIA, MPS IIB, MPS II-AR Autosomal Recessive Hunter Syndrome, MPS II-XR, MPS II-XR Severe Autosomal Recessive, MPS III, MPS III A B C and D Sanfiloppo A, MPS IV, MPS IV A and B Morquio A, MPS V, MPS VI, MPS VI Severe Intermediate Mild Maroteaux-Lamy, MPS VII, MPS VII Sly Syndrome, MPS VIII, MPS Disorder, MPS Disorder I, MPS Disorder II, MPS Disorder III MPS Disorder VI, MPS Disorder Type VII, MRS, MS, MSA, MSD, MSL, MSS, MSUD, MSUD Type Ib, MSUD Type II, Mucocutaneous Lymph Node Syndrome, Mucolipidosis I, Mucolipidosis II, Mucolipidosis III, Mucolipidosis IV, Mucopolysaccharidosis, Mucopolysaccharidosis I-H, Mucopolysaccharidosis I-S, Mucopolysaccharidosis II, Mucopolysaccharidosis III, Mucopolysaccharidosis IV, Mucopolysaccharidosis VI, Mucopolysaccharidosis VII, Mucopolysaccharidosis Type I, Mucopolysaccharidosis Type II, Mucopolysaccharidosis Type III, Mucopolysaccharidosis Type VII, Mucosis, Mucosulfatidosis, Mucous Colitis, Mucoviscidosis, Mulibrey Dwarfism, Mulibrey Nanism Syndrome, Mullerian Duct Aplasia-Renal Aplasia-Cervicothoracic Somite Dysplasia, Mullerian Duct-Renal-Cervicothoracic-Upper Limb Defects, Mullerian Duct and Renal Agenesis with Upper Limb and Rib Anomalies, Mullerian-Renal-Cervicothoracic Somite Abnormalities, Multi-Infarct Dementia Binswanger's Type, Multicentric Castleman's Disease, Multifocal Eosinophilic Granuloma, Multiple Acyl-CoA Dehydrogenase Deficiency, Multiple Acyl-CoA Dehydrogenase Deficiency, Multiple Acyl-CoA Dehydrogenase Deficiency / Glutaric Aciduria Type II, Multiple Angiomas and Endochondromas, Multiple Carboxylase Deficiency, Multiple Cartilaginous Enchondroses, Multiple Cartilaginous Exostoses, Multiple Enchondromatosis, Multiple Endocrine Deficiency Syndrome Type II, Multiple Epiphyseal Dysplasia, Multiple Exostoses, Multiple Exostoses Syndrome, Multiple Familial Polyposis, Multiple Lentigines Syndrome, Multiple Myeloma, Multiple Neuritis of the Shoulder Girdle, Multiple Osteochondromatosis, Multiple Peripheral Neuritis, Multiple Polyposis of the Colon, Multiple Pterygium Syndrome, Multiple Sclerosis, Multiple Sulfatase Deficiency, Multiple Symmetric Lipomatosis, Multiple System Atrophy, Multisynostotic Osteodysgenesis, Multisynostotic Osteodysgenesis with Long Bone Fractures, Mulvihill-Smith Syndrome, MURCS Association, Murk Jansen Type Metaphyseal Chondrodysplasia, Muscle Carnitine Deficiency, Muscle Core Disease, Muscle Phosphofructokinase Deficiency, Muscular Central Core Disease, Muscular Dystrophy, Muscular Dystrophy Classic X-linked Recessive, Muscular Dystrophy Congenital With Central Nervous System Involvement, Muscular Dystrophy Congenital Progressive with Mental Retardation, Muscular Dystrophy Facioscapulohumeral, Muscular Rheumatism, Muscular Rigidity - Progressive Spasm, Musculoskeletal Pain Syndrome, Mutilating Acropathy, Mutilating Acropathy, Mutism, mvp, MVP, MWS, Myasthenia Gravis, Myasthenia Gravis, Myasthenia Gravis Pseudoparalytica, Myasthenic Syndrome of Lambert-Eaton, Myelinoclastic Diffuse Sclerosis, Myelomatosis, Myhre Syndrome, Myoclonic Astatic Petit Mal Epilepsy, Myoclonic Dystonia, Myoclonic Encephalopathy of Infants, Myoclonic Epilepsy, Myoclonic Epilepsy Hartung Type, Myoclonus Epilepsy Associated with Ragged Red Fibers, Myoclonic Epilepsy and Ragged-Red Fiber Disease, Myoclonic Progressive Familial Epilepsy, Myoclonic Progressice Familial Epilepsy, Myoclonic Seizure, Myoclonus, Myoclonus Epilepsy, Myoencephalopathy Ragged-Red Fiber Disease, Myofibromatosis, Myofibromatosis Congenital, Myogenic Facio-ScapuloPeroneal Syndrome, Myoneurogastointestinal Disorder and Encephalopathy, Myopathic Arthrogryposis Multiplex Congenita, Myopathic Camitine Deficiency, myopathy Central Fibrillar, myopathy Congenital Nonprogressive, myopathy Congenital Nonprogressive with Central Axis, myopathy with Deficiency of Camitine Palmitoyltransferase, myopathy-Marinesco-Sjogren Syndrome, myopathy-Metabolic Carnitine Palmitoyltransderase Deficiency, myopathy Mitochondrial-Encephalopathy-Lactic Acidosis-Stroke, myopathy with Sarcoplasmic Bodies and Intermediate Filaments, Myophosphorylase Deficiency, Myositis Ossificans Progressiv, Myotonia Atrophica, Myotonia Congenita, Myotonia Congenita Intermittens, Myotonic Dystrophy, Myotonic myopathy Dwarfism Chondrodystrophy Ocular and Facial Anomalies, Myotubular myopathy, Myotubular myopathy X-linked, Myproic Acid, Myriachit (Observed in Siberia), Myxedema, N-Acetylglucosamine-1-Phosphotransferase Deficiency, N-Acetyl Glutamate Synthetase Deficiency, NADH-CoQ reductasedeficiency, Naegeli Ectodermal Dysplasias, Nager Syndrome, Nager Acrofacial Dysostosis Syndrome, Nager Acrofacial Dysostosis Syndrome, Nager Syndrome, NAGS Deficiency, Nail Dystrophy-Deafness Syndrome, Nail Dysgenesis and Hypodontia, Nail-Patella Syndrome, Nance-Horan Syndrome, Nanocephalic Dwarfism, Nanocephaly, Nanophthalmia, Narcolepsy, Narcoleptic syndrome, NARP, Nasal-fronto-faciodysplasia, Nasal Alar Hypoplasia Hypothyroidism Pancreatic Achylia Congenital Deafness, Nasomaxillary Hypoplasia, Nasu Lipodystrophy, NBIA1, ND, NDI, NDP, Necrotizing Encephalomyelopathy of Leigh's, Necrotizing Respiratory Granulomatosis, Neill-Dingwall Syndrome, Nelson Syndrome, Nemaline myopathy, Neonatal Adrenoleukodystrophy (NALD), Neonatal Adrenoleukodystrophy (ALD), Neonatal Autosomal Recessive Polycystic Kidney Disease, Neonatal Dwarfism, Neonatal Hepatitis, Neonatal Hypoglycemia, Neonatal Lactose Intolerance, Neonatal Lymphedema due to Exudative Enteropathy, Neonatal Progeroid Syndrome, Neonatal Pseudo-Hydrocephalic Progeroid Syndrome of Wiedemann-Rautenstrauch, Neoplastic Arachnoiditis, Nephroblastom, Nephrogenic Diabetes Insipidus, Nephronophthesis Familial Juvenile, Nephronophthesis Familial Juvenile, Nephropathic Cystinosis, Nephropathy-Pseudohermaphroditism-Wilms Tumor, Nephrosis-Microcephaly Syndrome, Nephrosis-Neuronal Dysmigration Syndrome, Nephrotic-Glycosuric-Dwarfism-Rickets-Hypophosphatemic Syndrome, Netherton Disease, Netherton Syndrome, Netherton Syndrome Ichthyosis, Nettleship Falls Syndrome (X-Linked), Neu-Laxova Syndrome, Neuhauser Syndrome, Neural-tube defects, Neuralgic Amyotrophy, Neuralgic Amyotrophy, Neuraminidase Deficiency, Neuraocutaneous melanosis, Neurinoma of the Acoustic Nerve, Neurinoma, Neuroacanthocytosis, Neuroaxonal Dystrophy Schindler Type, Neurodegeneration with brain iron accumulation type 1 (NBIA1), Neuro fibroma of the Acoustic Nerve, Neurogenic Arthrogryposis Multiplex Congenita, Neuromyelitis Optica, Neuromyotonia, Focal, Neuromyotonia, Generalized, Familial, Neuromytonia, Generalized, Sporadic, Neuronal Axonal Dystrophy Schindler Type, Neuronal Ceroid Lipofuscinosis Adult Type, Neuronal Ceroid Lipofuscinosis Juvenile Type, Neuronal Ceroid Lipofuscinosis Type 1, Neuronopathic Acute Gaucher Disease, Neuropathic Amyloidosis, Neuropathic Beriberi, Neuropathy Ataxia and Retinitis Pigmentosa, Neuropathy of Brachialpelxus Syndrome, Neuropathy Hereditary Sensory Type I, Neuropathy Hereditary Sensory Type II, Neutral Lipid Storage Disease, Nevii, Nevoid Basal Cell Carcinoma Syndrome, Nevus, Nevus Cavernosus, Nevus Comedonicus, Nevus Depigmentosus, Nevus Sebaceous of Jadassohn, Nezelof's Syndrome, Nezelof's Thymic Aplasia, Nezelof Type Severe Combined Immunodeficiency, NF, NF1, NF2, NF-1, NF-2, NHS, Niemann Pick Disease, Nieman Pick disease Type A (acute neuronopathic form), Nieman Pick disease Type B, Nieman Pick Disease Type C (chronic neuronopathic form), Nieman Pick disease Type D (Nova Scotia variant), Nieman Pick disease Type E, Nieman Pick disease Type F (sea-blue histiocyte disease), Night Blindness, Nigrospinodentatal Degeneration, Niikawakuroki Syndrome, NLS, NM, Noack Syndrome Type I, Nocturnal Myoclonus Hereditary Essential Myoclonus, Nodular Cornea Degeneration, Non-Bullous CIE, Non-Bullous Congenital Ichthyosiform Erythroderma, Non-Communicating Hydrocephalus, Non-Deletion Type Alpha-Thalassemia / Mental Retardation syndrome, Non-Ketonic Hyperglycinemia Type I (NKHI), Non-Ketotic Hyperglycinemia, Non-Lipid Reticuloendotheliosis, Non-Neuronopathic Chronic Adult Gaucher Disease, Non-Scarring Epidermolysis Bullosa, Nonarteriosclerotic Cerebral Calcifications, Nonarticular Rheumatism, Noncerebral, Juvenile Gaucher Disease, Nondiabetic Glycosuria, Nonischemic Cardio myopathy, Nonketotic Hypoglycemia and Carnitine Deficiency due to MCAD Deficiency, Nonketotic Hypoglycemia Caused by Deficiency of Acyl-CoA Dehydrogenase, Nonketotic Glycinemia, Nonne's Syndrome, Nonne-Milroy-Meige Syndrome, Nonopalescent Opalescent Dentine, Nonpuerperal Galactorrhea-Amenorrhea, Nonsecretory Myeloma, Nonspherocytic Hemolytic Anemia, Nontropical Sprue, Noonan Syndrome, Norepinephrine, Normal Pressure Hydrocephalus, Norman-Roberts Syndrome, Norrbottnian Gaucher Disease, Norrie Disease, Norwegian Type Hereditary Cholestasis, NPD, NPS, NS, NSA, Nuchal Dystonia Dementia Syndrome, Nutritional Neuropathy, Nyhan Syndrome, OAV Spectrum, Obstructive Apnea, Obstructive Hydrocephalus, Obstructive Sleep Apnea, OCC Syndrome, Occlusive Thromboaortopathy, OCCS, Occult Intracranial Vascular Malformations, Occult Spinal Dysraphism Sequence, Ochoa Syndrome, Ochronosis, Ochronotic Arthritis, OCR, OCRL, Octocephaly, Ocular Albinism, Ocular Herpes, Ocular Myasthenia Gravis, Oculo-Auriculo-Vertebral Dysplasia, Oculo-Auriculo-Vertebral Spectrum, Oculo-Bucco-Genital Syndrome, Oculocerebral Syndrome with Hypopigmentation, Oculocerebrocutaneous Syndrome, Oculo-Cerebro-Renal, Oculocerebrorenal Dystrophy, Oculocerebrorenal Syndrome, Oculocraniosomatic Syndrome (obsolete), Oculocutaneous Albinism, Oculocutaneous Albinism Chediak-Higashi Type, Oculo-Dento-Digital Dysplasia, Oculo-Dento-Digital Dysplasia, Oculodentodigital Syndrome, Oculo-Dento-Osseous Dysplasia, Oculo-Dento-Osseous Dysplasia, Oculo Gastrointestinal Muscular Dystrophy, Oculo Gastrointestinal Muscular Dystrophy, Oculogastrointestinal Muscular Dystrophy, Oculomandibulodyscephaly with hypotrichosis, Oculomandibulofacial Syndrome, Oculomotor with Congenital Contractures and Muscle Atrophy, Oculosympathetic Palsy, ODD Syndrome, ODD Syndrome, ODOD, Odontogenic Tumor, Odontotrichomelic Syndrome, OFD, OFD Syndrome, Ohio Type Amyloidosis (Type VII), OI, OI Congenita, OI Tarda, Oldfield Syndrome, Oligohydramnios Sequence, Oligophrenia Microphthalmos, Oligophrenic Polydystrophy, Olivopontocerebellar Atrophy, Olivopontocerebellar Atrophy, Olivopontocerebellar Atrophy with Dementia and Extrapyramidal Signs, Olivopontocerebellar Atrophy with Retinal Degeneration, Olivopontocerebellar Atrophy I, Olivopontocerebellar Atrophy II, Olivopontocerebellar Atrophy III, Olivopontocerebellar Atrophy IV, Olivopontocerebellar Atrophy V, Ollier Disease, Ollier Osteochondromatosis, Omphalocele-Visceromegaly-Macroglossia Syndrome, Ondine's Curse, Onion-Bulb Neuropathy, Onion Bulb Polyneuropathy, Onychoosteodysplasia, Onychotrichodysplasia with Neutropenia, OPCA, OPCA I, OPCA II, OPCA III, OPCA IV, OPCA V, OPD Syndrome, OPD Syndrome Type I, OPD Syndrome Type II, OPD I Syndrome, OPD II Syndrome, Ophthalmoarthropathy, Ophthalmoplegia-Intestinal Pseudoobstruction, Ophthalmoplegia, Pigmentary Degeneration of the Retina and Cadio myopathy, Ophthalmoplegia Plus Syndrome, Ophthalmoplegia Syndrome, Opitz BBB Syndrome, Opitz BBB/G Compound Syndrome, Opitz BBBG Syndrome, Opitz-Frias Syndrome, Opitz G Syndrome, Opitz G/BBB Syndrome, Opitz Hypertelorism-Hypospadias Syndrome, Opitz-Kaveggia Syndrome, Opitz Oculogenitolaryngeal Syndrome, Opitz Trigonocephaly Syndrome, Opitz Syndrome, Opsoclonus, Opsoclonus-Myoclonus, Opthalmoneuromyelitis, Optic Atrophy Polyneuropathy and Deafness, Optic Neuroencephalomyelopathy, Optic Neuromyelitis, Opticomyelitis, Optochiasmatic Arachnoiditis, Oral-Facial Clefts, Oral-facial Dyskinesia, Oral Facial Dystonia, Oral-Facial-Digital Syndrome, Oral-Facial-Digital Syndrome Type I, Oral-Facial-Digital Syndrome II, Oral-Facial-Digital Syndrome III, Oral-Facial-Digital Syndrome IV, Orbital Cyst with Cerebral and Focal Dermal Malformations, Ornithine Carbamyl Transferase Deficiency, Ornithine Transcarbamylase Deficiency, Orocraniodigital Syndrome, Orofaciodigital Syndrome, Oromandibular Dystonia, Orthostatic Hypotension, Osler-Weber-Rendu disease, Osseous-Oculo-Dento Dysplasia, Osseous-Oculo-Dento Dysplasia, Osteitis deformans, Osteochondrodystrophy Deformans, Osteochondroplasia, Osteodysplasty of Melnick and Needles, Osteogenesis Imperfect, Osteogenesis Imperfecta, Osteogenesis Imperfecta Congenita, Osteogenesis Imperfecta Tarda, Osteohypertrophic Nevus Flammeus, Osteopathia Hyperostotica Scleroticans Multiplex Infantalis, Osteopathia Hyperostotica Scleroticans Multiplex Infantalis, Osteopathyrosis, Osteopetrosis, Osteopetrosis Autosomal Dominant Adult Type, Osteopetrosis Autosomal Recessive Malignant Infantile Type, Osteopetrosis Mild Autosomal Recessive Intermediate Typ, Osteosclerosis Fragilis Generalisata, Osteosclerotic Myeloma, Ostium Primum Defect (endocardial cushion defects included), Ostium Secundum Defect, OTC Deficiency, Oto Palato Digital Syndrome, Oto-Palato-Digital Syndrome Type I, Oto-Palatal-Digital Syndrome Type II, Otodental Dysplasia, Otopalatodigital Syndrome, Otopalataldigital Syndrome Type II, Oudtshoorn Skin, Ovarian Dwarfism Turner Type, Ovary Aplasia Turner Type, OWR, Oxalosis, Oxidase deficiency, Oxycephaly, Oxycephaly, Oxycephaly-Acrocephaly, P-V, PA, PAC, Pachyonychia Ichtyosiforme, Pachyonychia Congenita with Natal Teeth, Pachyonychia Congenita, Pachyonychia Congenita Keratosis Disseminata Circumscripta (follicularis), Pachyonychia Congenita Jadassohn-Lewandowsky Type, PAF with MSA, Paget's Disease, Paget's Disease of Bone, Paget's Disease of the Breast, Paget's Disease of the Nipple, Paget's Disease of the Nipple and Areola, Pagon Syndrome, Painful Ophthalmoplegia, PAIS, Palatal Myoclonus, Palato-Oto-Digital Syndrome, Palatal-Oto-Digital Syndrome Type I, Palatal-Oto-Digital SyndromeType II, Pallister Syndrome, Pallister-Hall Syndrome, Pallister-Killian Mosaic Syndrome, Pallister Mosaic Aneuploidy, Pallister Mosaic Syndrome, Pallister Mosaic Syndrome Tetrasomy 12p, Pallister-W Syndrome, Palmoplantar Hyperkeratosis and Alopecia, Palsy, Pancreatic Fibrosis, Pancreatic Insufficiency and Bone Marrow Dysfunction, Pancreatic Ulcerogenic Tumor Syndrome, Panmyelophthisis, Panmyelopathy, Pantothenate kinase associated neurodegeneration (PKAN), Papillon-Lefevre Syndrome, Papillotonic Psuedotabes, Paralysis Periodica Paramyotonica, Paralytic Beriberi, Paralytic Brachial Neuritis, Paramedian Lower Lip Pits-Popliteal Pyerygium Syndrome, Paramedian Diencephalic Syndrome, Paramyeloidosis, Paramyoclonus Multiple, Paramyotonia Congenita, Paramyotonia Congenita of Von Eulenburg, Parkinson's disease, Paroxysmal Atrial Tachycardia, Paroxysmal Cold Hemoglobinuria, Paroxysmal Dystonia, Paroxysmal Dystonia Choreathetosis, Paroxysmal Kinesigenic Dystonia, Paroxysmal Nocturnal Hemoglobinuria, Paroxysmal Normal Hemoglobinuria, Paroxysmal Sleep, Parrot Syndrome, Parry Disease, Parry-Romberg Syndrome, Parsonage-Turner Syndrome, Partial Androgen Insensitivity Syndrome, Partial Deletion of the Short Arm of Chromosome 4, Partial Deletion of the Short Arm of Chromosome 5, Partial Deletion of Short Arm of Chromosome 9, Partial Duplication 3q Syndrome, Partial Duplication 15q Syndrome, Partial Facial Palsy With Urinary Abnormalities, Partial Gigantism of Hands and Feet- Nevi-Hemihypertrophy-Macrocephaly, Partial Lipodystrophy, Partial Monosomy of Long Arm of Chromosome 11, Partial Monosomy of the Long Arm of Chromosome 13, Partial Spinal Sensory Syndrome, Partial Trisomy 11q, Partington Syndrome, PAT, Patent Ductus Arteriosus, Pathological Myoclonus, Pauciarticular-Onset Juvenile Arthritis, Pauciarticular-Onset Juvenile Arthritis, Paulitis, PBC, PBS, PC Deficiency, PC Deficiency Group A, PC Deficiency Group B, PC, Eulenburg Disease, PCC Deficiency, PCH, PCLD, PCT, PD, PDA, PDH Deficiency, Pearson Syndrome Pyruvate Carboxylase Deficiency, Pediatric Obstructive Sleep Apnea, Peeling Skin Syndrome, Pelizaeus-Merzbacher Disease, Pelizaeus-Merzbacher Brain Sclerosis, Pelizaeus-Merzbacher Brain Sclerosis, Pellagra-Cerebellar Ataxia-Renal Aminoaciduria Syndrome, Pelvic Pain Syndrome, Pemphigus Vulgaris, Pena Shokeir II Syndrome, Pena Shokeir Syndrome Type II, Penile Fibromatosis, Penile Fibrosis, Penile Induration, Penta X Syndrome, Pentalogy of Cantrell, Pentalogy Syndrome, Pentasomy X, PEPCK Deficiency, Pepper Syndrome, Perheentupa Syndrome, Periarticular Fibrositis, Pericardial Constriction with Growth Failure, Pericollagen Amyloidosis, Perinatal Polycystic Kidney Diseases, Perineal Anus, Periodic Amyloid Syndrome, Periodic Peritonitis Syndrome, Periodic Somnolence and Morbid Hunger, Periodic Syndrome, Peripheral Cystoid Degeneration of the Retina, Peripheral Dysostosis-Nasal Hypoplasia-Mental Retardation, Peripheral Neuritis, Peripheral Neuropathy, Peritoneopericardial Diaphragmatic Hernia, Pernicious Anemia, Pernicious Anemia, Pernicious Anemia, Peromelia with Micrognathia, Peroneal Muscular Atrophy, Peroneal Nerve Palsy, Peroutka Sneeze, Peroxisomal Acyl-CoA Oxidase, Peroxisomal Beta-Oxidation Disorders, Peroxisomal Bifunctional Enzyme, Peroxisomal Thiolase, Peroxisomal Thiolase Deficiency, Persistent Truncus Arteriosus, Perthes Disease, Petit Mal Epilepsy, Petit Mal Variant, Peutz-Jeghers Syndrome, Peutz-Jeghers Syndrome, Peutz-Touraine Syndrome, Peutz-Touraine Syndrome, Peyronie Disease, Pfeiffer, Pfeiffer Syndrome Type I, PGA I, PGA II, PGA III, PGK, PH Type I, PH Type I, Pharyngeal Pouch Syndrome, PHD Short-Chain Acyl-CoA Dehydrogenase Deficiency, Phenylalanine Hydroxylase Deficiency, Phenylalaninemia, Phenylketonuria, Phenylketonuria, Phenylpyruvic Oligophrenia, Phocomelia, Phocomelia Syndrome, Phosphoenolpyruvate Carboxykinase Deficiency, Phosphofructokinase Deficiency, Phosphoglycerate Kinase Deficiency, Phosphoglycerokinase, Phosphorylase 6 Kinase Deficiency, Phosphorylase Deficiency Glycogen Storage Disease, Phosphorylase Kinase Deficiency of Liver, Photic Sneeze Reflex, Photic Sneezing, Phototherapeutic keratectomy, PHS, Physicist John Dalton, Phytanic Acid Storage Disease, Pi Phenotype ZZ, PI, Pick Disease of the Brain, Pick's Disease, Pick's Disease, Pickwickian Syndrome, Pierre Robin Anomalad, Pierre Robin Complex, Pierre Robin Sequence, Pierre Robin Syndrome, Pierre Robin Syndrome with Hyperphalangy and Clinodactyly, Pierre-Marie's Disease, Pigmentary Degeneration of Globus Pallidus Substantia Nigra Red Nucleus, Pili Torti and Nerve Deafness, Pili Torti-Sensorineural Hearing Loss, Pituitary Dwarfism II, Pituitary Tumor after Adrenalectomy, Pityriasis Pilaris, Pityriasis Rubra Pilaris, PJS, PJS, PKAN, PKD, PKD1, PKD2, PKD3, PKU, PKU1, Plagiocephaly, Plasma Cell Myeloma, Plasma Cell Leukemia, Plasma Thromboplastin Component Deficiency, Plasma Transglutaminase Deficiency, Plastic Induration Corpora Cavernosa, Plastic Induration of the Penis, PLD, Plicated Tongue, PLS, PMD, Pneumorenal Syndrome, PNH, PNM, PNP Deficiency, POD, POH, Poikiloderma Atrophicans and Cataract, Poikiloderma Congenitale, Poland Anomaly, Poland Sequence, Poland Syndactyly, Poland Syndrome, Poliodystrophia Cerebri Progressiva, Polyarthritis Enterica, Polyarteritis Nodosa, Polyarticular-Onset Juvenile Arthritis Type I, Polyarticular-Onset Juvenile Arthritis Type II, Polyarticular-Onset Juvenile Arthritis Types I and II, Polychondritis, Polycystic Kidney Disease, Polycystic Kidney Disease Medullary Type, Polycystic Kidney Disease Medullary Type, Polycystic Liver Disease, Polycystic Ovary Disease, Polycystic Renal Diseases, Polydactyly-Joubert Syndrome, Polydysplastic Epidermolysis Bullosa, Polydystrophia Oligophrenia, Polydystrophic Dwarfism, Polyglandular Autoimmune Syndrome Type III, Polyglandular Autoimmune Syndrome Type II, Polyglandular Autoimmune Syndrome Type I, Polyglandular Autoimmune Syndrome Type II, Polyglandular Deficiency Syndrome Type II, Polyglandular Syndromes, Polymorphic Macula Lutea Degeneration, Polymorphic Macular Degeneration, Polymorphism of Platelet Glycoprotien Ib, Polymorphous Corneal Dystrophy Hereditary, Polymyalgia Rheumatica, Polymyalgia Rheumatica, Polymyositis and Dermatomyositis,Primary Agammag-lobulinemi, Polyneuritis Peripheral, Polyneuropathy-Deafness-Optic Atrophy, Polyneuropathy Peripheral, Polyneuropathy and Polyradiculoneuropathy, Polyostotic Fibrous Dysplasia, Polyostotic Sclerosing Histiocytosis, Polyposis Familial, Polyposis Gardner Type, Polyposis Hamartomatous Intestinal, Polyposis Hamartomatous Intestinal, Polyposis-Osteomatosis-Epidermoid Cyst Syndrome, Polyposis Skin Pigmentation Alopecia and Fingernail Changes, Polyps and Spots Syndrome, Polyps and Spots Syndrome, Polyserositis Recurrent, Polysomy Y, Polysyndactyly with Peculiar Skull Shape, Polysyndactyly-Dysmorphic Craniofacies Greig Type, Pompe Disease, Pompe Disease, Popliteal Pterygium Syndrome, Porcupine Man, Porencephaly, Porencephaly, Porphobilinogen deaminase (PBG-D), Porphyria, Porphyria Acute Intermittant, Porphyria Acute Intermittent, Porphyria ALA-D, Porphyria Cutanea Tarda, Porphyria Cutanea Tarda, Porphyria Cutanea Tarda Hereditaria, Porphyria Cutanea Tarda Symptomatica, Porphyria Hepatica Variegate, Porphyria Swedish Type, Porphyria Variegate, Porphyriam Acute Intermittent, Porphyrins, Porrigo Decalvans, Port Wine Stains, Portuguese Type Amyloidosis, Post-Infective Polyneuritis, Postanoxic Intention Myoclonus, Postaxial Acrofacial Dysostosis, Postaxial Polydactyly, Postencephalitic Intention Myoclonus, Posterior Corneal Dystrophy Hereditary, Posterior Thalamic Syndrome, Postmyelographic Arachnoiditis, Postnatal Cerebral Palsy, Postoperative Cholestasis, Postpartum Galactorrhea-Amenorrhea Syndrome, Postpartum Hypopituitarism, Postpartum Panhypopituitary Syndrome, Postpartum Panhypopituitarism, Postpartum Pituitary Necrosis, Postural Hypotension, Potassium-Losing Nephritis, Potassium Loss Syndrome, Potter Type I Infantile Polycystic Kidney Diseases, Potter Type III Polycystic Kidney Disease, PPH, PPS, Prader-Willi Syndrome, Prader-Labhart-Willi Fancone Syndrome, Prealbumin Tyr-77 Amyloidosis, Preexcitation Syndrome, Preexcitation Syndrome, Pregnenolone Deficiency, Premature Atrial Contractions, Premature Senility Syndrome, Premature Supraventricular Contractions, Premature Ventricular Complexes, Prenatal or Connatal Neuroaxonal Dystrophy, Presenile Dementia, Presenile Macula Lutea Retinae Degeneration, Primary Adrenal Insufficiency, Primary Agammaglobulinemias, Primary Aldosteronism, Primary Alveolar Hypoventilation, Primary Amyloidosis, Primary Anemia, Primary Anemia, Primary Beriberi, Primary Biliary, Primary Biliary Cirrhosis, Primary Brown Syndrome, Primary Camitine Deficiency, Primary Central Hypoventilation Syndrome, Primary Ciliary Dyskinesia Kartagener Type, Primary Cutaneous Amyloidosis, Primary Dystonia, Primary Failure Adrenocortical Insufficiency, Primary Familial Hypoplasia of the Maxilla, Primary Hemochromatosis, Primary Hyperhidrosis, Primary Hyperoxaluria [Type I], Primary Hyperoxaluria Type 1 (PH1), Primary Hyperoxaluria Type 1, Primary Hyperoxaluria Type II, Primary Hyperoxaluria Type III, Primary Hypogonadism, Primary Intestinal Lymphangiectasia, Primary Lateral Sclerosis, Primary Nonhereditary Amyloidosis, Primary Obliterative Pulmonary Vascular Disease, Primary Progressive Multiple Sclerosis, Primary Pulmonary Hypertension, Primary Reading Disability, Primary Renal Glycosuria, Primary Sclerosing Cholangitis, Primary Thrombocythemia, Primary Tumors of Central Nervous System, Primary Visual Agnosia, Proctocolitis Idiopathic, Proctocolitis Idiopathic, Progeria of Adulthood, Progeria of Childhood, Progeroid Nanism, Progeriod Short Stature with Pigmented Nevi, Progeroid Syndrome of De Barsy, Progressive Autonomic Failure with Multiple System Atrophy, Progressive Bulbar Palsy, Progressive Bulbar Palsy Included, Progressive Cardiomyopathic Lentiginosis, Progressive Cerebellar Ataxia Familial, Progressive Cerebral Poliodystrophy, Progressive Choroidal Atrophy, Progressive Diaphyseal Dysplasia, Progressive Facial Hemiatrophy, Progressive Familial Myoclonic Epilepsy, Progressive Hemifacial Atrophy, Progressive Hypoerythemia, Progressive Infantile Poliodystrophy, Progressive Lenticular Degeneration, Progressive Lipodystrophy, Progressive Muscular Dystrophy of Childhood, Progressive Myoclonic Epilepsy, Progressive Osseous Heteroplasia, Progressive Pallid Degeneration Syndrome, Progressive Pallid Degeneration Syndrome, Progressive Spinobulbar Muscular Atrophy, Progressive Supranuclear Palsy, Progressive Systemic Sclerosis, Progressive Tapetochoroidal Dystrophy, Proline Oxidase Deficiency, Propionic Acidemia, Propionic Acidemia, Propionic Acidemia Type I (PCCA Deficiency), Propionic Acidemia Type II (PCCB Deficiency), Propionyl CoA Carboxylase Deficiency, Propionyl CoA Carboxylase Deficiency, Protanomaly, Protanopia, Protein-Losing Enteropathy Secondary to Congestive Heart Failure, Proteus Syndrome, Proximal Deletion of 4q Included, Proximal Deletion of 4q-Included, PRP, PRS, Prune Belly Syndrome, PS, Pseudo-Hurler Polydystrophy, Pseudo-Polydystrophy, Pseudoacanthosis Nigricans, Pseudoachondroplasia, Pseudocholinesterase Deficiency, Pseudogout Familial, Pseudohemophilia, Pseudohermaphroditism, Pseudohermaphroditism-Nephron Disorder-Wilm's Tumor, Pseudohypertrophic Muscular Dystrophy, Pseudohypoparathyroidism, Pseudohypophosphatasia, Pseudopolydystrophy, Pseudothalidomide Syndrome, Pseudoxanthoma Elasticum, Psoriasis, Psorospermosis Follicularis, PSP, PSS, Psychomotor Convulsion, Psychomotor Epilepsy, Psychomotor Equivalent Epilepsy, PTC Deficiency, Pterygium, Pterygium Colli Syndrome, Pterygium Universale, Pterygolymphangiectasia, Pulmonary Atresia, Pulmonary Lymphangiomyomatosis, Pulmonary Stenosis, Pulmonic Stenosis-Ventricular Septal Defect, Pulp Stones, Pulpal Dysplasia, Pulseless Disease, Pure Alymphocytosis, Pure Cutaneous Histiocytosis, Purine Nucleoside Phosphorylase Deficiency, Purpura Hemorrhagica, Purtilo Syndrome, PXE, PXE Dominant Type, PXE Recessive Type, Pycnodysostosis, Pyknodysostosis, Pyknoepilepsy, Pyroglutamic Aciduria, Pyroglutamicaciduria, Pyrroline Carboxylate Dehydrogenase Deficiency, Pyruvate Carboxylase Deficiency, Pyruvate Carboxylase Deficiency Group A, Pyruvate Carboxylase Deficiency Group B, Pyruvate Dehydrogenase Deficiency, Pyruvate Dehydrogenase Deficiency, Pyruvate Dehydrogenase Deficiency, Pyruvate Kinase Deficiency, q25-qter, q26 or q27-qter, q31 or 32-qter, QT Prolongation with Extracellular Hypohypocalcinemia, QT Prolongation without Congenital Deafness, QT Prolonged with Congenital Deafness, Quadriparesis of Cerebral Palsy, Quadriplegia of Cerebral Palsy, Quantal Squander, Quantal Squander, r4, r6, r14, r 18, r21, r22, Rachischisis Posterior, Radial Aplasia-Amegakaryocytic Thrombocytopenia, Radial Aplasia-Thrombocytopenia Syndrome, Radial Nerve Palsy, Radicular Neuropathy Sensory, Radicular Neuropathy Sensory Recessive, Radicular Dentin Dysplasia, Rapid-onset Dystonia-parkinsonism, Rapp-Hodgkin Syndrome, Rapp-Hodgkin (hypohidrotic) Ectodermal Dysplasia syndrome, Rapp-Hodgkin Hypohidrotic Ectodermal Dysplasias, Rare hereditary ataxia with polyneuritic changes and deafness caused by a defect in the enzyme phytanic acid hydroxylase, Rautenstrauch-Wiedemann Syndrome, Rautenstrauch-Wiedemann Type Neonatal Progeria, Raynaud's Phenomenon, RDP, Reactive Functional Hypoglycemia, Reactive Hypoglycemia Secondary to Mild Diabetes, Recessive Type Kenny-Caffe Syndrome, Recklin Recessive Type Myotonia Congenita, Recklinghausen Disease, Rectoperineal Fistula, Recurrent Vomiting, Reflex Neurovascular Dystrophy, Reflex Sympathetic Dystrophy Syndrome, Refractive Errors, Refractory Anemia, Refrigeration Palsy, Refsum Disease, Refsum's Disease, Regional Enteritis, Reid-Barlow's syndrome, Reifenstein Syndrome, Reifenstein Syndrome, Reiger Anomaly-Growth Retardation, Reiger Syndrome, Reimann Periodic Disease, Reimann's Syndrome, Reis-Bucklers Corneal Dystrophy, Reiter's Syndrome, Reiter's Syndrome, Relapsing Guillain-Barre Syndrome, Relapsing-Remitting Multiple Sclerosis, Renal Agenesis Renal Dysplasia-Blindness Hereditary, Renal Dysplasia-Retinal Aplasia Loken-Senior Type, Renal Glycosuria, Renal Glycosuria Type A, Renal Glycosuria Type B, Renal Glycosuria Type O, Renal-Oculocerebrodystrophy, Renal-Retinal Dysplasia with Medullary Cystic Disease, Renal-Retinal Dysplasia with Medullary Cystic Disease, Renal-Retinal Dystrophy Familial, Renal-Retinal Syndrome, Rendu-Osler-Weber Syndrome, Respiratory Acidosis, Respiratory Chain Disorders, Respiratory Myoclonus, Restless Legs Syndrome, Restrictive Cardio myopathy, Retention Hyperlipemia, Rethore Syndrome (obsolete), Reticular Dysgenesis, Retinal Aplastic-Cystic Kidneys-Joubert Syndrome, Retinal Cone Degeneration, Retinal Cone Dystrophy, Retinal Cone-Rod Dystrophy, Retinitis Pigmentosa, Retinitis Pigmentosa and Congenital Deafness, Retinoblastoma, Retinol Deficiency, Retinoschisis, Retinoschisis Juvenile, Retraction Syndrome, Retrobulbar Neuropathy, Retrolenticular Syndrome, Rett Syndrome, Reverse Coarction, Reye Syndrome, Reye's Syndrome, RGS, Rh Blood Factors, Rh Disease, Rh Factor Incompatibility, Rh Incompatibility, Rhesus Incompatibility, Rheumatic Fever, Rheumatoid Arthritis, Rheumatoid Myositis, Rhinosinusogenic Cerebral Arachnoiditis, Rhizomelic Chondrodysplasia Punctata (RCDP), Acatalasemia,Classical Refsum disease, RHS, Rhythmical Myoclonus, Rib Gap Defects with Micrognathia, Ribbing Disease (obsolete), Ribbing Disease, Richner-Hanhart Syndrome, Rieger Syndrome, Rieter's Syndrome, Right Ventricular Fibrosis, Riley-Day Syndrome, Riley-Smith syndrome, Ring Chromosome 14, Ring Chromosome 18, Ring 4, Ring 4 Chromosome, Ring 6, Ring 6 Chromosome, Ring 9, Ring 9 Chromosome R9, Ring 14, Ring 15, Ring 15 Chromosome (mosaic pattern), Ring 18, Ring Chromosome 18, Ring 21, Ring 21 Chromosome, Ring 22, Ring 22 Chromosome, Ritter Disease, Ritter-Lyell Syndrome, RLS, RMSS, Roberts SC-Phocomelia Syndrome, Roberts Syndrome, Roberts Tetraphocomelia Syndrome, Robertson's Ectodermal Dysplasias, Robin Anomalad, Robin Sequence, Robin Syndrome, Robinow Dwarfism, Robinow Syndrome, Robinow Syndrome Dominant Form, Robinow Syndrome Recessive Form, Rod myopathy, Roger Disease, Rokitansky's Disease, Romano-Ward Syndrome, Romberg Syndrome, Rootless Teeth, Rosenberg-Chutorian Syndrome, Rosewater Syndrome, Rosewater Syndrome, Rosselli-Gulienatti Syndrome, Rothmund-Thomson Syndrome, Roussy-Levy Syndrome, RP, RS X-Linked, RS, RS, RSDS, RSH Syndrome, RSS, RSTS, RTS, Rubella Congenital, Rubinstein Syndrome, Rubinstein-Taybi Syndrome, Rubinstein Taybi Broad Thumb-Hallux syndrome, Rufous Albinism, Ruhr's Syndrome, Russell's Diencephalic Cachexia, Russell's Syndrome, Russell Syndrome, Russell-Silver Dwarfism, Russell-Silver Syndrome, Russell-Silver Syndrome X-linked, Ruvalcaba-Myhre-Smith syndrome (RMSS), Ruvalcaba Syndrome, Ruvalcaba Type Osseous Dysplasia with Mental Retardation, Sacral Regression, Sacral Agenesis Congenital, SAE, Saethre-Chotzen Syndrome, Sakati, Sakati Syndrome, Sakati-Nyhan Syndrome, Salaam Spasms, Salivosudoriparous Syndrome, Salzman Nodular Corneal Dystrophy, Sandhoff Disease, Sanfilippo Syndrome, Sanfilippo Type A, Sanfilippo Type B, Santavuori Disease, Santavuori-Haltia Disease, Sarcoid of Boeck, Sarcoidosis, Sathre-chotzen, Saturday Night Palsy, SBMA, SC Phocomelia Syndrome, SC Syndrome, SCA 3, SCAD Deficiency, SCAD Deficiency Adult-Onset Localized, SCAD Deficiency Congenital Generalized, SCAD, SCADH Deficiency, Scalded Skin Syndrome, Scalp Defect Congenital, Scaphocephaly, Scapula Elevata, Scapuloperoneal myopathy, Scapuloperoneal Muscular Dystrophy, Scapuloperoneal Syndrome Myopathic Type, Scarring Bullosa, Scarring Bullosa, SCHAD, Schaumann's Disease, Scheie Syndrome, Schereshevkii-Turner Syndrome, Schilder Disease, Schilder Encephalitis, Schilder's Disease, Schindler Disease Type I (Infantile Onset), Schindler Disease Infantile Onset, Schindler Disease, Schindler Disease Type II (Adult Onset), Schinzel Syndrome, Schinzel-Giedion Syndrome, Schinzel Acrocallosal Syndrome, Schinzel-Giedion Midface-Retraction Syndrome, Schizencephaly, Schmid Type Metaphyseal Chondrodysplasia, Schmid Metaphyseal Dysostosis, Schmid-Fraccaro Syndrome, Schmidt Syndrome, Schopf-Schultz-Passarge Syndrome, Schueller-Christian Disease, Schut-Haymaker Type, Schwartz-Jampel-Aberfeld Syndrome, Schwartz-Jampel Syndrome Types 1A and 1B, Schwartz-Jampel Syndrome, Schwartz-Jampel Syndrome Type 2, SCI, D SCID, Scleroderma, Scleroderma, Sclerosis Familial Progressive Systemic, Sclerosis Diffuse Familial Brain, Scott Craniodigital Syndrome With Mental Retardation, Scrotal Tongue, SCS, SD, SDS, SDYS, Seasonal Conjunctivitis, Sebaceous Nevus Syndrome, Sebaceous nevus, Seborrheic Keratosis, Seborrheic Warts, Seckel Syndrome, Seckel Type Dwarfism, Second Degree Congenital Heart Block, Secondary Amyloidosis, Secondary Blepharospasm, Secondary Non-tropical Sprue, Secondary Brown Syndrome, Secondary Beriberi, Secondary Generalized Amyloidosis, Secondary Dystonia, Secretory Component Deficiency, Secretory IgA Deficiency, SED Tarda, SED Congenital, SEDC, Segmental linear achromic nevus, Segmental Dystonia, Segmental Myoclonus, Seip Syndrome, Seitelberger Disease, Seizures, Selective Deficiency of IgG Subclasses, Selective Mutism, Selective Deficiency of IgG Subclass, Selective IgM Deficiency, Selective Mutism, Selective IgA Deficiency, Self-Healing Histiocytosis, Semilobar Holoprosencephaly, Seminiferous Tubule Dysgenesis, Senile Retinoschisis, Senile Warts, Senior-Loken Syndrome, Sensory Neuropathy Hereditary Type I, Sensory Neuropathy Hereditary Type II, Sensory Neuropathy Hereditary Type I, Sensory Radicular Neuropathy, Sensory Radicular Neuropathy Recessive, Septic Progressive Granulomatosis, Septo-Optic Dysplasia, Serous Circumscribed Meningitis, Serum Protease Inhibitor Deficiency, Serum Camosinase Deficiency, Setleis Syndrome, Severe Combined Immunodeficiency, Severe Combined Immunodeficiency with Adenosine Deaminase Deficiency, Severe Combined Immunodeficiency (SCID), Sex Reversal, Sexual Infantilism, SGB Syndrome, Sheehan Syndrome, Shields Type Dentinogenesis Imperfecta, Shingles, varicella-zoster virus, Ship Beriberi, SHORT Syndrome, Short Arm 18 Deletion Syndrome, Short Chain Acyl CoA Dehydrogenase Deficiency, Short Chain Acyl-CoA Dehydrogenase (SCAD) Deficiency, Short Stature and Facial Telangiectasis, Short Stature Facial/Skeletal Anomalies-Retardation-Macrodontia, Short Stature-Hyperextensibility-Rieger Anomaly-Teething Delay, Short Stature-Onychodysplasia, Short Stature Telangiectatic Erythema of the Face, SHORT Syndrome, Shoshin Beriberi, Shoulder girdle syndrome, Shprintzen-Goldberg Syndrome, Shulman Syndrome, Shwachman-Bodian Syndrome, Shwachman-Diamond Syndrome, Shwachman Syndrome, Shwachman-Diamond-Oski Syndrome, Shwachmann Syndrome, Shy Drager Syndrome, Shy-Magee Syndrome, SI Deficiency, Sialidase Deficiency, Sialidosis Type I Juvenile, Sialidosis Type II Infantile, Sialidosis, Sialolipidosis, Sick Sinus Syndrome, Sickle Cell Anemia, Sickle Cell Disease, Sickle Cell-Hemoglobin C Disease, Sickle Cell-Hemoglobin D Disease, Sickle Cell-Thalassemia Disease, Sickle Cell Trait, Sideroblastic Anemias, Sideroblastic Anemia, Sideroblastosis, Sideroblastosis, SIDS, Siegel-Cattan-Mamou Syndrome, Siemens-Bloch type Pigmented Dermatosis, Siemens Syndrome, Siewerling-Creutzfeldt Disease, Siewert Syndrome, Silver Syndrome, Silver-Russell Dwarfism, Silver-Russell Syndrome, Simmond's Disease, Simons Syndrome, Simplex Epidermolysis Bullosa, Simpson Dysmorphia Syndrome, Simpson-Golabi-Behmel Syndrome, Sinding-Larsen-Johansson Disease, Singleton-Merten Syndrome, Sinus Arrhythmia, Sinus Venosus, Sinus tachycardia, Sirenomelia Sequence, Sirenomelus, Situs Inversus Bronchiectasis and Sinusitis, SJA Syndrome, Sjogren Larsson Syndrome Ichthyosis, Sjogren Syndrome, Sjogren Larsson Syndrome Ichthyosis, Sjögren's Syndrome, SJS, Skeletal dysplasia, Skeletal Dysplasia Weismann Netter Stuhl Type, Skin Peeling Syndrome, Skin Neoplasms, Skull Asymmetry and Mild Retardation, Skull Asymmetry and Mild Syndactyly, SLE, Sleep Epilepsy, Sleep Apnea, SLO, Sly Syndrome, SMA, SMA Infantile Acute Form, SMA I, SMA III, SMA type I, SMA type II, SMA type III, SMA3, SMAXI, SMCR, Smith Lemli Opitz Syndrome, Smith Magenis Syndrome, Smith-Magenis Chromosome Region, Smith-McCort Dwarfism, Smith-Opitz-Inborn Syndrome, Smith Disease, Smoldering Myeloma, SMS, SNE, Sneezing From Light Exposure, Sodium valproate, Solitary Plasmacytoma of Bone, Sorsby Disease, Sotos Syndrome, Souques-Charcot Syndrome, South African Genetic Porphyria, Spasmodic Dysphonia, Spasmodic Torticollis, Spasmodic Wryneck, Spastic Cerebral Palsy, Spastic Colon, Spastic Dysphonia, Spastic Paraplegia, SPD Calcinosis, Specific Antibody Deficiency with Normal Immunoglobulins, Specific Reading Disability, SPH2, Spherocytic Anemia, Spherocytosis, Spherophakia-Brachymorphia Syndrome, Sphingomyelin Lipidosis, Sphingomyelinase Deficiency, Spider fingers, Spielmeyer-Vogt Disease, Spielmeyer-Vogt-Batten Syndrome, Spina Bifida, Spina Bifida, Spina Bifida Aperta, Spinal Arachnoiditis, Spinal Arteriovenous Malformation, Spinal Ataxia Hereditofamilial, Spinal and Bulbar Muscular Atrophy, Spinal Diffuse Idiopathic Skeletal Hyperostosis, Spinal DISH, Spinal Muscular Atrophy, Spinal Muscular Atrophy All Types, Spinal Muscular Atrophy Type ALS, Spinal Muscular Atrophy-Hypertrophy of the Calves, Spinal Muscular Atrophy Type I, Spinal Muscular Atrophy Type III, Spinal Muscular Atrophy type 3, Spinal Muscular Atrophy-Hypertrophy of the Calves, Spinal Ossifying Arachnoiditis, Spinal Stenosis, Spino Cerebellar Ataxia, Spinocerebellar Atrophy Type I, Spinocerebellar Ataxia Type I (SCA1), Spinocerebellar Ataxia Type II (SCAII), Spinocerebellar Ataxia Type III (SCAIII), Spinocerebellar Ataxia Type III (SCA 3), Spinocerebellar Ataxia Type IV (SCAIV), Spinocerebellar Ataxia Type V (SCAV), Spinocerebellar Ataxia Type VI (SCAVI), Spinocerebellar Ataxia Type VII (SCAVII), Spirochetal Jaundice, Splenic Agenesis Syndrome, Splenic Ptosis, Splenoptosis, Split Hand Deformity-Mandibulofacial Dysostosis, Split Hand Deformity-Mandibulofacial Dysostosis, Split Hand Deformity, Split-Hand Deformity, Spondyloarthritis, Spondylocostal Dysplasia - Type I, Spondyloepiphyseal Dysplasia Tarda, Spondylothoracic Dysplasia, Spondylotic Caudal Radiculopathy, Sponge Kidney, Spongioblastoma Multiforme, Spontaneous Hypoglycemia, Sprengel Deformity, Spring Ophthalmia, SRS, ST, Stale Fish Syndrome, Staphyloccal Scalded Skin Syndrome, Stargardt's Disease, Startle Disease, Status Epilepticus, Steele-Richardson-Olszewski Syndrome, Steely Hair Disease, Stein-Leventhal Syndrome, Steinert Disease, Stengel's Syndrome, Stengel-Batten-Mayou-Spielmeyer-Vogt-Stock Disease, Stenosing Cholangitis, Stenosis of the Lumbar Vertebral Canal, Stenosis, Steroid Sulfatase Deficiency, Stevanovic's Ectodermal Dysplasias, Stevens Johnson Syndrome, Stevens-Johnson Syndrome, STGD, Stickler Syndrome, Stickler Syndrome, Stiff-Man Syndrome, Stiff Person Syndrome, Still's Disease, Stilling-Turk-Duane Syndrome, Stillis Disease, Stimulus-Sensitive Myoclonus, Stone Man Syndrome, Stone Man, Streeter Anomaly, Striatonigral Degeneration Autosomal Dominant Type, Striopallidodentate Calcinosis, Stroma, Descemet's Membrane, Stromal Corneal Dystrophy, Struma Lymphomatosa, Sturge-Kalischer-Weber Syndrome, Sturge Weber Syndrome, Sturge-Weber Phakomatosis, Subacute Necrotizing Encephalomyelopathy, Subacute Spongiform Encephalopathy, Subacute Necrotizing Encephalopathy, Subacute Sarcoidosis, Subacute Neuronopathic, Subaortic Stenosis, Subcortical Arteriosclerotic Encephalopathy, Subendocardial Sclerosis, Succinylcholine Sensitivity, Sucrase-Isomaltase Deficiency Congenital, Sucrose-Isomaltose Malabsorption Congenital, Sucrose Intolerance Congenital, Sudanophilic Leukodystrophy ADL, Sudanophilic Leukodystrophy Pelizaeus-Merzbacher Type, Sudanophilic Leukodystrophy Included, Sudden Infant Death Syndrome, Sudeck's Atrophy, Sugio-Kajii Syndrome, Summerskill Syndrome, Summit Acrocephalosyndactyly, Summitt's Acrocephalosyndactyly, Summitt Syndrome, Superior Oblique Tendon Sheath Syndrome, Suprarenal glands, Supravalvular Aortic Stenosis, Supraventricular tachycardia, Surdicardiac Syndrome, Surdocardiac Syndrome, SVT, Sweat Gland Abscess, Sweating Gustatory Syndrome, Sweet Syndrome, Swiss Cheese Cartilage Syndrome, Syndactylic Oxycephaly, Syndactyly Type I with Microcephaly and Mental Retardation, Syndromatic Hepatic Ductular Hypoplasia, Syringomyelia, Systemic Aleukemic Reticuloendotheliosis, Systemic Amyloidosis, Systemic Carnitine Deficiency, Systemic Elastorrhexis, Systemic Lupus Erythematosus, Systemic Mast Cell Disease, Systemic Mastocytosis, Systemic-Onset Juvenile Arthritis, Systemic-Onset Juvenile Arthritis, Systemic Sclerosis, Systopic Spleen, T-Lymphocyte Deficiency, Tachyalimentation Hypoglycemia, Tachycardia, Takahara syndrome, Takayasu Disease, Takayasu Arteritis, Takayasu Arteritis, Talipes Calcaneus, Talipes Equinovarus, Talipes Equinus, Talipes Varus, Talipes Valgus, Tandem Spinal Stenosis, Tangier Disease, Tapetoretinal Degeneration, TAR Syndrome, Tardive Dystonia, Tardive Muscular Dystrophy, Tardive Dyskinesia, Tardive Oral Dyskinesia, Tardive Dyskinesia, Tardive Dystonia, Tardy Ulnar Palsy, Target Cell Anemia, Tarsomegaly, Tarui Disease, TAS Midline Defects Included, TAS Midline Defect, Tay Sachs Disease, Tay Sachs Sphingolipidosis, Tay Sachs Disease, Tay Syndrome Ichthyosis, Tay Sachs Sphingolipidosis, Tay Syndrome Ichthyosis, Taybi Syndrome Type I, Taybi Syndrome, TCD, TCOFI, TCS, TD, TDO Syndrome, TDO-I, TDO-II, TDO-III, Telangiectasis, Telecanthus with Associated Abnormalities, Telecanthus With Associated Abnormalities, Telecanthus-Hypospadias Syndrome, Temporal Lobe Epilepsy, Temporal Arteritis/Giant Cell Arteritis, Temporal Arteritis, TEN, Tendon Sheath Adherence Superior Obliqu, Tension Myalgia, Terminal Deletion of 4q Included, Terminal Deletion of 4q- Included, Terrian Corneal Dystrophy, Teschler-Nicola/Killian Syndrome, Tethered Spinal Cord Syndrome, Tethered Cord Malformation Sequence, Tethered Cord Syndrome, Tethered Cervical Spinal Cord Syndrome, Tetrahydrobiopterin Deficiencies, Tetrahydrobiopterin Deficiencies, Tetralogy of Fallot, Tetralogy of Fallot, Tetraphocomelia-Thrombocytopenia Syndrome, Tetrasomy Short Arm of Chromosome 9, Tetrasomy 9p, Tetrasomy Short Arm of Chromosome 18, Thalamic Syndrome, Thalamic Pain Syndrome, Thalamic Hyperesthetic Anesthesia, Thalassemia Intermedia, Thalassemia Minor, Thalassemia Major, Thiamine Deficiency, Thiamine-Responsive Maple Syrup Urine Disease, Thin-Basement-Membrane Nephropathy, Thiolase deficiency,RCDP,Acyl-CoA dihydroxyacetonephosphate acyltransferase, Third and Fourth Pharyngeal Pouch Syndrome, Third Degree Congenital (Complete) Heart Block, Thomsen Disease, Thoracic-Pelvic-Phalangeal Dystrophy, Thoracic Spinal Canal, Thoracoabdominal Syndrome, Thoracoabdominal Ectopia Cordis Syndrome, Three M Syndrome, Three-M Slender-Boned Nanism, Thrombasthenia of Glanzmann and Naegeli, Thrombocythemia Essential, Thrombocytopenia-Absent Radius Syndrome, Thrombocytopenia-Hemangioma Syndrome, Thrombocytopenia-Absent Radii Syndrome, Thrombophilia Hereditary Due to AT III, Thrombotic Thrombocytopenic Purpura, Thromboulcerative Colitis, Thymic Dysplasia with Normal Immunoglobulins, Thymic Agenesis, Thymic Aplasia DiGeorge Type, Thymic Hypoplasia Agammaglobulinemias Primary Included, Thymic Hypoplasia DiGeorge Type, Thymus Congenital Aplasia, Tic Douloureux, Tics, Tinel's syndrome, Tolosa Hunt Syndrome, Tonic Spasmodic Torticollis, Tonic Pupil Syndrome, Tooth and Nail Syndrome, Torch Infection, TORCH Syndrome, Torsion Dystonia, Torticollis, Total Lipodystrophy, Total anomalous pulmonary venous connection, Touraine's Aphthosis, Tourette Syndrome, Tourette's disorder, Townes-Brocks Syndrome, Townes Syndrome, Toxic Paralytic Anemia, Toxic Epidermal Necrolysis, Toxopachyosteose Diaphysaire Tibio-Peroniere, Toxopachyosteose, Toxoplasmosis Other Agents Rubella Cytomegalovirus Herpes Simplex, Tracheoesophageal Fistula with or without Esophageal Atresia, Tracheoesophageal Fistula, Transient neonatal myasthenia gravis, Transitional Atrioventricular Septal Defect, Transposition of the great arteries, Transtelephonic Monitoring, Transthyretin Methionine-30 Amyloidosis (Type I), Trapezoidocephaly-Multiple Synostosis Syndrome, Treacher Collins Syndrome, Treacher Collins-Franceschetti Syndrome 1, Trevor Disease, Triatrial Heart, Tricho-Dento-Osseous Syndrome, Trichodento Osseous Syndrome, Trichopoliodystrophy, Trichorhinophalangeal Syndrome, Trichorhinophalangeal Syndrome, Tricuspid atresia, Trifunctional Protein Deficiency, Trigeminal Neuralgia, Triglyceride Storage Disease Impaired Long-Chain Fatty Acid Oxidation, Trigonitis, Trigonocephaly, Trigonocephaly, Trigonocephaly, Trigonocephaly Syndrome, Trigonocephaly "C" Syndrome, Trimethylaminuria, Triphalangeal Thumbs-Hypoplastic Distal Phalanges-Onychodystrophy, Triphalangeal Thumb Syndrome, Triple Symptom Complex of Behcet, Triple X Syndrome, Triplo X Syndrome, Triploid Syndrome, Triploidy, Triploidy Syndrome, Trismus-Pseudocamptodactyly Syndrome, Trisomy, Trisomy G Syndrome, Trisomy X, Trisomy 6q Partial, Trisomy 6q Syndrome Partial, Trisomy 9 Mosaic, Trisomy 9P Syndrome (Partial) Included, Trisomy 11q Partial, Trisomy 14 Mosaic, Trisomy 14 Mosaicism Syndrome, Trisomy 21 Syndrome, Trisomy 22 Mosaic, Trisomy 22 Mosaicism Syndrome, TRPS, TRPS1, TRPS2, TRPS3, True Hermaphroditism, True Hermaphroditism, Truncus arteriosus, Tryptophan Malabsorption, Tryptophan Pyrrolase Deficiency, TS, TTP, TTTS, Tuberous Sclerosis, Tubular Ectasia, Turcot Syndrome, Turner Syndrome, Tumer-Kieser Syndrome, Turner Phenotype with Normal Chromosomes (Karyotype), Turner-Varny Syndrome, Turricephaly, Twin-Twin Transfusion Syndrome, Twin-to-Twin Transfusion Syndrome, Type A, Type B, Type AB, Type O, Type I Diabetes, Type I Familial Incomplete Male, Type I Familial Incomplete Male Pseudohermaphroditism, Type I Gaucher Disease, Type I (PCCA Deficiency), Type I Tyrosinemia, Type II Gaucher Disease, Type II Histiocytosis, Type II (PCCB Deficiency), Type II Tyrosinnemia, Type IIA Distal Arthrogryposis Multiplex Congenita, Type III Gaucher Disease, Type III Tyrosinemia, Type III Dentinogenesis Imperfecta, Typical Retinoschisis, Tyrosinase Negative Albinism (Type I), Tyrosinase Positive Albinism (Type II), Tyrosinemia type 1 acute form, Tyrosinemia type 1 chronic form, Tyrosinosis, UCE, Ulcerative Colitis, Ulcerative Colitis Chronic Non-Specific, Ulnar-Mammary Syndrome, Ulnar-Mammary Syndrome of Pallister, Ulnar Nerve Palsy, UMS, Unclassified FODs, Unconjugated Benign Bilirubinemiav, Underactivity of Parathyroid, Unilateral Ichthyosiform Erythroderma with Ipsilateral Malformations Limb, Unilateral Chondromatosis, Unilateral Defect of Pectoralis Muscle and Syndactyly of the Hand, Unilateral Hemidysplasia Type, Unilateral Megalencephaly, Unilateral Partial Lipodystrophy, Unilateral Renal Agenesis, Unstable Colon, Unverricht Disease, Unverricht-Lundborg Disease, Unverricht-Lundborg-Laf Disease, Unverricht Syndrome, Upper Limb - Cardiovascular Syndrome (Holt-Oram), Upper Motor Neuron Disease, Upper Airway Apnea, Upper Airway Apnea, Urea Cycle Defects or Disorders, Urea Cycle Disorder Arginase Type, Urea Cycle Disorder Arginino Succinase Type, Urea Cycle Disorders Carbamyl Phosphate Synthetase Type, Urea Cycle Disorder Citrullinemia Type, Urea Cycle Disorders N-Acrtyl Glutamate Synthetase Typ, Urea Cycle Disorder OTC Type, Urethral Syndrome, Urethro-Oculo-Articular Syndrome, Uridine Diphosphate Glucuronosyltransferase Severe Def. Type I, Urinary Tract Defects, Urofacial Syndrome, Uroporphyrinogen III cosynthase, Urticaria pigmentosa, Usher Syndrome, Usher Type I, Usher Type II, Usher Type III, Usher Type IV, Uterine Synechiae, Uoporphyrinogen I-synthase, Uveitis, Uveomeningitis Syndrome, V-CJD, VACTEL Association, VACTERL Association, VACTERL Syndrome, Valgus Calcaneus, Valine Transaminase Deficiency, Valinemia, Valproic Acid, Valproate acid exposure, Valproic acid exposure, Valproic acid, Van Buren's Disease, Van der Hoeve-Habertsma-Waardenburg-Gauldi Syndrome, Variable Onset Immunoglobulin Deficiency Dysgammaglobulinemia, Variant Creutzfeldt-Jakob Disease (V-CJD), Varicella Embryopathy, Variegate Porphyria, Vascular Birthmarks, Vascular Dementia Binswanger's Type, Vascular Erectile Tumor, Vascular Hemophilia, Vascular Malformations, Vascular Malformations of the Brain, Vasculitis, Vasomotor Ataxia, Vasopressin-Resistant Diabetes Insipidus, Vasopressin-Sensitive Diabetes Insipidus, VATER Association, Vcf syndrome, Vcfs, Velocardiofacial Syndrome, VeloCardioFacial Syndrome, Venereal Arthritis, Venous Malformations, Ventricular Fibrillation, Ventricular Septal Defects, Congenital Ventricular Defects, Ventricular Septal Defect, Ventricular Tachycardia, Venual Malformations, VEOHD, Vermis Aplasia, Vermis Cerebellar Agenesis, Vernal Keratoconjunctivitis, Verruca, Vertebral Anal Tracheoesophageal Esophageal Radial, Vertebral Ankylosing Hyperostosis, Very Early Onset Huntington's Disease, Very Long Chain Acyl-CoA Dehydrogenase (VLCAD) Deficiency, Vestibular Schwannoma, Vestibular Schwannoma Neurofibromatosis, Vestibulocerebellar, Virchow's Oxycephaly, Visceral Xanthogranulomatosis, Visceral Xantho-Granulomatosis, Visceral myopathy-External Ophthalmoplegia, Visceromegaly-Umbilical Hernia-Macroglossia Syndrome, Visual Amnesia, Vitamin A Deficiency, Vitamin B-1 Deficiency, Vitelline Macular Dystrophy, Vitiligo, Vitiligo Capitis, Vitreoretinal Dystrophy, VKC, VKH Syndrome, VLCAD, Vogt Syndrome, Vogt Cephalosyndactyly, Vogt Koyanagi Harada Syndrome, Vogt Koyanagi Harada Syndrome, Vogt Koyanagi Harada Syndrome, Von Bechterew-Strumpell Syndrome, Von Eulenburg Paramyotonia Congenita, Von Frey's Syndrome, Von Gierke Disease, Von Hippel-Lindau Syndrome, Von Mikulicz Syndrome, Von Recklinghausen Disease, Von Willebrandt Disease, VP, Vrolik Disease (Type II), VSD, Vulgaris Type Disorder of Cornification, Vulgaris Type Ichthyosis, W Syndrome, Waardenburg Syndrome, Waardenburg-Klein Syndrome, Waardenburg Syndrome Type I (WS1), Waardenburg Syndrome Type II (WS2), Waardenburg Syndrome Type IIA (WS2A), Waardenburg Syndrome Type IIB (WS2B), Waardenburg Syndrome Type III (WS3), Waardenburg Syndrome Type IV (WS4), Waelsch's Syndrome, WAGR Complex, WAGR Syndrome, WAGR Syndrome, Waldenstroem's Macroglobulinemia, Waldenstrom's Purpura, Waldenstrom's Syndrome, Waldmann Disease, Walker-Warburg Syndrome, Wandering Spleen, Warburg Syndrome, Warm Antibody Hemolytic Anemia, Warm Reacting Antibody Disease, Wartenberg Syndrome, WAS, Water on the Brain, Watson Syndrome, Watson-Alagille Syndrome, Waterhouse-Friderichsen syndrome, Waxy Disease, WBS, Weaver Syndrome, Weaver-Smith Syndrome, Weber-Cockayne Disease, Wegener's Granulomatosis, Wegener's Granulomatosis, Weil Disease, Weil Syndrome, Weill-Marchesani, Weill-Marchesani Syndrome, Weill-Reyes Syndrome, Weismann-Netter-Stuhl Syndrome, Weissenbacher-Zweymuller Syndrome, Wells Syndrome, Wenckebach, Werdnig-Hoffman Disease, Werdnig-Hoffmann Disease, Werdnig-Hoffmann disease, Werdnig-Hoffman Disease, Werdnig-Hoffman Paralysis, Werlhof's Disease, Werner Syndrome, Wernicke's (C) I Syndrome, Wernicke's aphasia, Wemicke-Korsakoff Syndrome, West Syndrome, Wet Beriberi, WHCR, Whipple's Disease, Whistling face syndrome, Whistling Face-Windmill Vane Hand Syndrome, White-Darier Disease, Whitnall-Norman Syndrome, Whorled nevoid hypermelanosis, WHS, Wieacker Syndrome, Wieacher Syndrome, Wieacker-Wolff Syndrome, Wiedmann-Beckwith Syndrome, Wiedemann-Rautenstrauch Syndrome, Wildervanck Syndrome, Willebrand-Juergens Disease, Willi-Prader Syndrome, Williams Syndrome, Williams Syndrome, Williams-Beuren Syndrome, Wilms' Tumor, Wilms' Tumor-Aniridia-Gonadoblastoma-Mental Retardation Syndrome, Wilms Tumor Aniridia Gonadoblastoma Mental Retardation, Wilms' Tumor-Aniridia-Genitourinary Anomalies-Mental Retardation Syndrome, Wilms Tumor-Pseudohermaphroditism-Nephropathy, Wilms Tumor and Pseudohermaphroditism, Wilms Tumor-Pseuodohermaphroditism-Glomerulopathy, Wilson's Disease, Winchester Syndrome, Winchester-Grossman Syndrome, Wiskott-Aldrich Syndrome, Wiskott-Aldrich Type Immunodeficiency, Witkop Ectodermal Dysplasias, Witkop Tooth-Nail Syndrome, Wittmaack-Ekbom Syndrome, WM Syndrome, WMS, WNS, Wohlfart-Disease, Wohlfart-Kugelberg-Welander Disease, Wolf Syndrome, Wolf-Hirschhorn Chromosome Region (WHCR), Wolf-Hirschhom Syndrome, Wolff-Parkinson-White Syndrome, Wolff-Parkinson-White syndrome, Wolff Parkinson White Syndrome, Wolfram Syndrome, Wolman Disease (Lysomal Acid Lypase Deficiency), Woody Guthrie's Disease, WPW Syndrome, WPW Syndrome, Writer's Cramp, WS, WS, WS, WSS, WWS, Wyburn-Mason Syndrome, Wybum-Mason Syndrome, X-Linked Addison's Disease, X-linked Adrenoleukodystrophy (X-ALD), X-linked Adult Onset Spinobulbar Muscular Atrophy, X-linked Adult Spinal Muscular Atrophy, X-Linked Agammaglobulinemia with Growth Hormone Deficiency, X-Linked Agammaglobulinemia, Lymphoproliferate X-Linked Syndrome, X-linked Cardio myopathy and Neutropenia, X-Linked Centronuclear myopathy, X-linked Copper Deficiency, X-linked Copper Malabsorption, X-Linked Dominant Conradi-Hunermann Syndrome, X-Linked Dominant Inheritance Agenesis of Corpus Callosum, X-Linked Dystonia-parkinsonism, X-Linked Ichthyosis, X-Linked Infantile Agammaglobulinemia, X-Linked Infantile Nectrotizing Encephalopathy, X-linked Juvenile Retinoschisis, X-linked Lissencephaly, X-linked Lymphoproliferative Syndrome, X-linked Mental Retardation-Clasped Thumb Syndrome, X-Linked Mental Retardation with Hypotonia, X-linked Mental Retardation and Macroorchidism, X-Linked Progressive Combined Variable Immunodeficiency, X-Linked Recessive Conradi-Hunermann Syndrome, X-Linked Recessive Severe Combined Immunodeficiency, X-Linked Recessive Severe Combined Immunodeficiency, X-Linked Retinoschisis, X-linked Spondyloepiphyseal Dysplasia, Xanthine Oxidase Deficiency (Xanthinuria Deficiency, Hereditary), Xanthinuria Deficiency, Hereditary (Xanthine Oxidase Deficiency), Xanthogranulomatosis Generalized, Xanthoma Tuberosum, Xeroderma Pigmentosum, Xeroderma Pigmentosum Dominant Type, Xeroderma Pigmentosum Type A I XPA Classical Form, Xeroderma Pigmentosum Type B II XPB, Xeroderma Pigmentosum Type E V XPE, Xeroderma Pigmentosum Type C III XPC, Xeroderma Pigmentosum Type D IV XPD, Xeroderma Pigmentosum Type F VI XPF, Xeroderma Pigmentosum Type G VII XPG, Xeroderma Pigmentosum Variant Type XP-V, Xeroderma-Talipes-and Enamel Defect, Xerodermic Idiocy, Xerophthalmia, Xerotic Keratitis, XLP, XO Syndrome, XP, XX Male Syndrome,Sex Reversal, XXXXX Syndrome, XXY Syndrome, XYY Syndrome, XYY Chromosome Pattern, Yellow Mutant Albinism, Yellow Nail Syndrome, YKL, Young Female Arteritis, Yunis-Varon Syndrome, YY Syndrome, Z-E Syndrome, Z- and -Protease Inhibitor Deficiency, Zellweger Syndrome, Zellweger cerebro-hepato-renal syndrome, ZES, Ziehen-Oppenheim Disease (Torsion Dystonia), Zimmermann-Laband Syndrome, Zinc Deficiency Congenital, Zinsser-Cole-Engman Syndrome, ZLS, Zollinger-Ellison Syndrome.

As used herein a "cancer" refers to a group of diseases and disorders that are characterized by uncontrolled cellular growth (e.g. formation of tumor) without any differentiation of those cells into specialized and different cells. Cancers which can be treated using the methods of the present invention include, without being limited to, ABL1 protooncogene, AIDS Related Cancers, Acoustic Neuroma, Acute Lymphocytic Leukaemia, Acute Myeloid Leukaemia, Adenocystic carcinoma, Adrenocortical Cancer, Agnogenic myeloid metaplasia, Alopecia, Alveolar soft-part sarcoma, Anal cancer, Angiosarcoma, Aplastic Anaemia, Astrocytoma, Ataxia-telangiectasia, Basal Cell Carcinoma (Skin), Bladder Cancer, Bone Cancers, Bowel cancer, Brain Stem Glioma, Brain and CNS Tumours, Breast Cancer, CNS tumours, Carcinoid Tumours, Cervical Cancer, Childhood Brain Tumours, Childhood Cancer, Childhood Leukaemia, Childhood Soft Tissue Sarcoma, Chondrosarcoma, Choriocarcinoma, Chronic Lymphocytic Leukaemia, Chronic Myeloid Leukaemia, Colorectal Cancers, Cutaneous T-Cell Lymphoma, Dermatofibrosarcoma-protuberans, Desmoplastic-Small-Round-Cell-Tumour, Ductal Carcinoma, Endocrine Cancers, Endometrial Cancer, Ependymoma, Esophageal Cancer, Ewing's Sarcoma, ExtraHepatic Bile Duct Cancer, Eye Cancer, Eye: Melanoma, Retinoblastoma, Fallopian Tube cancer, Fanconi Anaemia, Fibrosarcoma, Gall Bladder Cancer, Gastric Cancer, Gastrointestinal Cancers, Gastrointestinal-Carcinoid-Tumour, Genitourinary Cancers, Germ Cell Tumours, Gestational-Trophoblastic-Disease, Glioma, Gynaecological Cancers, Haematological Malignancies, Hairy Cell Leukaemia, Head and Neck Cancer, Hepatocellular Cancer, Hereditary Breast Cancer, Histiocytosis, Hodgkin's Disease, Human Papillomavirus, Hydatidiform mole, Hypercalcemia, Hypopharynx Cancer, IntraOcular Melanoma, Islet cell cancer, Kaposi's sarcoma, Kidney Cancer, Langerhan's-Cell-Histiocytosis, Laryngeal Cancer, Leiomyosarcoma, Leukaemia, Li-Fraumeni Syndrome, Lip Cancer, Liposarcoma, Liver Cancer, Lung Cancer, Lymphedema, Lymphoma, Hodgkin's Lymphoma, Non-Hodgkin's Lymphoma, Male Breast Cancer, Malignant-Rhabdoid-Tumour-of-Kidney, Medulloblastoma, Melanoma, Merkel Cell Cancer, Mesothelioma, Metastatic Cancer, Mouth Cancer, Multiple Endocrine Neoplasia, Mycosis Fungoides, Myelodysplastic Syndromes, Myeloma, Myeloproliferative Disorders, Nasal Cancer, Nasopharyngeal Cancer, Nephroblastoma, Neuroblastoma, Neurofibromatosis, Nijmegen Breakage Syndrome, Non-Melanoma Skin Cancer, Non-Small-Cell-Lung-Cancer-(NSCLC), Ocular Cancers, Oesophageal Cancer, Oral cavity Cancer, Oropharynx Cancer, Osteosarcoma, Ostomy Ovarian Cancer, Pancreas Cancer, Paranasal Cancer, Parathyroid Cancer, Parotid Gland Cancer, Penile Cancer, Peripheral-Neuroectodermal-Tumours, Pituitary Cancer, Polycythemia vera, Prostate Cancer, Rare-cancers-and-associated-disorders, Renal Cell Carcinoma, Retinoblastoma, Rhabdomyosarcoma, Rothmund-Thomson Syndrome, Salivary Gland Cancer, Sarcoma, Schwannoma, Sezary syndrome, Skin Cancer, Small Cell Lung Cancer (SCLC), Small Intestine Cancer, Soft Tissue Sarcoma, Spinal Cord Tumours, Squamous-Cell-Carcinoma-(skin), Stomach Cancer, Synovial sarcoma, Testicular Cancer, Thymus Cancer, Thyroid Cancer, Transitional-Cell-Cancer-(bladder), Transitional-Cell-Cancer-(renal-pelvis-/- ureter), Trophoblastic Cancer, Urethral Cancer, Urinary System Cancer, Uroplakins, Uterine sarcoma, Uterus Cancer, Vaginal Cancer, Vulva Cancer, Waldenstrom's-Macroglobulinemia and Wilms' Tumor.

The present invention also provides for the treatment of conditions associated with inflammatory diseases and disorders. As used herein "inflammatory diseases and disorders" encompass those disease and disorders which result in a response of redness, swelling, pain, and a feeling of heat in certain areas that is meant to protect tissues affected by injury or disease. Inflammatory diseases which can be treated using the methods of the present invention, include, without being limited to, acne, angina, arthritis, aspiration pneumonia, disease, empyema, gastroenteritis, inflammation, intestinal flu, NEC, necrotizing enterocolitis, pelvic inflammatory disease, pharyngitis, PID, pleurisy, raw throat, redness, rubor, sore throat, stomach flu and urinary tract infections.

Such compositions can be formulated according to conventional pharmaceutical compounding techniques. See, for example, Remington's Pharmaceutical Sciences, 18th Ed. (1990, Mack Publishing, Company, Easton, PA, U.S.A.). The composition may contain the active agent or pharmaceutically acceptable salts of the active agent. These compositions may comprise, in addition to one of the active substances, a pharmaceutically acceptable excipient, carrier, buffer, stabilizer or other materials well known in the art. Such materials should be non-toxic and should not interfere with the efficacy of the active ingredient. The carrier may take a wide variety of forms depending on the form of preparation desired for administration, e.g. intravenous, oral, intrathecal, epineural or parenteral. For antibodies, parenteral administration is particularly useful.

For oral administration, the compounds can be formulated into solid or liquid preparations such as capsules, pills, tablets, lozenges, melts, powders, suspensions or emulsions. In preparing the compositions in oral dosage form, any of the usual pharmaceutical media may be employed, such as, for example, water, glycols, oils, alcohols, flavoring agents, preservatives, coloring agents, suspending agents, and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are obviously employed. If desired, tablets may be sugar-coated or enteric-coated by standard techniques. The active agent can be encapsulated to make it stable to passage through the gastrointestinal tract while at the same time allowing for passage across the blood brain barrier. See for example, International Patent Publication No. WO 96/11698.

For parenteral administration, the compound may dissolved in a pharmaceutical carrier and administered as either a solution of a suspension. Illustrative of suitable carriers are water, saline, dextrose solutions, fructose solutions, ethanol, or oils of animal, vegetative or synthetic origin. The carrier may also contain other ingredients, for example, preservatives, suspending agents, solubilizing agents, buffers and the like. When the compounds are being administered intrathecally, they may also be dissolved in cerebrospinal fluid.

The active agent is preferably administered in a therapeutically effective amount. The actual amount administered and the rate and time-course of administration will depend on the nature and severity of the condition being treated. Prescription of treatment, e.g. decisions on dosage, timing, etc. is within the responsibility of general practitioners or specialists and typically takes account of the disorder to be treated, the condition of the individual patient, the site of delivery, the method of administration and other factors known to practitioners. Examples of techniques and protocols can be found in *Remington's Pharmaceutical Sciences, supra*.

Instead of administering these agents directly, they may also be produced in the target cell, e.g. in a viral vector or in a cell based delivery system such as described in U.S. Patent No. 5,550,050 and International Patent Publication Nos. WO 92/19195, WO 94/25503, WO 95/01203, WO 95/05452, WO 96/02286, WO 96/02646, WO 96/40871, WO 96/40959 and WO 97/12635. The vector could be targeted to the target cells. The cell based delivery system is designed to be implanted in a patient's body at the desired target site and contains a coding sequence for the target agent. Alternatively, the agent could be administered in a precursor form for conversion to the active form by an activating agent produced in, or targeted to, the cells to be treated. See, for example, European Patent Application No. 0 425 731 A and International Patent Publication No. WO 90/07936.

Alternative aspects of the invention include:
1. An isolated nucleic acid molecule comprising a sequence selected from the group consisting of:
   (a) a sequence provided in SEQ ID NOs:1, 3, 5, 7, 9, 1 l, 13, 15, 17, 19, 21, 23 and 25;
   (b) a complement of a sequence provided in SEQ ID NOs:1, 3, 5, 7, 9, 11, 13, 15, 17, 19, 21, 23 or 25;
   (c) a sequence consisting of at least 10 contiguous nucleotides of a sequence provided in SEQ ID NOs:1, 3, 5, 7, 9, 11, 13, 15, 17, 19, 21, 23 or 25 or complementary form thereof;
   (d) a sequence which hybridizes to the complement of a sequence provided in SEQ ID NOs:1, 3, 5, 7, 9, 11, 13, 15, 17, 19, 21, 23 or 25, under conditions of low stringency;
   (e) a sequence having at least 70% identity after optimal alignment to a sequence provided in SEQ ID NOs:1, 3, 5, 7, 9, 11, 13, 15, 17, 19, 21, 23 or 25;
   (f) a derivative of a sequence provided in SEQ ID NOs:1, 3, 5, 7, 9, 11, 13, 15, 17, 19, 21, 23 or 25; and
   (g) a homolog of a sequence provided in SEQ ID NOs:1, 3, 5, 7, 9, 11, 13, 15, 17, 19, 21, 23 or 25.
2. A vector comprising a nucleic acid molecule of aspect 1 operably linked to an expression control sequence.
3. The vector of aspect 2, wherein the vector is an artificial chromosome.
4. The vector of aspect 3, wherein the vector is an artificial human chromosome.
5. A host cell transformed or transfected with the vector of aspect 2 or 3 or 4.
6. An isolated polypeptide comprising an amino acid sequence selected from the group consisting of:
   (a) a sequence provided in SEQ ID NOs:2, 4, 6, 8, 10, 12, 14, 16, 18, 20, 22, 24, 26, 27 and 28;
   (b) a sequence having at least 70% similarity after optimal alignment to an amino acid sequence provided in SEQ ID NOs:2, 4, 6, 8, 10, 12, 14, 16, 18, 20, 22, 24, 26, 27 and 28;
   (c) a derivative, homolog, analog, chemical equivalent or mimetic of a sequence provided in SEQ ID NOs:2, 4, 6, 8, 10, 12, 14, 16, 18, 20, 22, 24, 26, 27 and 28;
   (d) a sequence encoded by a nucleic acid molecule of aspect 1; and
   (e) a sequence having at least 70% similarity after optimal alignment to a sequence encoded by a nucleic acid molecule of aspect 1.
7. A vector comprising a nucleic acid molecule which encodes a polypeptide of aspect 6 operably linked to an expression control sequence.
8. The vector of aspect 7, wherein the vector is an artificial chromosome.
9. The vector of aspect 8, wherein the vector is a human artificial chromosome.
10. A host cell transformed or transfected with the vector of aspect 7 or 8 or 9.
11. An isolated immunointeractive molecule which specifically binds to a polypeptide of aspect 6 or an immunogenic fragment thereof.
12. The immunointeractive molecule of aspect 11, wherein the molecule is an antibody or an antigen binding fragment thereof.
13. The isolated antibody of aspect 12, wherein said antibody is selected from a polyclonal antibody, a monoclonal antibody, a humanized antibody or a deimmunized antibody.
14. The antibody of aspect 12 or aspect 13 conjugated to an immunotoxin.
15. A composition comprising a first component selected from the group consisting of:
   (a) a nucleic acid molecule of aspect 1;
   (b) a polypeptide of aspect 6; and
   (c) an immunointeractive molecule of aspect 11 or 12 or 13,
   and a second component selected from a pharmaceutical carrier, diluent and an immunostimulant.
16. A method for detecting the presence of a disease or condition in a subject, comprising the steps of:
   (a) obtaining a biological sample from said subject;
   (b) contacting said biological sample with an molecule that binds to a nucleic acid molecule of aspect 1 or a polypeptide aspect 6;
   (c) detecting in said biological sample the presence of binding of said molecule; and
   (d) comparing the presence of bound molecule with a pre-determined cut-off value to make a determination as to the presence or absence of a disease or condition in said subject.
17. The method of aspect 16, wherein said disease or condition is AML.
18. The method of aspect 16, wherein said molecule is an antibody.
19. A method for detecting a target cell which produces a member of the 35-LM family of proteins, comprising the steps of:
   (a) obtaining a sample comprising cells;
   (b) contacting said sample with an molecule that binds to a member of the 35-LM family of proteins; and
   (c) detecting the presence of a target cell conjugated to said molecule specific for a member of the 35-LM family of proteins.
20. The method of aspect 19, wherein the 35-LM protein is selected from the group consisting of 35-L1, 35-L2, 35-L3, 35-L4 and 35-L5.
21. The method of aspect 19, wherein the target cell is of myeloid lineage.
22. The method of aspect 21, wherein the myeloid cell is selected from a monocyte, a macrophage, a dendritic cell and a stem cell.
23. The method of aspect 22, wherein said dendritic cell is CD11c⁺.
24. A method for assessing a disease or condition including the ability for a subject to mount an immune response, said method comprising determining the level or pattern of expression of the nucleic acid molecule in aspect 1, wherein the pattern of presence or absence of expression correlates with a disease condition, a propensity for developing a disease condition and/or an ability for a subject to maintain an immune response.
25. A method for assessing a disease or condition including the ability for a subject to mount an immune response, said method comprising determining the level or pattern of the protein in aspect 6, wherein the pattern of presence or absence or level of said protein correlates with a disease condition, a propensity for developing a disease condition and/or an ability for a subject to maintain an immune response.
26. The method of aspect 24 or aspect 25, wherein said disease or condition is selected from cancer or a genetic disorder or an inflammatory disease.

The present invention is further described by the following non-limiting Examples.

### EXAMPLE 1

### Identification of CMRF-35 family members

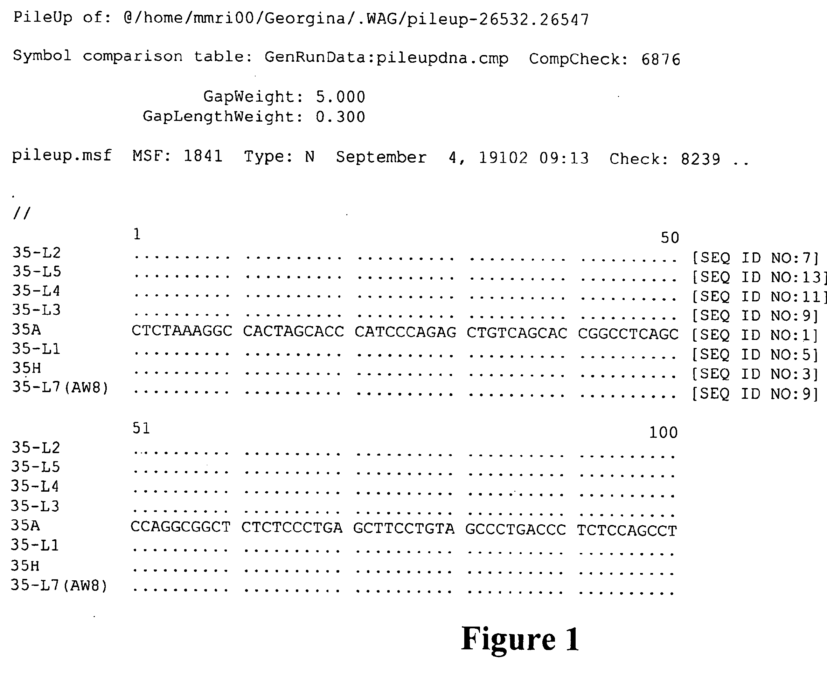

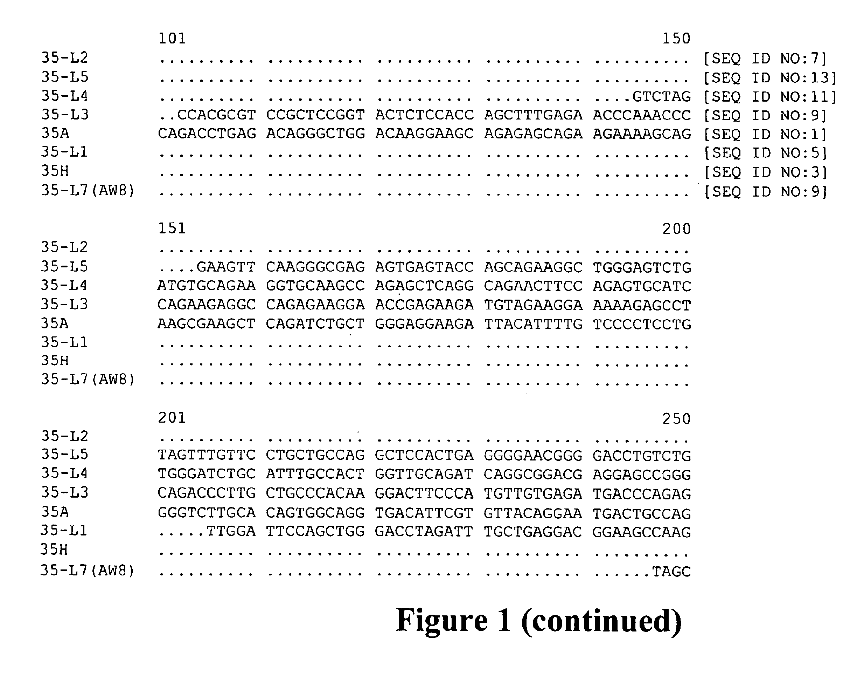

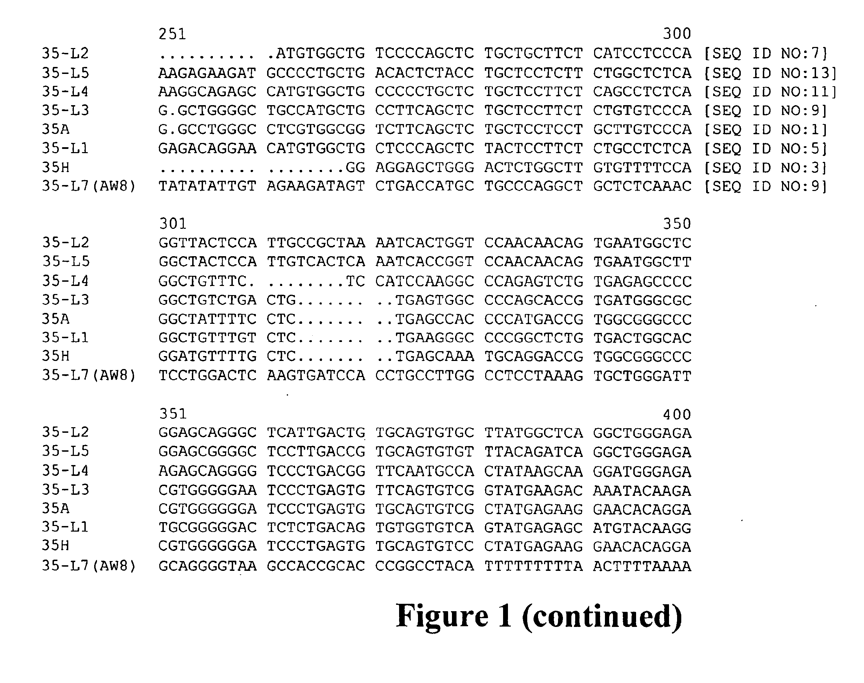

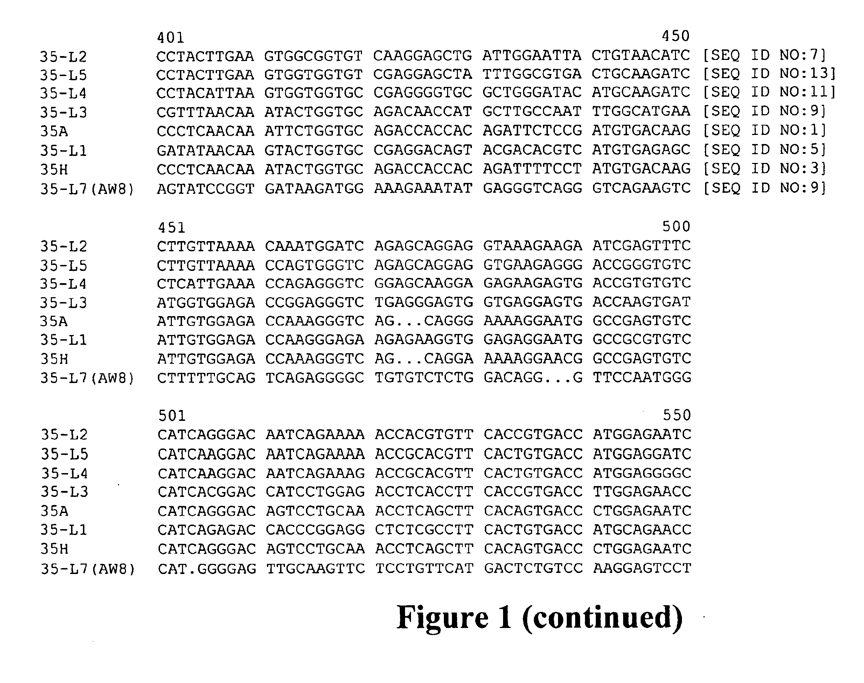

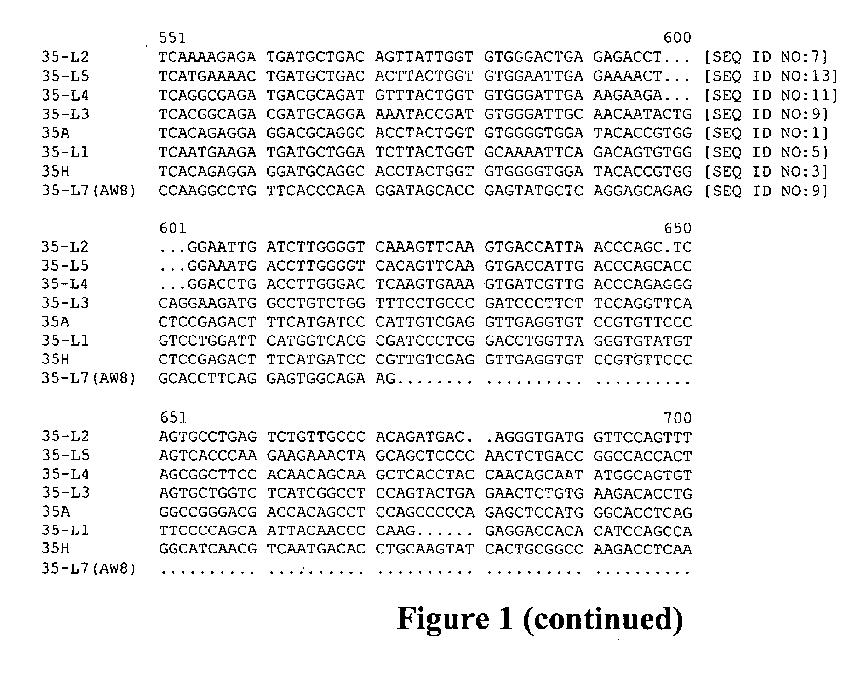

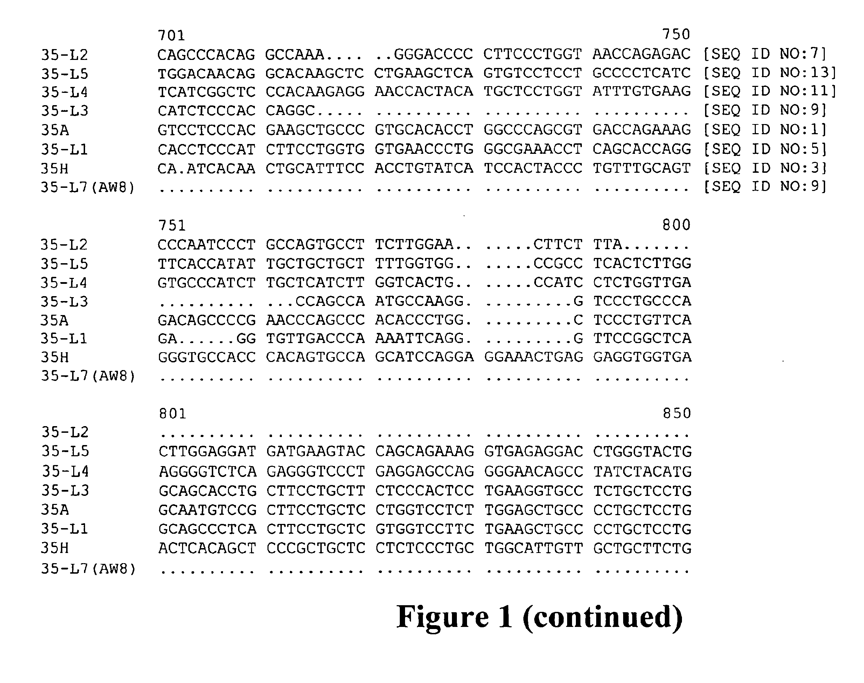

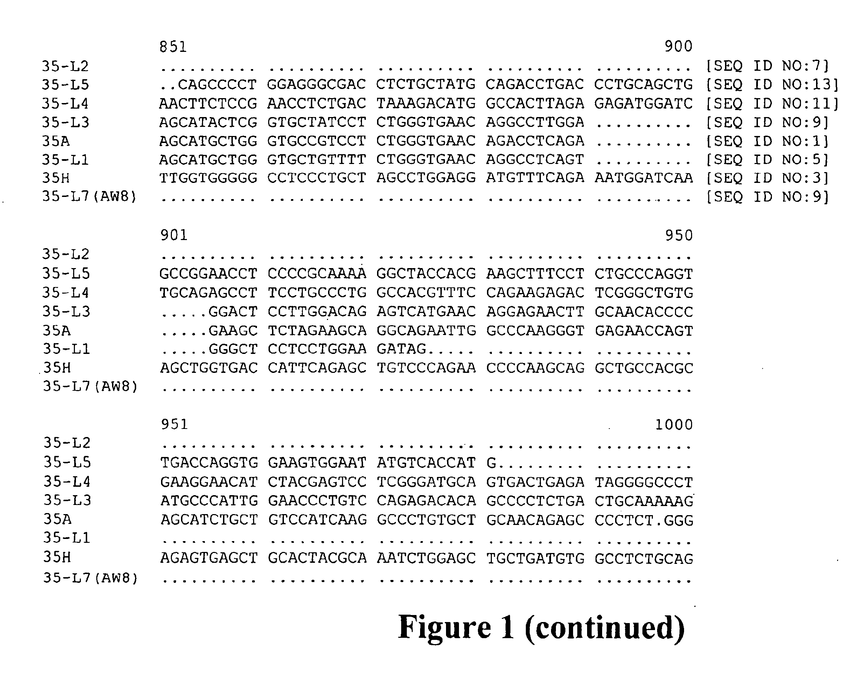

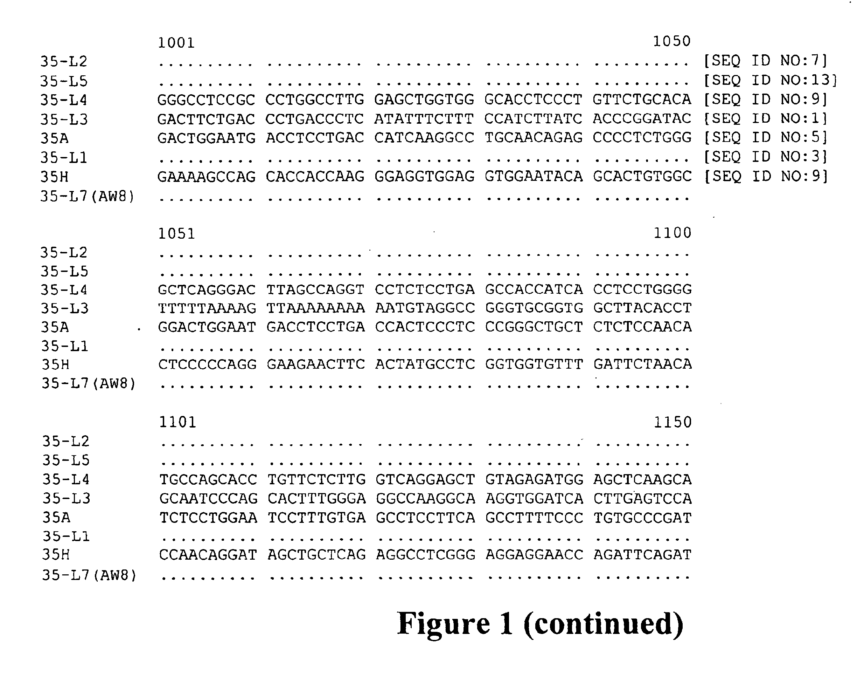

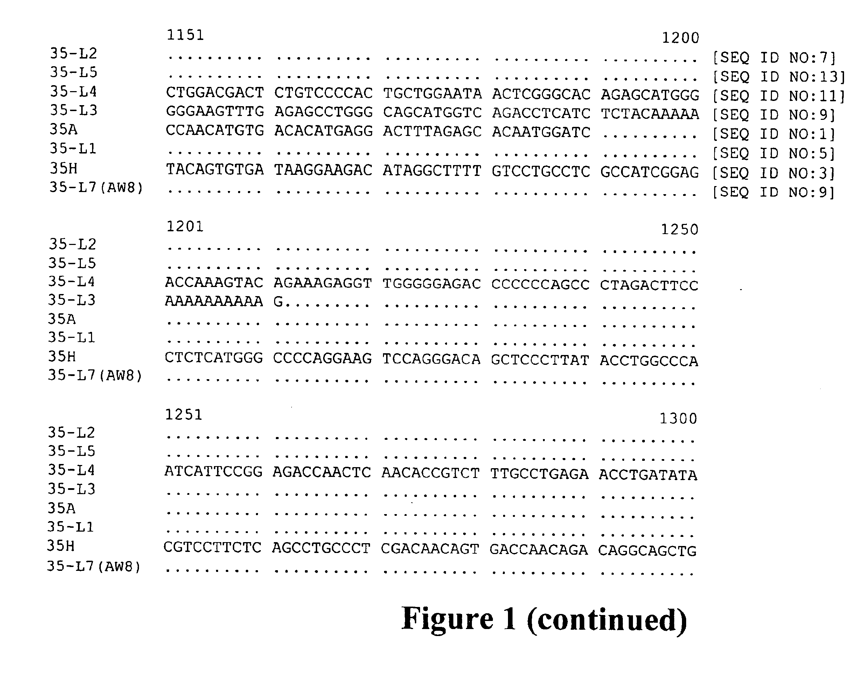

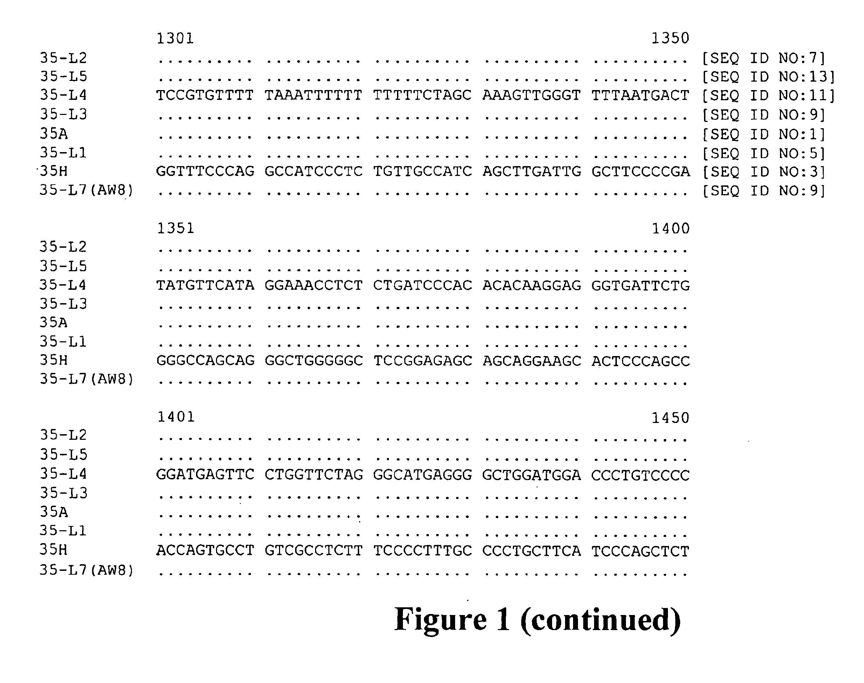

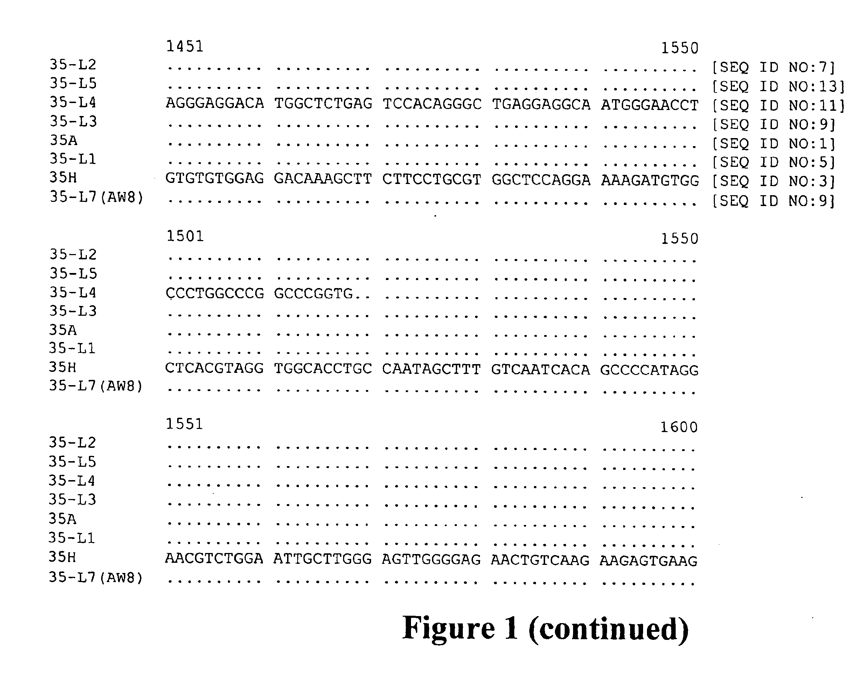

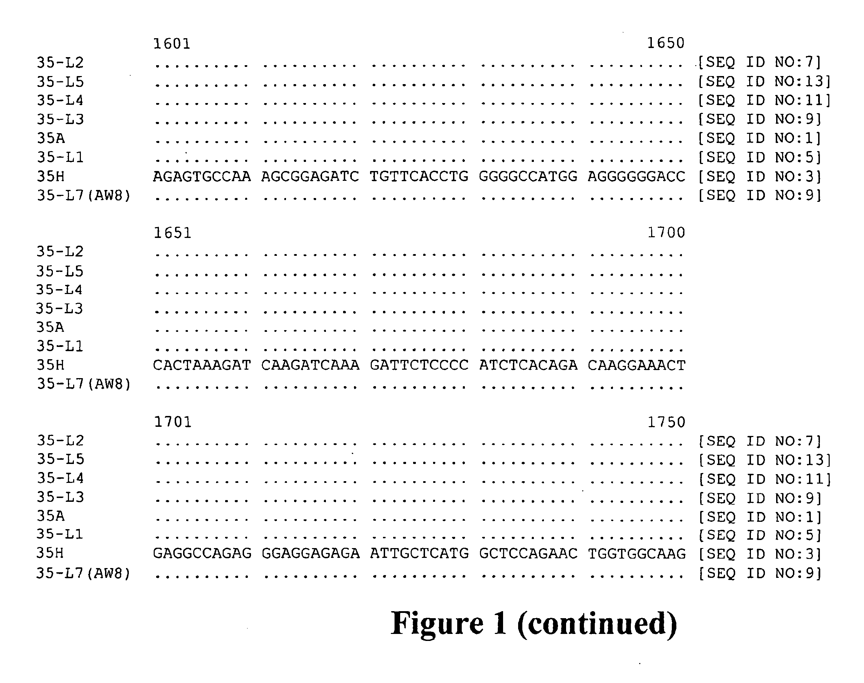

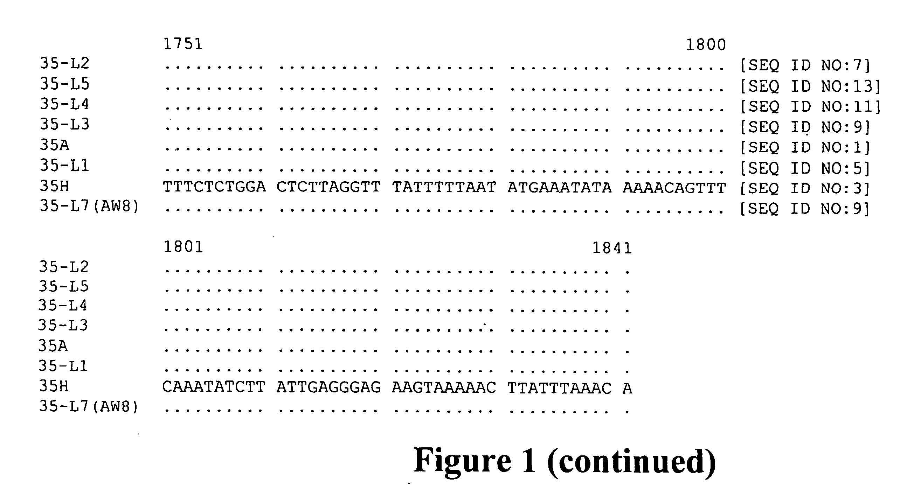

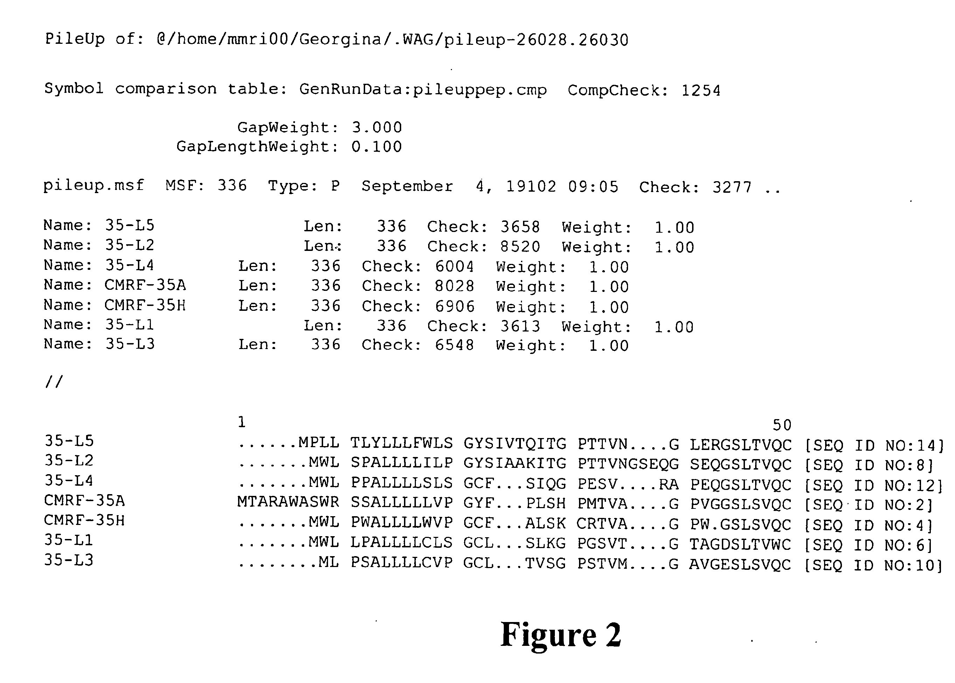

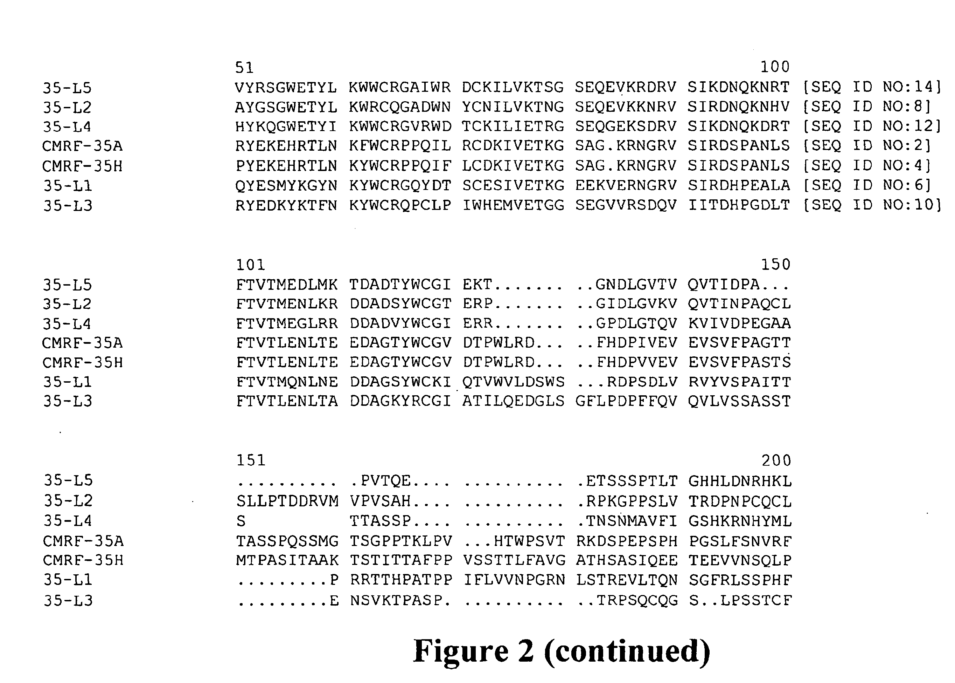

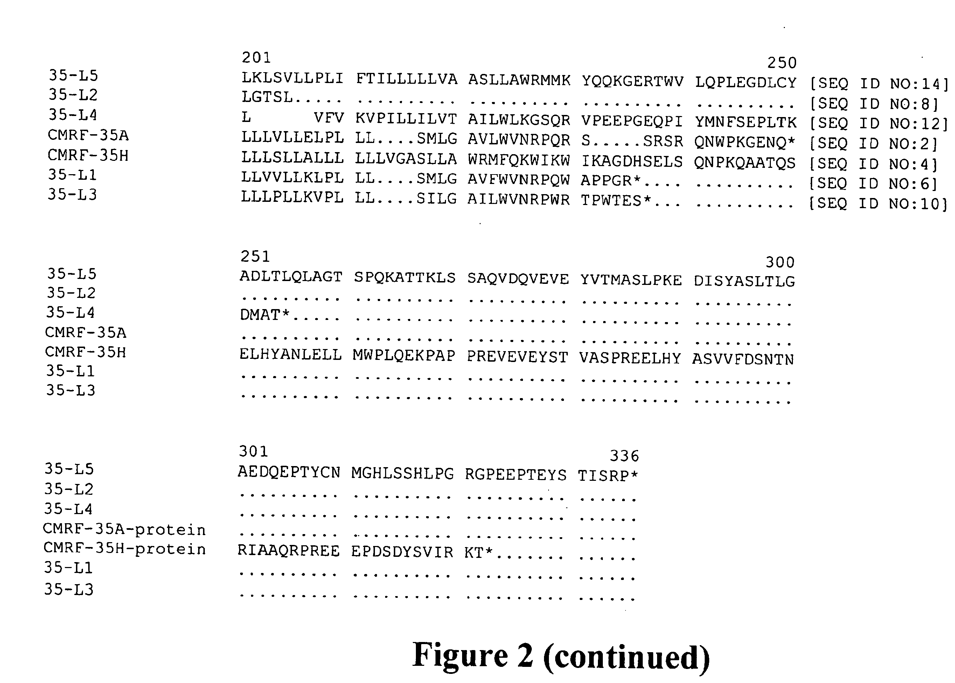

cDNA probes specific for CMRF-35A and CMRF-35H Ig domains were identified as binding to a large number of independent, non-overlapping PAC clones. Partial and full length cDNA molecules which map to human chromosome 17q22-24 were identified from EST and 5' RACE studies. Alignment of the sequences with CMRF-35A and CMRF-35H indicated similarities over the transmembrane region. cDNA and gDNA sequences were also used to further RT-PCR based expression studies. An alignment of the nucleic acid sequences of the human cDNAs is shown in Figure 1. An alignment of the protein sequences of the human cDNAs is shown in Figure 2.

An RT-PCR assay was established to characterize the expression of the novel members of the 35-LM family in normal hematopoietic lineages and cell lines. Screening of public and commercial databases was used to confirm that the EST used for the RT-PCR represents a single exon. The sequence of the complete cDNAs is used to design RT-PCR primers that cross intron-exon junctions. The primers are used to confirm the expression data. This ensures the identification of any splice variants.

### EXAMPLE 2

### Expression studies for CMRF-35

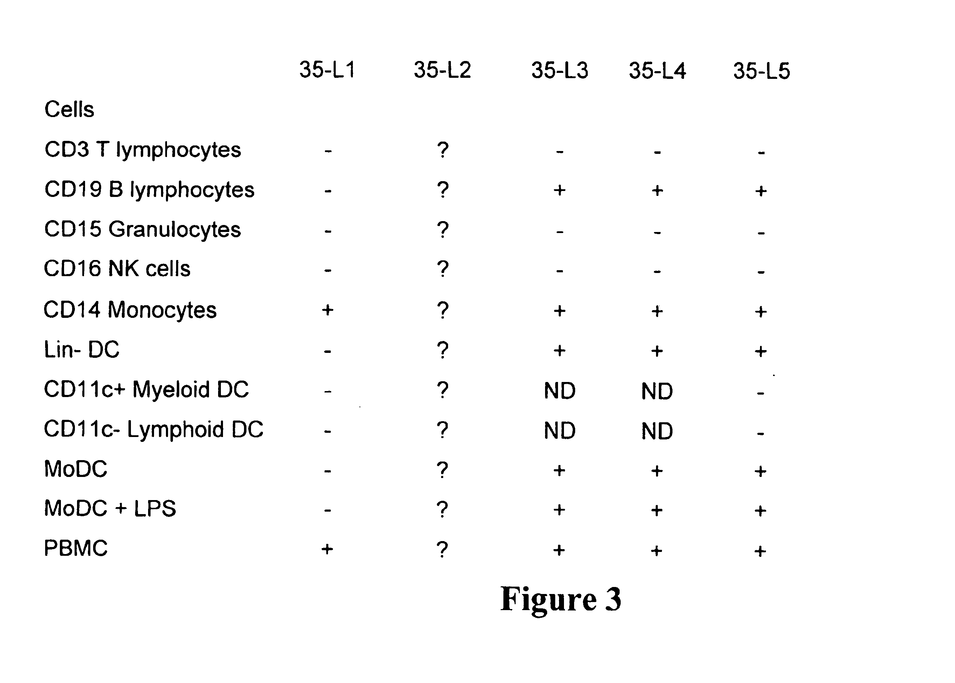

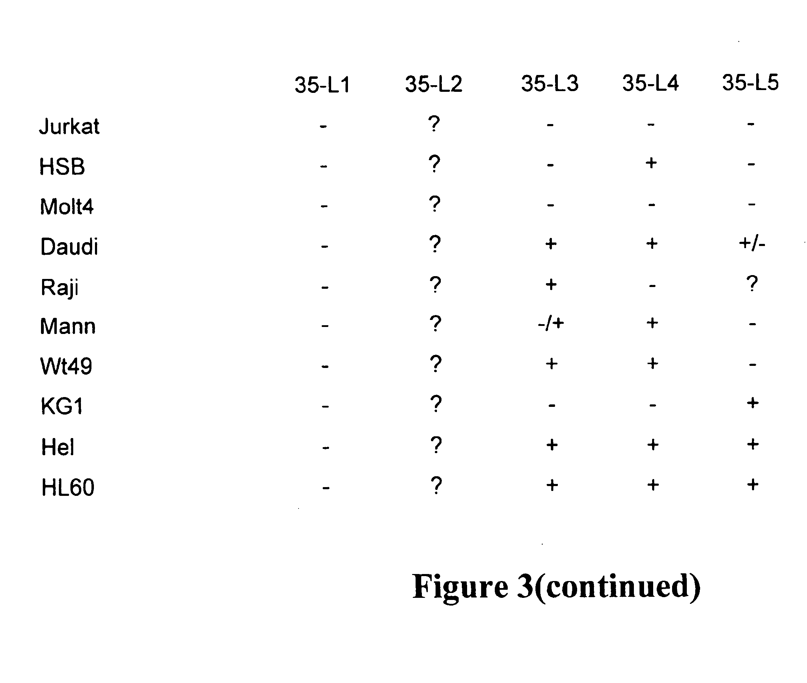

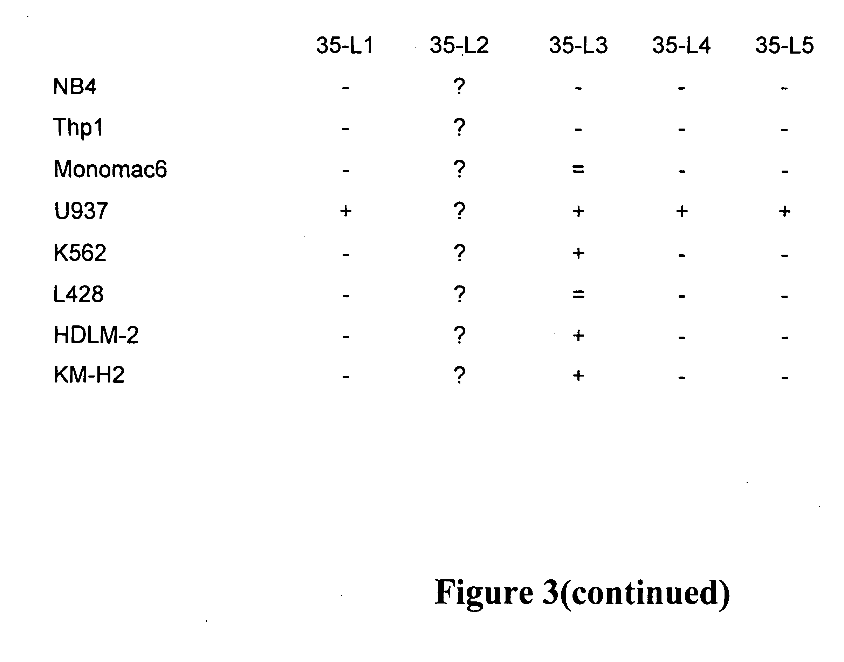

Figure 3 summarizes the expression analysis of the h35-LMs on cell lines and freshly purified hematopoietic populations.

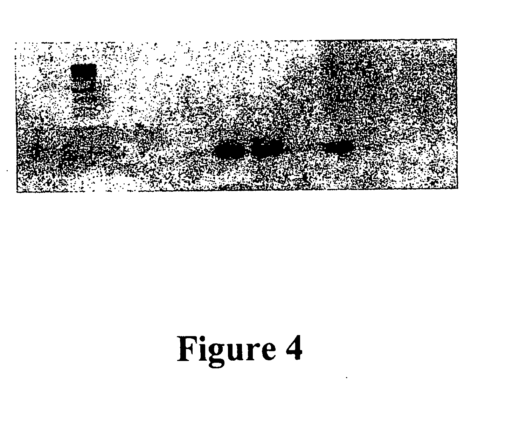

RT-PCR was performed to determine the expression of h35-L3 (AW8) on cDNA made from RNA isolated from hematopoietic cell lines (leukemic derived) and cells of different hematopoietic lineages. Analysis of hematopoietic cell line data indicate that 35-L3 is expressed by the derived cell lines HEL, HL60, KG-1, Monomac 6, U937 and K562 and the Hodgkins disease derived cell lines HDLM-2 and KM-H2. 35-L3 was not found in lines of T or B cell origin. The RNA for this molecule is predominantly expressed by cells of the myeloid lineage as shown in Figure 4. Further analysis of dendritic cell populations indicate that 35-L3 (AW8) is expressed only by the CD11c⁺ myeloid derived DC and not the CD11c⁻ lymphoid derived DC. Thus, in addition CD33, CD13, and CD14 this molecule appears to be expressed by cells of the myeloid lineage. The inventors have shown that 35-L3 is expressed by leukemic cells from single AML patients. Blast cells from a patient newly diagnosed with AML was selected by flow sorting. RNA isolated from these cells, when used in RT-PCR show the expression of the CMRF-35-L3 specific PCR products.

### EXAMPLE 3

### Homologs of hCMRF-35 molecules

To locate mouse homologs of h35-LM (i.e. murine orthologs), a series of searches were conducted in the public and commercial databases around the region 11E2.

Initially, six computationally predicted genes sharing significant homology with h35-LMs were chosen for further analysis. These genes were termed m35a, m35c, m35d, m35f and m35g. Of these, m35a, m35d and m35f contained complete coding regions. Comparison to mouse ESTs in NCBI provided overlapping sequences from which a complete coding sequence could be obtained for m35c and m35g. The ESTs were as follows: 3' end of m35c (gi: 16445999) and middle region of m35g (EST gi: 15562326).

Further database searches revealed two new homologs termed m35h and DIgR2 (86% similar to DIgR1) with NCBI Accession Nos. XM_126721 and XM_126696. Only m35h contained a complete coding region.

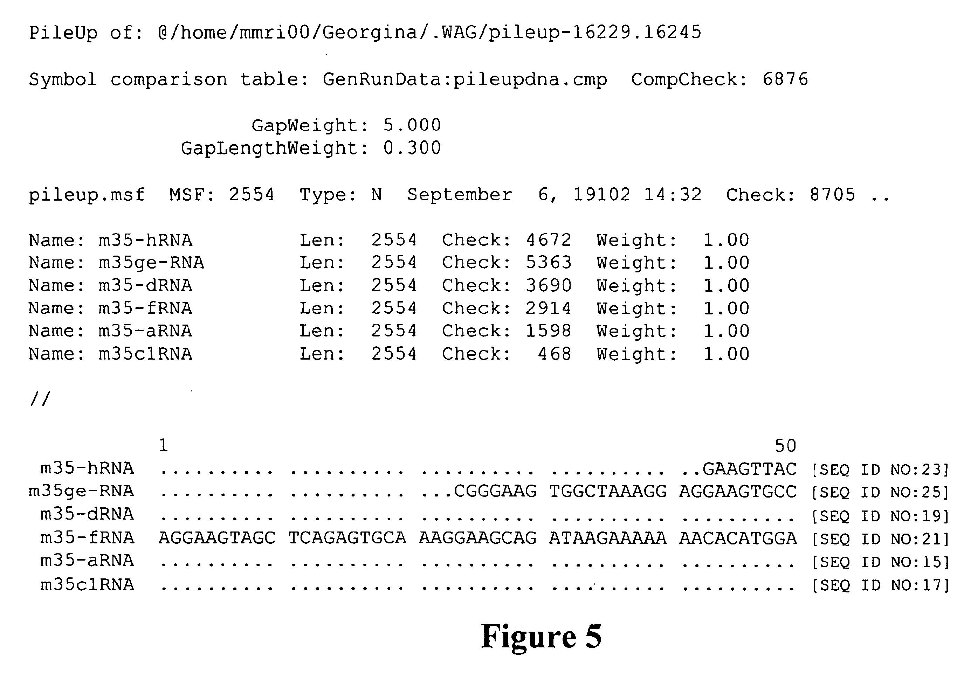

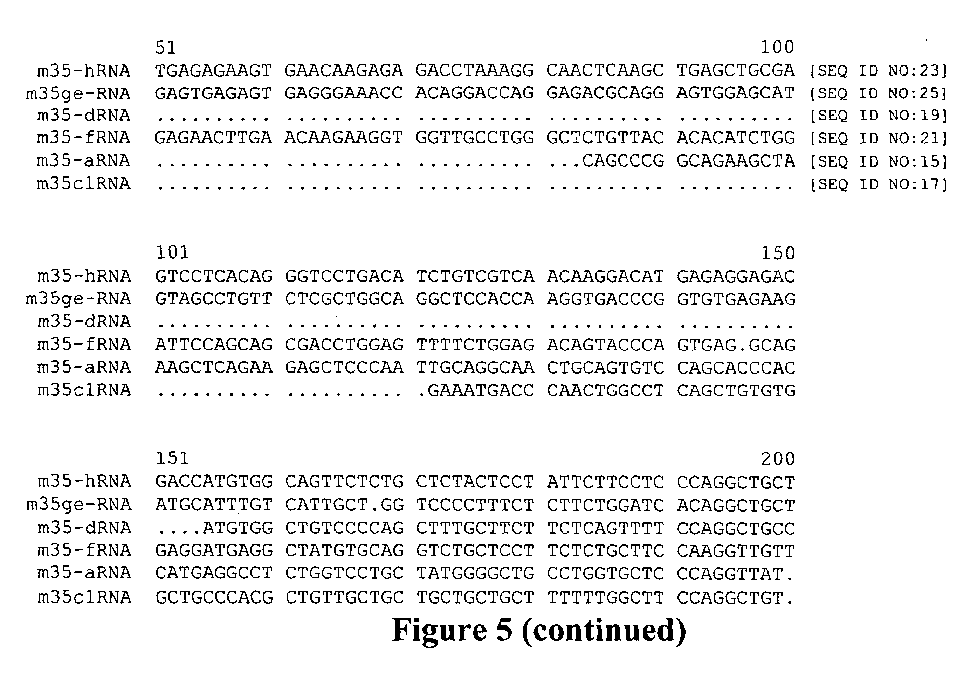

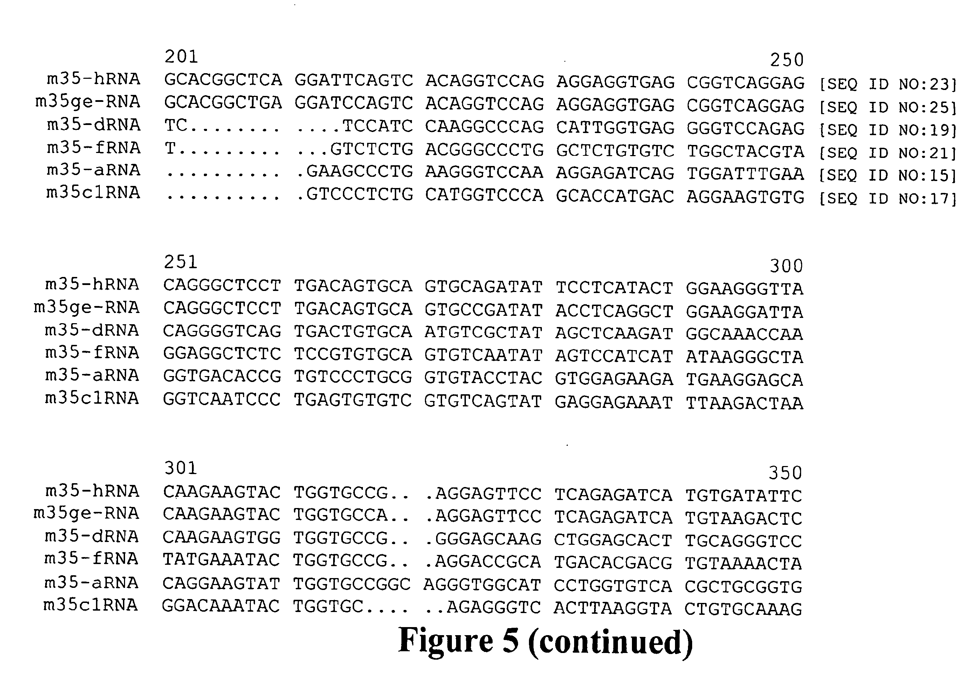

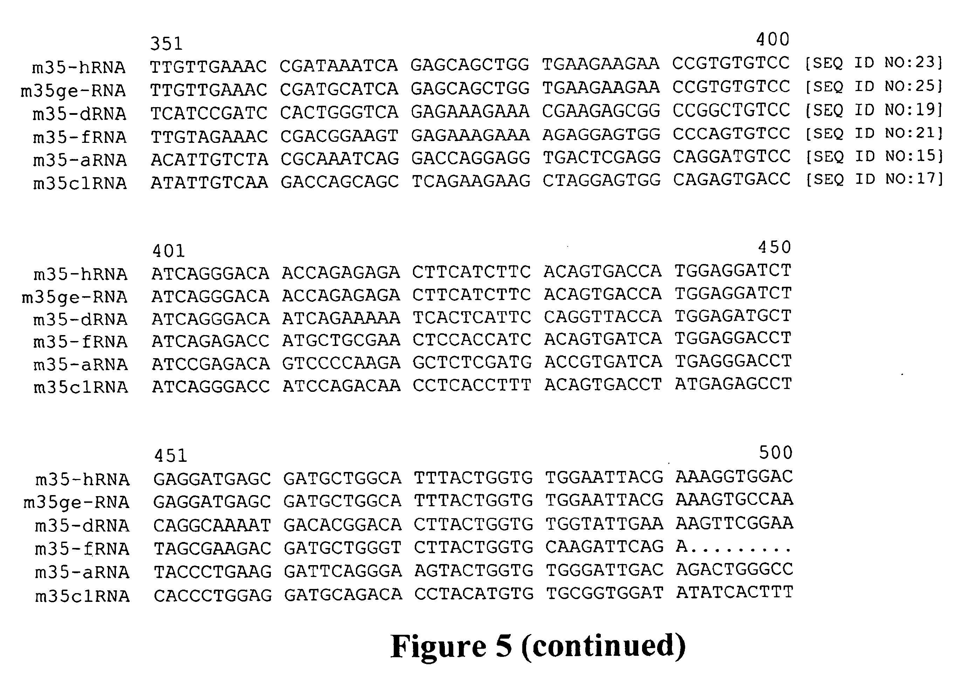

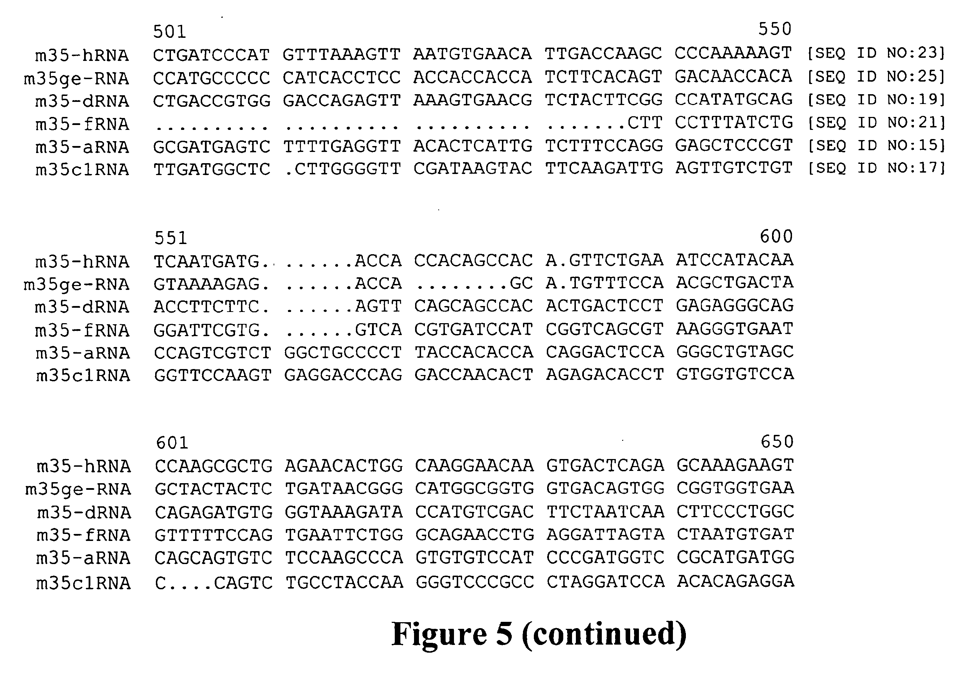

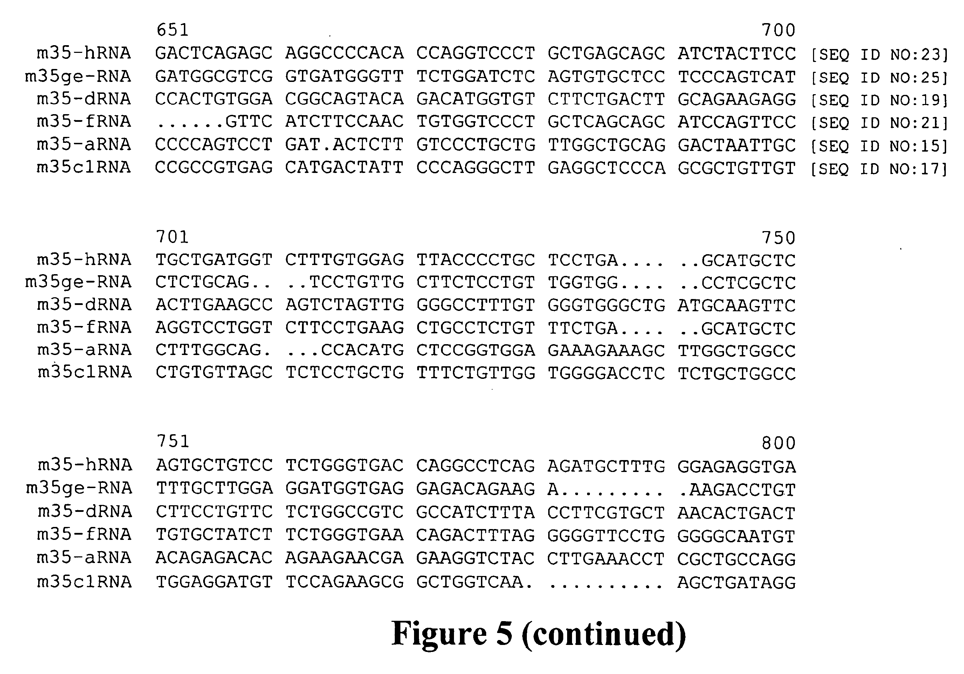

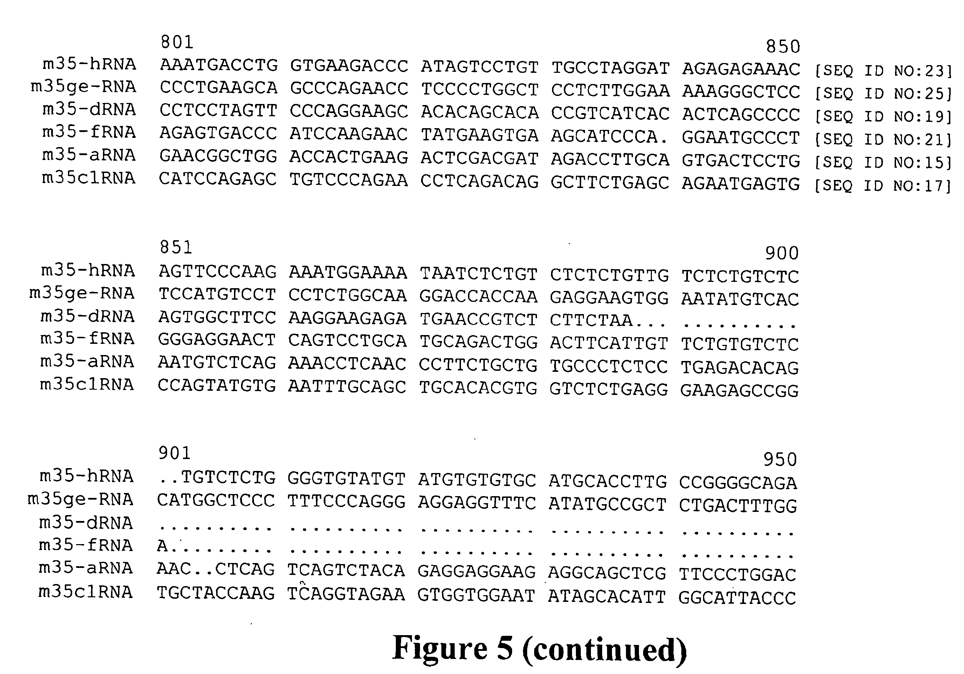

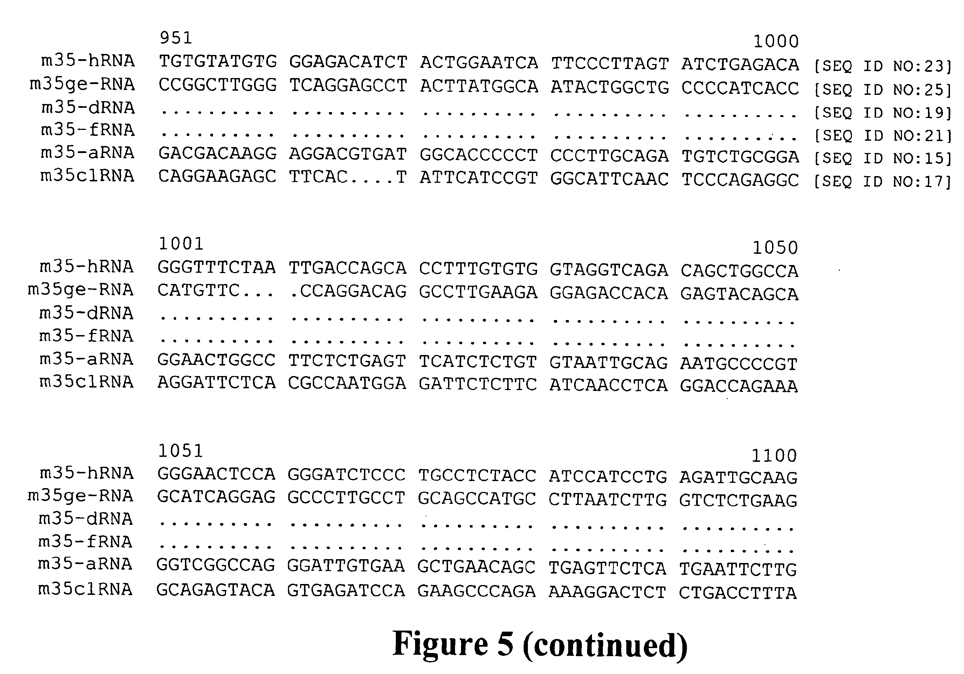

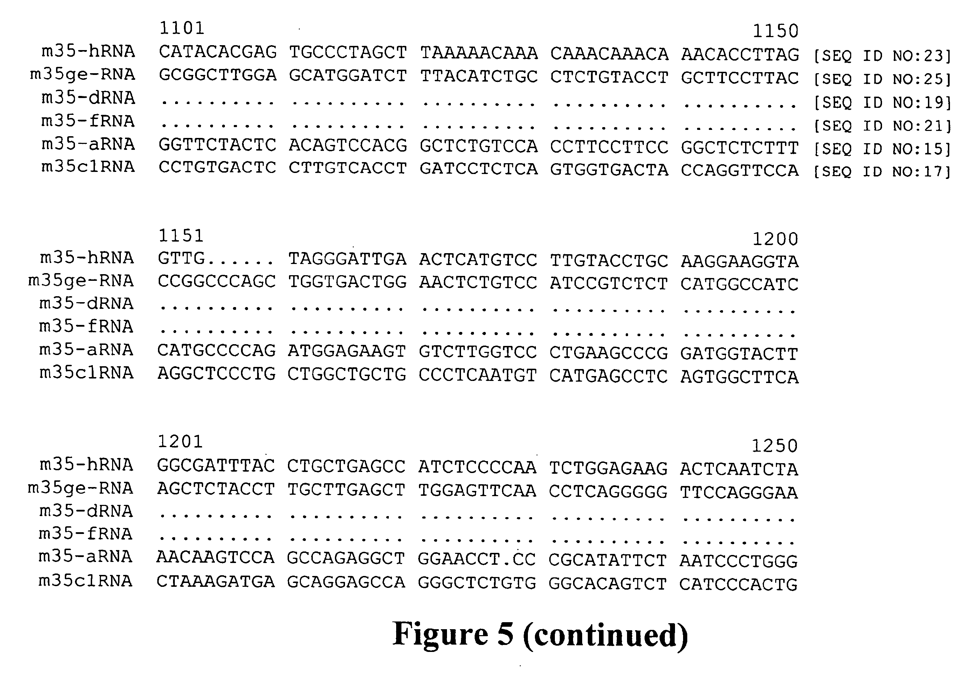

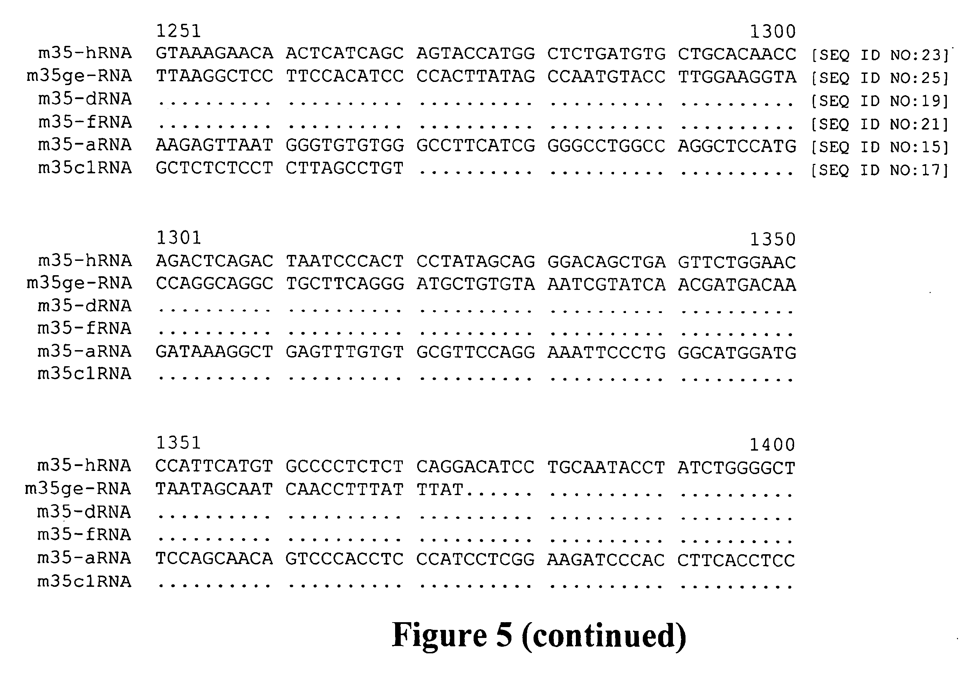

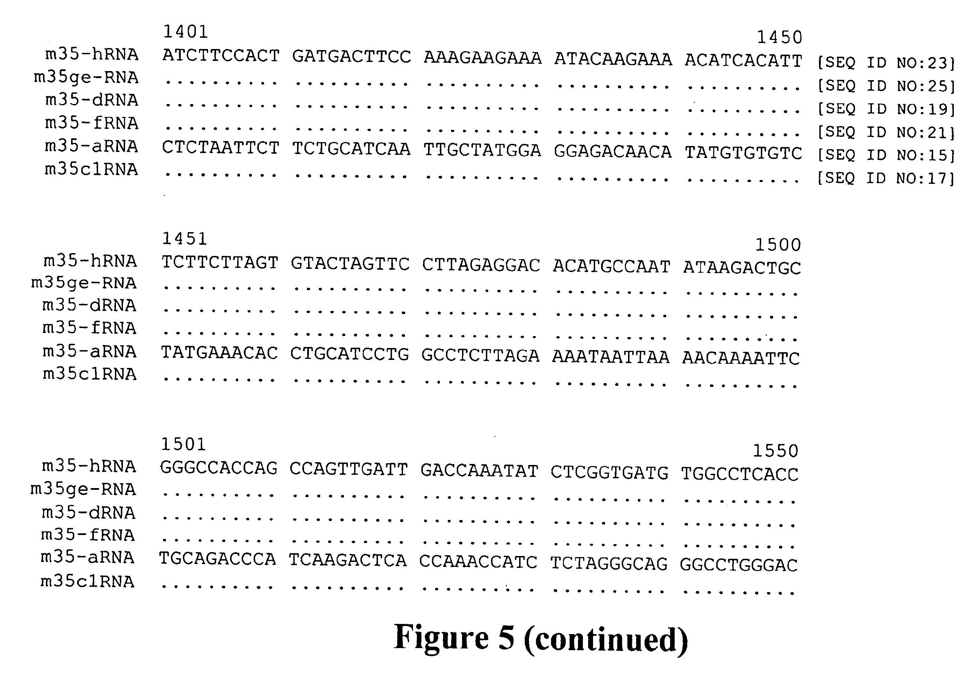

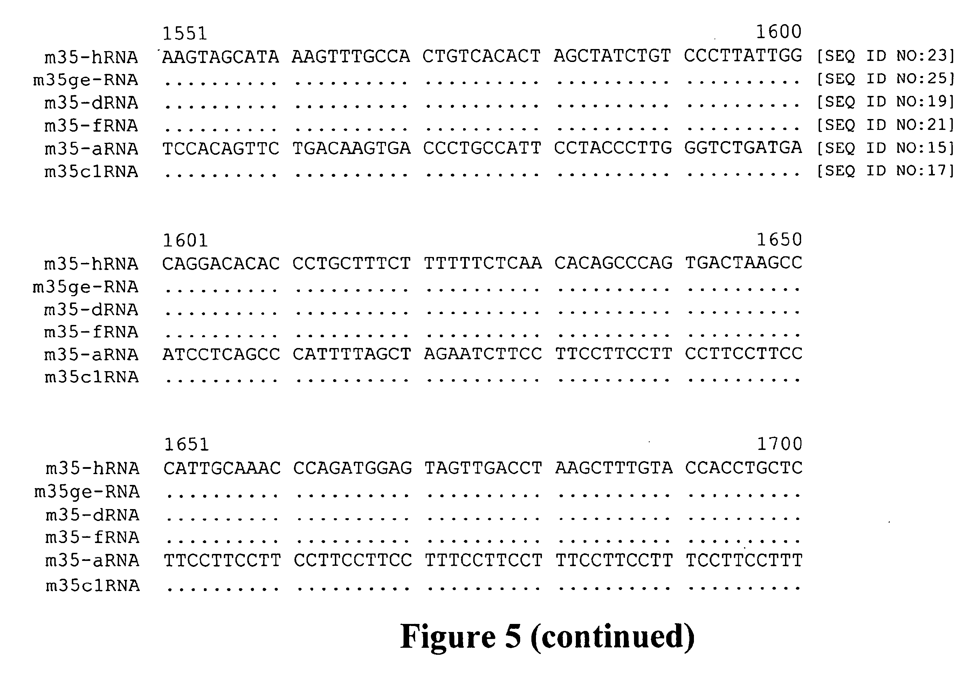

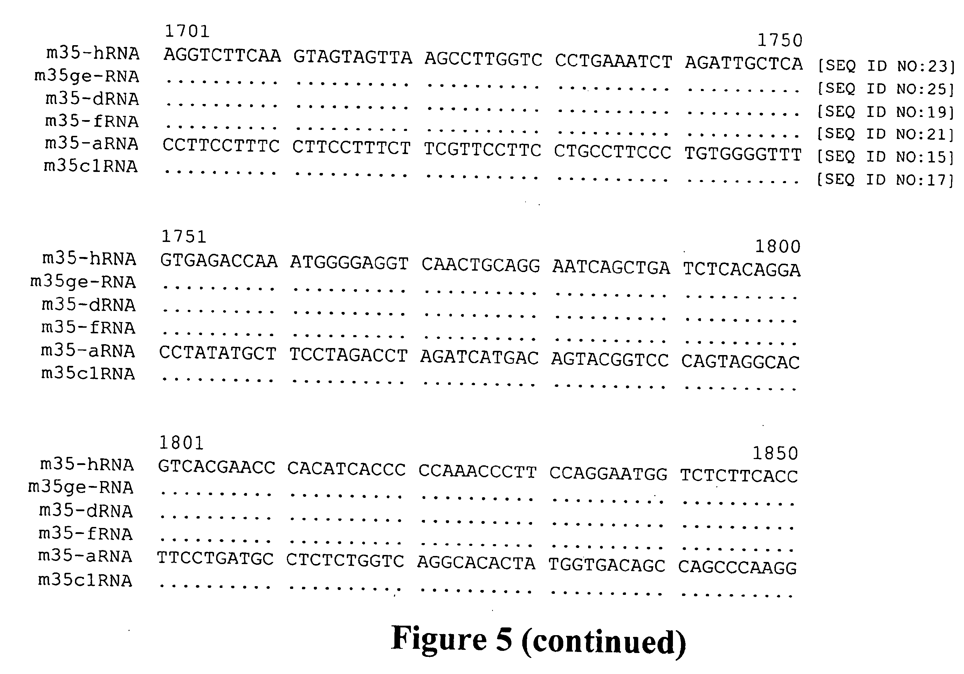

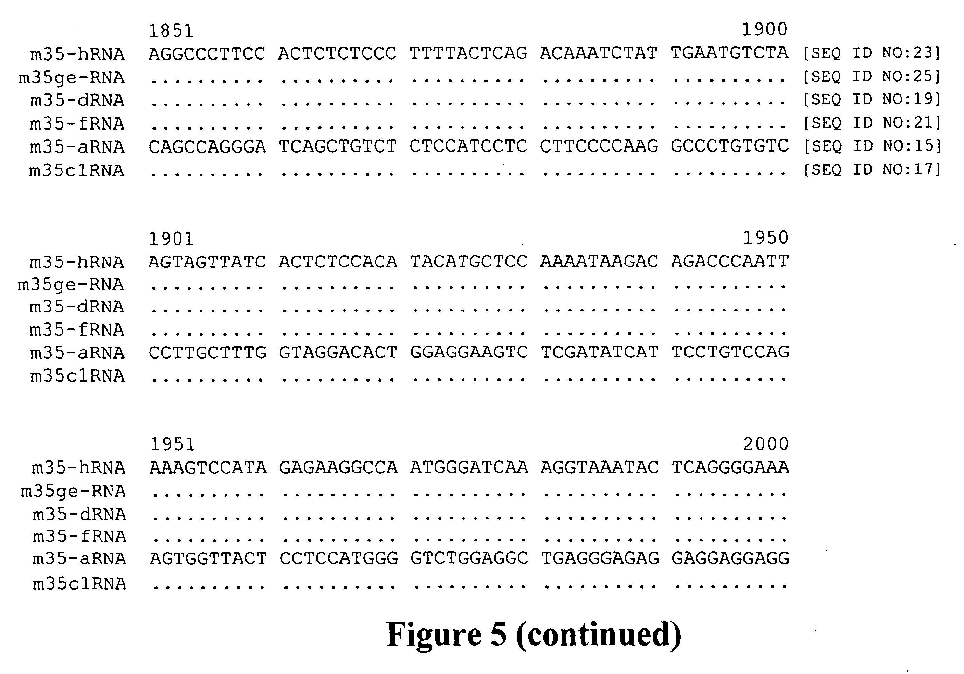

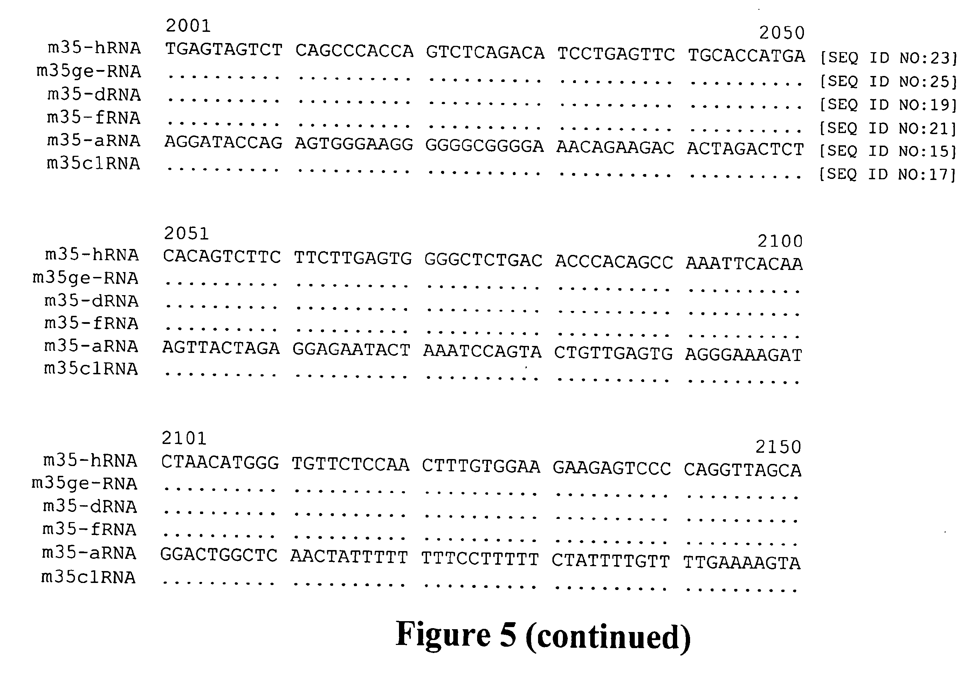

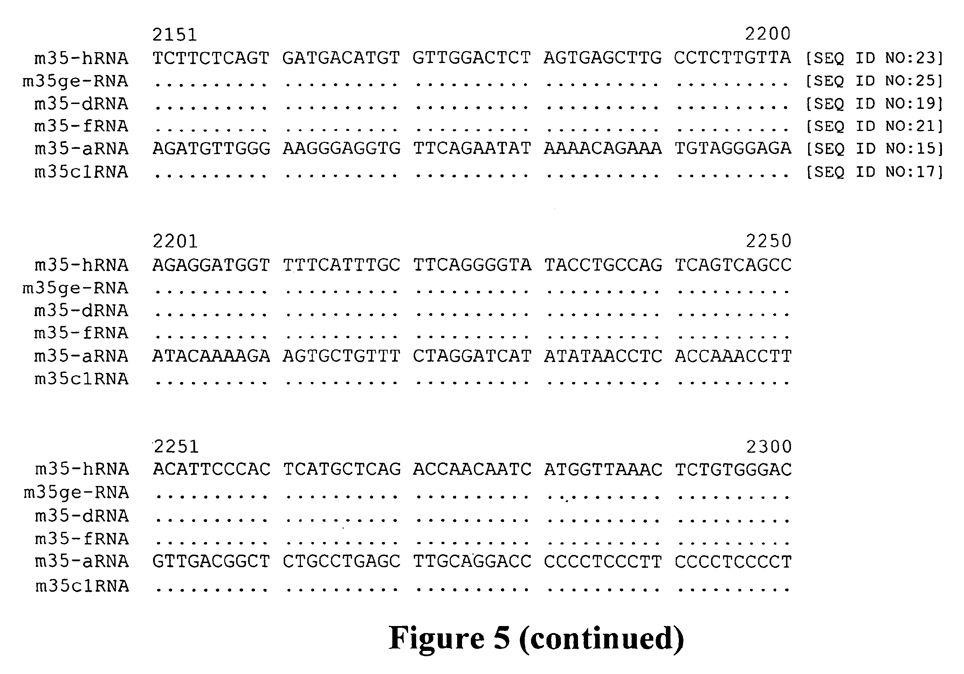

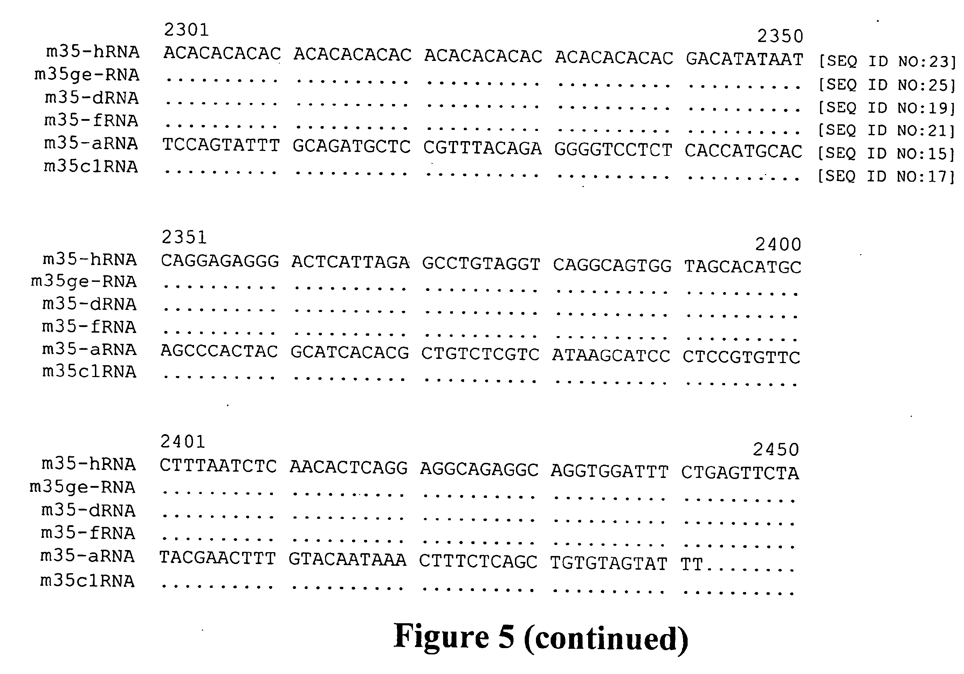

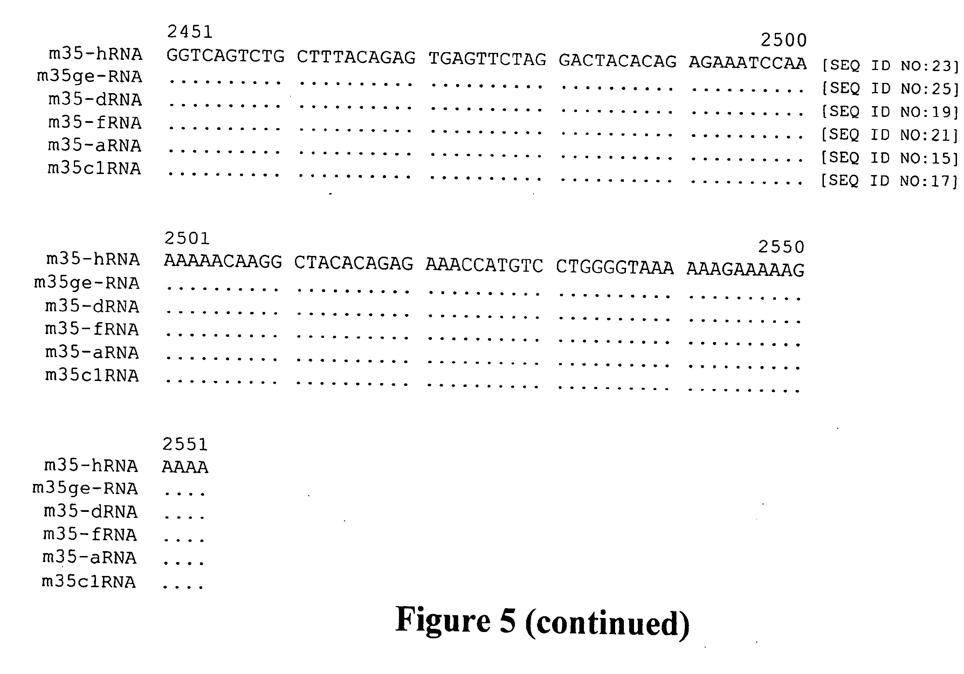

The alignment of the nucleic acid sequences of the mouse cDNAs is shown in Figure 4 and the alignment of the protein sequences of the mouse cDNAs is shown in Figure 5.

### EXAMPLE 4

### Expression analysis of mouse homologs

To study the expression of m35a, m35c, m35d, m35e, m35f, m35g, m35h and DIgR1 in cell lines and freshly prepared haemopoietic cell populations, primers were designed that were specific for each transcript and cross-checked for sequence similarity against other family members. DIgRI was included for comparison to published data (Luo et al., Biochem. Biophys. Res. Commun. 287: 35-41, 2001). Optimization of RT-PCR conditions was necessary before analysis of expression could be performed (Table 5).

**TABLE 5**

| ***Optimization of RT-PCR conditions**** | | | | | | | | | |
|---|---|---|---|---|---|---|---|---|---|
| | | **m35a** | **m35c** | **m35d** | **m35e** | **m35f** | **m35g** | **m35h** | **DIgR1** |
| A | Optimal RT-PCR conditions | AT: 60°C | AT: 60°C | AT: 60°C | AT: 60°C; 3' primer: 10 mM | AT: 60°C | AT: 60°C | Touchdown MgCl₂: 2.0 mM | AT: 60°C |
| | RT-PCR fragment size | 239 | 266 | 159 | 217 | 142 | 111 | 246 | 244 |
| B | Ig domain RT-PCR conditions | AT: 65°C | AT: 65°C | AT: 65°C | AT: 64°C; 3' primer: 10 mM | AT: 53°C | na | na | na |
| | Ig RT-PCR fragment size | 444 | 447 | 393 | 405 | 462 | na | na | na |

| | | | | | | | | | |
|---|---|---|---|---|---|---|---|---|---|
| * (A) refers to RT-PCRs used for expression analysis while (B) refers to RT-PCRs used for amplification of Ig domains. Only annealing temperature (AT) for RT-PCRs are indicated, unless the PCR cycle varied from standard conditions. | | | | | | | | | |

Optimization involved performing a temperature gradient RT-PCR on each primer set, which altered the annealing temperature between 50°C and 65°C. If multiple products were amplified making interpretation difficult, MgCl₂ concentrations were titrated between 1.5 mM and 3.5 mM. Further optimization was necessary for m35e, which involved varying forward and reverse primer concentrations and m35h, which involved designing a touchdown RT-PCR program. The touchdown program contained an initial denaturation of 94°C for 5 min, followed by 20 cycles of [94°C for 15 sec; 65°C for 15 sec - 0.5°C/cycles; 72°C for 1 min], then 15 cycles of [94°C for 15 sec; 55°C for 15 sec; 72°C for 1 min] and a final extension of 72°C for 5 min. This cycles prevents early false priming, while facilitating amplification, by lowering the annealing temperature in later stage of the program.

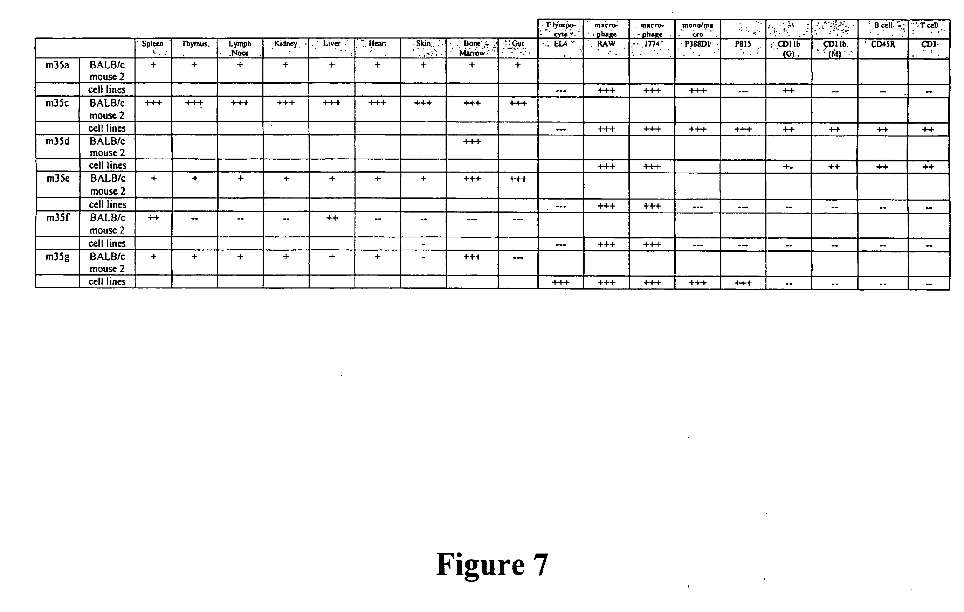

The expression of m35a, m35c, m35d, m35e, m35f, m35g, m35h and DIgRI was examined by RT-PCR and Southern blotting (Figure 9 and Figure 7). Amplified template included cDNA synthesized from selected tissues of BALB/c mice, mouse cell lines, C57BL/6 mouse spleen cell subsets and bone marrow derived DCs. Expression of m35-LMs in tissue was generally widespread with only m35d and m35f showing restricted expression for lymphoid tissue. m35a, m35c and DIgR1 were expressed in all tested tissues and m35e and m35h were negative only in skin. Spleen was the only tissue positive for all family members.

### EXAMPLE 5

### Characterization of the molecular structure of a novel myeloid restricted molecule, 35-L3

Preliminary studies identified the partial sequence of the 35-L3 molecule from an EST database (AW880126). The gene for the molecule has been localized to human chromosome 17. The inventors have established an RT-PCR that identifies this molecule and shows that it is an expressed product. The PCR product has been cloned and sequenced, confirming its identity as the 35-L3 EST. 5' and 3' RACE protocols were used to further identify the full length molecule. PBMC cDNA library in an expression vector, pCMV-SPORT.6 (Life Technologies) is used to isolate a full length clone. PCR and hybridization screening is used. The full length 35-L3 molecule (cDNA) sequence corresponds to an ORF with sequence similarity to the CMRF-35A and CMRF-35H sequences which, in accordance with the present invention, is identified on chromosome 17.

The isolated cDNA(s) is sequenced by Big Dye chain termination sequencing. The 5' RACE data are used to confirm that a full clone has been isolated. The complete sequence of the cDNA is used to analyze the 35-L3 gene structure. Two sequence BLAST searches are performed using the 35-L3 cDNA sequence and the chromosome 17 sequence. This will provide the sequence of the putative promoter region.

RT-PCR has been used to establish the expression of the 35-L3 EST in normal haemopoietic lineages and cell lines. This RT-PCR was designed from a single EST. Screening of the public databases indicates that this EST represents a single exon. The sequence of the complete cDNA is used to design RT-PCR primers that cross intron-exon junctions. These primers are used to confirm the expression data. This will ensure that any splice variants are identified. Variants identified are characterized at the molecular level to determine the presence of alternative exon usage.

### EXAMPLE 6

### To express 35-L3 and generate monoclonal antibodies (mAb) to 35-L3 to study its expression in leukocytes and other tissues

Constructs are made to allow expression of recombinant forms of the 35-L3 molecule in mammalian and prokaryotic systems. The cDNA isolated from the pCMV-SPORT library is inserted in an expression vector. This is used to transiently transfect COS cells. Mice are immunized using a tolerance procedure (Dzionek et al., J Immunol 165(11): 6037, 2000) that allows the induction of tolerance to the parental COS cells, whilst immunizing against the transfected cells. Expression of the cDNA is monitored by RT-PCR and Northern blotting to ensure at least RNA is transcribed. DNA immunization was also used in place of the tolerance procedure.

The cDNA sequence is used to design PCR primers to produce a range of fragments that is used to make recombinant proteins. These include the potential extracellular domains of the 35-L3 molecule fused to (1) the human IgG1 Fc portion, (2) a HIS tag or (3) a myc tag.

The fusion products are expressed in mammalian cells or *E. coli* as appropriate. The fusion proteins will be purified by affinity chromatography using protein A for IgG1 Fc fusion proteins, and anti-His or anti myc monoclonal antibodies as appropriate. Purified recombinant proteins are monitored by SDS-PAGE.

The recombinant proteins are used to immunize rabbits to produced rabbit polyclonal serum. Recombinant proteins or cDNA in expression vectors are used to immunize mice to produce mAb. Specific mAb are identified by ELISA using the recombinant fusion proteins or by flow cytometry using RT-PCR expression data to determine appropriate cell lines as targets.

The mAb is used to analyze the expression of the 35-L3 molecule on normal haemopoietic populations by flow cytometry. Basic biochemical characterization (immunoprecipitation or Western Blots) of the 35-L3 molecule is performed to identify its molecular size.

### EXAMPLE 7

### To analyze 35-L1 to L5 expression in leukemias

Blast populations are isolated from bone marrow or peripheral blood samples of new and relapsed AML and ALL patients. A standard cell surface phenotype of the leukemic cells are determined and this is used in three color analysis to phenotype the cells. If necessary, the leukemic cells are sorted for more detailed phenotypic analysis.

Aliquots of 5 ml peripheral blood is collected from newly diagnosed leukemic patients according to ethical consent. Patients of each subtype is tested and reported according to the new classification.

In addition, sorted blast cells are used to prepare RNA and cDNA for quantitative real time (RT) polymerase chain reaction (PCR) analysis. This allows information to be collected on the expression of the 35-L1, 35-L2, 35-L3, 35-L4 or 35-L5 (referred to as "35-L1 to L5") prior to the generation of monoclonal or polyclonal reagents.

### EXAMPLE 8

### Functional aspects of 35-L1 to L5 specific mAb

Given the potential of this molecule to be used, for example, as a marker for leukemic cells, mAbs generated herein are assessed for their ability to target or purge 35-L1⁺ to L5⁺ cells. Reference to "35-L1 to L5" means any one of 35-L1, 35-L2, 35-L3, 35-L4 or 35-L5 or combinations thereof. The following experiments are performed to assess their potential:
(a) to deliver intracellular toxins or radionuleotides *via* internalization;
(b) to effect the growth of 35-L1⁺ to L5⁺ cells in culture; and
(c) their ability to target and lyse 35-L1⁺ to L5⁺ and homolog bearing cells.

(a) The ability of the mAb bound to surface 35-L1⁺ to L5⁺ to internalize. Biotinylated mAb will be bound to 35-L1⁺ to L5⁺ targets. Cells are incubated at 37°C, 4°C with and without fixation. Internalization is assessed by flow cytometry. In addition, these assays will allow determination of the shedding or production of soluble 35-L1⁺ to L5⁺ protein from the cell surface.
(b) The effects of crosslinking the 35-L1⁺ to L5⁺ mAb on growth kinetics, cell cycle disruption or apoptosis will also be assayed on leukemic cell lines and AML samples. Apoptosis is assessed by Annexin V staining or expression of the bcl-2 molecule by cells in culture.
(c) Complement dependent cytotoxicity of 35-L1⁺ to L5⁺ targets using anti-35-L1⁺ to L5⁺ mAb and complement is assayed. Targets to be used for this analysis depend on the results of AIM 2.The ability of the mAb to lyse tumor cells *via* antibody dependent cell mediated cytotoxicity (ADCC) is also tested using standard assays. Assays for complement dependent cytotoxicity (CDC) and ADCC have been developed for assessing the ability of the CMRF-44 mAb to lyse target cells.

In a variation of this assay, CMRF-35A or CMRF-35H is crosslinked with 35-LM antibodies.

### EXAMPLE 9

### In vivo model

A NOD-SCID mouse model is developed to conduct *in vivo* assays on AML. Such a model provides valuable information of the *in vivo* effects of antagonists and agonists of 35-L1 to L5 (e.g. 35-L1 to L5 mAbs).

### EXAMPLE 10

### Generation of mAbs to CMFR-35A, CMFR-35H, 35-L1, 35-L2, 35-L3, 35-L5 and 35-L5

Twelve-week old female BALB/c mice were injected in the tibialis anterior muscle with 50µl of 2µg/ml cDNA construct in 25% sucrose. -4 immunizations were performed at 3 week intervals. Approximately one month after the final immunization, the mice were boosted with either purified protein corresponding to 35-L1, 35-L3, 35-L4, or 35-L5 or 5 x 10⁶ U937 cells. Spleens were collected three days later and fused to NS-1 myeloma by standard techniques. The cDNA constructs were either the full-length cDNA in pcDNA3.1 expression vector or the Ig fusion protein construct in the pIg vector

### EXAMPLE 11

### 35-L1 is expressed predominantly on cells of the monocytes lineage

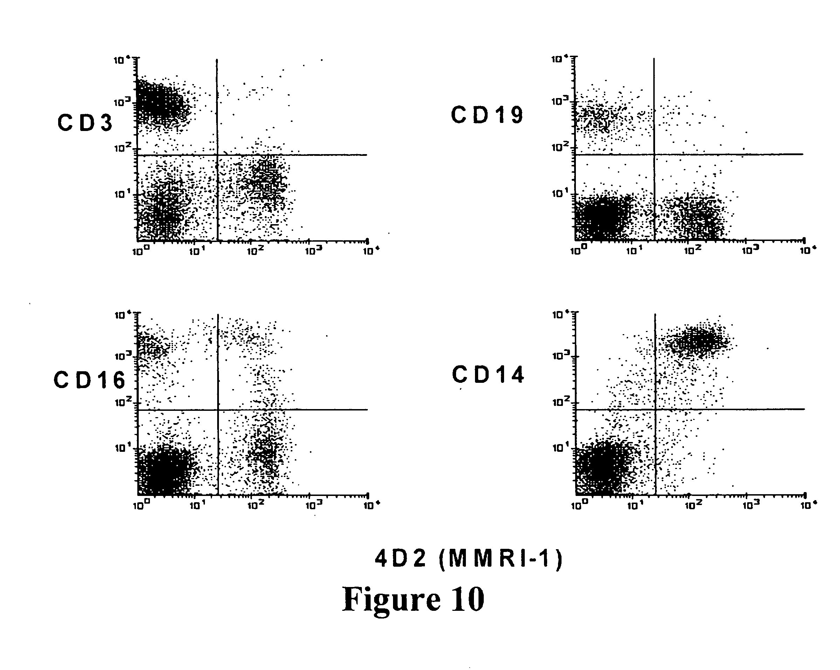

Using the mAbs generated in Example 11, the cell surface expression 35-L1 was examined on T cells, B cells, natural killer cells and monocytes populations by staining for CD3⁺, CD19⁺, CD16⁺ and CD14⁺, respectively and analyzing the staining profiles using flow cytometric analysis. Analysis revealed that the majority of CD14⁺ monocytes were positive for surface expressed 35-L1, while T cells and B cells were negative, and there was minimal staining of CD16⁺ natural killer cells (see Figure 10).

Further evaluation of 35-L1 expression was performed analyzing monocyte derived DCs (MoDCs) and blood DCs. Cells were examined for the level of surface expression of both CMRF-35 and 35-L1. Flow cytometric analysis demonstrated that surface expression of both CMRF-35 and 35-L1 were significantly higher in monocytes than blood DCs.

Evaluation of the cell surface expression on cells from cord blood was performed using antibodies to 35-L3, 35-L4 and 35-L5. Co-staining was performed using an antibody to CD38 which is an antigen expressed during the early stages of T- and B-lymphocyte differentiation. It is also expressed on activated T- and B-cells, NK lymphocytes and plasma cells. Flow cytometric analysis revealed that 6.07%, 6.10% and 4.70% of cord blood cells expressed 35-L3, 35-L4 and/or 35-L5 on their cell surface respectively.

### EXAMPLE 12

### 35-L1, 35-L3 and 35-L5 expression in AML

In order to determine the expression levels of 35-L1, 35-L3 and 35-L5 in AML, multiple samples of AML cells were examined for both mRNA levels and cell surface expression of 35-L1, 35-L3 and 35-L5.

Samples were analyzed for their levels of 35-L3- and 35-L5-specific mRNA. These levels were then standardized against β-Actin-specific mRNA. All AML samples tested were positive for both 35-L3 and 35-L5 specific mRNA (see Table 6).

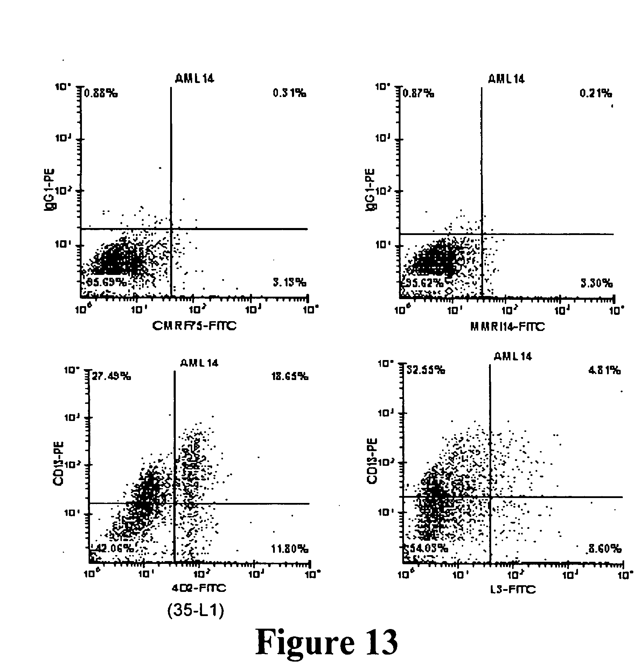

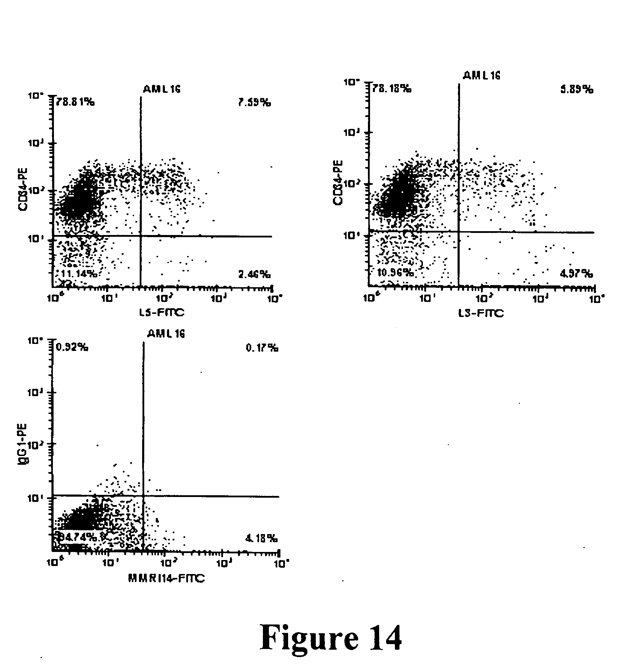

AML samples were also tested for the surface expression of 35-L1, 35-L3 and 35-L5 using flow cytometric analysis. AML sample #14 stained positive for both 35-L3 and 35-L1 (see Figure 13) and AML sample #16 tested positive for 35-L3 and 35-L5 (see Figure 14).

These findings demonstrate that 35-L1, 35-L3 and 35-L5 are useful molecules for the diagnosis and treatment of AML infection in a subject.

**TABLE 6**

| ***Expression of 35L3-mMRNA and 35-L5 mRNA in AML samples*** | | | |
|---|---|---|---|
| **Sample** | **FAB phenotype** | **Expression of 35-L3 mRNA relative to** | **Expression of 35-L5 mRNA relative to ßactin** |
| **AML #1** | M4/5: | 1:1320 | 1:5083 |
| | (CD33⁺CD13⁺CD34⁺CD14⁺) | | |
| **AML #2** | M4: | 1:3551 | 1:1102 |
| | (CD33⁺CD13⁺CD34⁺CD14⁻) | | |
| **AML #3** | M4: | 1:684 | 1:449 |
| | (CD33⁺CD13⁺CD34⁺CD14⁺) | | |
| **AML #4** | M5: (CD33⁺CD13⁺CD34⁻ | 1:5542 | 1:800 |
| | CD14⁺) | | |
| **AML #5** | M5/6: (CD33⁺CD13⁺CD34⁻ | 1:2471 | 1:1230 |
| | CD14⁺) | | |
| **AML #6** | M4/5: (CD33⁺CD13⁺CD34⁻ | 1:431 | 1:8412 |
| | CD14⁺) | | |
| **AML #7** | M1: (CD33⁺CD13⁺CD34⁻ | 1:2954 | 1:4324 |
| | CD14⁻) | | |
| **AML #8** | M4/5: | 1:1492 | 1:950 |
| | (CD33⁺CD13⁺CD34⁺CD14⁺) | | |
| **AML #9** | M1/2: (CD33⁺CD13⁺CD34⁻ | 1:30400 | 1:2553 |
| | CD14⁻) | | |
| **Normal** | CD14⁺ | 1:1722 | 1:3546 |

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### BIBLIOGRAPHY

Altschul et al., Nucleic Acid Research, 25: 3389-3402, 1997
Ausubel et al, Current Protocols in Molecular Biology, John Wiley & Sons, Inc., Chapter 15, 1994-1998
Beaucage and Carruthers, Tetra Letts 22: 1859-1862, 1981
Biggs et al., Cytometry 36: 36-45, 1999
Bonner and Laskey, Eur. J. Biochem. 46: 83, 1974
Bouchon et al., J. Immunol. 164: 4991-4995, 2000
Clark et al., Tissue Antigens 57: 415-423, 2001
Clark et al., Tissue Antigens 55: 101-109, 2000
Dangl and Herzenberg, J. Immunol. Methods 52: 1-14, 1982
Darzynkiewica and Crissman., Eds., "Methods in Cell Biology", Vol. 33, Academic Press
Douillard and Hoffman, Basic Facts about Hybridomas, in Compendium of Immunology Vol. II, ed. by Schwartz, 1981
Dzionek et al., Immunol 165(11): 6037, 2000
Elghanian et al., Science 277: 1078-1081, 1997
Fiers et al., Nature 273: 113-120, 1978
Fu et al., Nature Biotechnology 17: 1109-1111, 1999
Green et al., Int Immunol. 10: 891-899, 1998
Grompe et al., Nature Genetics 5: 111-117, 1993
Grompe et al., Proc. Natl. Acad. Sci. USA 86: 5855-5892, 1989
Jackson et al., Eur. J. Immunol. 22: 1157-1163, 1992
Jakoby and Pastan (Eds.), Cell Culture. Methods in Enzymology, Vol. 58, Academic Press, Inc., Harcour Brace Jovanovich, New York, 1979
Kohler and Milstein, European Journal of Immunology 6: 511-519, 1976
Kohler and Milstein, Nature 256: 495-499, 1975
Kubo et al., FEBS Lett. 241: 119, 1988
Kyte and Dolittle, J. Mol. Biol. 157: 105-132, 1982
Luo et al., Biochem. Biophys. Res. Commun. 287: 35-41, 2001
Malemed et al., "Flow cytometry and sorting", 2nd Ed., New York, Wiley-Liss, 1990
Matteucci and Caruthers, J. Am. Chem. Soc, 103: 3185, 1981
Orita et al., Proc. Natl. Acad. Sci. USA 86: 2776-2770, 1989
Pessino et al., J. Exp. Med. 188: 953-960, 1998
Remington's Pharmaceutical Sciences, 18th Ed. (1990, Mack Publishing, Company, Easton, PA, USA
Sambrook et al., Molecular Cloning: A Laboratory Manual, Cold Spring Harbor Laboratory, 2nd Edition, 1989
Sambrook et al., Molecular Cloning: A Laboratory Manual, Cold Spring Harbor Laboratory, 3rd Edition, 2001
Selvakumar et al., Tissue Antigens 49:564, 1997
Shapiro, "Practical flow cytometry", 3rd ed., Brisbane, Wiley-Liss, 1995
Sheffield et al., Am. J. Hum. Genet. 49: 699-706, 1991
Vitale et al., J. Exp. Med. 187: 2065-2072, 1998
Wagtmann et al., Current Biol 7 :615, 1997
Wende et al., Mamm Genome 10(2): 154, 1999
Wende et al., Immunogenetics 51:703, 2000
White et al., Genomics 12: 301-306, 1992
Wilson et al., Immunol Res 22: 21, 2000
Wilson et a/., Methods Mol Biol 121: 251, 2000
Wilson et al., Tissue Antigens 49:574, 1997

## Claims

1. An isolated immunointeractive molecule which specifically binds to a polypeptide selected from:
(A) a polypeptide comprising an amino acid sequence selected from the group consisting of:
(a) a sequence provided in SEQ ID NO: 14; and
(b) a sequence having at least 90% similarity after optimal alignment to an amino acid sequence provided in SEQ ID NO: 14, or
(B) a polypeptide encoded by a nucleic acid molecule comprising a sequence selected from the group consisting of:
(a) a sequence provided in SEQ ID NO: 13;
(b) a complement of a sequence provided in SEQ ID NO: 13;
(c) a sequence which hybridizes to a sequence provided in SEQ ID NO: 13 or the complement thereof, under conditions of high stringency; and
(d) a sequence having at least 90% identity after optimal alignment to a sequence provided in SEQ ID NO: 13 or its complement,
or an immunogenic fragment of the immunointeractive molecule.

2. The immunointeractive molecule of claim 1, wherein the molecule is an antibody or an antigen binding fragment thereof.

3. The isolated antibody of claim 2, wherein said antibody is selected from the group consisting of: a polyclonal antibody, a monoclonal antibody, a humanized antibody, and a deimmunized antibody.

4. The antibody of claim 3 conjugated to an immunotoxin.

5. A pharmaceutical composition comprising a first component comprising an immunointeractive molecule of any one of claims 1 to 4 and a second component selected from the group consisting of: a pharmaceutical carrier, diluent and an immunostimulant.

6. An immunointeractive molecule of any one of claims 1 to 4 for use in treating a cancer.

7. The use of claim 6, wherein said cancer is leukemia.

8. The use of claim 6, wherein said cancer is AML.

9. A method for detecting the presence of a disease or condition in a subject, comprising the steps of:
(a) contacting a biological sample obtained from said subject with a molecule that binds to a nucleic acid molecule or polypeptide as defined in claim 1;
(b) detecting in said biological sample the presence of binding of said molecule; and
(c) comparing the presence of bound molecule with a pre-determined cut-off value to make a determination as to the presence or absence of a disease or condition in said subject.

10. A method for assessing a disease or condition including the ability for a subject to mount an immune response, said method comprising determining the level or pattern of expression of a nucleic acid molecule or polypeptide as defined in claim 1, wherein the pattern of presence or absence of expression correlates with a disease condition, a propensity for developing a disease condition and/or an ability for a subject to maintain an immune response.

11. The method of claim 9 or claim 10, wherein said disease or condition is leukemia.

12. The method of claim 9 or claim 10, wherein said disease or condition is AML.

13. The method of claim 9, wherein said molecule is an immunointeractive molecule of any one of claims 1 to 4.

14. Use of an immunointeractive molecule of any one of claims 1 to 4 in the manufacture of a medicament for treating a cancer.
